# EUROPEAN PATENT APPLICATION

(11) **EP 4 155 349 A1**
(43) Date of publication of application: **29.03.2023**
(21) Application number: 22197531.1
(22) Date of filing: 23.09.2022
(51) Int. Cl.: C09B 69/10, C07F 5/02, G01N 33/52, G01N 33/58, C09B 23/04

(54) **WATER-SOLUBLE YELLOW GREEN ABSORBING DYES**

(30) Priority: 24.09.2021 US 202163248041 P; 21.03.2022 US 202217699790; 21.03.2022 WO PCT/US2022/021137; 28.07.2022 US 202217876349
(71) Applicant: Becton, Dickinson and Company, Franklin Lakes, NJ 07417 (US)
(72) Inventor: BARTHOLOMEW, Glenn, Escondido (US); LIANG, Yongchao, Irvine (US); WALL, Brian, Poway (US); MOUREAU, David Michel, San Diego (US); HOLLINGER, Jon, Poway (US)
(74) Representative: Mewburn Ellis LLP

(57) **Abstract**

Water-soluble dipyrromethene-based dyes are provided herein. In certain cases, the water-soluble dye is a dipyrromethene-boron dye. Embodiments of the subject water-soluble dipyrromethene-based dyes have a narrow absorption maxima in the yellow green range close to or at the 561 nm laser line, and are suitable for use in tandem dye systems. Embodiments of the subject dyes efficiently transfer energy to an acceptor chromophore while a second dipyrromethene-based dye of the same or different chemical structure is within energy-receiving proximity therewith. Also provided herein are tandem dyes that include the subject water-soluble dipyrromethene-based dyes, e.g., as donor chromophores. Methods of evaluating a sample for the presence of a target analyte and methods of labelling a target molecule in which the subject dyes find use are also provided. Systems and kits for practicing the subject methods are also provided.

## Description

Fluorescent dyes are compounds which, when irradiated with light of a wavelength which they absorb, emit light of a (usually) different wavelength. Fluorescent dyes find use in a variety of applications in biochemistry, biology and medicine, e.g. in diagnostic kits, in microscopy or in drug screening. Fluorescent dyes are characterized by a number of parameters allowing a user to select a suitable dye depending on the desired purpose. Parameters of interest include the excitation wavelength maximum, the emission wavelength maximum, the Stokes shift, the extinction coefficient, the fluorescence quantum yield and the fluorescence lifetime. Dyes may be selected according to the application of interest in order to, e.g., allow penetration of exciting radiation into biological samples, to minimize background fluorescence and/or to achieve a high signal-to-noise ratio.

Molecular recognition involves the specific binding of two molecules. Molecules which have binding specificity for a target biomolecule find use in a variety of research and diagnostic applications, such as the labelling and separation of analytes, flow cytometry, in situ hybridization, enzyme-linked immunosorbent assays (ELISAs), western blot analysis, magnetic cell separations and chromatography. Target biomolecules may be detected by labelling with a fluorescent dye.

### SUMMARY

Water-soluble dipyrromethene-based dyes are provided herein. In certain cases, the water-soluble dye is a dipyrromethene-boron dye. Embodiments of the subject water-soluble dipyrromethene-based dyes have a narrow absorption maxima in the yellow green range close to or at the 561 nm laser line, and are suitable for use in tandem dye systems. Embodiments of the subject dyes efficiently transfer energy to an acceptor chromophore while a second dipyrromethene-based dye of the same or different chemical structure is within energy-receiving proximity therewith. Also provided herein are tandem dyes that include the subject water-soluble dipyrromethene-based dyes, e.g., as donor chromophores. The tandem dyes include a pendant acceptor fluorophore configured as an energy acceptor in energy-receiving proximity to a donor water-soluble dipyrromethene-based dye. Methods of evaluating a sample for the presence of a target analyte and methods of labelling a target molecule in which the subject dyes find use are also provided. Systems and kits for practicing the subject methods are also provided.

### BRIEF DESCRIPTION OF THE DRAWINGS

It is understood that the drawings, described below, are for illustration purposes only. The drawings are not intended to limit the scope of the present teachings in any way.
FIG. 1 shows example yellow green water-soluble dipyrromethene-based dyes of embodiments of the invention.
FIG. 2 shows additional example yellow green water-soluble dipyrromethene-based dyes of embodiments of the invention.
FIG. 3 shows the absorption spectra for four example dipyrromethene-boron dyes of embodiments of the invention.

### DEFINITIONS

As used herein, the terms "chemoselective functional group" and "chemoselective tag" are used interchangeably and refer to a functional group that can selectively react with another compatible functional group to form a covalent bond, in some cases, after optional activation of one of the functional groups. Chemoselective functional groups of interest include, but are not limited to, thiols and maleimide or iodoacetamide, amines and carboxylic acids or active esters thereof, as well as groups that can react with one another via Click chemistry, e.g., azide and alkyne groups (e.g., cyclooctyne groups), tetrazine, transcyclooctene, dienes and dienophiles, and azide, sulfur(VI) fluoride exchange chemistry (SuFEX), sulfonyl fluoride, as well as hydroxyl, hydrazido, hydrazino, aldehyde, ketone, azido, alkyne, phosphine, epoxide, and the like.

As used herein, the term "sample" relates to a material or mixture of materials, in some cases in liquid form, containing one or more analytes of interest. In some embodiments, the term as used in its broadest sense, refers to any plant, animal or bacterial material containing cells or producing cellular metabolites, such as, for example, tissue or fluid isolated from an individual (including without limitation plasma, serum, cerebrospinal fluid, lymph, tears, saliva and tissue sections) or from in vitro cell culture constituents, as well as samples from the environment. The term "sample" may also refer to a "biological sample". As used herein, the term "a biological sample" refers to a whole organism or a subset of its tissues, cells or component parts (e.g. body fluids, including, but not limited to, blood, mucus, lymphatic fluid, synovial fluid, cerebrospinal fluid, saliva, amniotic fluid, amniotic cord blood, urine, vaginal fluid and semen). A "biological sample" can also refer to a homogenate, lysate or extract prepared from a whole organism or a subset of its tissues, cells or component parts, or a fraction or portion thereof, including but not limited to, plasma, serum, spinal fluid, lymph fluid, the external sections of the skin, respiratory, intestinal, and genitourinary tracts, tears, saliva, milk, blood cells, tumors and organs. In certain embodiments, the sample has been removed from an animal or plant. Biological samples may include cells. The term "cells" is used in its conventional sense to refer to the basic structural unit of living organisms, both eukaryotic and prokaryotic, having at least a nucleus and a cell membrane. In certain embodiments, cells include prokaryotic cells, such as from bacteria. In other embodiments, cells include eukaryotic cells, such as cells obtained from biological samples from animals, plants or fungi.

The terms "support bound" and "linked to a support" are used interchangeably and refer to a moiety (e.g., a specific binding member) that is linked covalently or non-covalently to a support of interest. Covalent linking may involve the chemical reaction of two compatible functional groups (e.g., two chemoselective functional groups, an electrophile and a nucleophile, etc.) to form a covalent bond between the two moieties of interest (e.g. a support and a specific binding member). In some cases, non-covalent linking may involve specific binding between two moieties of interest (e.g., two affinity moieties such as a hapten and an antibody or a biotin moiety and a streptavidin, etc.). In certain cases, non-covalent linking may involve absorption to a substrate.

The term "polypeptide" refers to a polymeric form of amino acids of any length, including peptides that range from 2-50 amino acids in length and polypeptides that are greater than 50 amino acids in length. The terms "polypeptide" and "protein" are used interchangeably herein. The term "polypeptide" includes polymers of coded and non-coded amino acids, chemically or biochemically modified or derivatized amino acids, and polypeptides having modified peptide backbones in which the conventional backbone has been replaced with non-naturally occurring or synthetic backbones. A polypeptide may be of any convenient length, e.g., 2 or more amino acids, such as 4 or more amino acids, 10 or more amino acids, 20 or more amino acids, 50 or more amino acids, 100 or more amino acids, 300 or more amino acids, such as up to 500 or 1000 or more amino acids. "Peptides" may be 2 or more amino acids, such as 4 or more amino acids, 10 or more amino acids, 20 or more amino acids, such as up to 50 amino acids. In some embodiments, peptides are between 5 and 30 amino acids in length.

As used herein the term "isolated," refers to an moiety of interest that is at least 60% free, at least 75% free, at least 90% free, at least 95% free, at least 98% free, and even at least 99% free from other components with which the moiety is associated with prior to purification.

A "plurality" contains at least 2 members. In certain cases, a plurality may have 5 or more, such as 6 or more, 7 or more, 8 or more, 9 or more, 10 or more, 20 or more, 30 or more, 40 or more, 50 or more, 60 or more, 70 or more, 80 or more, 90 or more, 100 or more, 300 or more, 1000 or more, 3000 or more, 10,000 or more, 100,000 or more members.

Numeric ranges are inclusive of the numbers defining the range.

The term "specific binding" refers to the ability of a capture agent (or a first member of a specific binding pair) to preferentially bind to a particular analyte (or a second member of a specific binding pair) that is present, e.g., in a homogeneous mixture of different analytes. In some instances, a specific binding interaction will discriminate between desirable and undesirable analytes in a sample with a specificity of 10-fold or more for a desirable analyte over an undesirable analytes, such as 100-fold or more, or 1000-fold or more. In some cases, the affinity between a capture agent and analyte when they are specifically bound in a capture agent/analyte complex is at least 10⁻⁸ M, at least 10⁻⁹ M, such as up to 10⁻¹⁰ M.

"Affinity" refers to the strength of binding, increased binding affinity being correlated with a lower Kd. In an embodiment, affinity is determined by surface plasmon resonance (SPR), e.g. as used by Biacore systems. The affinity of one molecule for another molecule is determined by measuring the binding kinetics of the interaction, e.g. at 25°C.

The methods described herein include multiple steps. Each step may be performed after a predetermined amount of time has elapsed between steps, as desired. As such, the time between performing each step may be 1 second or more, 10 seconds or more, 30 seconds or more, 60 seconds or more, 5 minutes or more, 10 minutes or more, 60 minutes or more and including 5 hours or more. In certain embodiments, each subsequent step is performed immediately after completion of the previous step. In other embodiments, a step may be performed after an incubation or waiting time after completion of the previous step, e.g., a few minutes to an overnight waiting time.

As used herein, the terms "evaluating", "determining," "measuring," and "assessing," and "assaying" are used interchangeably and include both quantitative and qualitative determinations.

The term "separating", as used herein, refers to physical separation of two elements (e.g., by size or affinity, etc.) as well as degradation of one element, leaving the other intact.

The term "linker" or "linkage" refers to a linking moiety that connects two groups and has a backbone of 100 atoms or less in length. A linker or linkage may be a covalent bond that connects two groups or a chain of between 1 and 100 atoms in length, for example a chain of 1, 2, 3, 4, 5, 6, 8, 10, 12, 14, 16, 18, 20 or more carbon atoms in length, where the linker may be linear, branched, cyclic or a single atom. In some cases, the linker is a branching linker that refers to a linking moiety that connects three or more groups. In certain cases, one, two, three, four or five or more carbon atoms of a linker backbone may be optionally substituted with a sulfur, nitrogen or oxygen heteroatom. In some cases, the linker backbone includes a linking functional group, such as an ether, thioether, amino, amide, sulfonamide, carbamate, thiocarbamate, urea, thiourea, ester, thioester or imine. The bonds between backbone atoms may be saturated or unsaturated, and in some cases not more than one, two, or three unsaturated bonds are present in a linker backbone. The linker may include one or more substituent groups, for example with an alkyl, aryl or alkenyl group. A linker may include, without limitations, polyethylene glycol; ethers, thioethers, tertiary amines, alkyls, which may be straight or branched, e.g., methyl, ethyl, n-propyl, 1-methylethyl (iso-propyl), n-butyl, n-pentyl, 1,1-dimethylethyl (t-butyl), and the like. The linker backbone may include a cyclic group, for example, an aryl, a heterocycle or a cycloalkyl group, where 2 or more atoms, e.g., 2, 3 or 4 atoms, of the cyclic group are included in the backbone. A linker may be cleavable or non-cleavable.

As used herein, the terms "water solubilizing group" , "water soluble group" and WSG are used interchangeably and refer to a group or substituent that is well solvated in aqueous environments e.g., under physiological conditions, and which imparts improved water solubility upon the molecule to which it is attached. A WSG can increase the solubility of a multichromophore in a predominantly aqueous solution, as compared to a control multichromophore which lacks the WSG. The water solubilizing groups may be any convenient hydrophilic group that is well solvated in aqueous environments.

The terms "polyethylene oxide", "PEO", "polyethylene glycol" and "PEG" are used interchangeably and refer to a polymeric group including a chain described by the formula --(CH₂----O--)ₙ- or a derivative thereof. In some embodiments, "n" is 5000 or less, such as 1000 or less, 500 or less, 200 or less, 100 or less, 50 or less, 40 or less, 30 or less, 20 or less, 15 or less, such as 3 to 15, or 10 to 15. It is understood that the PEG polymeric group may be of any convenient length and may include a variety of terminal groups and/or further substituent groups, including but not limited to, alkyl, aryl, hydroxyl, amino, acyl, acyloxy, and amido terminal and/or substituent groups. PEG groups that may be adapted for use in the subject multichromophores include those PEGs described by S. Zalipsky in "Functionalized poly(ethylene glycol) for preparation of biologically relevant conjugates", Bioconjugate Chemistry 1995, 6 (2), 150-165; and by Zhu et al in "Water-Soluble Conjugated Polymers for Imaging, Diagnosis, and Therapy", Chem. Rev., 2012, 112 (8), pp 4687-4735 .

The term "alkyl" by itself or as part of another substituent refers to a saturated branched or straight-chain monovalent hydrocarbon radical derived by the removal of one hydrogen atom from a single carbon atom of a parent alkane. Alkyl groups of interest include, but are not limited to, methyl; ethyl, propyls such as propan-1-yl or propan-2-yl; and butyls such as butan-1-yl, butan-2-yl, 2-methyl-propan-1-yl or 2-methyl-propan-2-yl. In some embodiments, an alkyl group includes from 1 to 20 carbon atoms. In some embodiments, an alkyl group includes from 1 to 10 carbon atoms. In certain embodiments, a lower alkyl group includes from 1 to 6 carbon atoms, such as from 1 to 4 carbon atoms. This term includes, by way of example, linear and branched hydrocarbyl groups such as methyl (CH₃-), ethyl (CH₃CH₂-), n-propyl (CH₃CH₂CH₂-), isopropyl ((CH₃)₂CH-), n-butyl (CH₃CH₂CH₂CH₂-), isobutyl ((CH₃)₂CHCH₂-), sec-butyl ((CH₃)(CH₃CH₂)CH-), t-butyl ((CH₃)₃C-), n-pentyl (CH₃CH₂CH₂CH₂CH₂-), and neopentyl ((CH₃)₃CCH₂-).

The term "substituted alkyl" refers to an alkyl group as defined herein wherein one or more carbon atoms in the alkyl chain have been optionally replaced with a heteroatom such as -O-, -N-, -S-, -S(O)ₙ- (where n is 0 to 2), -NR- (where R is hydrogen or alkyl) and having from 1 to 5 substituents selected from the group consisting of alkoxy, substituted alkoxy, cycloalkyl, substituted cycloalkyl, cycloalkenyl, substituted cycloalkenyl, acyl, acylamino, acyloxy, amino, aminoacyl, aminoacyloxy, oxyaminoacyl, azido, cyano, halogen, hydroxyl, oxo, thioketo, carboxyl, carboxylalkyl, thioaryloxy, thioheteroaryloxy, thioheterocyclooxy, thiol, thioalkoxy, substituted thioalkoxy, aryl, aryloxy, heteroaryl, heteroaryloxy, heterocyclyl, heterocyclooxy, hydroxyamino, alkoxyamino, nitro, -SO-alkyl, -SO-aryl, - SO-heteroaryl, -SO₂-alkyl, -SO₂-aryl, -SO₂-heteroaryl, and -NR^{a}R^{b}, wherein R' and R" may be the same or different and are chosen from hydrogen, optionally substituted alkyl, cycloalkyl, alkenyl, cycloalkenyl, alkynyl, aryl, heteroaryl and heterocyclic.

"Alkoxy" refers to the group -O-alkyl, wherein alkyl is as defined herein. Alkoxy includes, by way of example, methoxy, ethoxy, n-propoxy, isopropoxy, n-butoxy, t-butoxy, sec-butoxy, n-pentoxy, and the like. The term "alkoxy" also refers to the groups alkenyl-O-, cycloalkyl-O-, cycloalkenyl-O-, and alkynyl-O-, where alkenyl, cycloalkyl, cycloalkenyl, and alkynyl are as defined herein.

The term "substituted alkoxy" refers to the groups substituted alkyl-O-, substituted alkenyl-O-, substituted cycloalkyl-O-, substituted cycloalkenyl-O-, and substituted alkynyl-O- where substituted alkyl, substituted alkenyl, substituted cycloalkyl, substituted cycloalkenyl and substituted alkynyl are as defined herein.

"Alkynyl" or "alkyne" refers to straight or branched monovalent hydrocarbyl groups having from 2 to 6 carbon atoms and preferably 2 to 3 carbon atoms and having at least 1 and preferably from 1 to 2 sites of triple bond unsaturation. Examples of such alkynyl groups include acetylenyl (-C≡CH), and propargyl (-CH₂C≡CH).

The term "substituted alkynyl" or "substituted alkyne" refers to an alkynyl group as defined herein having from 1 to 5 substituents, or from 1 to 3 substituents, selected from alkoxy, substituted alkoxy, cycloalkyl, substituted cycloalkyl, cycloalkenyl, substituted cycloalkenyl, acyl, acylamino, acyloxy, amino, substituted amino, aminoacyl, aminoacyloxy, oxyaminoacyl, azido, cyano, halogen, hydroxyl, oxo, thioketo, carboxyl, carboxylalkyl, thioaryloxy, thioheteroaryloxy, thioheterocyclooxy, thiol, thioalkoxy, substituted thioalkoxy, aryl, aryloxy, heteroaryl, heteroaryloxy, heterocyclyl, heterocyclooxy, hydroxyamino, alkoxyamino, nitro, -SO-alkyl, -SO-substituted alkyl, -SO-aryl, -SO-heteroaryl, -SO₂-alkyl, -SO₂-substituted alkyl, -SO₂-aryl, and -SO₂-heteroaryl.

"Amino" refers to the group -NH₂. The term "substituted amino" refers to the group -NRR where each R is independently selected from the group consisting of hydrogen, alkyl, substituted alkyl, cycloalkyl, substituted cycloalkyl, alkenyl, substituted alkenyl, cycloalkenyl, substituted cycloalkenyl, alkynyl, substituted alkynyl, aryl, heteroaryl, and heterocyclyl provided that at least one R is not hydrogen.

"Aryl" by itself or as part of another substituent refers to a monovalent aromatic hydrocarbon radical derived by the removal of one hydrogen atom from a single carbon atom of an aromatic ring system. Aryl groups of interest include, but are not limited to, groups derived from aceanthrylene, acenaphthylene, acephenanthrylene, anthracene, azulene, benzene, chrysene, coronene, fluoranthene, fluorene, hexacene, hexaphene, hexalene, as-indacene, s-indacene, indane, indene, naphthalene, octacene, octaphene, octalene, ovalene, penta-2,4-diene, pentacene, pentalene, pentaphene, perylene, phenalene, phenanthrene, picene, pleiadene, pyrene, pyranthrene, rubicene, triphenylene, trinaphthalene and the like. In certain embodiments, an aryl group includes from 6 to 20 carbon atoms. In certain embodiments, an aryl group includes from 6 to 12 carbon atoms. Examples of an aryl group are phenyl and naphthyl.

"Substituted aryl", unless otherwise constrained by the definition for the aryl substituent, refers to an aryl group substituted with from 1 to 5 substituents, or from 1 to 3 substituents, selected from acyloxy, hydroxy, thiol, acyl, alkyl, alkoxy, alkenyl, alkynyl, cycloalkyl, cycloalkenyl, substituted alkyl, substituted alkoxy, substituted alkenyl, substituted alkynyl, substituted cycloalkyl, substituted cycloalkenyl, amino, substituted amino, aminoacyl, acylamino, alkaryl, aryl, aryloxy, azido, carboxyl, carboxylalkyl, cyano, halogen, nitro, heteroaryl, heteroaryloxy, heterocyclyl, heterocyclooxy, aminoacyloxy, oxyacylamino, thioalkoxy, substituted thioalkoxy, thioaryloxy, thioheteroaryloxy, -SO-alkyl, -SO-substituted alkyl, -SO-aryl, -SO-heteroaryl, -SO₂-alkyl, -SO₂-substituted alkyl, -SO₂-aryl, -SO₂-heteroaryl and trihalomethyl.

"Heteroaryl" by itself or as part of another substituent, refers to a monovalent heteroaromatic radical derived by the removal of one hydrogen atom from a single atom of a heteroaromatic ring system. Heteroaryl groups of interest include, but are not limited to, groups derived from acridine, arsindole, carbazole, β-carboline, chromane, chromene, cinnoline, furan, imidazole, indazole, indole, indoline, indolizine, isobenzofuran, isochromene, isoindole, isoindoline, isoquinoline, isothiazole, isoxazole, naphthyridine, oxadiazole, oxazole, perimidine, phenanthridine, phenanthroline, phenazine, phthalazine, pteridine, purine, pyran, pyrazine, pyrazole, pyridazine, pyridine, pyrimidine, pyrrole, pyrrolizine, quinazoline, quinoline, quinolizine, quinoxaline, tetrazole, thiadiazole, thiazole, triazole, benzotriazole, thiophene, triazole, xanthene, benzodioxole and the like. In certain embodiments, the heteroaryl group is from 5-20 membered heteroaryl. In certain embodiments, the heteroaryl group is from 5-10 membered heteroaryl. In certain embodiments, heteroaryl groups are those derived from thiophene, pyrrole, benzothiophene, benzofuran, indole, pyridine, quinoline, imidazole, oxazole and pyrazine.

"Heterocycle," "heterocyclic," "heterocycloalkyl," and "heterocyclyl" refer to a saturated or unsaturated group having a single ring or multiple condensed rings, including fused bridged and spiro ring systems, and having from 3 to 20 ring atoms, including 1 to 10 hetero atoms. These ring atoms are selected from the group consisting of nitrogen, sulfur, or oxygen, wherein, in fused ring systems, one or more of the rings can be cycloalkyl, aryl, or heteroaryl, provided that the point of attachment is through the non-aromatic ring. In certain embodiments, the nitrogen and/or sulfur atom(s) of the heterocyclic group are optionally oxidized to provide for the N-oxide, -S(O)-, or -SO₂- moieties.

Examples of heterocycles and heteroaryls include, but are not limited to, azetidine, pyrrole, imidazole, pyrazole, pyridine, pyrazine, pyrimidine, pyridazine, indolizine, isoindole, indole, dihydroindole, indazole, purine, quinolizine, isoquinoline, quinoline, phthalazine, naphthylpyridine, quinoxaline, quinazoline, cinnoline, pteridine, carbazole, carboline, phenanthridine, acridine, phenanthroline, isothiazole, phenazine, isoxazole, phenoxazine, phenothiazine, imidazolidine, imidazoline, piperidine, piperazine, indoline, phthalimide, 1,2,3,4-tetrahydroisoquinoline, 4,5,6,7-tetrahydrobenzo[b]thiophene, thiazole, thiazolidine, thiophene, benzo[b]thiophene, morpholinyl, thiomorpholinyl (also referred to as thiamorpholinyl), 1,1-dioxothiomorpholinyl, piperidinyl, pyrrolidine, tetrahydrofuranyl, and the like.

"Substituted heteroaryl", unless otherwise constrained by the definition for the substituent, refers to an heteroaryl group substituted with from 1 to 5 substituents, or from 1 to 3 substituents, selected from acyloxy, hydroxy, thiol, acyl, alkyl, alkoxy, alkenyl, alkynyl, cycloalkyl, cycloalkenyl, substituted alkyl, substituted alkoxy, substituted alkenyl, substituted alkynyl, substituted cycloalkyl, substituted cycloalkenyl, amino, substituted amino, aminoacyl, acylamino, alkaryl, aryl, aryloxy, azido, carboxyl, carboxylalkyl, cyano, halogen, nitro, heteroaryl, heteroaryloxy, heterocyclyl, heterocyclooxy, aminoacyloxy, oxyacylamino, thioalkoxy, substituted thioalkoxy, thioaryloxy, thioheteroaryloxy, -SO-alkyl, -SO-substituted alkyl, -SO-aryl, - SO-heteroaryl, -SO₂-alkyl, -SO₂-substituted alkyl, -SO₂-aryl, -SO₂-heteroaryl and trihalomethyl.

The term "alkaryl" or "aralkyl" refers to the groups -alkylene-aryl and substituted alkylene-aryl where alkylene, substituted alkylene and aryl are defined herein.

"Alkylene" refers to divalent aliphatic hydrocarbyl groups preferably having from 1 to 6 and more preferably 1 to 3 carbon atoms that are either straight-chained or branched, and which are optionally interrupted with one or more groups selected from -O-, -NR¹⁰-, -NR¹⁰C(O)-, -C(O)NR¹⁰- and the like. This term includes, by way of example, methylene (-CH₂-), ethylene (-CH₂CH₂-), n-propylene (-CH₂CH₂CH₂-), isopropylene (-CH₂CH(CH₃)-), (-C(CH₃)₂CH₂CH₂-), (-C(CH₃)₂CH₂C(O)-), (-C(CH₃)₂CH₂C(O)NH-), (-CH(CH₃)CH₂-), and the like. "Substituted alkylene" refers to an alkylene group having from 1 to 3 hydrogens replaced with substituents as described for carbons in the definition of "substituted" below.

"Substituted" refers to a group in which one or more hydrogen atoms are independently replaced with the same or different substituent(s). Substituents of interest include, but are not limited to, alkylenedioxy (such as methylenedioxy), -M, -R⁶⁰, -O-, =O, -OR⁶⁰, -SR⁶⁰, -S⁻, =S, -NR⁶⁰R⁶¹, =NR⁶⁰, -CF₃, -CN, -OCN, -SCN, -NO, -NO₂, =N₂, -N₃, -S(O)₂O⁻, -S(O)₂OH, -S(O)₂R⁶⁰, -OS(O)₂O⁻, -OS(O)₂R⁶⁰, -P(O)(O⁻)₂, -P(O)(O R⁶⁰)(O⁻), -OP(O)(OR⁶⁰)(OR⁶¹), -C(O)R⁶⁰, -C(S)R⁶⁰, -C(O)OR⁶⁰, -C(O)NR⁶⁰R⁶¹,-C(O)O-, -C(S)OR⁶⁰, -NR⁶²C(O)NR⁶⁰R⁶¹, -NR⁶²C(S)NR⁶⁰R⁶¹, -NR⁶²C(NR⁶³)NR⁶⁰R⁶¹ and -C(NR⁶²)NR⁶⁰R⁶¹ where M is halogen; R⁶⁰, R⁶¹, R⁶² and R⁶³ are independently hydrogen, alkyl, substituted alkyl, alkoxy, substituted alkoxy, cycloalkyl, substituted cycloalkyl, cycloheteroalkyl, substituted cycloheteroalkyl, aryl, substituted aryl, heteroaryl or substituted heteroaryl, or optionally R⁶⁰ and R⁶¹ together with the nitrogen atom to which they are bonded form a cycloheteroalkyl or substituted cycloheteroalkyl ring; and R⁶⁴ and R⁶⁵ are independently hydrogen, alkyl, substituted alkyl, aryl, cycloalkyl, substituted cycloalkyl, cycloheteroalkyl, substituted cycloheteroalkyl, aryl, substituted aryl, heteroaryl or substituted heteroaryl, or optionally R⁶⁴ and R⁶⁵ together with the nitrogen atom to which they are bonded form a cycloheteroalkyl or substituted cycloheteroalkyl ring. In certain embodiments, substituents include -M, -R⁶⁰, =O, -OR⁶⁰, -SR⁶⁰, -S⁻, =S, -NR⁶⁰R⁶¹, =NR⁶⁰, -CF₃, -CN, -OCN, -SCN, -NO, -NO₂, =N₂, -N₃, -S(O)₂R⁶⁰, -OS(O)₂O⁻, -OS(O)₂R⁶⁰, -P(O)(O⁻)₂, -P(O)(OR⁶⁰)(O⁻), -OP(O)(OR ⁶⁰)(OR⁶¹), -C(O)R⁶⁰, -C(S)R⁶⁰, -C(O)OR⁶⁰, -C(O)NR⁶⁰R⁶¹,-C(O)O-, -NR⁶²C(O)NR⁶⁰R⁶¹. In certain embodiments, substituents include -M, -R⁶⁰, =O, -OR⁶⁰, -SR⁶⁰, -NR⁶⁰R⁶¹, -CF₃, -CN, -NO₂, -S(O)₂R⁶⁰, -P(O)(OR⁶⁰)(O⁻), -OP(O)(OR ⁶⁰)(OR⁶¹), -C(O)R⁶⁰, -C(O)OR⁶⁰, -C(O)NR⁶⁰R⁶¹,-C(O)O⁻. In certain embodiments, substituents include -M, -R⁶⁰, =O, -OR⁶⁰, -SR⁶⁰, -NR⁶⁰R⁶¹, -CF₃, -CN, -NO₂, -S(O)₂R⁶⁰, -OP(O)(OR⁶⁰)(OR⁶¹), -C(O)R ⁶⁰, -C(O)OR⁶⁰ ,-C(O)O⁻, where R⁶⁰, R⁶¹ and R⁶² are as defined above. For example, a substituted group may bear a methylenedioxy substituent or one, two, or three substituents selected from a halogen atom, a (1-4C)alkyl group and a (1-4C)alkoxy group. When the group being substituted is an aryl or heteroaryl group, the substituent(s) (e.g., as described herein) may be referred to as "aryl substituent(s)".

It is understood that in all substituted groups defined above, polymers arrived at by defining substituents with further substituents to themselves (e.g., substituted aryl having a substituted aryl group as a substituent which is itself substituted with a substituted aryl group, which is further substituted by a substituted aryl group, etc.) are not intended for inclusion herein. In such cases, the maximum number of such substitutions is three. For example, serial substitutions of substituted aryl groups specifically contemplated herein are limited to substituted aryl-(substituted aryl)-substituted aryl.

Unless indicated otherwise, the nomenclature of substituents that are not explicitly defined herein are arrived at by naming the terminal portion of the functionality followed by the adjacent functionality toward the point of attachment. For example, the substituent "arylalkyloxycarbonyl" refers to the group (aryl)-(alkyl)-O-C(O)-.

As to any of the groups disclosed herein which contain one or more substituents, it is understood, of course, that such groups do not contain any substitution or substitution patterns which are sterically impractical and/or synthetically non-feasible. In addition, the subject compounds include all stereochemical isomers arising from the substitution of these compounds.

It will be appreciated that the definitions provided herein are not intended to be mutually exclusive. Accordingly, some chemical moieties may fall within the definition of more than one term.

Other definitions of terms may appear throughout the specification.

### DETAILED DESCRIPTION

As summarized above, water-soluble dipyrromethene-based dyes are provided herein. In certain cases, the water-soluble dye is a dipyrromethene-boron dye. Embodiments of the subject water-soluble dipyrromethene-based dyes have a narrow absorption maxima in the yellow green range close to or at the 561 nm laser line, and are suitable for use in tandem dye systems. Embodiments of the subject dyes efficiently transfer energy to an acceptor chromophore while a second dipyrromethene-based dye of the same or different chemical structure is within energy-receiving proximity therewith. Also provided herein are tandem dyes that include the subject water-soluble dipyrromethene-based dyes, e.g., as donor chromophores. The tandem dyes include a pendant acceptor fluorophore configured as an energy acceptor in energy-receiving proximity to a donor water-soluble dipyrromethene-based dye. Methods of evaluating a sample for the presence of a target analyte and methods of labelling a target molecule in which the subject tandem dyes find use are also provided. Systems and kits for practicing the subject methods are also provided.

Before the present invention is described in greater detail, it is to be understood that this invention is not limited to particular embodiments described, as such may, of course, vary. It is also to be understood that the terminology used herein is for the purpose of describing particular embodiments only, and is not intended to be limiting, since the scope of the present invention will be limited only by the appended claims.

Where a range of values is provided, it is understood that each intervening value, to the tenth of the unit of the lower limit unless the context clearly dictates otherwise, between the upper and lower limit of that range and any other stated or intervening value in that stated range, is encompassed within the invention. The upper and lower limits of these smaller ranges may independently be included in the smaller ranges and are also encompassed within the invention, subject to any specifically excluded limit in the stated range. Where the stated range includes one or both of the limits, ranges excluding either or both of those included limits are also included in the invention.

Certain ranges are presented herein with numerical values being preceded by the term "about." The term "about" is used herein to provide literal support for the exact number that it precedes, as well as a number that is near to or approximately the number that the term precedes. In determining whether a number is near to or approximately a specifically recited number, the near or approximating unrecited number may be a number which, in the context in which it is presented, provides the substantial equivalent of the specifically recited number.

Unless defined otherwise, all technical and scientific terms used herein have the same meaning as commonly understood by one of ordinary skill in the art to which this invention belongs. Although any methods and materials similar or equivalent to those described herein can also be used in the practice or testing of the present invention, representative illustrative methods and materials are now described.

All publications and patents cited in this specification are herein incorporated by reference as if each individual publication or patent were specifically and individually indicated to be incorporated by reference and are incorporated herein by reference to disclose and describe the methods and/or materials in connection with which the publications are cited. The citation of any publication is for its disclosure prior to the filing date and should not be construed as an admission that the present invention is not entitled to antedate such publication by virtue of prior invention. Further, the dates of publication provided may be different from the actual publication dates which may need to be independently confirmed.

It is noted that, as used herein and in the appended claims, the singular forms "a", "an", and "the" include plural referents unless the context clearly dictates otherwise. It is further noted that the claims may be drafted to exclude any optional element. As such, this statement is intended to serve as antecedent basis for use of such exclusive terminology as "solely," "only" and the like in connection with the recitation of claim elements, or use of a "negative" limitation.

As will be apparent to those of skill in the art upon reading this disclosure, each of the individual embodiments described and illustrated herein has discrete components and features which may be readily separated from or combined with the features of any of the other several embodiments without departing from the scope or spirit of the present invention. Any recited method can be carried out in the order of events recited or in any other order which is logically possible.

While the apparatus and method has or will be described for the sake of grammatical fluidity with functional explanations, it is to be expressly understood that the claims, unless expressly formulated under 35 U.S.C. §112, are not to be construed as necessarily limited in any way by the construction of "means" or "steps" limitations, but are to be accorded the full scope of the meaning and equivalents of the definition provided by the claims under the judicial doctrine of equivalents, and in the case where the claims are expressly formulated under 35 U.S.C. §112 are to be accorded full statutory equivalents under 35 U.S.C. §112.

In further describing various aspects of the invention, embodiments of the dyes of the invention are reviewed first in greater detail, followed by a review of tandem dyes that include the subject dyes, as well as methods of using the dyes.

### DIPYRROMETHENE-BASED DYES

As summarized above, the present disclosure provides water-soluble dipyrromethene-based dyes. The term "dipyrromethene-based dyes" refers to a dye including the following core structure:

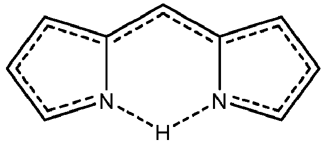

In certain cases, the subject dipyrromethene-based dyes are yellow-green absorbing dyes. In certain cases, the subject water-soluble dipyrromethene-based dye absorbs in the yellow-green range at 561 nm. In certain cases, the water-soluble dipyrromethene-based dye has an absorption maxima of from 540 nm to 590 nm, of from 555 to 585 nm, such as 555 to 575 nm, such as 555 to 574 nm, 555 to 573 nm, 555 to 572 nm, 555 to 571 nm, 555 to 570 nm, 555 to 569 nm, 555 to 568 nm, 555 to 567 nm, 555 to 566 nm, 555 to 565 nm, 555 to 564 nm, 555 to 563 nm, or 555 to 562 nm. In certain cases, the water-soluble dipyrromethene-based dye has an absorption maxima of from 558 to 568 nm, such as 558 to 567 nm, 558 to 566 nm, 558 to 565 nm, 558 to 564 nm, 558 to 563 nm, or 558 to 562 nm. In certain cases, the subject water-soluble dipyrromethene-based dye has an absorption maxima of about, such as at or close to, 561 nm or 562 nm.

In certain embodiments, the subject water-soluble dipyrromethene-based dyes exhibit narrow absorption spectrum. A narrow absorption spectrum is one that has an absorption maxima peak having a full-width half maximum (FWHM) of 35 nm or less, such as 25nm or less, 20nm or less, 15 nm or less, 12 nm or less or 10nm or less, e.g., as measured in ethanol or in phosphate buffered saline (PBS).

In some instances, the dipyromethene-based dye has an extinction coefficient of 5 × 10⁵ cm⁻¹M⁻¹ or more, such as 6 × 10⁵ cm⁻¹M⁻¹ or more, 7 × 10⁵ cm⁻¹M⁻¹ or more, 8 × 10⁵ cm⁻¹M⁻¹ or more, 9 × 10⁵ cm⁻¹M⁻¹ or more, such as 1 × 10⁶ cm⁻¹M⁻¹ or more, 1.5 × 10⁶ cm⁻¹M⁻¹ or more, 2 × 10⁶ cm⁻¹M⁻¹ or more, 2.5 × 10⁶ cm⁻¹M⁻¹ or more, 3 × 10⁶ cm⁻¹M⁻¹ or more, 4 × 10⁶ cm⁻¹M⁻¹ or more, 5 × 10⁶ cm⁻¹M⁻¹ or more, 6 × 10⁶ cm⁻¹M⁻¹ or more, 7 × 10⁶ cm⁻¹M⁻¹ or more, or 8 × 10⁶ cm⁻¹M⁻¹ or more. In some embodiments, the polymeric tandem dye has a molar extinction coefficient of 5 × 105 M-1cm-1 or more. In certain embodiments, the tandem dye has a molar extinction coefficient of 1 × 10⁶ M⁻¹cm⁻¹ or more.

The subject water-soluble dipyrromethene-dyes are substituted with one or more water solubilizing groups (WSG). A water-soluble dye of the present disclosure has solubility under aqueous conditions that makes it especially suitable for application to a variety of biological assays. The subject water soluble dyes, tandem dyes, and conjugates thereof, can be resistant to undesirable aggregation which provides advantageous fluorescence and spectroscopic properties in various biological assays. Aggregation of dyes is undesirable because it can lead to reduced fluorescent signals, e.g., via aggregation-caused quenching of dye fluorescence. The subject water-soluble dyes and tandem dyes that include the same can be used as fluorescent reporters for a variety of biosensors and provide signals of exceptional brightness with a range of options for excitation and emission wavelength for applications such as Flow Cytometry, and imaging.

A variety of water soluble polymer groups can be adapted for use in the WSG of the subject dyes. Any convenient water solubilizing groups (WSG's) may be included in the dyes described herein to provide for increased water-solubility. While the increase in solubility may vary, in some instances the increase (as compared to the compound without the WSG(s)) is 2 fold or more, e.g., 5 fold, 10 fold, 25 fold, 50 fold, 100 fold or more. In some cases, the hydrophilic water solubilizing group is charged, e.g., positively or negatively charged. In certain cases, the hydrophilic water solubilizing group is a neutral hydrophilic group. In some embodiments, the WSG is branched (e.g., as described herein). In certain instances, the WSG is linear. In some embodiments, the WSG is a hydrophilic polymer, e.g., a polyethylene glycol, a modified PEG, a peptide sequence, a peptoid, a carbohydrate, an oxazoline, a polyol, a dendron, a dendritic polyglycerol, a cellulose, a chitosan, or a derivative thereof. Water solubilizing groups of interest include, but are not limited to, carboxylate, phosphonate, phosphate, sulfonate, sulfate, sulfinate, sulfonium, ester, polyethylene glycols (PEG) and modified PEGs, hydroxyl, amine, amino acid, ammonium, guanidinium, pyridinium, polyamine and sulfonium, polyalcohols, straight chain or cyclic saccharides, primary, secondary, tertiary, or quaternary amines and polyamines, phosphonate groups, phosphinate groups, ascorbate groups, glycols, including, polyethers, -COOM', -SO₃M', -PO₃M', -NR₃⁺, Y', (CH₂CH₂O)ₚR and mixtures thereof, where Y' can be any halogen, sulfate, sulfonate, or oxygen containing anion, p can be 1 to 500, each R can be independently H or an alkyl (such as methyl) and M' can be a cationic counterion or hydrogen,-(CH₂CH₂O)_{yy}CH₂CH₂XR^{yy}, --(CH₂CH₂O)_{yy}CH₂CH₂X--, --X(CH₂CH₂O)_{yy}CH₂CH₂--, glycol, and polyethylene glycol, wherein yy is selected from 1 to 1000, X is selected from O, S, and NR^{ZZ}, and R^{ZZ} and R^{YY} are independently selected from H and C₁₋₃ alkyl. In some cases, a WSG is (CH₂)ₓ(OCH₂CH₂)_{y}OCH₃ where each x is independently an integer from 0-20, each y is independently an integer from 0 to 50. In some cases, the water solubilizing group includes a non-ionic polymer (e.g., a PEG polymer) substituted at the terminal with an ionic group (e.g., a sulfonate).

In some embodiments of the formulae, the pendant group of interest includes a substituent selected from (CH₂)ₓ(OCH₂CH₂)_{y}OCH₃ where each x is independently an integer from 0-20, each y is independently an integer from 0 to 50; and a benzyl optionally substituted with one or more halogen, hydroxyl, C₁-C₁₂ alkoxy, or (OCH₂CH₂)_{z}OCH₃ where each z is independently an integer from 0 to 50. In some instances, the substituent is (CH₂)₃(OCH₂CH₂)₁₁OCH₃. In some embodiments, one or more of the substituents is a benzyl substituted with at least one WSG groups (e.g., one or two WSG groups) selected from (CH₂)ₓ(OCH₂CH₂)_{y}OCH₃ where each x is independently an integer from 0-20 and each y is independently an integer from 0 to 50.

Multiple WSGs may be included at a single location in the subject dyes via a branching linker. In certain embodiments, the branching linker is an aralkyl substituent, further di-substituted with water solubilizing groups. As such, in some cases, the branching linker group is a substituent of the dye that connects the dye to two or more water solubilizing groups. In certain embodiments, the branching linker is an amino acid, e.g., a lysine amino acid that is connected to three groups via the amino and carboxylic acid groups. In some cases, the incorporation of multiple WSGs via branching linkers imparts a desirable solubility on the dye. In some instances, the WSG is a non-ionic sidechain group capable of imparting solubility in water in excess of 50 mg/mL. In some instances, the WSG is a non-ionic sidechain group capable of imparting solubility in water in excess of 100 mg/mL. In some embodiments, the dye includes substituent(s) selected from the group consisting of, an alkyl, an aralkyl and a heterocyclic group, each group further substituted with a include water solubilizing groups hydrophilic polymer group, such as a polyethylglycol (PEG) (e.g., a PEG group of 6-24 units).

Water soluble polymers of interest that can be utilized in the WSG include polyethylene glycol (PEG) groups or modified PEG groups. Water-soluble polymers of interest include, but are not limited to, polyalkylene oxide based polymers, such as polyethylene glycol "PEG" (See. e.g., "Poly(ethylene glycol) Chemistry: Biotechnical and Biomedical Applications", J. M. Harris, Ed., Plenum Press, New York, N.Y. (1992); and "Poly(ethylene glycol) Chemistry and Biological Applications", J. M. Harris and S. Zalipsky, Eds., ACS (1997); and International Patent Applications: WO 90/13540, WO 92/00748, WO 92/16555, WO 94/04193,WO 94/14758, WO 94/17039, WO 94/18247, WO 94/28937, WO 95/11924, WO 96/00080, WO 96/23794, WO 98/07713, WO 98/41562, WO 98/48837, WO 99/30727, WO 99/32134, WO 99/33483, WO 99/53951, WO 01/26692, WO 95/13312, WO 96/21469, WO 97/03106, WO 99/45964, and U.S. Pat. Nos. 4,179,337; 5,075,046; 5,089,261; 5,100,992; 5,134,192; 5,166,309; 5,171,264; 5,213,891; 5,219,564; 5,275,838; 5,281,698; 5,298,643; 5,312,808; 5,321,095; 5,324,844; 5,349,001; 5,352,756; 5,405,877; 5,455,027; 5,446,090; 5,470,829; 5,478,805; 5,567,422; 5,605,976; 5,612,460; 5,614,549; 5,618,528; 5,672,662; 5,637,749; 5,643,575; 5,650,388; 5,681,567; 5,686,110; 5,730,990; 5,739,208; 5,756,593; 5,808,096; 5,824,778; 5,824,784; 5,840,900; 5,874,500; 5,880,131; 5,900,461; 5,902,588; 5,919,442; 5,919,455; 5,932,462; 5,965,119; 5,965,566; 5,985,263; 5,990,237; 6,011,042; 6,013,283; 6,077,939; 6,113,906; 6,127,355; 6,177,087; 6,180,095; 6,194,580; 6,214,966).

Examples of water soluble polymers of interest include, but are not limited to, those containing a polyalkylene oxide, polyamide alkylene oxide, or derivatives thereof, including polyalkylene oxide and polyamide alkylene oxide comprising an ethylene oxide repeat unit of the formula -(CH₂-CH₂-O)-. Further examples of polymers of interest include a polyamide having a molecular weight greater than 1,000 Daltons of the formula -[C(O)-X-C(O)-NH-Y-NH]n- or -[NH-Y-NH-C(O)-X-C(O)]ₙ-, where X and Y are divalent radicals that may be the same or different and may be branched or linear, and n is a discrete integer from 2-100, such as from 2 to 50, and where either or both of X and Y comprises a biocompatible, substantially non-antigenic water-soluble repeat unit that may be linear or branched. Further examples of water-soluble repeat units comprise an ethylene oxide of the formula - (CH₂-CH₂-O)- or -(O-CH₂-CH₂)- . The number of such water-soluble repeat units can vary significantly, with the number of such units being from 2 to 500, 2 to 400, 2 to 300, 2 to 200, 2 to 100, 6-100, for example from 2 to 50 or 6 to 50. An example of an embodiment is one in which one or both of X and Y is selected from: -((CH₂)ₙ₁-(CH₂-CH₂-O)ₙ₂-(CH₂)- or -((CH₂)ₙ₁-O-CH₂-CH₂)ₙ₂-(CH₂)ₙ₋₁-), where n1 is 1 to 6, 1 to 5, 1 to 4, or 1 to 3, and where n2 is 2 to 50, 2 to 25, 2 to 15, 2 to 10, 2 to 8, or 2 to 5. A further example of an embodiment is one in which X is -(CH₂-CH₂)-, and where Y is - (CH₂-(CH₂-CH₂-O)₃-CH₂-CH₂-CH₂)- or-(CH₂-CH₂-CH₂-(O-CH₂-CH₂)₃-CH₂)-.

The term modified polymer, such as a modified PEG, refers to water soluble polymers that have been modified or derivatized at either or both terminals, e.g., to include a terminal substituent (e.g., a terminal alkyl, substituted alkyl, alkoxy or substituted alkoxy, etc.) and/or a terminal linking functional group (e.g., an amino or carboxylic acid group suitable for attachment via amide bond formation) suitable for attached of the polymer to a molecule of interest (e.g., to a light harvesting chromophore via a branching group). The subject water soluble polymers can be adapted to include any convenient linking groups. It is understood that in some cases, the water soluble polymer can include some dispersity with respect to polymer length, depending on the method of preparation and/or purification of the polymeric starting materials. In some instances, the water soluble polymers are monodisperse.

The water soluble polymer can include one or more spacers or linkers. Examples of spacers or linkers include linear or branched moieties comprising one or more repeat units employed in a water-soluble polymer, diamino and or diacid units, natural or unnatural amino acids or derivatives thereof, as well as aliphatic moieties, including alkyl, aryl, heteroalkyl, heteroaryl, alkoxy, and the like, which can contain, for example, up to 18 carbon atoms or even an additional polymer chain.

The water soluble polymer moiety, or one or more of the spacers or linkers of the polymer moiety when present, may include polymer chains or units that are biostable or biodegradable. For example, polymers with repeat linkages have varying degrees of stability under physiological conditions depending on bond lability. Polymers with such bonds can be categorized by their relative rates of hydrolysis under physiological conditions based on known hydrolysis rates of low molecular weight analogs, e.g., from less stable to more stable, e.g., polyurethanes (-NH-C(O)-O-) > polyorthoesters (-O-C((OR)(R'))-O-) > polyamides (-C(O)-NH-). Similarly, the linkage systems attaching a water-soluble polymer to a target molecule may be biostable or biodegradable, e.g., from less stable to more stable: carbonate (-O-C(O)-O-) > ester (-C(O)-O-) > urethane (-NH-C(O)-O-) > orthoester (-O-C((OR)(R'))-O-) > amide (-C(O)-NH-). In general, it may be desirable to avoid use of a sulfated polysaccharide, depending on the lability of the sulfate group. In addition, it may be less desirable to use polycarbonates and polyesters. These bonds are provided by way of example, and are not intended to limit the types of bonds employable in the polymer chains or linkage systems of the water-soluble polymers useful in the WSGs disclosed herein.

The water soluble group (WSG) can be capable of imparting solubility in water in excess of 10 mg/mL to the subject dye or polymeric tandem dye, such as in excess of 20 mg/mL, in excess of 30 mg/mL, in excess of 40 mg/mL, in excess of 50 mg/mL, in excess of 60 mg/mL, in excess of 70 mg/mL, in excess of 80 mg/mL, in excess of 90 mg/mL or in excess of 100 mg/mL. In certain cases, the branched non-ionic water soluble group (WSG) is capable of imparting solubility in water (e.g., an aqueous buffer) of 20 mg/mL or more to the subject dye or polymeric tandem dye, such as 30 mg/mL or more, 40 mg/mL or more, 50 mg/mL or more, 60 mg/mL or more, 70 mg/mL or more, 80 mg/mL or more, 90 mg/mL or more, 100 mg/mL or more, or even more. It is understood that water-soluble dipyrromethene-based dye may, under certain conditions, form discrete water solvated nanoparticles in aqueous systems. In certain cases, the water solvated nanoparticles are resistant to aggregation and find use in a variety of biological assays.

In certain cases, the dipyrromethene-based dye is a dipyrromethene-boron based dye. In certain cases, the water-soluble dipyrromethene-based dye is of formula (I): 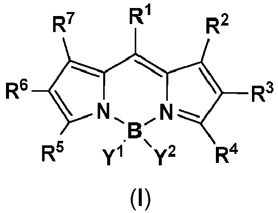 wherein:
R¹-R⁷ are each independently selected from H, alkyl, halogen, substituted alkyl, alkoxy, substituted alkoxy, amino, substituted amino, amido, substituted amido, thiol, substituted thiol, aryl, substituted aryl, heteroaryl, substituted heteroaryl, heterocycle, substituted heterocycle, alkenyl, substituted alkenyl, alkynyl, substituted alkynyl, water solubilizing group (WSG), and -T¹-J¹, or
optionally any one or more pairs of substituents selected from R⁶ and R⁷, R² and R³, R⁵ and R⁶, R³ and R⁴, R² and R¹ and R⁷ and R¹, together form a divalent radical and are cyclically linked and together with the carbon atoms to which they are bound provide a 5- or 6-membered fused heterocycle, carbocycle, aryl or heteroaryl ring (e.g., a 5- or 6-membered ring comprising carbon atoms and 0-3 heteroatoms selected from O, S and N), which ring may be unsubstituted or further substituted with a substituent independently selected from alkyl, substituted alkyl, alkoxy, substituted alkoxy, aryl, substituted aryl, heteroaryl, substituted heteroaryl, alkenyl, substituted alkenyl, alkynyl, substituted alkynyl, water solubilizing group (WSG), and -T¹-J¹;
T¹ is an optional linker;
J¹ is a repeat unit of a compound of formula (I); and
Y¹ and Y² are independently selected from F, OH, H, cyano, alkyl, substituted alkyl, aryl, substituted aryl, heteroaryl, substituted heteroaryl, alkoxy, substituted alkoxy, alkenyl, substituted alkenyl, alkynyl, substituted alkynyl, and water solubilizing group (WSG), or
Y¹ and Y² together with the boron atom to which they are bound form a 5- or 6-membered ring (e.g., a 5- or 6-membered ring comprising a boron atom, carbon atoms and 0-3 heteroatoms selected from O, S and N), which ring may be unsubstituted or further substituted with a substituent independently selected from alkyl, substituted alkyl, alkoxy, substituted alkoxy, aryl, substituted aryl, heteroaryl, substituted heteroaryl, alkenyl, substituted alkenyl, alkynyl, substituted alkynyl, water solubilizing group (WSG), and -T¹-Z¹; and
wherein at least one moiety selected from Y¹, Y² and R¹-R⁷ is a WSG, or at least one moiety selected from Y¹, Y² and R¹-R⁷ is substituted with a WSG, where the WSG is of the formula (Ia):

   -(L¹)ₙ₁ - (X¹)ₙ₂ - ((L²)ₙ₃-Z¹)ₘ (Ia)

   wherein:
   n1, n2 and n3 are each independently 0 or 1;
   L¹, if present, is a linker;
   L² if present, is a linker;
   X¹, if present, is a branching point;
   Z¹ is a water soluble polymer comprising 6-24 monomeric units; and
   m is an integer from 1 to 3.

In some cases of the WSG of formula (la), L¹, L², and X¹ are absent (i.e., n1-n3 are 0), and the WSG is Z¹, i.e., a water soluble polymer comprising 6-24 monomeric units. It certain cases, at least one moiety selected from Y¹, Y² and R¹-R⁷ is substituted with a Z¹ group, and Y¹, Y² and R¹-R⁷ are defined as described herein above. In certain instances, Y¹ and Y² are both substituted with a Z¹ group (e.g., as described herein). In certain instances, Z¹ is a PEG or modified PEG polymer of 6-24 monomeric units, such as 10-30, 10-24, 10-20, 12-24, 12-20, 12-16 or 16-20 monomeric units.

In certain cases of the WSG of formula (la), L² and X¹ are absent, n1 is 1, and the WSG is -L¹-Z¹, where L¹ is a linker (e.g., as described herein) having a backbone of 20 atoms or less in length and Z¹ is a WSG (e.g., as described herein). In some instances, L¹ is selected from an alkyl or substituted alkyl linker, an alkenyl or substituted alkenyl linker, an alkynyl or substituted alkynyl linker, an alkoxy or substituted alkoxy linker, a PEG linker, a sulfonamido-alkyl or substituted sulfonamido-alkyl linker, an amido-alkyl or substituted amido-alkyl linker and an alkyl-amido-alkyl or substituted alkyl-amido-alkyl linker. In certain instances L¹ is selected from a C₁-C₁₂ alkyl or substituted alkyl linker, a C₁-C₁₂ alkenyl or substituted alkenyl linker, a C₁-C₁₂ alkynyl or substituted alkynyl linker, a C₁-C₁₂ alkoxy or substituted alkoxy linker, a C₁-C₁₂ amido-alkyl or substituted amido-alkyl linker and a C₁-C₁₂ alkyl-amido-alkyl or substituted alkyl-amido-alkyl linker. Z¹ is a water soluble polymer (e.g., as described herein). In some instances, the water soluble polymer is a PEG group. In certain instances, Z¹ is a PEG or modified PEG polymer of 6-24 monomeric units, such as 10-30, 10-24, 10-20, 12-24, 12-20, 12-16 or 16-20 monomeric units.

In some other embodiments of the WSG of formula (la), n1 and n2 are each 1, n3 is 1 or 0, m is 2, such that the WSG has formula (Ic) or (Id):

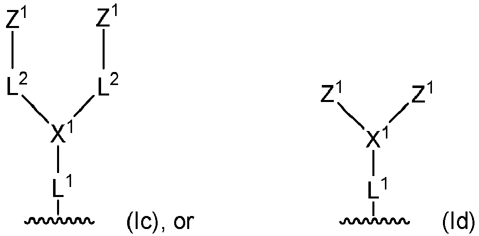

Wherein, L¹, X¹, L² and Z¹ are as defined herein above. In some instances, L¹ is a linker having a backbone of 20 atoms or less in length and Z¹ is a WSG (e.g., as described herein). In some instances, L¹ is selected from an alkyl or substituted alkyl linker, an alkenyl or substituted alkenyl linker, an alkynyl or substituted alkynyl linker, an acyl or substituted acyl, an alkoxy or substituted alkoxy linker, a PEG linker, a sulfonamido-alkyl or substituted sulfonamido-alkyl linker, an amido-alkyl or substituted amido-alkyl linker and an alkyl-amido-alkyl or substituted alkyl-amido-alkyl linker. In certain instances, L¹ and L² are each independently selected from a C₁-C₁₂ alkyl or substituted alkyl linker, a C₁-C₁₂ alkenyl or substituted alkenyl linker, a C₁-C₁₂ alkynyl or substituted alkynyl linker, a C₁-C₁₂ acyl or substituted acyl linker, a C₁-C₁₂ alkoxy or substituted alkoxy linker, a C₁-C₁₂ amido-alkyl or substituted amido-alkyl linker and a C₁-C₁₂ alkyl-amido-alkyl or substituted alkyl-amido-alkyl linker. In certain cases, the linker comprises a carbonyl group. In certain cases, L¹ comprises a carbonyl group, and L² is a C₁-C₁₂ alkyl or substituted alkyl. In certain instances, the branching point X¹ is selected from N, CR', C(=O)N, SO₂N, a tri-substituted aryl group (e.g., a 1,3,5-phenyl), a tetra-substituted aryl group, and a tri-substituted heteroaryl group. In certain instances, the branching point X¹ is a nitrogen atom. In other instances, the branching point X¹ is CR', where R' is selected from hydrogen, alkyl, substituted alkyl, or -L²-Z¹ (e.g., as described herein). Z¹ is a water soluble polymer (e.g., as described herein). In some instances, the water soluble polymer is a PEG group. In certain instances, each Z¹ is independently a PEG or modified PEG polymer of 6-24 monomeric units, such as 10-30, 10-24 or 10-20, 12-24, 12-20, 12-16 or 16-20 monomeric units. In certain cases, at least one of R⁵ and R⁴ comprises a WSG of formula (Ic) or (Id).

In some other embodiments, the WSG is a branched group of the formula (Ie): 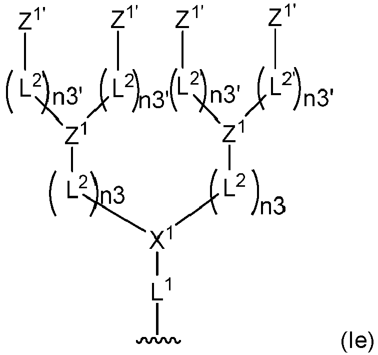 wherein each of L¹, X¹, L², n3 and Z¹ is as defined above; L^{2'}, n3' and Z^{1'} is as defined herein for L², n3 and Z¹ respectively.

In some other embodiments, the WSG is a branched group of formula (1f): 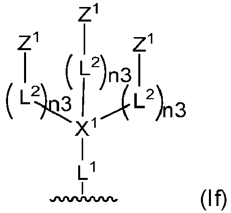 wherein each of L¹, X¹, L², n3 and Z¹ is as defined above; and X¹ is a branching group selected from a carbon atom and a tetra-substituted aryl group (e.g., a 1, 3, 4, 5-phenyl).

In some embodiments, the water-soluble dipyrromethene-based dye of formula (I), further includes a WSG of formula (Ib):

-(L¹)ₙ₁ - (X¹)ₙ₂ - ((L²)ₙ₃-Z²)ₘ (Ib)

wherein:
n1, n2 and n3 are each independently 0 or 1;
L¹, if present, is a linker;
L² if present, is a linker;
X¹, if present, is a branching point;
Z² is a water soluble polymer comprising 1-50 monomeric units; and
m is an integer from 1 to 3.

In some cases of the WSG of formula (lb), L¹, L², and X¹ are absent (i.e., n1-n3 are 0), and the WSG is Z², i.e., a water soluble polymer comprising 1-50 monomeric units. In certain instances, Z² is a PEG or modified PEG polymer of 1-50 monomeric units, such as 1-40, 1-30, 1-20, 2-24, 2-20, 2-10 or 2-6 monomeric units.

In certain cases of the WSG of formula (lb), L² and X¹ are absent, n1 is 1, and the WSG is -L¹-Z², where L¹ is a linker (e.g., as described herein) having a backbone of 20 atoms or less in length and Z² is a WSG (e.g., as described herein). In some instances, L¹ is selected from an alkyl or substituted alkyl linker, an alkenyl or substituted alkenyl linker, an alkynyl or substituted alkynyl linker, an alkoxy or substituted alkoxy linker, a PEG linker, a sulfonamido-alkyl or substituted sulfonamido-alkyl linker, an amido-alkyl or substituted amido-alkyl linker and an alkyl-amido-alkyl or substituted alkyl-amido-alkyl linker. In certain instances L¹ is selected from a C₁-C₁₂ alkyl or substituted alkyl linker, a C₁-C₁₂ alkenyl or substituted alkenyl linker, a C₁-C₁₂ alkynyl or substituted alkynyl linker, a C₁-C₁₂ alkoxy or substituted alkoxy linker, a C₁-C₁₂ amido-alkyl or substituted amido-alkyl linker and a C₁-C₁₂ alkyl-amido-alkyl or substituted alkyl-amido-alkyl linker. Z¹ is a water soluble polymer (e.g., as described herein). In some instances, the water soluble polymer is a PEG group. In certain instances, Z² is a PEG or modified PEG polymer of 1-50 monomeric units, such as 1-40, 1-30, 1-20, 2-24, 2-20, 2-10 or 2-6 monomeric units.

In some other embodiments of the WSG of formula (lb), n1 and n2 are each 1, n3 is 1 or 0, m is 2, such that the WSG has formula (Ig) or (Ih):

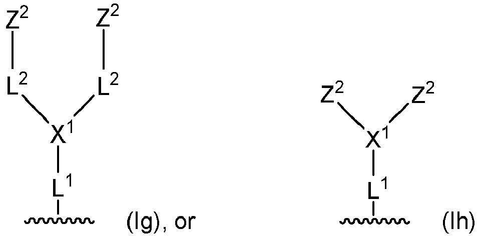

Wherein, L¹, X¹, L² and Z² are as defined herein above. In some instances, L¹ is a linker having a backbone of 20 atoms or less in length and Z² is a WSG (e.g., as described herein). In some instances, L¹ is selected from an alkyl or substituted alkyl linker, an alkenyl or substituted alkenyl linker, an alkynyl or substituted alkynyl linker, an acyl or substituted acyl, an alkoxy or substituted alkoxy linker, a PEG linker, a sulfonamido-alkyl or substituted sulfonamido-alkyl linker, an amido-alkyl or substituted amido-alkyl linker and an alkyl-amido-alkyl or substituted alkyl-amido-alkyl linker. In certain instances, L¹ and L² are each independently selected from a C₁-C₁₂ alkyl or substituted alkyl linker, a C₁-C₁₂ alkenyl or substituted alkenyl linker, a C₁-C₁₂ alkynyl or substituted alkynyl linker, a C₁-C₁₂ acyl or substituted acyl linker, a C₁-C₁₂ alkoxy or substituted alkoxy linker, a C₁-C₁₂ amido-alkyl or substituted amido-alkyl linker and a C₁-C₁₂ alkyl-amido-alkyl or substituted alkyl-amido-alkyl linker. In certain cases, the linker comprises a carbonyl group. In certain cases, L¹ comprises a carbonyl group, and L² is a C₁-C₁₂ alkyl or substituted alkyl. In certain instances, the branching point X¹ is selected from N, CR', C(=O)N, SO₂N, a tri-substituted aryl group (e.g., a 1,3,5-phenyl), a tetra-substituted aryl group, and a tri-substituted heteroaryl group. In certain instances, the branching point X¹ is a nitrogen atom. In other instances, the branching point X¹ is CR', where R' is selected from hydrogen, alkyl, substituted alkyl, or -L²-Z² (e.g., as described herein). Z² is a water soluble polymer (e.g., as described herein). In some instances, the water soluble polymer is a PEG group. In certain instances, Z² is a PEG or modified PEG polymer of 1-50 monomeric units, such as 1-40, 1-30, 1-20, 2-24, 2-20, 2-10 or 2-6 monomeric units. In certain cases, at least one of R⁵ and R⁴ comprises a WSG of formula (Ig) or (Ih).

In some other embodiments, the WSG is a branched group of the formula (Ii): 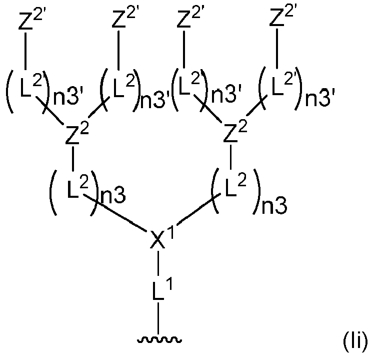 wherein each of L¹, X¹, L², n3 and Z² is as defined above; L^{2'}, n3' and Z^{2'} is as defined herein for L², n3 and Z² respectively.

In some other embodiments, the WSG is a branched group of formula (1j): 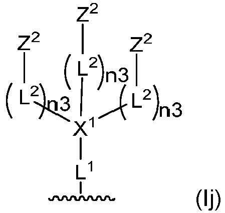 wherein each of L¹, X¹, L², n3 and Z² is as defined above; and X¹ is a branching group selected from a carbon atom and a tetra-substituted aryl group (e.g., a 1, 3, 4, 5-phenyl).

In certain embodiments of the water-soluble dipyrromethene-based dye of formula (I), the water solubilizing group (WSG) comprises a PEG moiety (e.g., as described herein). In some cases, the subject water-soluble dipyrromethene-based dye is substituted with one or more water solubilizing groups (WSGs) independently selected from the following formulae: 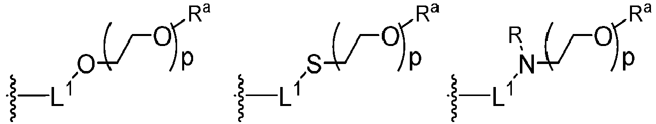 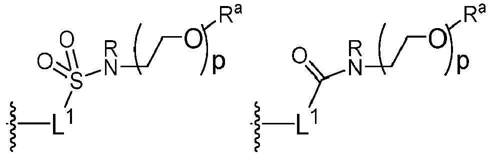 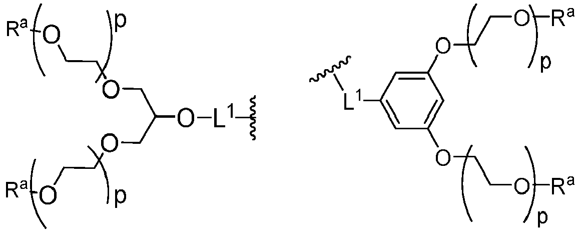 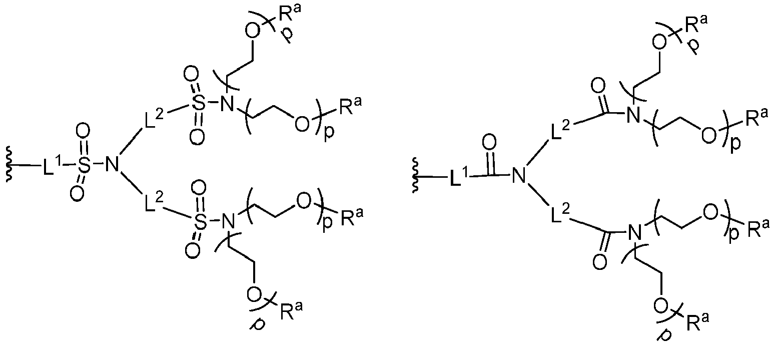 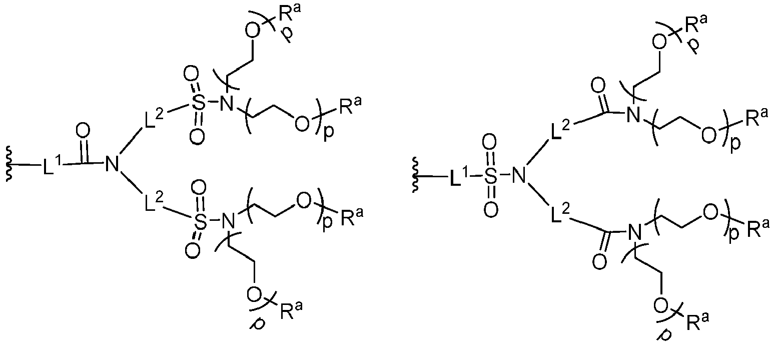 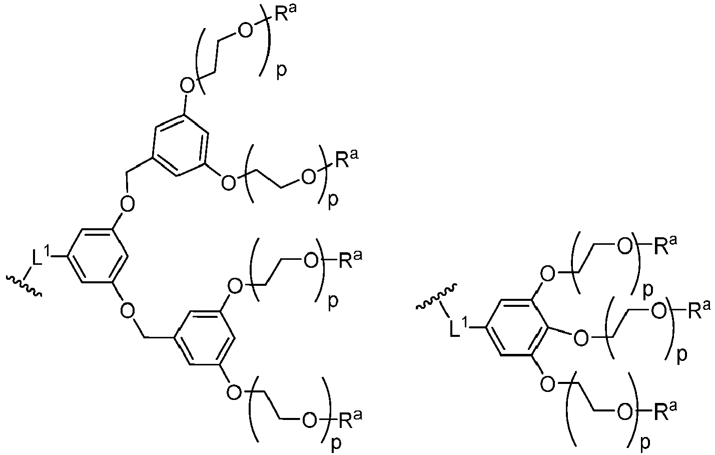 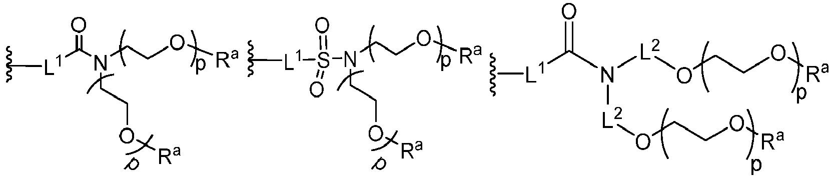 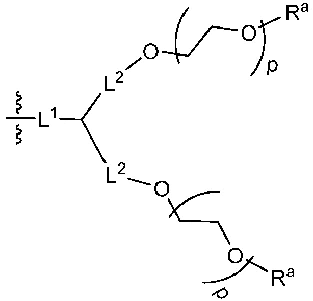 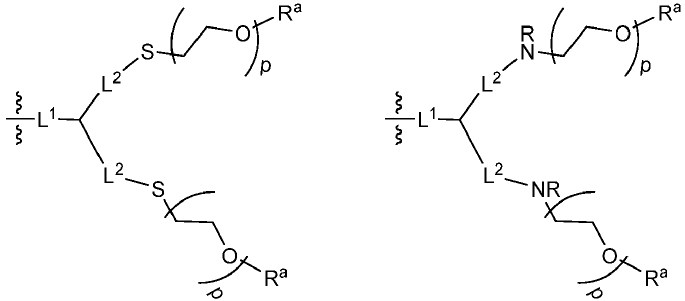 wherein:
L¹ and L² are each independently an optional linker;
R^{a} and R are each independently H, alkyl or substituted alkyl; and each p is an integer from 6 to 24.

In certain instances, each p is independently 6 to 24, such as 6 to 20, 11 to 20, 12 to 20, 12 to 18 or 12 to 16. In certain instances, each p is independently 6 to 24, such as 8 to 24, 10 to 24, 12 to 24, 13 to 24, 14 to 24, 15 to 22 or 16 to 20. In some cases, each p is independently 11, 12, 13, 14, 15, 16, 17, 18, 19, 20, 21, 22, 23 or 24. In some embodiments, each p is independently 7 or more, such as 8, 9 or more, 10 or more, 11 or more, 12 or more, 13 or more, 14 or more, 15 or more, or even more, and in some cases, have up to 24 monomeric units, such as up to 20, up to 15 or up to 12 monomeric units. In some cases, each p is the same. In some embodiments of the WSG, L¹ and/or L² is a C1-C12-alkyl linker, e.g., a C1-C6-alkyl linker, wherein one or more backbone atoms are optionally substituted with a heteroatom (e.g., an -O-). In some embodiments of the WSG, the L¹ and/or L² linker includes a carbonyl group. In some embodiments of the WSG, each R^{a} is H. In some embodiments of the WSG, each R^{a} is methyl.

It is understood that hydroxy-terminated PEG chains instead of methoxy-terminated PEG chains may be utilized in any of the WSG groups described above. In certain instances, any one of the formulae described herein may be substituted with a WSG that is a dendron selected from one of the following structures:

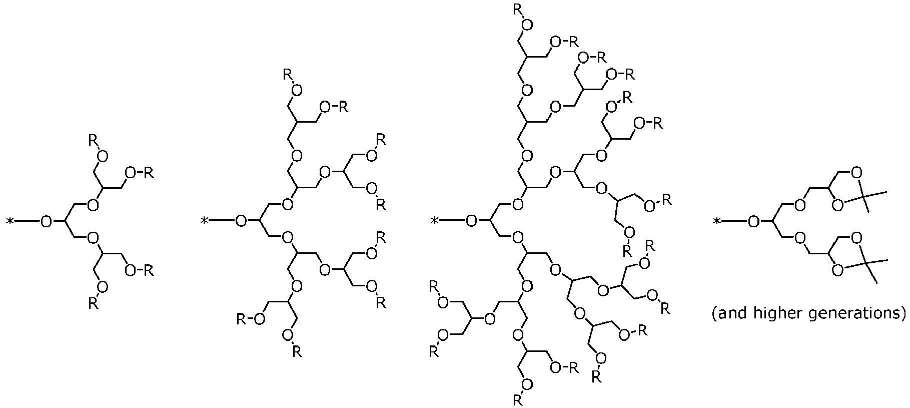

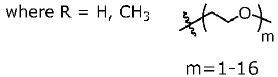

In certain instances, any one of the formulae described herein may be substituted with a WSG that is a polyol selected from one of the following structures:

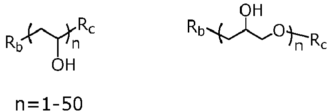

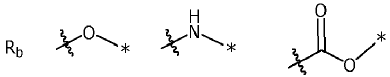

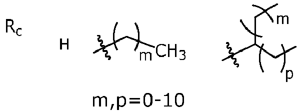

In certain instances, any one of the formulae described herein may be substituted with WSG that is an oxazoline of the following structure:

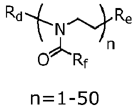

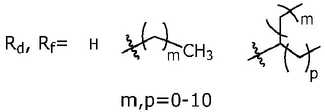

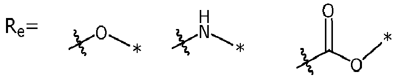

In certain instances, any one of the formulae described herein may be substituted with a WSG that is a peptoid selected from one of the following structures:

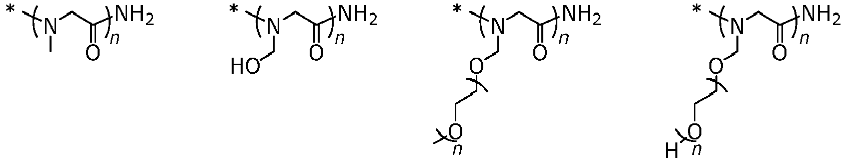

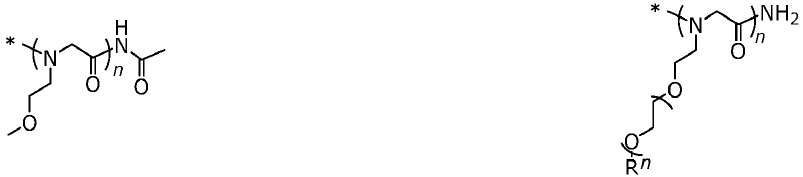

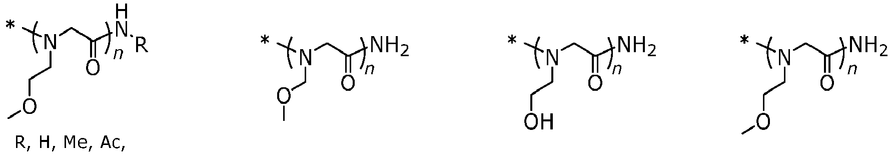

In certain embodiments of the subject water-soluble dipyrromethene-based dyes Y¹ and Y² each comprise a water solubilizing group (WSG). In certain other cases, R¹ comprises a water solubilizing group (WSG). In certain cases, all of Y¹, Y² and R¹ include WSGs. In certain cases, at least one of R⁴ or R⁵ includes a WSG. In certain cases, any one or more pairs of substituents selected from R⁶ and R⁷, R² and R³, R⁵ and R⁶, R³ and R⁴, R² and R¹ and R⁷ and R¹ together with the atoms to which they are attached form a cyclic group (e.g., as described herein), and the cyclic group is substituted with a WSG (e.g., as described herein).

In certain cases, of the subject water-soluble dipyrromethene-based dyes Y¹ is alkynyl substituted with WSG. Y² can be the same as Y¹ or different. In some cases of the subject water-soluble dipyrromethene-based dyes, Y² is selected from F, OH, H, cyano, alkyl, substituted alkyl, aryl, substituted aryl, heteroaryl, substituted heteroaryl, alkenyl, substituted alkenyl, alkynyl and substituted alkynyl. In some instances, Y² is selected from F, CN, phenyl and substituted phenyl.

In some cases of the subject water-soluble dipyrromethene-based dyes, Y¹ and Y² each comprises a water solubilizing group (WSG). In certain instances, Y¹ and/or Y² is alkynyl substituted with a WSG (e.g., as described herein). In certain instances, Y¹ and/or Y² is alkynyl substituted with polyethylene glycol (PEG) or modified (PEG). In certain cases of any of the subject water-soluble dipyrromethene-based dyes, Y¹ and/or Y² is of the formula: 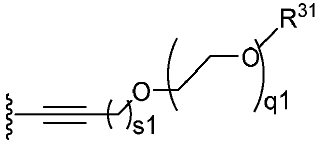 wherein:
s1 is 1 to 12;
q1 is 0 to 50; and
R³¹ is H, alkyl or substituted alkyl. In certain cases, s1 is 1 to 6, such as 1, 2 or 3. In some instances, q1 is 1 to 50, 1 to 30, 2 to 30, 4 to 30, 6 to 30, 8 to 30, 10 to 30, 10 to 20 or 11 to 16. In certain cases, q1 is 10 to 50, such as 10 to 30, 10 to 20 or 11 to 16.

In certain embodiments, the water-soluble dipyrromethene-based dye is of the formula (II): 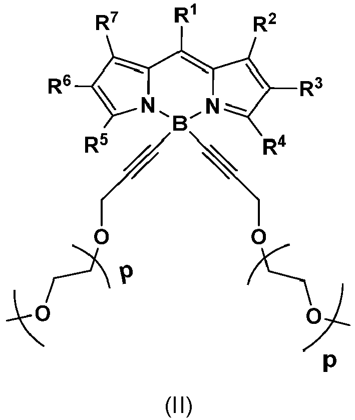 wherein R¹-R⁷ are as defined herein, and p is an integer from 6-24.

In certain instances, each p is independently 6 to 20, such as 11 to 20, 12 to 20, 12 to 18 or 12 to 16. In certain instances, each p is independently 8 to 24, such as 10 to 24, 12 to 24, 13 to 24, 14 to 24, 15 to 22 or 16 to 20. In some cases, each p is independently 11, 12, 13, 14, 15, 16, 17, 18, 19, 20, 21, 22, 23 or 24. In some embodiments, each p is independently 7 or more, such as 8, 9 or more, 10 or more, 11 or more, 12 or more, 13 or more, 14 or more, 15 or more, or even more, and in some cases, have up to 24 monomeric units, such as up to 20, up to 15 or up to 12 monomeric units. In some cases, each p is the same. In certain cases, each p is 11. In certain cases, each p is 12. In certain cases, each p is 13. In certain cases, each p is 14. In certain cases, each p is 15. In certain cases, each p is 16. In certain cases, each p is 17. In certain cases, each p is 18. In certain cases, each p is 19. In certain cases, each p is 20.

In certain embodiments, the water-soluble dipyrromethene-based dye of the formula (I) or (II), R⁵ is selected from halogen, substituted alkyl, substituted alkenyl, alkenyl aryl, aryl, substituted aryl, heterocycle, substituted heterocycle, spiro heterocycle, substituted spiro heterocycle, heteroaryl, substituted heteroaryl, substituted amino, substituted amido, and substituted thiol. In certain cases, R⁵ is halogen. In certain cases, R⁵ is chloride. In certain cases, R⁵ is substituted alkyl or substituted alkenyl. In certain cases, R⁵ is alkenyl aryl, such as alkenyl-phenyl (e.g., a styrene derivative). In certain cases, R⁵ is aryl or substituted aryl. In certain cases, R⁵ is substituted aryl, wherein the aryl is substituted with one or more groups selected from C₁₋₆ alkyl, halogen, hydroxyl, cyano, amino, carboxamide, and a PEG group (e.g., as described herein), In certain cases, R⁵ is heterocycle or substituted heterocycle. In certain cases, R⁵ is spiro heterocycle or substituted spiro heterocycle. In certain cases, the spiro heterocycle is based on a spiro[3,3]heptane core. In certain cases, R⁵ is heteroaryl or substituted heteroaryl. In certain cases R⁵ is substituted amino. In certain cases, R⁵ is substituted amido. In certain cases, R⁵ is substituted thiol. In certain other cases, R⁵ together with R⁶ and the atoms to which they are attached form a cyclic group, e.g., a 5 or 6 membered carbocyclic or heterocyclic group.

In certain embodiments, the water-soluble dipyrromethene-based dye of the formula (I) or (II), R⁴ is selected from halogen, substituted alkyl, substituted alkenyl, alkenyl aryl, aryl, substituted aryl, heterocycle, substituted heterocycle, spiro heterocycle, substituted spiro heterocycle, heteroaryl, substituted heteroaryl, substituted amino, substituted amido, and substituted thiol. In certain cases, R⁴ is halogen. In certain cases, R⁴ is chloride. In certain cases, R⁴ is substituted alkyl or substituted alkenyl. In certain cases, R⁴ is alkenyl aryl, such as alkenyl-phenyl (e.g., a styrene derivative). In certain cases, R⁴ is aryl or substituted aryl. In certain cases, R⁴ is substituted aryl, wherein the aryl is substituted with one or more groups selected from C₁₋₆ alkyl, halogen, hydroxyl, cyano, amino, carboxamide, and a PEG group (e.g., as described herein), In certain cases, R⁴ is heterocycle or substituted heterocycle. In certain cases, R⁴ is spiro heterocycle or substituted spiro heterocycle. In certain cases, the spiro heterocycle is based on a spiro[3,3]heptane core. In certain cases, R⁴ is heteroaryl or substituted heteroaryl. In certain cases R⁴ is substituted amino. In certain cases, R⁴ is substituted amido. In certain cases, R⁴ is substituted thiol. In certain other cases, R⁴ together with R³ and the atoms to which they are attached form a cyclic group, e.g., a 5 or 6 membered carbocyclic or heterocyclic group.

In certain embodiments of the subject water-soluble dipyrromethene-based dyes, R⁵ and R⁴ are the same.

In certain embodiments, the water-soluble dipyrromethene-based dye of the formula (I) or (II), R⁵ is alkenyl aryl, such as alkenyl-phenyl (e.g., a styrene derivative). In certain cases, the water-soluble dipyrromethene-based dye is of the formula (IIa): 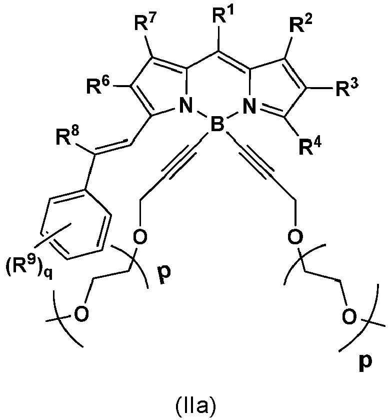 wherein:
R¹-R⁴, R⁶-R⁷ and p are as previously defined herein;
R⁸ is selected from H, halogen, cyano, alkyl, substituted alkyl, aryl, substituted aryl, heteroaryl, substituted heteroaryl, alkoxy, substituted alkoxy, alkenyl, substituted alkenyl, alkynyl, substituted alkynyl, water solubilizing group (WSG), or
R⁸ and R⁶ together with the atoms to which they are bound form a 5- or 6-membered heterocycle, carbocycle, aryl or heteroaryl ring (e.g., a 5- or 6-membered ring comprising carbon atoms and 0-3 heteroatoms selected from O, S and N), which ring may be unsubstituted or further substituted with a substituent independently selected from alkyl, substituted alkyl, alkoxy, substituted alkoxy, aryl, substituted aryl, heteroaryl, substituted heteroaryl, alkenyl, substituted alkenyl, alkynyl, substituted alkynyl, and water solubilizing group (WSG);
q is an integer from 0 to 5; and
each R⁹, if present, is independently selected from alkyl, substituted alkyl, hydroxy, alkoxy, substituted alkoxy, amino, substituted amino, thiol, substituted thiol, cyano, aryl, substituted aryl, heteroaryl, substituted heteroaryl, alkenyl, substituted alkenyl, alkynyl, substituted alkynyl, and water solubilizing group (WSG), or
optionally two adjacent R⁹ groups, together form a divalent radical and are cyclically linked and together with the carbon atoms to which they are bound provide a 5- or 6-membered fused heterocycle, carbocycle, aryl or heteroaryl ring (e.g., a 5- or 6-membered ring comprising carbon atoms and 0-3 heteroatoms selected from O, S and N), which ring may be unsubstituted or further substituted with a substituent independently selected from alkyl, substituted alkyl, alkoxy, substituted alkoxy, aryl, substituted aryl, heteroaryl, substituted heteroaryl, alkenyl, substituted alkenyl, alkynyl, substituted alkynyl, and water solubilizing group (WSG).

In certain embodiments of the water-soluble dipyrromethene-based dye of formula (IIa), R⁸ is H. In certain other cases, R⁸ and R⁶ together with the atoms to which they are bound form a 5- membered carbocycle, which carbocycle is optionally further substituted with a substituent independently selected from alkyl, substituted alkyl, alkoxy, substituted alkoxy, aryl, substituted aryl, heteroaryl, substituted heteroaryl, alkenyl, substituted alkenyl, alkynyl, substituted alkynyl, and water solubilizing group (WSG). In certain cases, the 5-membered carbocycle is substituted with one or more alkyl substituents. In certain cases, the alkyl substituent is C₁₋₆ alkyl. In certain cases, the alkyl substituent is methyl. In certain cases, the alkyl substituents are present bonded to the same carbon atom of the carbocycle.

In certain embodiments of the water-soluble dipyrromethene-based dye of formula (IIa), q is 0, such that there are no R⁹ substituents present. In certain cases, q is 1, 2, or 3, and each R⁹ is independently selected from alkyl, substituted alkyl, hydroxy, alkoxy, amino, thiol, cyano, aryl, heteroaryl, alkenyl, alkynyl, and water solubilizing group (WSG). In certain cases, q is 1, 2 or 3 and each R⁹ is independently selected from alkyl, substituted alkyl, and WSG. In certain cases, each WSG is independently a PEG group (e.g., as described herein). In certain cases, each alkyl is independently C₁₋₆ alkyl. In certain cases each alkyl group is independently selected from, methyl, ethyl, propyl, iso-propyl, butyl, and tert-butyl. In certain cases, q is 1 and R⁹ is selected from C₁₋₆ alkyl, C₁₋₆ substituted alkyl and PEG. In some cases, the C₁₋₆ substituted alkyl is substituted with a PEG group.

In certain embodiments of formula (IIa), R², R³, R⁴, R⁶ and R⁷ are independently selected from H and C₁₋₆ alkyl. In certain cases, R² is C₁₋₆ alkyl. In certain cases, R³ is H. In certain cases, R⁴ is C₁₋₆ alkyl. In certain cases R⁶ is H. In certain cases, R⁷ is C₁₋₆ alkyl. In certain cases, the C₁₋₆ alkyl is selected from methyl, ethyl, propyl, iso-propyl, butyl, and t-butyl. In certain cases, the C₁₋₆ alkyl is methyl.

In certain embodiments of formula (IIa), the compound is of any one of the following structures:

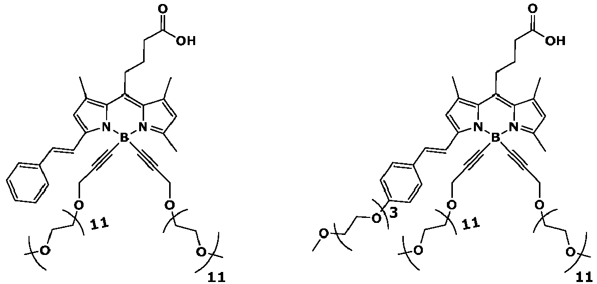

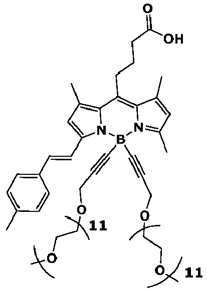

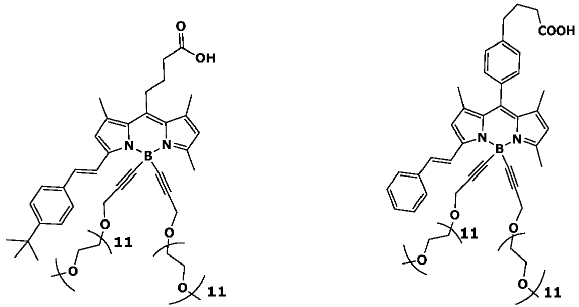

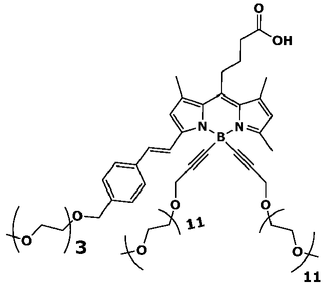

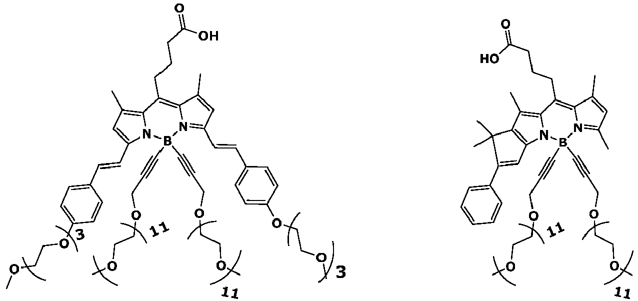

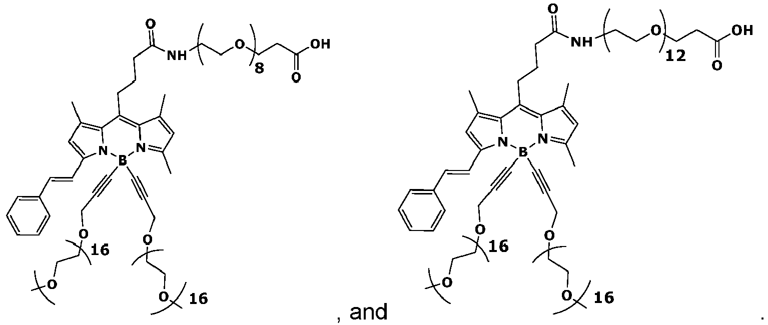

In certain other embodiments of the compound of formula (IIa), R⁴ is alkenyl aryl, such as alkenyl-phenyl. In some cases, R⁴ is a substituted styrene derivative. In certain cases, the compound of formula (IIa) is of the following structure:

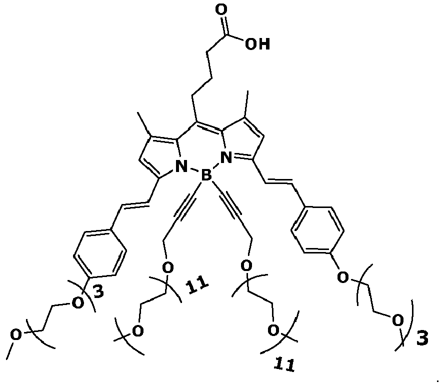

In certain embodiments of the subject water-soluble dipyrromethene-based dyes, R⁵ and R⁴ are the same. In certain cases, both R⁵ and R⁴ are aryl or substituted aryl. In certain cases, the water-soluble dipyrromethene-based dye is of the formula (IIb): 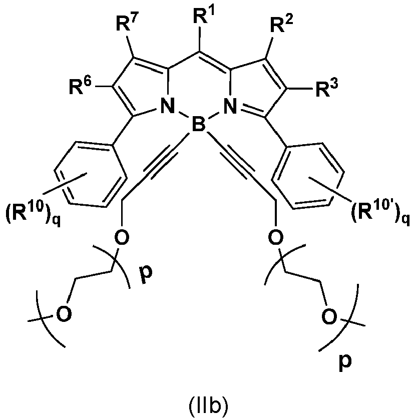 wherein:
R¹-R³, R⁶-R⁷ and p are as previously defined herein;
each q is independently an integer from 0 to 5; and
R¹⁰ and R^{10'}, if present, are each independently selected from halogen, hydroxyl, cyano, carboxamide, substituted carboxamide, amino, substituted amino, thiol, substituted thiol, alkyl, substituted alkyl, aryl, substituted aryl, heteroaryl, substituted heteroaryl, alkoxy, substituted alkoxy, alkenyl, substituted alkenyl, alkynyl, substituted alkynyl, and water soluble group (WSG), or
at least one R¹⁰ group is at an ortho position, and R¹⁰ and R⁶ together with the atoms to which they are bound form a 5- or 6-membered ring (e.g., a 5- or 6-membered ring comprising a boron atom, carbon atoms and 0-3 heteroatoms selected from O, S and N), which ring may be unsubstituted or further substituted with a substituent independently selected from alkyl, substituted alkyl, alkoxy, substituted alkoxy, aryl, substituted aryl, heteroaryl, substituted heteroaryl, alkenyl, substituted alkenyl, alkynyl, substituted alkynyl, and water solubilizing group (WSG); and
at least one R^{10'} is an ortho position, and R¹⁰ and R³ together with the atoms to which they are bound form a 5- or 6-membered ring (e.g., a 5- or 6-membered ring comprising a boron atom, carbon atoms and 0-3 heteroatoms selected from O, S and N), which ring may be unsubstituted or further substituted with a substituent independently selected from alkyl, substituted alkyl, alkoxy, substituted alkoxy, aryl, substituted aryl, heteroaryl, substituted heteroaryl, alkenyl, substituted alkenyl, alkynyl, substituted alkynyl, and water solubilizing group (WSG).

In certain embodiments of the water-soluble dipyrromethene-based dye of formula (IIb), each q is 0, such that there are no R¹⁰ or R^{10'} substituents present. In certain cases, each q is independently 1, 2, or 3, and R¹⁰ and R^{10'} are each independently selected from cyano, carboxamide, substituted carboxamide, alkyl, substituted alkyl, hydroxy, alkoxy, amino, thiol, aryl, heteroaryl, alkenyl, alkynyl, and water solubilizing group (WSG). In certain cases, each q is 1, 2 or 3 and R¹⁰ and R^{10'} are each independently selected from cyano, carboxamide, substituted carboxamide and WSG. In certain cases, each WSG is independently a PEG group (e.g., as described herein). In certain cases, each q is 1 and R¹⁰ and R^{10'} are each selected from cyano, carboxamide, substituted carboxamide and PEG. In some cases R¹⁰ and R^{10'} are the same. In some cases, the carboxamide is substituted with a PEG group.

In certain cases of the formula (IIb), each q is 1, and R¹⁰ and R^{10'} are both present in their respective para positions. In certain cases, R¹⁰ and R^{10'} are both present in their respective ortho positions. In certain cases, R¹⁰ and R^{10'} are both present in their respective meta positions.

In certain embodiments of formula (IIb), R¹⁰ and R^{10'} comprise a PEG moiety. In certain cases, the PEG moiety is selected from one of the following formulae: 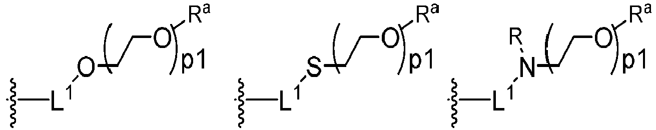 wherein:
L¹ is an optional linker (e.g., as described herein);
R^{a} and R is H, alkyl or substituted alkyl; and
each p1 is an integer from 1 to 50.

In certain instances, each p1 is independently 1 to 40, such as 1 to 30, 1 to 20, 1 to 10 or 1 to 6. In certain instances, each p1 is independently 1-10, such as 1-9, 1-8, 1-7, 1-6, or 1-5. In some cases, each p is independently 1, 2, 3, 4, 5, 6, 7, 8, 9, or 10. In certain cases, each p is 3. In certain cases, each p is 4. In certain cases, each p is 5. In certain cases, each p is 6. In certain cases, each p is 7. In certain cases, each p is 8. In certain cases, each p is 9. In certain cases, each p is 10. In certain cases, each p is 11. In certain cases, each p is 12.

In certain embodiments of formula (IIb), R², R³, R⁶ and R⁷ are independently selected from H and C₁₋₆ alkyl. In certain cases, R² is C₁₋₆ alkyl. In certain cases, R³ is H. In certain cases R⁶ is H. In certain cases, R⁷ is C₁₋₆ alkyl. In certain cases, the C₁₋₆ alkyl is selected from methyl, ethyl, propyl, iso-propyl, butyl, and t-butyl. In certain cases, the C₁₋₆ alkyl is methyl. In certain other cases, all of R², R³, R⁶ and R⁷ are H.

In certain embodiments of formula (IIb), the compound is of any one of the following structures:

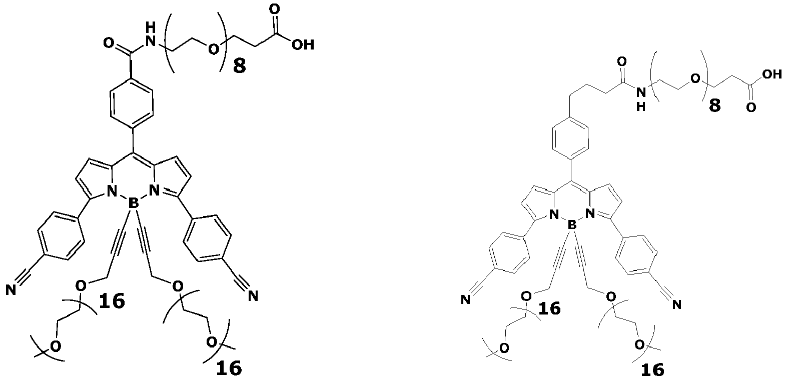

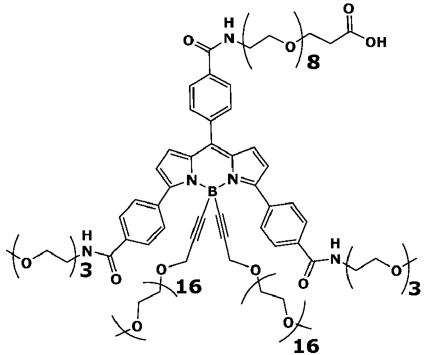

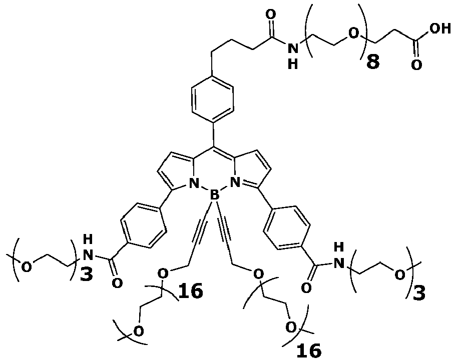

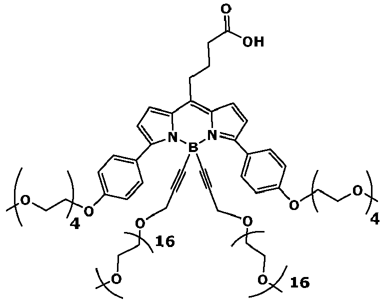

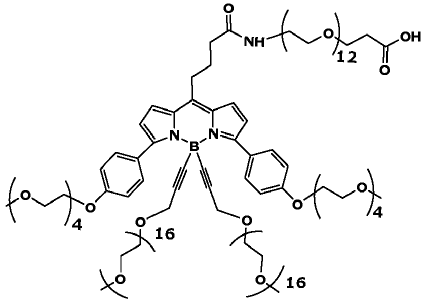

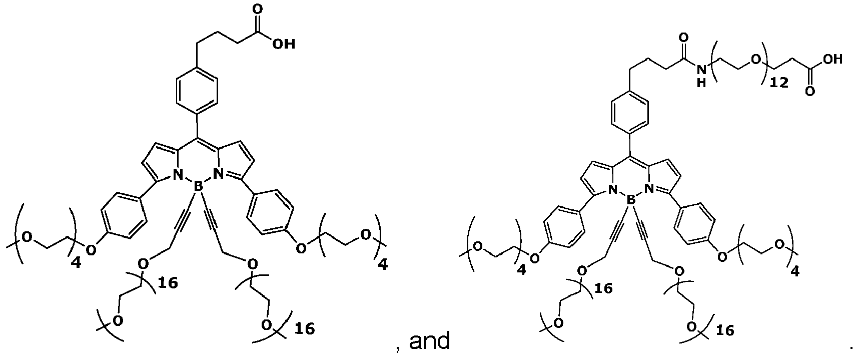

In certain embodiments of the subject water-soluble dipyrromethene-based dyes, R⁵ is halogen. In certain cases, R⁶ and R⁷ together with the atoms to which they are attached form a 5 or 6 membered cyclic group. In certain cases, the water-soluble dipyrromethene-based dye is of the formula (IIc): 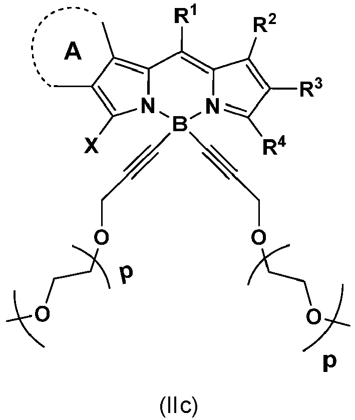 wherein:
R¹-R⁴ and p are as previously defined herein;
X is a halogen; and
ring A is a 5- or 6-membered fused heterocycle, carbocycle, aryl or heteroaryl ring (e.g., a 5- or 6-membered ring comprising carbon atoms and 0-3 heteroatoms selected from O, S and N), which ring may be unsubstituted or further substituted with a substituent independently selected from alkyl, substituted alkyl, alkoxy, substituted alkoxy, aryl, substituted aryl, heteroaryl, substituted heteroaryl, alkenyl, substituted alkenyl, alkynyl, substituted alkynyl, and water solubilizing group (WSG).

In certain embodiments of formula (IIc), ring A is a 6-membered heterocycle, carbocycle, aryl or heteroaryl ring. In certain cases, ring A is a heterocycle. In certain cases, ring A is a carbocycle. In certain cases, ring A is a heteroaryl. In certain cases, ring A is an aryl ring.

In certain embodiments of formula (IIc), X is CI, F, Br or I. In certain cases X is F. In certain cases, X is Br. In certain cases X is I. In certain cases, X is CI.

In certain embodiments of formula (IIc), R², R³, and R⁴ are independently selected from H and C₁₋₆ alkyl. In certain cases, R² is C₁₋₆ alkyl. In certain cases, R³ is H. In certain cases, R⁴ is C₁₋₆ alkyl. In certain cases, the C₁₋₆ alkyl is selected from methyl, ethyl, propyl, iso-propyl, butyl, and t-butyl. In certain cases, the C₁₋₆ alkyl is methyl.

In certain embodiments of formula (IIc), the compound is of any one of the following structures: 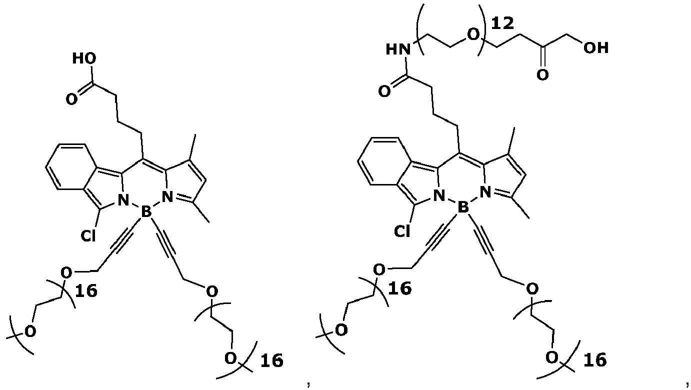 and

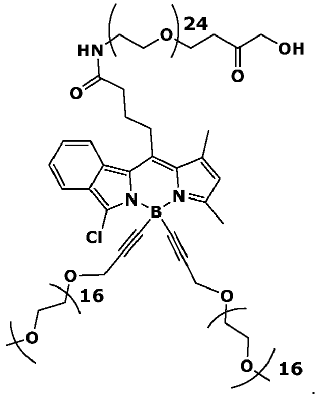

In certain cases, both R⁵ and R⁴ are selected from substituted amino, substituted amido, substituted thiol, and spiro heterocycle. In certain cases, the water-soluble dipyrromethene-based dye is of the formula (IId): 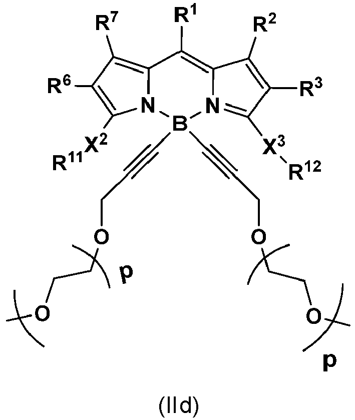 wherein:
R¹-R^{3'}, R⁶-R⁷, and p are as previously defined herein;
X² and X³ are each independently selected from NR¹³, S and O;
R¹³ is selected from H, alkyl, and substituted alkyl;
R¹¹ and R¹² are each independently selected from aryl, substituted aryl, heteroaryl, substituted heteroaryl, and a water soluble group (WSG), or
when X² is NR¹³, optionally R¹³ and R¹¹ together with the nitrogen atom to which they are attached, form a heterocyclic or spiro heterocyclic ring, which ring may be unsubstituted or further substituted with a substituent independently selected from alkyl, substituted alkyl, alkoxy, substituted alkoxy, aryl, substituted aryl, heteroaryl, substituted heteroaryl, alkenyl, substituted alkenyl, alkynyl, substituted alkynyl, and a water solubilizing group (WSG); and
when X³ is NR¹³, optionally R¹³ and R¹² together with the nitrogen atom to which they are attached, form a heterocyclic or spiro heterocyclic ring, which ring may be unsubstituted or further substituted with a substituent independently selected from alkyl, substituted alkyl, alkoxy, substituted alkoxy, aryl, substituted aryl, heteroaryl, substituted heteroaryl, alkenyl, substituted alkenyl, alkynyl, substituted alkynyl, and a water solubilizing group (WSG).

In certain embodiments of the water-soluble dipyrromethene-based dye of formula (Id), X² and X³ are each NR¹³, wherein R¹³ is H. In certain other embodiments, X² and X³ are each O. In certain other embodiments, X² and X³ are each S.

In certain embodiments of the water soluble dipyrromethene-based dye of formula (Id), R¹¹ is selected from aryl, substituted aryl, and a water soluble group (WSG). In certain cases, R¹¹ is a phenyl group. In certain cases, R¹¹ is a PEG group (e.g., as described herein). In certain cases, the PEG group has 2 or more monomeric units, such as 3 or more, 4 or more, or 5 or more monomeric units. In certain cases, the PEG group has 1-5 monomeric units, such as 2, 3, or 4 monomeric units.

In certain embodiments of the water soluble dipyrromethene-based dye of formula (Id), R¹² is selected from aryl, substituted aryl, and a water soluble group (WSG). In certain cases, R¹² is a phenyl group. In certain cases, R¹² is a PEG group (e.g., as described herein). In certain cases, the PEG group has 2 or more monomeric units, such as 3 or more, 4 or more, or 5 or more monomeric units. In certain cases, the PEG group has 1-5 monomeric units, such as 2, 3, or 4 monomeric units.

In certain embodiments of the water soluble dipyrromethene-based dye of formula (Id), R¹¹ and R¹² are the same. In some cases, both R¹¹ and R¹² are phenyl. In some cases, both R¹¹ and R¹² are a PEG group (e.g., as described herein).

In certain embodiments of the water soluble dipyrromethene-based dye of formula (Id), X² is NR¹³, and R¹³ and R¹¹ together with the nitrogen atom to which they are attached form a heterocyclic or a spiro heterocyclic ring; and X³ is NR¹³, and R¹³ and R¹² together with the nitrogen atom to which they are attached form a heterocyclic or a spiro heterocyclic ring. In certain cases, the spiro heterocyclic ring has core structure selected from, spiro[3,3]heptane, spiro[3,4]octane, spiro[3,5]nonane, spiro[4,4]nonane; spiro[4,5]decane, and spiro[5,5]undecane. In certain cases, the spiro heterocyclic ring is based on a spiro[3,3]heptane core structure.

In certain embodiments of formula (IId), R², R³, R⁶ and R⁷ are independently selected from H and C₁₋₆ alkyl. In certain cases, R² is C₁₋₆ alkyl. In certain cases, R³ is H. In certain cases R⁶ is H. In certain cases, R⁷ is C₁₋₆ alkyl. In certain cases, the C₁₋₆ alkyl is selected from methyl, ethyl, propyl, iso-propyl, butyl, and t-butyl. In certain cases, the C₁₋₆ alkyl is methyl. In certain other cases, all of R², R³, R⁶ and R⁷ are H.

In certain embodiments of formula (IId), the compound is of any one of the following structures: 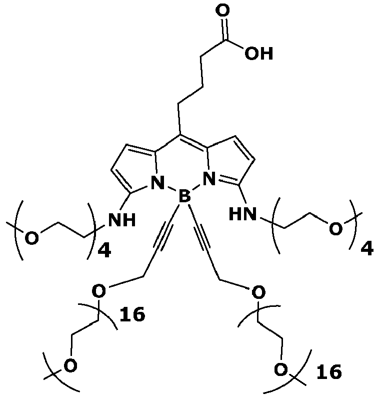 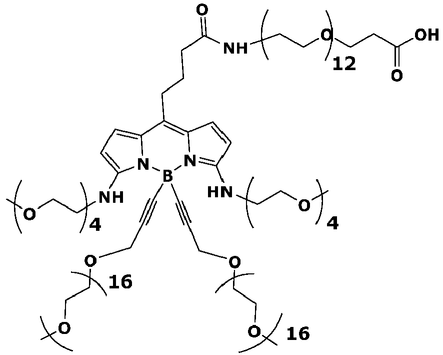 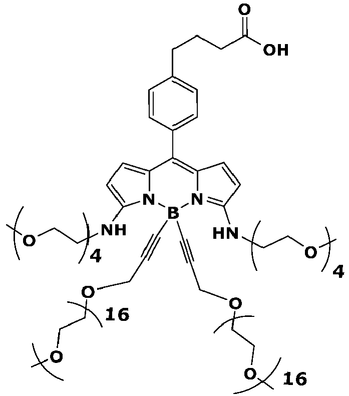 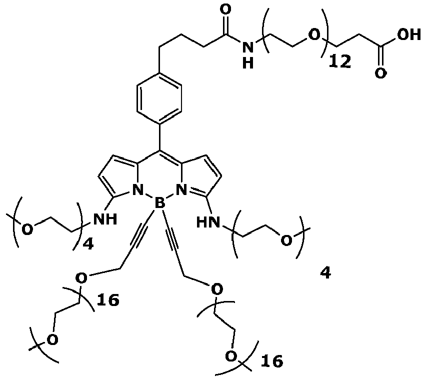 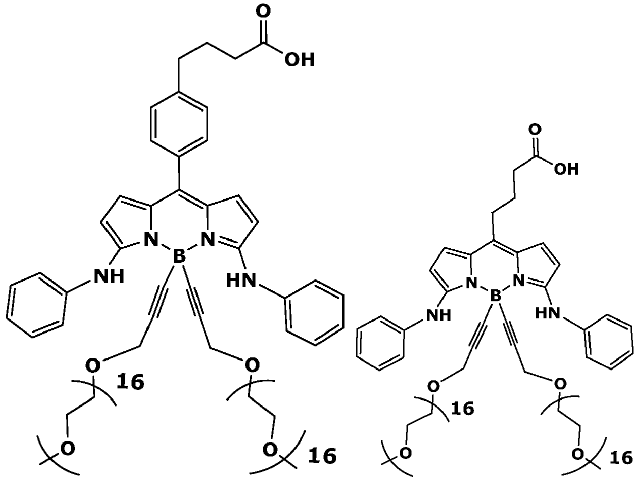 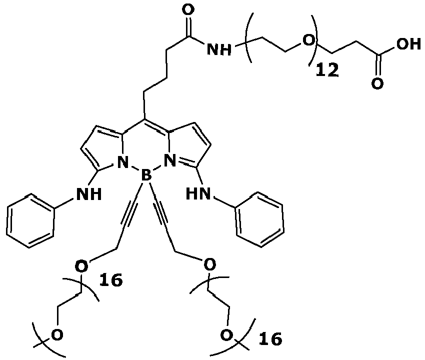 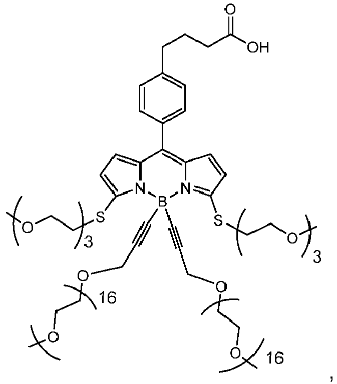 and

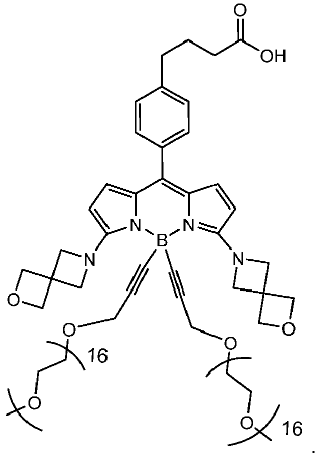

In some embodiments of the water-soluble dipyrromethene-based dye of formula (I), Y¹ and Y² together with the boron atom to which they are bound form a 5- or 6-membered ring (e.g., a 5- or 6-membered ring comprising a boron atom, carbon atoms and 0-3 heteroatoms selected from O, S and N), which ring may be unsubstituted or further substituted (e.g., as described herein). In certain cases, the water-soluble dipyrromethene-based dye is of the formula (III): 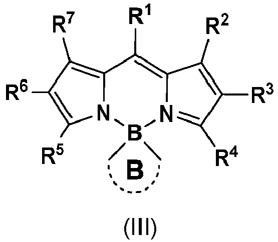 wherein:
R¹-R⁷ are as previously defined herein; and
ring B is a 5- or 6-membered cyclic group (e.g., a 5- or 6-membered ring comprising a boron atom, carbon atoms and 0-3 heteroatoms selected from O, S and N), which cyclic group may be unsubstituted or further substituted with one or more substituents independently selected from alkyl, substituted alkyl, alkoxy, substituted alkoxy, aryl, substituted aryl, heteroaryl, substituted heteroaryl, alkenyl, substituted alkenyl, alkynyl, substituted alkynyl, and water solubilizing group (WSG).

In certain cases, ring B is a 6-membered cyclic group. In certain cases the 6-membered cyclic group is further substituted with one or more substituents selected from alkyl, substituted alkyl, alkoxy, substituted alkoxy, aryl, substituted aryl, heteroaryl, substituted heteroaryl, alkenyl, substituted alkenyl, alkynyl, substituted alkynyl, and water solubilizing group (WSG). In certain cases, the 6-membered cyclic group is further substituted with one or more substituents selected from aryl and substituted aryl. In certain cases, the 6-membered cyclic group is substituted with two phenyl groups.

In certain embodiments of the water-soluble dipyrromethene-based dye of formula (III), the compound is of the formula (IIIa): 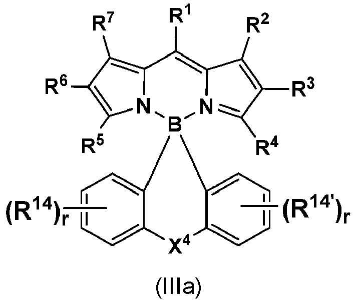 wherein:
X⁴ is selected from O, S, NR, and CR₂;
each R is independently selected from H, alkyl, and substituted alkyl;
each r is independently an integer from 0 to 4; and
each R¹⁴ and R^{14'}, if present, is independently selected from halogen, hydroxyl, cyano, amino, substituted amino, thiol, substituted thiol, amido, substituted amido, acyl, substituted acyl, alkyl, substituted alkyl, aryl, substituted aryl, heteroaryl, substituted heteroaryl, alkoxy, substituted alkoxy, alkenyl, substituted alkenyl, alkynyl, substituted alkynyl, and water solubilizing group (WSG).

In certain embodiments of the water-soluble dipyrromethene-based dye formula (IIIa), at least one of R⁵ and R⁶, and R⁴ and R³ together form a divalent radical and are cyclically linked and together with the carbon atoms to which they are bound provide a 5- or 6-membered fused heterocycle, carbocycle, aryl or heteroaryl ring (e.g., a 5- or 6-membered ring comprising carbon atoms and 0-3 heteroatoms selected from O, S and N), which ring may be unsubstituted or further substituted with a substituent independently selected from alkyl, substituted alkyl, alkoxy, substituted alkoxy, aryl, substituted aryl, heteroaryl, substituted heteroaryl, alkenyl, substituted alkenyl, alkynyl, substituted alkynyl, and water solubilizing group (WSG). In certain cases, only one of R⁵ and R⁶, or R⁴ and R³ together form a divalent radical and are cyclically linked and together with the carbon atoms to which they are bound provide a 5- or 6-membered ring. In certain other cases, both of R⁵ and R⁶ and R⁴ and R³ together form a divalent radical and are cyclically linked and together with the carbon atoms to which they are bound provide a 5- or 6-membered ring. In certain cases, R⁵ and R⁶, and R⁴ and R³ together with the carbon atoms to which they are bound form a 6-membered ring (e.g., as described herein). In certain cases, R⁵ and R⁶, and R⁴ and R³ together with the carbon atoms to which they are bound form a 5-membered ring ( e.g., as described herein). In certain embodiments, at least one of R⁵ and R⁶, or R⁴ and R³ together with the carbon atoms to which they are bound provide a 5-membered ring represented by one of the following structures, where with wavy line represents the point of attachment to the dipyrromethene core of R⁵ and R⁶, or R⁴ and R³: 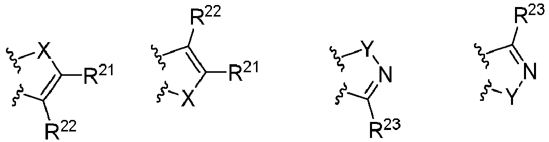 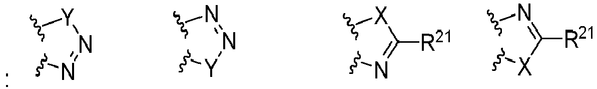 wherein:
X is O or S;
Y is O, S or NR, wherein R is H, alkyl, substituted alkyl or a substituent as defined for R¹- R⁷ in formula (I); and
R²¹-R²³ are independently selected from H and a substituent as defined for R¹- R⁷ in formula (I). In certain instances the 5-membered ring is independently selected from the following rings: 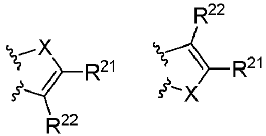 as defined above.

In certain cases of formulae (III)-(IIIa), R⁵ and R⁶, or R⁴ and R³ form the same rings. In certain instances, R⁵ and R⁶, or R⁴ and R³ form different rings.

In certain embodiments, the water-soluble dipyrromethene-based dye of claim is of the formula (IIIb) or (IIIc): 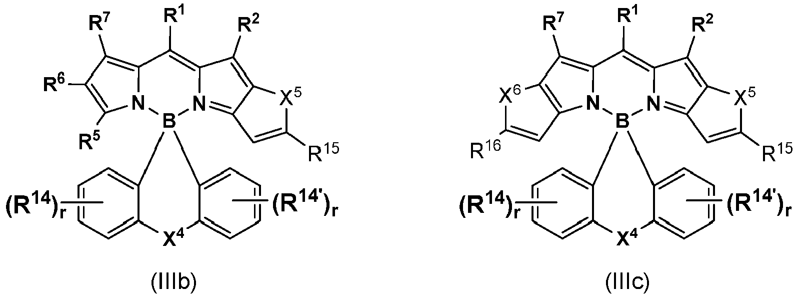 wherein:
X⁵ and X⁶ are each independently selected from O, S, NR, and CR₂;
each R is independently selected from H, alkyl, and substituted alkyl; and
R¹⁵ and R¹⁶, are each independently selected from hydrogen, halogen, hydroxyl, cyano, amino, substituted amino, thiol, substituted thiol, amido, substituted amido, acyl, substituted acyl, alkyl, substituted alkyl, aryl, substituted aryl, heteroaryl, substituted heteroaryl, alkoxy, substituted alkoxy, alkenyl, substituted alkenyl, alkynyl, substituted alkynyl, and water solubilizing group (WSG).

In certain cases of formula (IIIb), X⁵ is O. In certain cases, X⁵ is S. In certain cases, X⁵ is NR. In certain other cases, X⁵ is CR₂.

In certain cases of formula (IIIc), X⁵ and X⁶ are each O. In certain cases, X⁵ and X⁶ are each S. In certain cases, X⁵ and X⁶ are each NR. In certain other cases, X⁵ and X⁶ are each CR₂.

In certain cases of formula (IIIb) and (IIIc), R¹⁵ is selected from alkyl, substituted alkyl, aryl, and substituted aryl. In certain cases, the substituted alkyl or substituted aryl group is substituted with a water-solubilizing group (WSG) (e.g., as described herein). In certain cases, the WSG includes a PEG moiety. In certain cases, the WSG includes a carboxamide group. In certain cases, the substituted alkyl or substituted aryl group is substituted with an alkoxy group or a substituted alkoxy group. In certain cases, the substituted aryl is a di-substituted aryl. In certain cases, the di-substituted aryl is a 2,4-disubstituted aryl moiety.

In certain cases of formula (IIIb) and (IIIc), R¹⁶ is selected from alkyl, substituted alkyl, aryl, and substituted aryl. In certain cases R¹⁶ is C₁₋₆ alkyl, e.g., methyl, ethyl, propyl, iso-propyl, butyl, tert-butyl, pentyl, hexyl. In certain cases, R¹⁶ is methyl. In certain cases, the substituted alkyl or substituted aryl group is substituted with a water-solubilizing group (WSG) (e.g., as described herein). In certain cases, the WSG includes a PEG moiety. In certain cases, the WSG includes a carboxamide group. In certain cases, the substituted alkyl or substituted aryl group is substituted with an alkoxy group or a substituted alkoxy group. In certain cases, the substituted aryl is a di-substituted aryl. In certain cases, the di-substituted aryl is a 2,4-disubstituted aryl moiety.

In certain embodiments the water-soluble dipyrromethene-based dye of formula (III)-(IIIc) is one of the following structures: 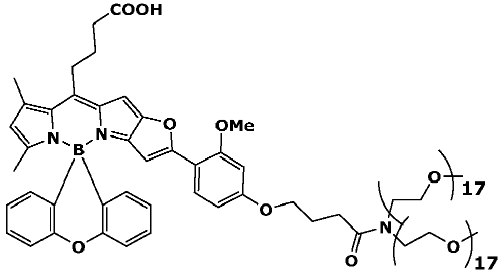 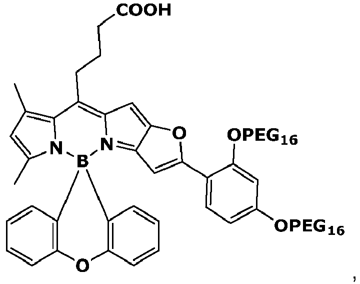 and

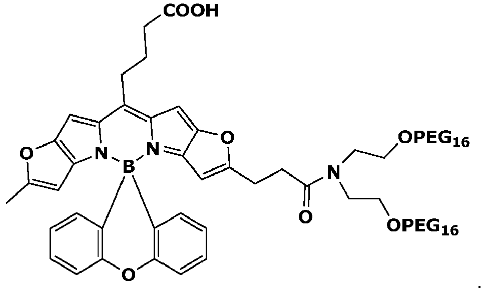

In some embodiments of the water-soluble dipyrromethene-based dye of formula (I), at least one of R¹-R⁷ is T¹-J¹, such that the compound of formula (I) comprises at least one repeat units of a compound of formula (I). In some cases, the compound of formula (I) is a dimer. In some cases R¹ is T¹-J¹. In some cases, R² is T¹-J¹. In some cases, R³ is T¹-J¹. In some cases, R⁴ is T¹-J¹. In some cases, R⁵ is T¹-J¹. In some cases, R⁶ is T¹-J¹. In some cases, R⁷ is T¹-J¹. In certain cases, T¹ is absent and at least one of R¹-R⁷ is J¹ (e.g., a repeat unit of a compound of formula (I)).

In some embodiments of the subject water-soluble dipyrromethene-based dye, R³ is T¹-J¹, and T¹ is absent, such that R³ is simply J¹. In some cases, the water-soluble dipyrromethene-based dye is of one of the following structures. 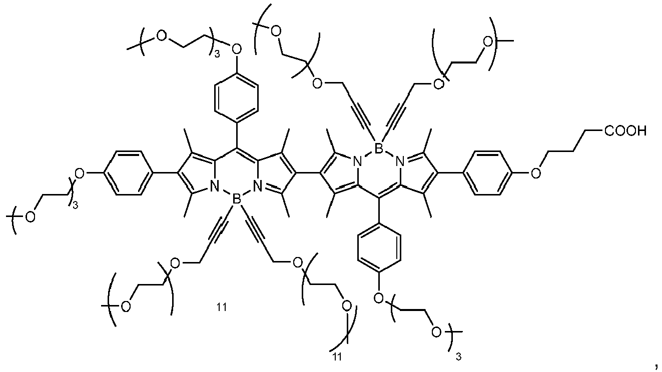 and

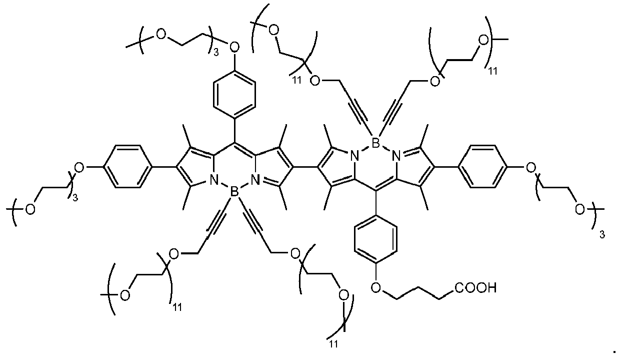

In certain cases, the dipyrromethene-based dye is of formula (IV): 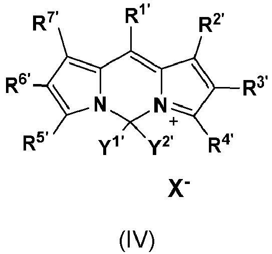 wherein:
X⁻ is a counterion;
R^{1'}-R^{7'} are each independently selected from H, alkyl, halogen, substituted alkyl, alkoxy, substituted alkoxy, amino, substituted amino, amido, substituted amido, thiol, substituted thiol, aryl, substituted aryl, heteroaryl, substituted heteroaryl, heterocycle, substituted heterocycle, alkenyl, substituted alkenyl, alkynyl, substituted alkynyl, water solubilizing group (WSG), and -T¹-Z¹, or
optionally any one or more pairs of substituents selected from R^{6'} and R^{7'}, R^{2'} and R^{3'}, R^{5'} and R^{6'}, R^{3'} and R^{4'}, R^{2'} and R^{1'} and R^{7'} and R^{1'}, together form a divalent radical and are cyclically linked and together with the carbon atoms to which they are bound provide a 5- or 6-membered fused heterocycle, carbocycle, aryl or heteroaryl ring (e.g., a 5- or 6-membered ring comprising carbon atoms and 0-3 heteroatoms selected from O, S and N), which ring may be unsubstituted or further substituted with a substituent independently selected from alkyl, substituted alkyl, alkoxy, substituted alkoxy, aryl, substituted aryl, heteroaryl, substituted heteroaryl, alkenyl, substituted alkenyl, alkynyl, substituted alkynyl, water solubilizing group (WSG), and -T¹-Z¹;
T¹ is an optional linker;
Z¹ is a repeat unit of a compound of formula (I); and
Y^{1'} and Y^{2'} are independently selected from F, OH, H, cyano, alkyl, substituted alkyl, aryl, substituted aryl, heteroaryl, substituted heteroaryl, alkoxy, substituted alkoxy, alkenyl, substituted alkenyl, alkynyl, substituted alkynyl, and WSG, or
Y^{1'} and Y^{2'} together with the boron atom to which they are bound form a 5- or 6-membered ring (e.g., a 5- or 6-membered ring comprising a boron atom, carbon atoms and 0-3 heteroatoms selected from O, S and N), which ring may be unsubstituted or further substituted with a substituent independently selected from alkyl, substituted alkyl, alkoxy, substituted alkoxy, aryl, substituted aryl, heteroaryl, substituted heteroaryl, alkenyl, substituted alkenyl, alkynyl, substituted alkynyl, water solubilizing group (WSG), and -T¹-Z¹; and
wherein at least one moiety selected from Y^{1'}, Y^{2'} and R^{1'}-R^{7'} is a WSG, or at least one moiety selected from Y^{1'}, Y^{2'} and R^{1'}-R^{7'} is substituted with a WSG, where the WSG is of the formula (Ia):

   -(L¹)ₙ₁ - (X¹)ₙ₂ - ((L²)ₙ₃-Z¹)ₘ (Ia)

   wherein:
   n1, n2 and n3 are each independently 0 or 1;
   L¹, if present, is a linker;
   L² if present, is a linker;
   X¹, if present, is a branching point;
   Z¹ is a water soluble polymer comprising 6-24 monomeric units; and m is an integer from 1 to 3.

In some embodiments of the water-soluble dipyrromethene-based dye of formula (IV), the dye further includes a WSG of formula (Ib):

-(L¹)ₙ₁ - (X¹)ₙ₂ - ((L²)ₙ₃-Z²)ₘ (Ib)

wherein:
n1, n2 and n3 are each independently 0 or 1;
L¹, if present, is a linker;
L² if present, is a linker;
X¹, if present, is a branching point;
Z² is a water soluble polymer comprising 1-50 monomeric units; and
m is an integer from 1 to 3.

In certain embodiments of the water-soluble dipyrromethene-based dye of formula (IV), the WSG comprises a PEG moiety (e.g., as described herein). In certain cases, one or more water solubilizing groups (WSGs) is independently selected from any one of the WSG formulae disclosed herein.

In certain embodiments of the water-soluble dipyrromethene-based dye of formula (IV), R^{1'}-R^{7'}, are as defined for R¹-R⁷ (e.g., as described here). In certain cases, Y^{1'} and Y^{2'} are as defined for Y¹ and Y² (e.g., as described herein). In certain cases, Y^{1'} and Y^{2'} are both hydrogen.

In certain embodiments of the water-soluble dipyrromethene-based dye of formula (IV), R^{5'} and R^{4'} both comprise a water solubilizing group (WSG). In certain cases, R^{5'} and R^{4'} are the same. In certain cases, both R^{5'} and R^{4'} are substituted aryl, and the aryl is substituted with at least one WSG (e.g., as described herein).

In certain cases, the water-soluble dipyrromethene-based dye of formula (IV) is of one of the following structures:

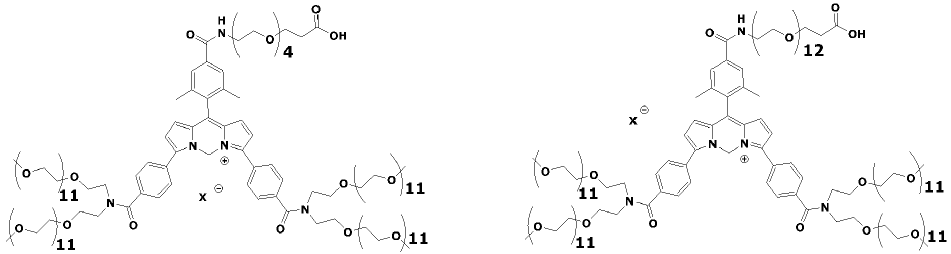

The counter ion (X⁻) in the water-soluble dipyrromethene-based dye of formula (IV), may be any convenient counterion. In certain cases, the counterion (X⁻) is selected from bromide, chloride, formate, tartrate, besylate, mesylate, acetate (e.g., trifluoroacetate), maleate, and oxalate.

In certain embodiments of the water-soluble dipyrromethene-based dye of any one of formulae (I)-(IV), R¹ or R^{1'} is selected from substituted alkyl, and substituted aryl, wherein the substituent comprises a chemoselective tag. In any of the embodiments of formulae (I)-(IV) described herein, R¹ or R^{1'} may include a particular -L^{a}-Z^{a} group as described in one of the following embodiments.

L^{a} can be a linker (e.g., as described herein) having a backbone of 20 atoms or less in length. In some instances, L^{a} is selected from an alkyl or substituted alkyl linker, an alkoxy or substituted alkoxy linker, an aryl or substituted aryl linker, a PEG linker, a sulfonamido-alkyl or substituted sulfonamido-alkyl linker, an amido-alkyl or substituted amido-alkyl linker and an alkyl-amido-alkyl or substituted alkyl-amido-alkyl linker. The linker may be substituted with a WSG, such as a PEG group. In certain instances, L^{a} is selected from a C₁-C₁₂ alkyl or substituted alkyl linker, a C₁-C₁₂ alkoxy or substituted alkoxy linker, a C₁-C₁₂ amido-alkyl or substituted amido-alkyl linker and a C₁-C₁₂ alkyl-amido-alkyl or substituted alkyl-amido-alkyl linker. The linker L^{a} can include several linked components, such as one or linking groups independently selected from lower alkylene, substituted lower alkylene, alkenylene (-CH=CH-), substituted alkenylene, alkynylene (-CC-), substituted or unsubstituted amido (e.g., -NRCO- or -CONR-, where R Is H, alkyl or substituted alkyl), substituted or unsubstituted sulfonamido (e.g., -NRSO₂- or -SO₂NR-, where R Is H, alkyl or substituted alkyl), oxo (-O-), thio (-S-), ethylene glycol (-OCH₂CH₂O-), polyethylene glycol (e.g., -(CH₂CH₂O)ₙ- where n is 2-20, such as 2-10 or 2-6, or 2, 3, 4, 5 or 6), arylene, substituted arylene, heteroarylene, substituted heteroarylene, alpha-amino acid residue, beta-amino acid residue, and the like.

Z^{a} can be a chemoselective functional group suitable for conjugation to a molecule of interest having a compatible functional group. Chemoselective functional groups of interest which find use in the subject dyes include, but are not limited to, amine groups (e.g., -NH₂), carboxylic acid (-CO₂H), active ester (e.g., NHS or sulfo-NHS ester), thiol, maleimide, iodoacetamide, hydroxyl, hydrazido, hydrazino, aldehyde, ketone, azido, alkyne, tetrazine, alkene, phosphine and epoxide. It is understood that in some cases, the chemoselective functional group of a subject dye is a synthetic precursor or protected version of the functional group of interest, which may be converted to a reactive functional group capable of conjugation to a molecule of interest. For example, a carboxylic acid is a chemoselective functional group which can be coupled with an amine group on a molecule of interest. The carboxylic acid may be converted to an active ester that couples with the amine group, either in situ or prior to coupling. In certain instances, Z^{a} is a protected carboxylic acid, such as an ester protected carboxylic acid. In some cases, Z^{a} is a synthetic precursor of a chemoselective functional group, such as an azido group which can be reduced to an amine, or a cyano group which can be hydrolyzed to carboxylic acid, using any convenient methods.

In certain embodiments of any water-soluble dipyrromethene-based dye of formulae (I)-(IV), R¹ or R^{1'} is -L^{a}-Z^{a}, where L^{a} is alkylene or substituted alkylene and Z^{a} is carboxylic acid or active ester of carboxylic acid. In some cases, -L^{a}-Z^{a} is -(C₁-C₁₂)alkylene-CO₂H, such as -(C₁-C₆)alkylene-CO₂H. In certain embodiments, L^{a} is arylene or substituted arylene and Z^{a} is carboxylic acid or active ester of carboxylic acid. In certain embodiments, L^{a} is arylene or substituted arylene and Z^{a} is carboxylic acid or active ester of carboxylic acid. In some cases, -L^{a}-Z^{a} is selected from - phenyl-(C₁-C₁₂)alkylene-CO₂H and -substituted phenyl-(C₁-C₁₂)alkylene-CO₂H.

In some embodiments of any water-soluble dipyrromethene-based dye of formulae (I)-(IV), R¹ or R^{1'} is -L^{a}-Z^{a}, where L^{a} includes an optionally substituted carbocyclic or heterocyclic group linked to a chemoselective functional group (Z^{a}). In certain instances, L^{a} is an optionally substituted aryl or heteroaryl. Bivalent carbocyclic or heterocycle groups of interest include, but are not limited to, 1,4-cyclohexyl, 1,3-cyclohexyl, piperidinyl (e.g., 1,4-piperidinyl), piperazinyl (e.g., 1,4-piperazinyl), and the like. Bivalent aryl or heteroaryl groups of interest include, but are not limited to, 1,4-phenyl, 1,3-phenyl, 2,5-pyridyl, 2,6-pyridyl, 3,5-pyridyl, and the like. The bivalent carbocyclic or heterocycle group or the bivalent aryl or heteroaryl group of L^{a} may be linked to -L^{b}-Z^{a}, where L^{b} is a linking group, e.g., as described in any one of the embodiments herein. In some embodiments of any water-soluble dipyrromethene-based dye of formulae (I)-(IV), R¹ or R^{1'} is described by one of the following structures: 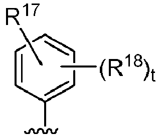 wherein:
R¹⁷ is L^{b}-z^{a};
L^{b} is a linker and Z^{a} is a chemoselective group or a molecule of interest;
t is 0-4; and each R¹⁸ is independently selected from alkyl, substituted alkyl, alkoxy, substituted alkoxy, hydroxy, aryl, substituted aryl, heteroaryl, substituted heteroaryl, halogen, sulfonic acid and water solubilizing group (WSG). In certain embodiments, R¹ or R^{1'} is described by one of the following structures: 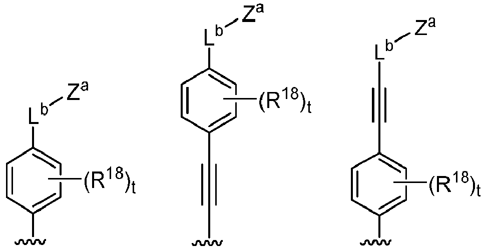 wherein:
L^{b} is a linker and Z^{a} is a chemoselective group or a molecule of interest;
t is 0-4; and each R¹⁸ is independently selected from alkyl, substituted alkyl, alkoxy, substituted alkoxy, hydroxy, aryl, substituted aryl, heteroaryl, substituted heteroaryl, halogen, sulfonic acid and water solubilizing group (WSG). In certain embodiments, R¹ or R^{1'} is described by one of the following structures: 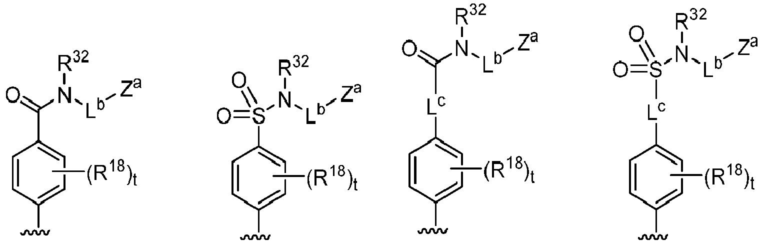 wherein:
L^{b} is a linker and Z^{a} is a chemoselective group or a molecule of interest;
R³² is H, alkyl, substituted alkyl, and water solubilizing group (WSG);
L^{c} is a linker selected from alkylene (e.g., C₁-C₆-alkylene), -O-alkylene and substituted versions thereof;
t is 0-4; and each R¹⁸ is independently selected from alkyl, substituted alkyl, alkoxy, substituted alkoxy, hydroxy, aryl, substituted aryl, heteroaryl, substituted heteroaryl, halogen, sulfonic acid and water solubilizing group (WSG).

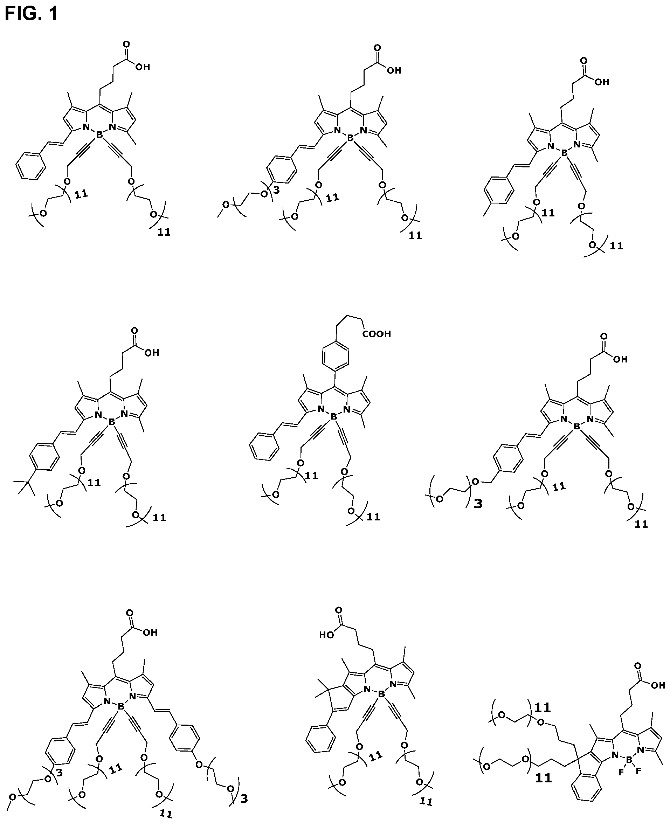

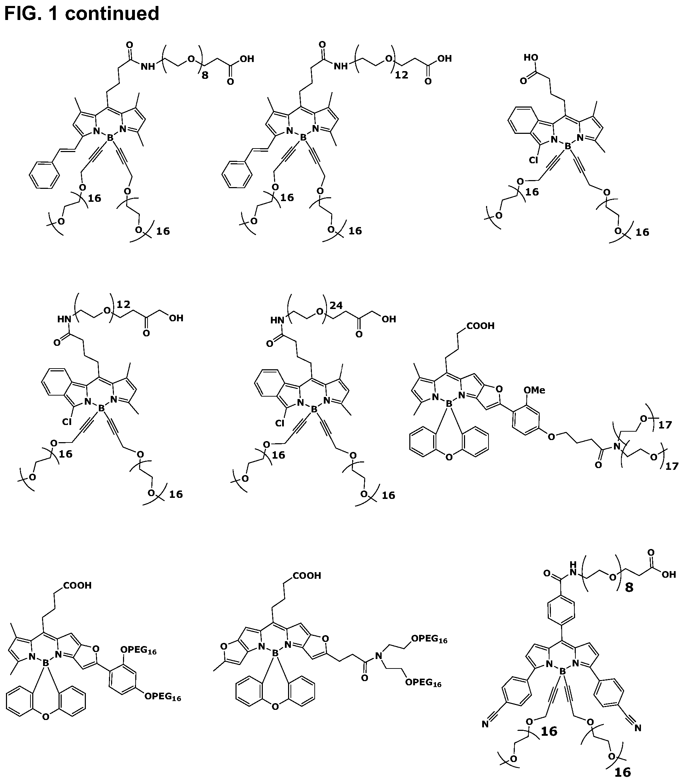

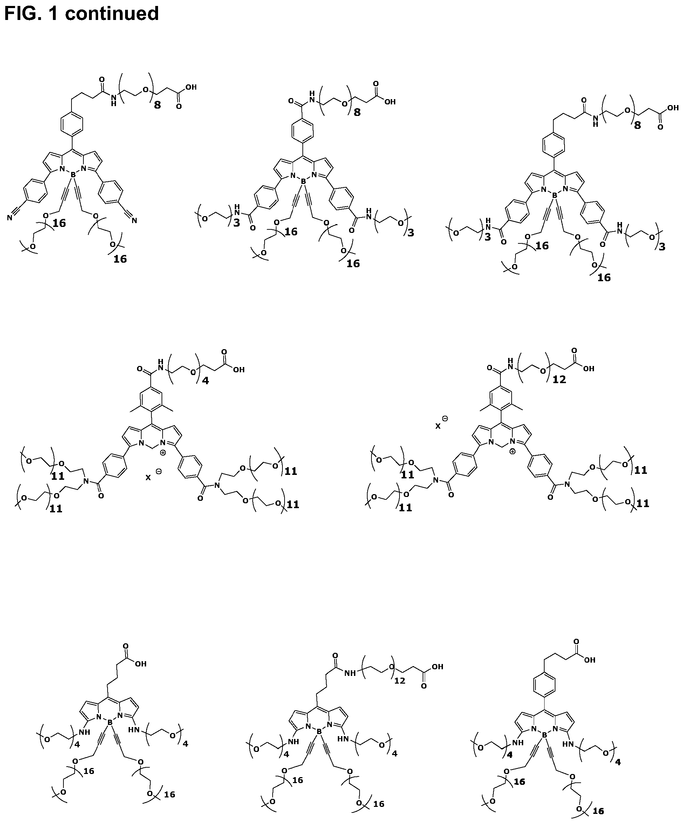

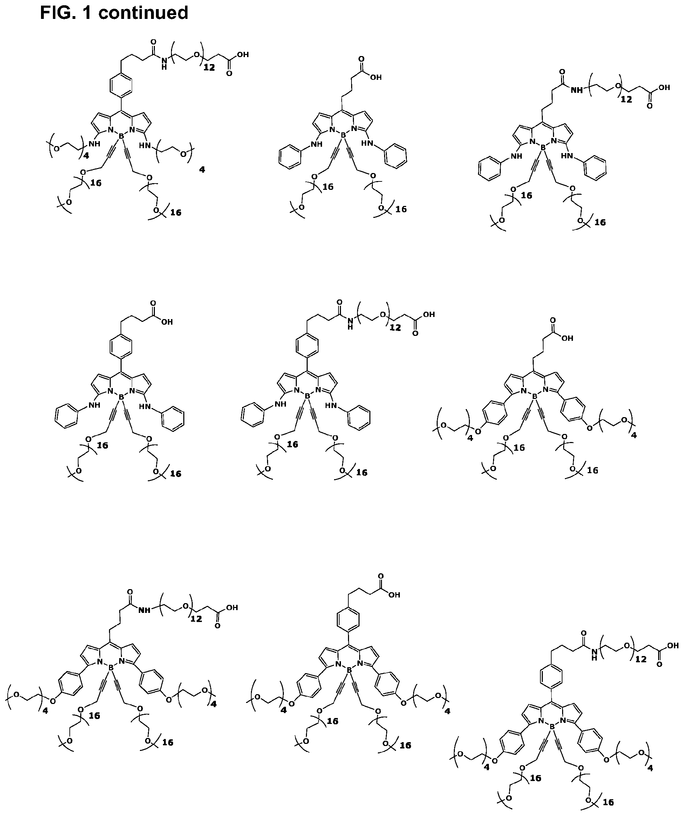

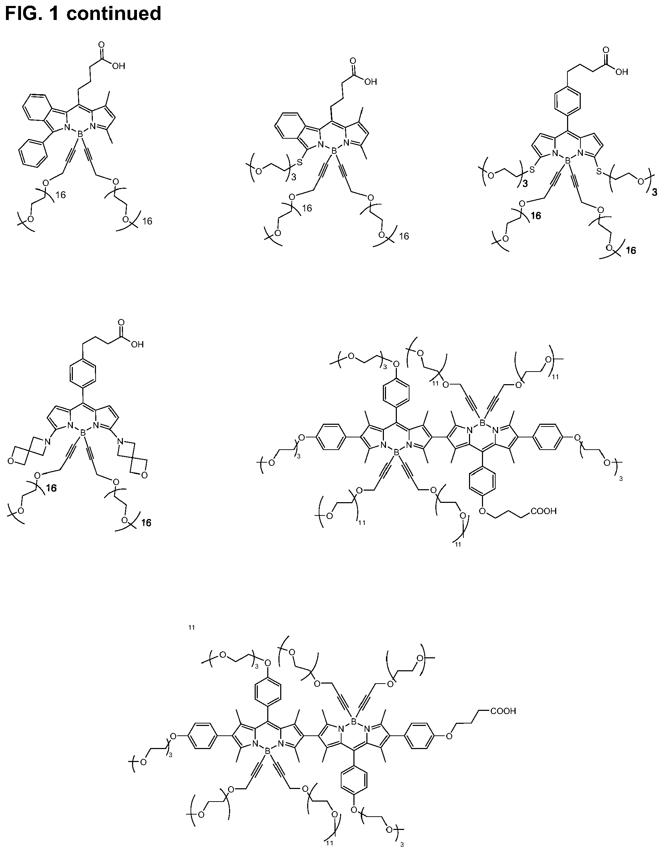

Aspects of the present disclosure include water-soluble dipyrromethene-based dyes including water solubilizing groups (e.g., a dye of any one of formulae (I)-(IV)), that are suitable for linking to molecules of interest, and tandem dyes including the same. In some cases, the subject water-soluble dipyrromethene-based dyes are substituted with a group or substituent including a chemoselective functional group that facilitates conjugation of the dye to a molecule of interest such as a polymeric donor dye. In certain cases, the water-soluble dipyrromethene-based dye is any compound of formulae (I)-(IV) or a compound as depicted in FIG. 1. It is understood that for any of the water-soluble dipyrromethene-based dyes described herein (e.g., dyes of formulae (I)-(IV)), the present disclosure includes novel water-soluble dipyrromethene-based dyes that are not conjugated to a polymeric dye backbone, and water-soluble dipyrromethene-based dye reagent derivatives thereof including a chemoselective functional group. Such dyes and dye reagents find use in a variety of contexts. The water-soluble dipyrromethene-based dye reagents of interest can be conjugated to any convenient molecule of interest, e.g., a biomolecule for a second dye such as a polymeric dye. As such, also included are polymeric tandem dyes including a linked water-soluble dipyrromethene-based dye as described herein (e.g., a polymeric tandem dye including one of formulae (I)-(IV)).

Embodiments of the invention include yellow-green absorbing dipyrromethene-based dyes that efficiently transfer energy to an acceptor chromophore while a second dipyrromethene-based dye of the same or different chemical structure is within energy-receiving proximity therewith. Accordingly, embodiments of the invention provide dipyrromethene-based dyes that efficiently transfer energy to an acceptor chromophore while a second dipyrromethene-based dye of the same or different chemical structure is within energy-receiving proximity therewith. Mechanisms for energy transfer between the dipyrromethene-based dye to an acceptor chromophore include, for example, resonant energy transfer (e.g., Forster (or fluorescence) resonance energy transfer, FRET), quantum charge exchange (Dexter energy transfer) and the like. These energy transfer mechanisms can be relatively short range; that is, close proximity of chromophores of the light harvesting multichromophore system to each other and/or to an acceptor fluorophore provides for efficient energy transfer. By "efficient" energy transfer is meant that 20% or more or 30% or more, 40% or more, 50 % or more, of the energy harvested by the donor chromophores is transferred to the acceptor.

The provided dipyrromethene-based dyes of embodiments of the invention efficiently transfer energy to an acceptor chromophore while a second dipyrromethene-based dye of the same or different chemical structure is within energy-receiving proximity therewith. Stated in another manner, such efficient energy transfer can occur even when a second dipyrromethene-based dye is located in energy-receiving proximity to the first dipyrromethene-based dye. The efficient energy transfer can also occur when no second dipyrromethene-based dye is located in energy-receiving proximity. The provided dipyrromethene-based dyes can selectively transfer energy to the acceptor chromophore instead of to a second dipyrromethene-based dye, which can thereby advantageously increase the fluorescence quantum yield. For example, the fluorescence quantum yield of the provided dipyrromethene-based dye and an acceptor chromophore can be 0.01 or more, such as 0.05 or more, 0.1 or more, 0.2 or more, 0.3 or more, 0.4 or more, or 0.5 or more. As used herein, the term "quantum yield" refers to the number of photons emitted by the acceptor chromophore divided by the number of photons absorbed by the provided dipyrromethene-based dye. In some cases, the second dipyrromethene-based dye has the same chemical structure as the first dipyrromethene-based dye.

In some cases, the dipyrromethene-based dyes have the formula (I): 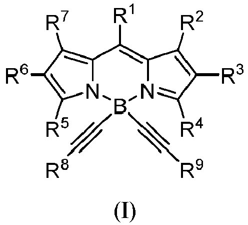 wherein:
R¹-R⁷ are each independently selected from H, alkyl, substituted alkyl, alkenyl, alkynyl, substituted alkynyl, aryl, substituted aryl, heteroaryl, substituted heteroaryl, heterocycle, substituted heterocycle, alkoxy, substituted alkoxy, amino, amido, substituted amido, thiol, halogen, hydroxyl, cyano, water solubilizing group (WSG), -T¹-J¹, wherein T¹ is an optional linker and J¹ has formula (I),
optionally one or more pairs of substituents selected from R¹ and R², R² and R³, R³ and R⁴, R⁵ and R⁶, R⁶ and R⁷, and R⁷ and R¹ together form a fused ring, and optionally a pair of further substituents on the fused ring together form a further fused ring;
R⁸ and R⁹ are each independently a poly(alkylene oxide) group.

In some cases, R¹ is aryl, substituted aryl, heteroaryl, or substituted heteroaryl. For example, in some cases R¹ is substituted aryl, e.g. R¹ has the formula: 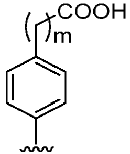 wherein m ranges from 0 to 10. In some cases, m is 0 and in other cases m ranges from 1 to 10, such as from 1 to 5.

In some embodiments, R¹ is alkyl or substituted alkyl, e.g., wherein R¹ is an alkyl group that has been substituted with a carboxy group. For instance, R¹ can have the formula: 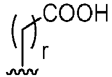 wherein r ranges from 0 to 10. In some cases, r is 0 and in other cases r ranges from 1 to 10, such as from 1 to 4.

In some cases, at least one of R²-R⁷ comprise a π bond that is π-conjugated to π electrons of a pyrrole ring of the formula (I) structure. Such a π bond can change the optical properties of the dipyrromethene-based dye, e.g., the absorption spectrum, the emission spectrum, the efficiency of energry transfer (e.g. FRET), or a combination thereof. For example, R⁴ can be a cyano group (i.e. -C≡N) that has a triple bond between the carbon atom and the nitrogen atom, wherein the two π bonds of the triple bond are π conjugated to the pyrrole ring on the right side of the formula (I) structure. As another example, R² and R³ can together form a fused aromatic ring, e.g., an aryl ring. Since such an aryl ring of R² and R³ is fused to the pyrrole ring on the right side of the formula (I) structure, the π bonds of the aryl ring are also π conjugated to π electrons of the pyrrole ring.

In some embodiments, R² and R³ together form a fused aromatic ring. The fused aromatic ring can be an aryl ring, a substituted aryl ring, a heteroaryl ring, or a substituted heteroaryl ring.

In some embodiments, R⁴ is selected from the group consisting of: alkyl, aryl, substituted aryl, heteroaryl, substituted heteroaryl, and cyano. In some cases, R⁴ is an alkyl group, e.g., methyl, ethyl, or propyl. In some cases R⁴ is an aryl group or a substituted aryl group, e.g., wherein the aryl group is substituted with an alkyl group, an alkoxy group, or a cyano group. The presence of π bonds and unpaired electrons (e.g., of an alkoxy group) of R⁴ can influence the electronic stucture of the dipyrromethene core, which can alter the optical properties of the provided dye.

In some cases, R⁵ and R⁶ together can form a fused ring. In some cases the fused ring of R⁵ and R⁶ is substituted by further substituents that form a further fused ring. In some embodiments, this further fused ring is aromatic, e.g., an aryl ring or a substituted aryl ring. In some cases, R⁵ and R⁶ are configured such that they provide a first ring fused to the dipyrromethene core and a second ring fused to the first ring, e.g., wherein the second ring is aromatic and is π conjugated to the dipyrromethene core. In some cases, R⁵ and R⁶ together provide a group of formula:

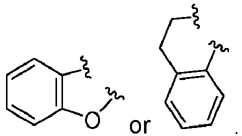

In some cases, R⁸ and R⁹ are each independently a poly(ethylene glycol) group. For instance, one or more of the poly(ethylene glycol) groups can have the formula: 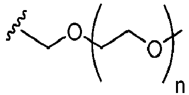 wherein n ranges from 1 to 50, such as from 3 to 35, from 6 to 24, from 10 to 20, or from 12 to 16. The presence of the poly(alkylene oxide) groups of R⁸ and R⁹ can increase the solubility of the dye in water.

Provided are dipyrromethene-based dyes of formula (I). In some cases, R¹ is aryl, substituted aryl, heteroaryl, substituted heteroaryl, alkyl, or substituted alkyl. In some cases R² and R³ together form a fused aromatic ring. In some cases R⁴ is wherein R⁴ is methyl, ethyl, aryl, substituted aryl, or cyano In some cases R⁵ and R⁶ together form a fused ring that is optionally substituted with a pair of further substitutents that form a further fused ring, e.g. that is aromatic and π conjugated to a pyrrole ring of the formula (I) structure. In some embodiments, if R⁴ is substituted aryl then R⁵ is not substituted aryl, if R⁴ is aryl then R⁵ is not alkyl, if R², R⁴, and R⁵ are methyl then R⁷ is not methyl, if R² and R³ or R⁵ and R⁶ together form a fused ring, then R³ and R⁶ are not hydrogen, or a combination thereof.

In some embodiments R² and R³ together form a fused ring, e.g., an aryl ring, and R⁴ is aryl, substituted aryl, or cyano. In some of such cases, R⁵-R⁷ are each hydrogen. In some cases R¹ is substituted alkyl or substituted aryl. Exemplary dyes with such R groups are shown below.

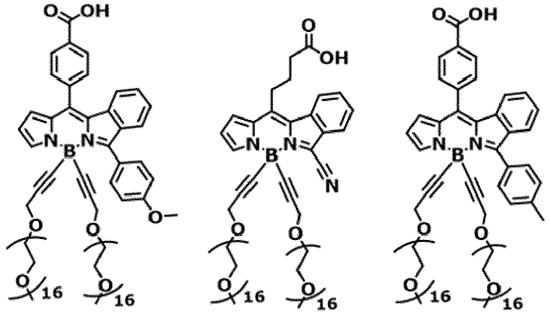

In some cases R⁵ and R⁶ together form a ring, e.g., a non-aromatic ring, that is substituted with further substitutents that form a further ring. Such an embodiment can also be referred to as R⁵ and R⁶ together forming a first ring that is fused to a pyrrole ring of the formula (I) structure and a second ring that is fused to the first ring. In some embodiments the further ring is an aromatic ring, e.g., an aryl ring. In some cases R¹ is substituted alkyl or substituted aryl. Exemplary dyes with such R groups are shown below.

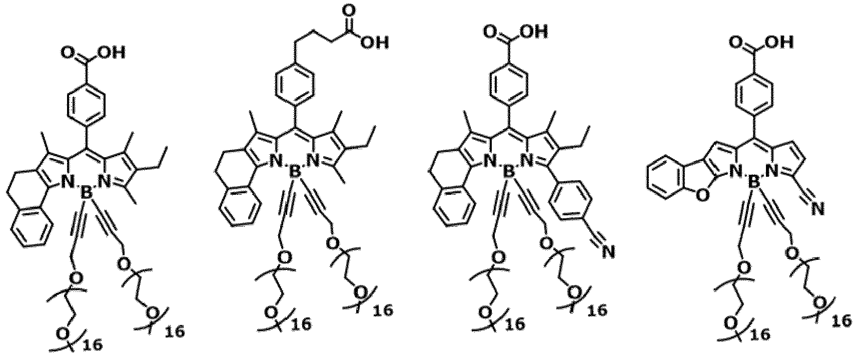

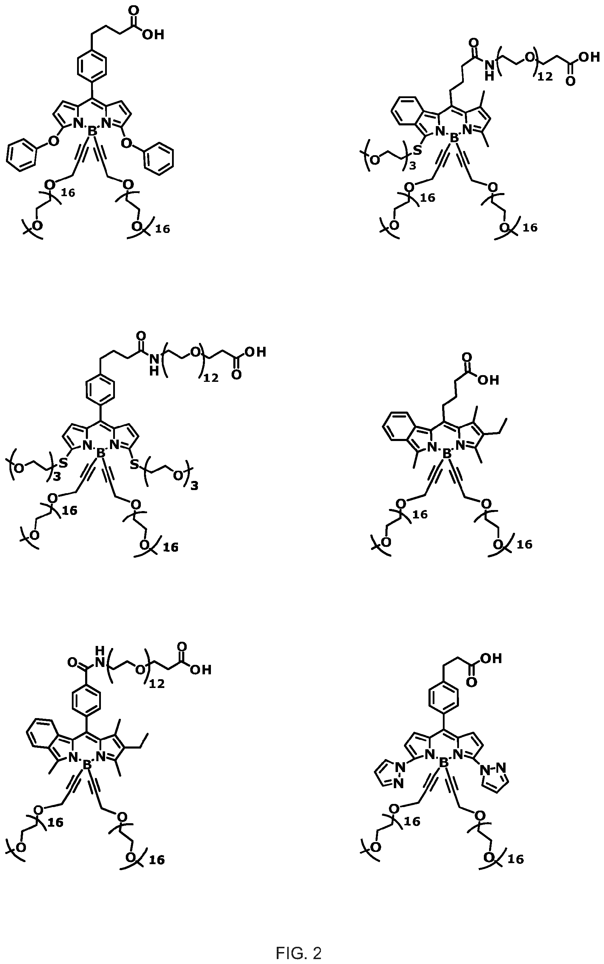

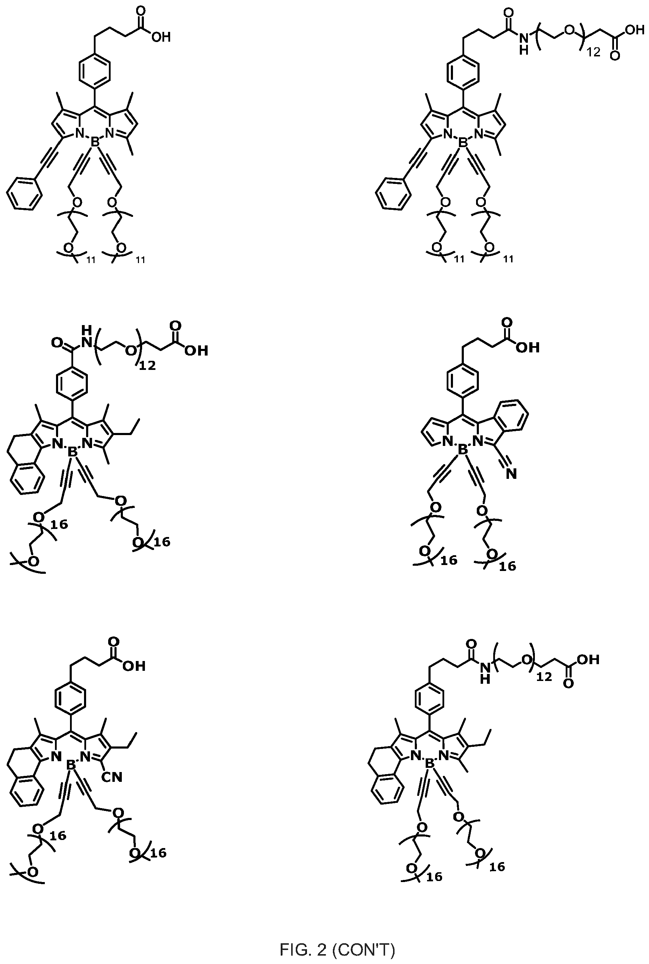

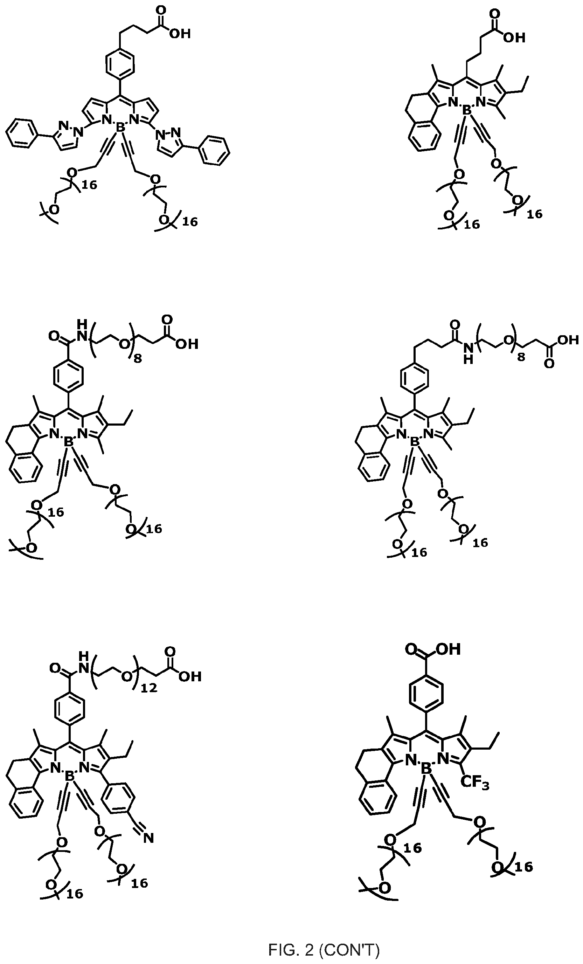

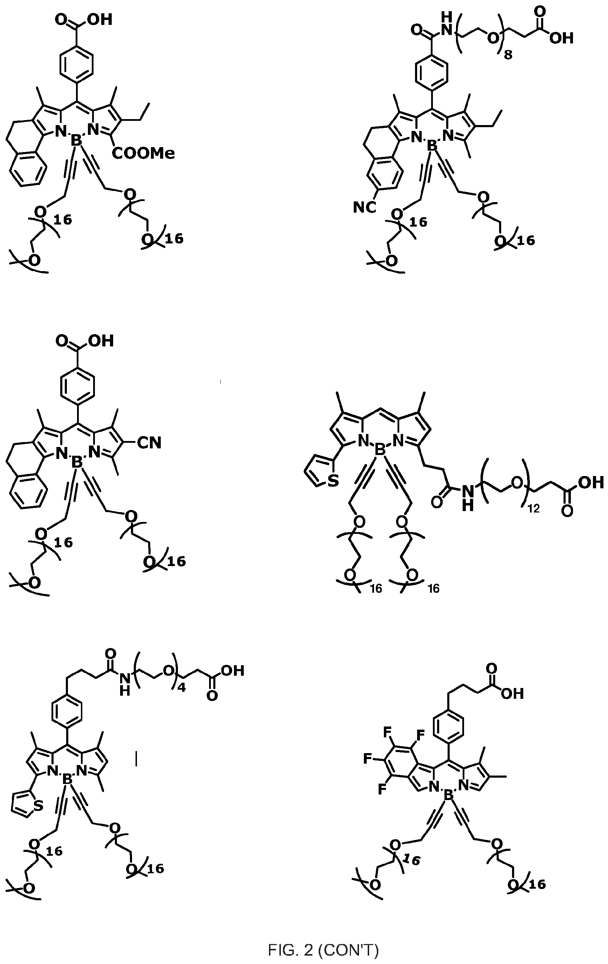

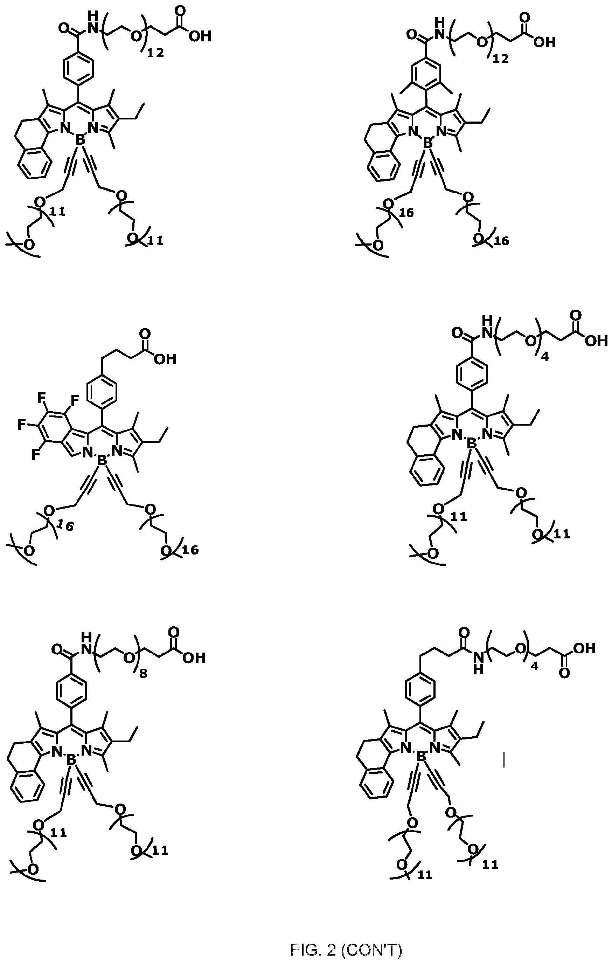

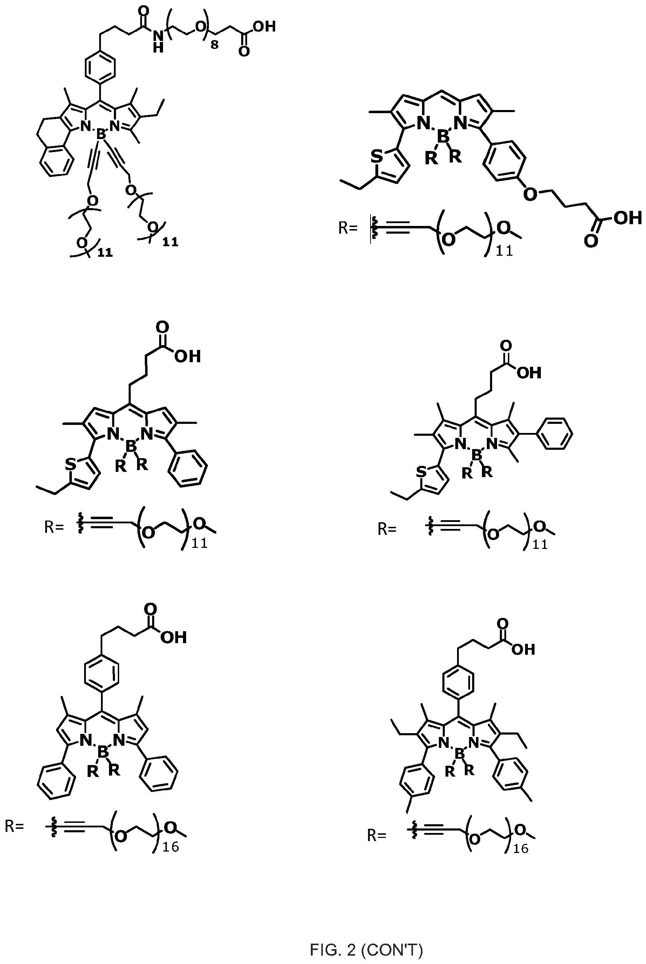

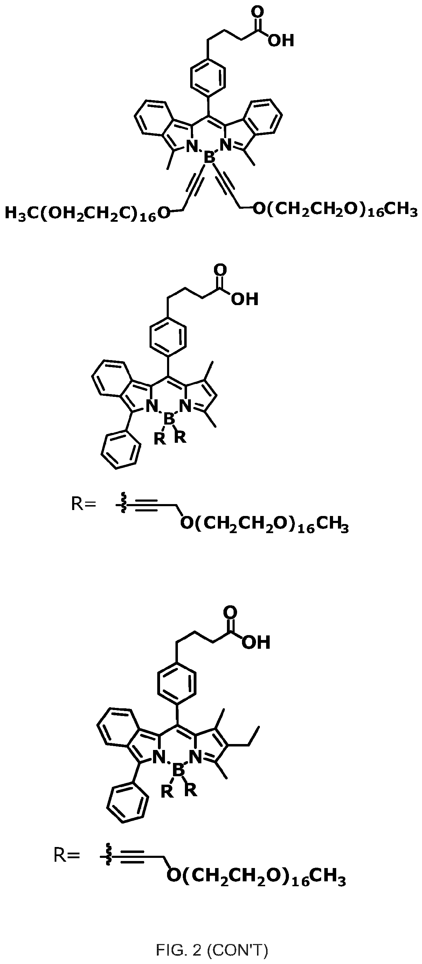

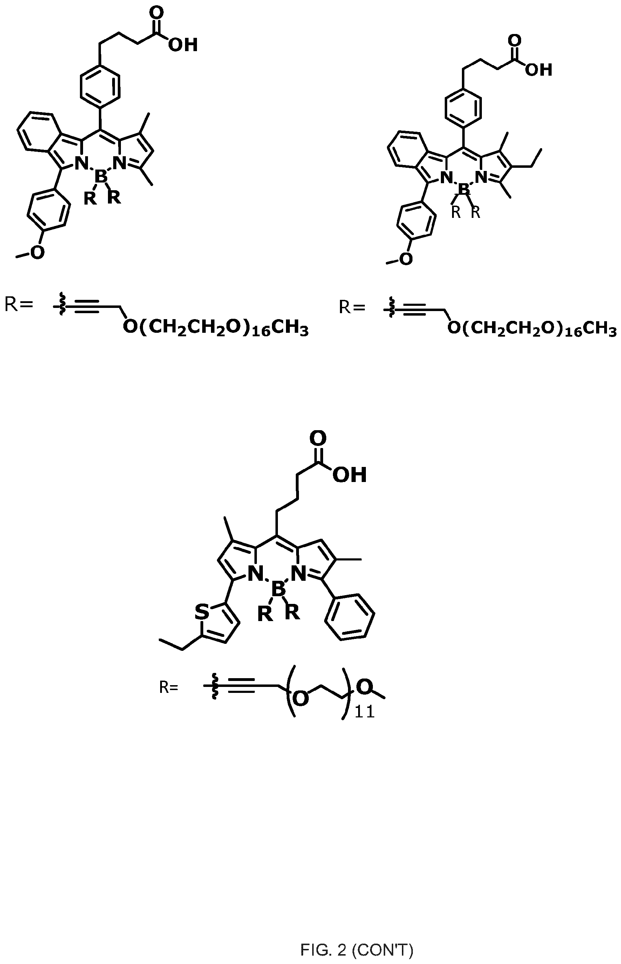

Additional dyes of interest include those provided in FIG. 2.

### TANDEM DYES

As summarized above, also provided are tandem dyes that include a water-soluble dipyrromethene-based dye that absorbs in the yellow green range at 561 nm (e.g., a dye of formulae (I)-(IV)), such as described above. Tandem dyes of the invention include the water-soluble dipyrromethene-based dye and a second dye, where the dyes are in energy transfer relationship, such as fluorescent energy transfer (FET) relationship, e.g., as describe further below. In such tandem dyes, the water-soluble dipyrromethene-based dye may be a donor or an acceptor. In such tandem dyes, the water-soluble dipyrromethene-based dye and second dye may be linked to a backbone, such as a polymeric backbone, e.g., a polypeptide backbone, that provides for the desired energy transfer relationship.

In some instances, the water-soluble dipyrromethene-based dye is a donor of a tandem dye. Tandem dyes of these embodiments include a donor chromophore that is a water-soluble dipyrromethene-based dye that absorbs in the yellow green range at 561 nm (e.g., a dye of formulae (I)-(IV)), e.g., as described above, and an energy acceptor that includes a pendant acceptor fluorophore, configured in energy-receiving proximity to the donor chromophore, e.g., where both are linked to a common backbone. In some cases of the subject tandem dyes, a plurality of pendant donor chromophore groups, e.g., water-soluble dipyrromethene-based dye that absorbs in the yellow green range at 561 nm (e.g., a dye of formulae (I)-(IV)), e.g., as described above, are present, and are configured in energy-transferring proximity to the pendant acceptor fluorophore. The term "pendant group" refers to a sidechain group that is connected to the backbone but which is not part of the backbone itself.

The present disclosure provides polymeric tandem dyes that can achieve narrow yellow green laser absorption features (i.e., at 561 nm), minimizing absorption at other common laser sources for applications such as flow cytometry. The subject polymeric tandem dyes allow for absorption in the yellow green laser range and emit at a wavelength determined by a chosen attached energy acceptor. The subject tandem dyes are water-soluble and can be dissolved into water and common aqueous buffer solutions to provide for good quantum yield in the aqueous media.

In certain embodiments of the tandem dye, the donor chromophore has an absorption maxima of from 555 nm to 585 nm, such as 555 nm to 575 nm, such as 555 to 575 nm, such as 555 to 574 nm, 555 to 573 nm, 555 to 572 nm, 555 to 571 nm, 555 to 570 nm, 555 to 569 nm, 555 to 568 nm, 555 to 567 nm, 555 to 566 nm, 555 to 565 nm, 555 to 564 nm, 555 to 563 nm, or 555 to 562 nm. In certain cases of the tandem dye, the donor chromophore has an absorption maxima of from 558 to 568 nm, such as 558 to 567 nm, 558 to 566 nm, 558 to 565 nm, 558 to 564 nm, 558 to 563 nm, or 558 to 562 nm. In certain cases of the tandem dye, the donor chromophore has an absorption maxima of about 561 nm.

In certain embodiments of the tandem dye, the donor chromophore is a water-soluble dipyrromethene-based dye as described herein (e.g., a dye of any one of formulae (I)-(IV) as described herein). In certain cases, the donor chromophore is a water-soluble dipyrromethene-boron dye (e.g., a dye of any one of formulae (I)-(IIIc) as described herein). In certain cases, the donor chromophore is a water-soluble dipyrromethene-based dye as depicted in FIG. 1.

The donor chromophore is capable of transferring energy to a linked acceptor fluorophore. As such, the subject tandem dyes include a linked acceptor signaling fluorophore in energy-receiving proximity to the donor water solvated light harvesting chromophore system, i.e., in energy-receiving proximity to at least one linked donor chromophore group. The terms "acceptor fluorophore" and "acceptor chromophore" are used interchangeably herein.

The acceptor signaling fluorophore can be linked to a repeat unit of a backbone as a pendant group. Excitation of the donor can lead to energy transfer to, and emission from, the covalently attached acceptor signaling fluorophore. The number of repeat units of the donor water solvated light harvesting chromophore having linked acceptor signaling fluorophore groups may vary, where in some instances the number ranges from 1 mol% to 50 mol% of the repeat units, such as from 1 mol% to 25 mol%, 2 mol% to 25 mol%, 3 mol% to 25 mol%, 4 mol% to 25 mol%, 5 mol% to 25 mol% or from 10 mol% to 25 mol%.

Mechanisms for energy transfer between the donor chromophores to a linked acceptor signaling fluorophore include, for example, resonant energy transfer (e.g., Forster (or fluorescence) resonance energy transfer, FRET), quantum charge exchange (Dexter energy transfer) and the like. These energy transfer mechanisms can be relatively short range; that is, close proximity of chromophores of the light harvesting multichromophore system to each other and/or to an acceptor fluorophore provides for efficient energy transfer.

Under conditions for efficient energy transfer, amplification of the emission from the acceptor fluorophore can occur where the emission from the luminescent acceptor fluorophore is more intense when the incident light (the "pump light") is at a wavelength which is absorbed by, and transferred from, the chromophores of the light harvesting chromophore than when the luminescent acceptor fluorophore is directly excited by the pump light.

By "efficient" energy transfer is meant 10% or more, such as 20% or more or 30% or more, 40% or more, 50 % or more, of the energy harvested by the donor chromophores is transferred to the acceptor. By "amplification" is meant that the signal from the acceptor fluorophore is 1.5x or greater when excited by energy transfer from the donor light harvesting chromophore system as compared to direct excitation of the acceptor fluorophore with incident light of an equivalent intensity. The signal may be measured using any convenient method. In some cases, the 1.5x or greater signal refers to an intensity of emitted light. In certain cases, the 1.5x or greater signal refers to an increased signal to noise ratio. In certain embodiments of the tandem dye, the acceptor fluorophore emission is 1.5 fold greater or more when excited by the chromophore as compared to direct excitation of the acceptor fluorophore with incident light, such as 2-fold or greater, 3-fold or greater, 4-fold or greater, 5-fold or greater, 6-fold or greater, 8-fold or greater, 10-fold or greater, 20-fold or greater, 50-fold or greater, 100-fold or greater, or even greater as compared to direct excitation of the acceptor fluorophore with incident light.

Any convenient fluorescent dyes may be utilized in the polymeric tandem dyes as an acceptor fluorophore. The terms "fluorescent dye" and "fluorophore" are used interchangeably herein. The acceptor fluorophore (e.g., each A) can be a small molecule fluorophore. The acceptor fluorophore (e.g., each A) can be a dye molecule selected from a rhodamine, a perylene, a diimide, a coumarin, a xanthene, a cyanine, a polymethine, a pyrene, a thiazine, an acridine, a dipyrromethene borondifluoride, a napthalimide, a phycobiliprotein, a peridinum chlorophyll protein, conjugates thereof, and combinations thereof. In certain embodiments, the acceptor fluorophore (A) is a cyanine dye, a xanthene dye, a coumarin dye, a thiazine dye or an acridine dye. In some instances, the acceptor fluorophore (A) is selected from DY 431, DY 485XL, DY 500XL, DY 610, DY 640, DY 654, DY 682, DY 700, DY 701, DY 704, DY 730, DY 731, DY 732, DY 734, DY 752, DY 778, DY 782, DY 800, DY 831, Biotium CF 555, Cy 3.5 and diethylamino coumarin. Fluorescent dyes of interest include, but are not limited to, fluorescein, 6-FAM, rhodamine, Texas Red, tetramethylrhodamine, carboxyrhodamine, carboxyrhodamine 6G, carboxyrhodol, carboxyrhodamine 110, Cascade Blue, Cascade Yellow, coumarin, Cy2, Cy3, Cy3.5, Cy5, Cy5.5, Cy-Chrome, phycoerythrin, PerCP (peridinin chlorophyll-a Protein), PerCP-Cy5.5, JOE (6-carboxy-4',5'-dichloro-2',7'-dimethoxyfluorescein), NED, ROX (5-(and-6)-carboxy-X-rhodamine), HEX, Lucifer Yellow, Marina Blue, Oregon Green 488, Oregon Green 500, Oregon Green 514, Alexa Fluor 350, Alexa Fluor 430, Alexa Fluor 488, Alexa Fluor 532, Alexa Fluor 546, Alexa Fluor 568, Alexa Fluor 594, Alexa Fluor 633, Alexa Fluor 647, Alexa Fluor 660, Alexa Fluor 680, Alexa Fluor 700, 7-amino-4-methylcoumarin-3-acetic acid, BODIPY FL, BODIPY FL-Br.sub.2, BODIPY 530/550, BODIPY 558/568, BODIPY 564/570, BODIPY 576/589, BODIPY 581/591, BODIPY 630/650, BODIPY 650/665, BODIPY R6G, BODIPY TMR, BODIPY TR, conjugates thereof, and combinations thereof. Lanthanide chelates of interest include, but are not limited to, europium chelates, terbium chelates and samarium chelates. In some embodiments, the polymeric tandem dye includes a multichromophore linked to an acceptor fluorophore selected from Cy3, Cy3.5, Cy5, Cy5.5, Cy7, Alexa488, Alexa 647 and Alexa700. In certain embodiments, the polymeric tandem dye includes a multichromophore linked to an acceptor fluorophore selected from Dyomics dyes (such as DY 431, DY 485XL, DY 500XL, DY 530, DY 610, DY 633, DY 640, DY 651, DY 654, DY 682, DY 700, DY 701, DY 704, DY 730, DY 731, DY 732, DY 734, DY 752, DY 754, DY 778, DY 782, DY 800 or DY 831), Biotium CF 555, Cy 3.5, and diethylamino coumarin.

In certain cases, the acceptor fluorophore (A) is selected from fluorescein, 6-FAM, rhodamine, Texas Red, California Red, iFluor594, tetramethylrhodamine, a carboxyrhodamine, carboxyrhodamine 6G, carboxyrhodol, carboxyrhodamine 110, Cascade Blue, Cascade Yellow, coumarin, Cy2^{®}, Cy3^{®}, Cy3.5^{®}, Cy5^{®}, Cy5.5^{®}, Cy7^{®}, Cy-Chrome, DyLight 350, DyLight 405, DyLight 488, DyLight 549, DyLight 594, DyLight 633, DyLight 649, DyLight 680, DyLight 750, DyLight 800, phycoerythrin, PerCP (peridinin chlorophyll-a Protein), PerCP-Cy5.5, JOE (6-carboxy-4',5'-dichloro-2',7'-dimelhoxyfluorescein), NED, ROX (5-(and -6)-carboxy-X-rhodamine), HEX, Lucifer Yellow, Marina Blue, Oregon Green 488, Oregon Green 500, Oregon Green 514, Alexa Fluor@ 350, Alexa Fluor@ 430, Alexa Fluor@ 488, Alexa Fluor@ 532, Alexa Fluor@ 546, Alexa Fluor@ 568, Alexa Fluor@ 594, Alexa Fluor@ 633, Alexa Fluor@ 647, Alexa Fluor@ 660, Alexa Fluor@ 680, 7-amino-4-methylcoumarin-3-acetic acid, BODIPY^{®} FL, BODIPY^{®} FL-Br2, BODIPY^{®} 530/550, BODIPY^{®} 558/568, BODIPY^{®} 564/570, BODIPY^{®} 576/589, BODIPY^{®} 581/591, BODIPY^{®} 630/650, BODIPY^{®} 650/665, BODIPY^{®} R6G, BODIPY^{®} TMR, BODIPY^{®} TR, conjugates thereof and combinations thereof.

In some instances, the acceptor fluorophore is a dipyrromethene-based dye, e.g., a water-soluble dipyrromethene-based dye of any one of formula (I)-(IV) or any embodiment thereof described herein. It is understood that any convenient dipyrromethene-based dye described herein having a suitable absorption and emission profile can be configured as an acceptor fluorophore in energy receiving proximity to the donor water solvated light harvesting chromophore system, i.e., in energy-receiving proximity to at least one compatible pendant donor chromophore group.

In some instances, the tandem dye exhibits an effective Stokes shift of 100 nm or more, such as 110nm or more, 120nm or more, 130nm or more, 140nm or more, 150nm or more, 160nm or more, 170nm or more, 180nm or more, 190nm or more, 200nm or more, 250nm or more when the light harvesting chromophore is directly excited with incident light. In some cases, the effective Stokes shift of the tandem dye is up to about 300nm such as 100-300nm, 100-250nm or 100-200nm.

The emission of the polymeric tandem dye can have a quantum yield of 0.03 or more, such as a quantum yield of 0.04 or more, 0.05 or more, 0.06 or more, 0.07 or more, 0.08 or more, 0.09 or more, 0.1 or more, 0.15 or more, 0.2 or more, 0.3 or more or even more. In some instances, the polymeric tandem dye has an extinction coefficient of 5 × 10⁵ cm⁻¹M⁻¹ or more, such as 6 × 10⁵ cm⁻¹M⁻¹ or more, 7 × 10⁵ cm⁻¹M⁻¹ or more, 8 × 10⁵ cm⁻¹M⁻¹ or more, 9 × 10⁵ cm⁻¹M⁻¹ or more, such as 1 × 10⁶ cm⁻¹M⁻¹ or more, 1.5 × 10⁶ cm⁻¹M⁻¹ or more, 2 × 10⁶ cm⁻¹M⁻¹ or more, 2.5 × 10⁶ cm⁻¹M⁻¹ or more, 3 × 10⁶ cm⁻¹M⁻¹ or more, 4 × 10⁶ cm⁻¹M⁻¹ or more, 5 × 10⁶ cm⁻¹M⁻¹ or more, 6 × 10⁶ cm⁻¹M⁻¹ or more, 7 × 10⁶ cm⁻¹M⁻¹ or more, or 8 × 10⁶ cm⁻¹M⁻¹ or more. In some embodiments, the polymeric tandem dye has a molar extinction coefficient of 5 × 10⁵ M⁻¹cm⁻¹ or more. In certain embodiments, the tandem dye has a molar extinction coefficient of 1 × 10⁶ M⁻¹cm⁻¹ or more.

In embodiments, the subject tandem dyes provide for fluorescence emissions from acceptor fluorophores that are brighter than the emissions which are possible from such fluorescent dyes in isolation. The emission of the polymeric tandem dye can have a brightness of 50 mM⁻¹cm⁻¹ or more, such as 60 mM⁻¹cm⁻¹ or more, 70 mM⁻¹cm⁻¹ or more, 80 mM⁻¹cm⁻¹ or more, 90 mM⁻¹cm⁻¹ or more, 100 mM⁻¹cm⁻¹ or more, 150 mM⁻¹cm⁻¹ or more, 200 mM⁻¹cm⁻¹ or more, 250 mM⁻¹cm⁻¹ or more, 300 mM⁻¹cm⁻¹ or more, or even more. In certain instances, the emission of the tandem dye has a brightness that is at least 5-fold greater than the brightness of a directly excited acceptor fluorophore, such as at least 10-fold greater, at least 20-fold greater, at least 30-fold greater, at least 50-fold greater, at least 100-fold greater, at least 300-fold greater, or even greater than the brightness of a directly excited acceptor fluorophore.

The terms "light harvesting chromophore" and "polymeric dye" are used interchangeably and refer to a polymer of the present disclosure which has a plurality of pendant chromophore groups capable of harvesting light with a particular absorption maximum wavelength and converting it to emitted light at a longer emission maximum wavelength. The polymer can have a backbone which is saturated or partially unsaturated.

In certain embodiments of the tandem dye, the backbone is a linear polymer. In certain cases, the linear polymer is selected from a peptide, a peptoid, a hydrocarbon polymer, and a PEG polymer. In certain cases, the linear polymer is a peptide. In certain cases, the linear polymer is a peptoid. In certain cases, the polymer is a hydrocarbon polymer. In certain other cases, the polymer is a PEG polymer.

The subject tandem dye may include a linear polymer backbone of any number of units. As used herein the term "unit" refers to a structural subunit of a polymer. The term unit is meant to include monomers, co-monomers, co-blocks, conjugated segments, repeating units, and the like. A "repeating unit" is a subunit of a polymer that is defined by the minimum number of distinct structural features that are required for the unit to be considered monomeric, such that when the unit is repeated n times, the resulting structure describes the polymer or a block thereof. In some cases, the polymer may include two or more different repeating units, e.g., when the polymer is a multiblock polymer or a random arrangement of units, each block may define a distinct repeating unit, e.g., an n-block and a m-block. It is understood that a variety of arrangements of n and/or m repeating units or blocks are possible and that in the depicted formula of the subject chromophores described herein any convenient linear arrangements of co-blocks of various lengths are included within the structure of the overall polymer. It is understood that the polymer may also be represented by a formula in terms of mol% values of each unit in the polymer and that such formula may represent a variety of arrangements of repeat unit, such as random or multiblock polymer. In some cases, a repeating unit of the polymer includes a single monomer group. In certain instances, a repeating unit of the polymer includes two or more monomer groups, i.e., co-monomer groups, such as two, three, four or more co-monomer groups. As used herein, the term "co-monomer" or "co-monomer group" refers to a structural unit of a polymer that may itself be part of a repeating unit of the polymer. In some embodiments, the conjugated polymer includes a block copolymer that is composed of blocks of polymerized monomers. In such cases, the block copolymer may be described as having distinct repeating units each corresponding to a distinct co-block of the polymer. In some cases, the polymer is a diblock copolymer that contains two different co-blocks. In such cases, the polymer may be described as including co-blocks, where each co-block may be composed of co-monomers, such as one, two, three or more co-monomers.

The backbone of the tandem dye may have any convenient length. In some cases, the particular number of monomeric repeating units or segments of the chromophore may fall within the range of 2 to 500,000, such as 2 to 100,000, 2 to 30,000, 2 to 10,000, 2 to 3,000 or 2 to 1,000 units or segments, or such as 5 to 100,000, 10 to 100,000, 100 to 100,000, 200 to 100,000, or 500 to 50,000 units or segments. In some instances, the particular number of monomeric repeating units or segments of the backbone may fall within the range of 2 to 1,000, such as 2 to 500, 2 to 100, 3 to 100, 4 to 100, 5 to 100, 6 to 100, 7 to 100, 8 to 100, 9 to 100 or 10 to 100 units or segments. In certain cases, the particular number of monomeric repeating units or segments of the backbone may fall within the range of 2 to 500, such as 2 to 400, 2 to 300, 2 to 200, or 2 to 100 units or segments. In certain cases, the particular number of monomeric repeating units or segments of the backone may fall within the range of 2 to 100 repeating monomeric units, such as 2 to 90, 2 to 80, 2 to 70, 2 to 60, 2 to 50, 2 to 40, or 2 to 30 units or segments.

The polymeric backbone may have a random configuration of non-conjugated repeat units. The polymeric backbone may include a block or co-block configuration of non-conjugated repeat units. Alternatively, the polymeric backbone may include a particular defined sequence of non-conjugated repeat units, e.g., amino acid residues of a polypeptide sequence. These configurations can be characterized by polymeric segments of repeat units (e.g., as described herein), which segments can themselves be repeated throughout the modular scaffold.

By "non-conjugated" is meant that at least a portion of the repeat unit includes a saturated backbone group (e.g., a group having two or more consecutive single covalent bonds) which precludes pi conjugation or an extended delocalized electronic structure along the polymeric backbone from one repeat unit to the next. It is understood that even though one repeat unit may not be conjugated to an adjacent repeat unit, such a repeat unit may include one or more isolated unsaturated groups including an unsaturated bond (e.g., of an alkenylene group or an alkynylene group) and/or an aryl or heteroaryl group, which groups can be a part of the backbone. In some cases, each repeat unit of the polymeric backbone includes one sidechain including a linked pendant group or a chemo-selective tag for linking to a pendant group.

In certain embodiments of the tandem dyes, the polymeric backbone is a linear polymer. In certain cases, the linear polymer is selected from a peptide, a peptoid, a hydrocarbon polymer, and a PEG polymer. In certain cases, the linear polymer is a peptide. In certain cases, the linear polymer is a peptoid. In certain cases, the polymer is a hydrocarbon polymer. In certain other cases, the polymer is a PEG polymer. Further details regarding polymeric backbones that may be employed in embodiments of the invention are found in PCT application serial no. PCT/US2019/024662 published as WO2019/191482 and PCT application serial no. PCT/ US2020/019510 published as WO2020/222894; the disclosures of which applications are herein incorporated by reference.

In certain instances, the tandem dye includes a linear peptide backbone of from 2 to 100 amino acids, such as 2 to 90, 2 to 80, 2 to 70, 2 to 60, 2 to 50, 2 to 40 or 2 to 30 amino acids. In some cases, the linear peptide backbone includes 2 or more amino acids, such as 5 or more, 10 or more, 15 or more, 20 or more, 25 or more, 30 or more, up to a maximum of 100 amino acids. In certain cases, the tandem dye includes a linear peptide backbone of from 5 to 30 amino acids, such as 5 to 25, 5 to 20, 5 to 15, or 5 to 10 amino acids.

Any convenient the polymeric backbone can be adapted to include a linked water-soluble dipyrromethene-based dye (e.g., as described herein). In some embodiments, the subject tandem dye are described by formula (VA) or (VB): 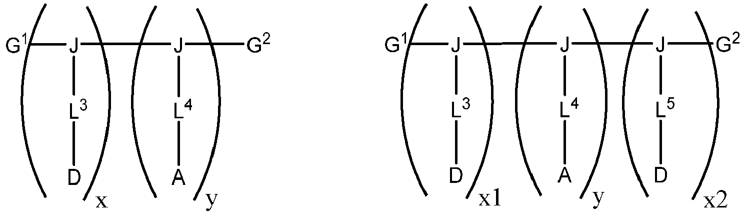 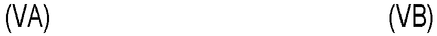 wherein:
each J are repeat units of the backbone;
L³-L⁵ are each independently a linker;
each D is a donor chromophore (e.g., as described herein);
each A is an acceptor fluorophore;
G¹ and G² are each independently selected from the group consisting of terminal group, a polymer segment, a donor chromophore, an acceptor fluorophore, a linker, and a linked specific binding member;
x is 75 mol% or more;
x1 + X2 is 75% or more; and
y is 25 mol% or less.

In certain cases of the tandem dye of formula (VA) or (VB), the donor chromophore (D) is a water-soluble dipyrromethene-based dye of any one of formulae (I)-(IV) or any compound depicted in FIGS. 1 & 2 (e.g., as described herein). In certain cases of the tandem dye of formula (VA) o (VB), each D pendant donor chromophore for each repeat units are all the same.

In certain cases of the tandem dye of formula (VA), x is 80 mol% or more, such as 85% or more, 90% or more, 95% or more. In certain embodiments of formula (VA), x is 90% or more, such as 92% or more, 94% or more, 96% or more.

In certain cases of the tandem dye is of formula (VB), the combination of x1 + X2 is 80 mol% or more, such as 85% or more, 90% or more, 95% or more. In certain embodiments of formula (VB), the combination of x1 and x2 is 90% or more, such as 92% or more, 94% or more, 96% or more.

In certain cases of tandem dye of formula (VA), y is 20 mol% or less, such as 18% or less, 15% or less, 10% or less. In certain embodiments of formula (VA), y is 10% or less, such as 8% or less, 6% or less, 5% or less.

In certain cases of tandem dye of formula (VB), y is 20 mol% or less, such as 18% or less, 15% or less, 10% or less. In certain embodiments of formula (VB), y is 10% or less, such as 8% or less, 6% or less, 5% or less.

In certain embodiments of the tandem dye of formula (VA) or (VB), at least one of G¹ or G² is a linked specific binding member (e.g., as described herein). In certain other cases of the light harvesting chromophore of formula (VA) or (VB), any of J, L³-L⁵, D or A comprise a linked specific binding member.

Any convenient co-monomers can be utilized to provide the repeat units of the backbone (J) in formulae (VA) or (VB). Co-monomers of interest which find use in preparing fully saturated or partially saturated polymeric backbones include, but are not limited to, co-monomers derived from an acrylate, a methacrylate, an acrylamide, a polystyrene, a ROMP (ring-opening metathesis polymerization) monomer, an ADMET (acyclic diene metathesis) monomer, a cyclic carbonate, monomers derived from polyethylene glycol and monomers derived from polyethylenimine. The co-monomers can be optionally substituted, e.g., with a chemoselective tag. Co-monomers can be polymerized or linked using any convenient chemistries, including but not limited, alkene polymerization, ring-opening polymerization, radical polymerization and Click chemistry or conjugations between compatible chemoselective functional groups or tags. ADMET monomers of interest include, but are not limited to those described by Mutlu et al. ("Acyclic diene metathesis: a versatile tool for the construction of defined polymer architectures", Chem. Soc. Rev., 2011,40, 1404-1445). In some instances, each J has the following formula: 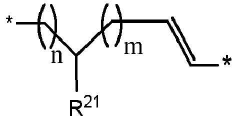 wherein:
R²¹ is -L³-D, -L⁵-D or -L⁴-A, where D is a pendant donor chromophore (e.g., as described herein), A is an acceptor chromophore, and L³-L⁵ are optional linkers;
n and m are independently an integer from 1 to 6 (e.g., 1 or 2); and
* is a connection to the polymeric backbone of the chromophore.

ROMP monomers of interest include, but are not limited to, those described by Song et al. ("Scope of the Ring-Opening Metathesis Polymerization (ROMP) Reaction of 1-Substituted Cyclobutenes", J. Am. Chem. Soc., 2010, 132 (30), pp 10513-10520). In some instances, each J independently has one of the following formulae: 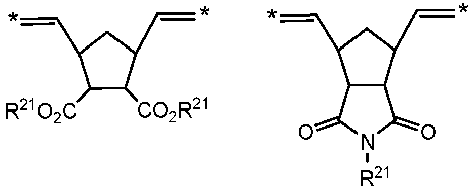 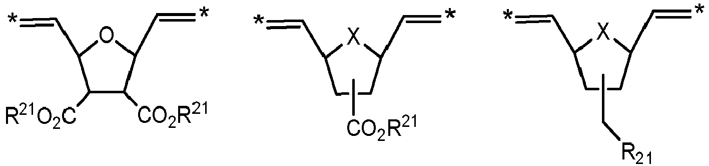 wherein:
R²¹ is -L³-D, -L⁵-D or -L⁴-A, where D is a pendant donor chromophore (e.g., as described herein), A is an acceptor chromophore, and L³-L⁵ are optional linkers;
X is CH₂ or O; and
* is a connection to the polymeric backbone of the chromophore.

In some instances, each J includes one of the following formulae: 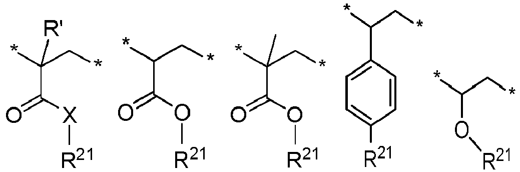 wherein:
R²¹ is -L³-D, -L⁵-D or -L⁴-A;
each D is a pendant donor chromophore;
A is an acceptor chromophore;
L³-L⁵ are optional linkers;
X is O or NR";
R' is H or lower alkyl (e.g., methyl);
R" is H, lower alkyl, substituted lower alkyl and WSG; and
* is a connection to the polymeric backbone of the chromophore. In some instances, the polymeric backbone includes a mixture of polystyrene and acrylate or acrylamide-derived co-monomers.

The tandem dye can have a hydrocarbon backbone prepared using any convenient polymerization methods. In some cases, the hydrocarbon backbone is derived from acrylate, acrylamide or styrene co-monomers, or a derivative thereof. In some cases, the chromophore is described by formula (VC): 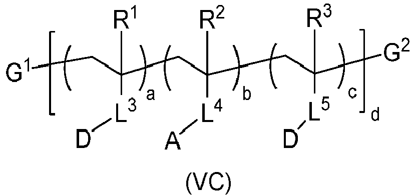 wherein:
each D is independently a pendant donor chromophore (e.g., as described herein);
each A is an acceptor chromophore;
each L³, L⁴ and L⁵ is independently a linker;
a, b and c are mol% values for each co-monomer;
d represents the total polymerization or average length of the polymer (e.g., d is 2-1000, such as 2-500, 2-200, 2-100 or 2-50); and
G¹ and G² are each independently selected from terminal group, polymer segment, donor chromophore group, acceptor fluorophore, linker and a linked specific binding member.

In some instances of formula (VC), c = 0. In some instances of formula (VC), a > 0 and b > 0. In some instances of formula (VC), a is 80 mol% or more, such as 85 mol% or more, 90 mol% or more, 95 mol% or more, 96 mol% or more, 97 mol% or more, 98 mol% or more, or 99 mol% or more. In some instances of formula (VC), b is 20 mol% or less, such as 15 mol% or less, 10 mol% or less, 5 mol% or less, 4 mol% or less, 3 mol% or less, 2 mol% or less, 1 mol% or less. In some instances of formula (VC), a is 65-95 mol%, b is 5-35 mol% and c is 0-30 mol%, where a+b+c=100%. In certain instances of formula (VC), L³-L⁵ includes a linkage to the backbone of the polymer selected from: -COO-, -CONR"-, -Ph-, -O-, where R" is H, lower alkyl, substituted lower alkyl or WSG. Such linkages cam be utilized to connect D, and A groups to the polymer backbone.

In certain instances of formula (VC), L³-D is described by one of the following: 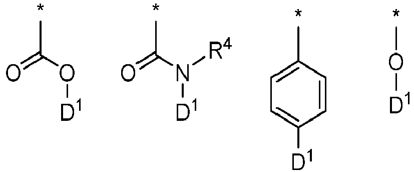 where R⁴ is H, lower alkyl, substituted lower alkyl or WSG.

The tandem dye can have a backbone derived from co-monomers connected via linkages or groups that are derived from Click chemistry conjugation reactions. Any convenient divalent co-monomer groups can be derivatized with terminal chemoselective tags and polymerized via conjugation of compatible chemoselective tag. In some cases, the co-monomers include one or more ethyleneoxide or ethyleneamino groups that make up part of the backbone of the polymer. Such groups can provide for desirable water solubility of the resulting polymeric dye. The co-monomer can further include a trivalent unit for linking to a sidechain group such as a donor or acceptor dye or a WSG. In some cases, the co-monomer includes a propyleneoxide or propyleneamino group in the backbone which is further substituted at the 2-position with a sidechain group or substituent. This group can include a linked chemoselective tag, linked donor or acceptor dye, or a linked WSG.

In some instances, the tandem dye is of formula (VD): 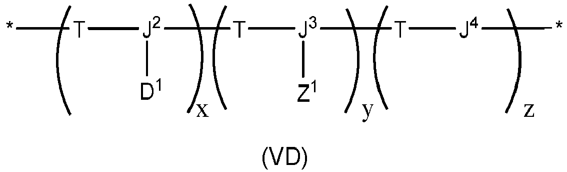 wherein:
the polymeric backbone of repeat units comprises J², J³ and J⁴ co-monomers that are each linked via a group T that is the product of a click chemistry or chemoselective group conjugation reaction (e.g., an azide-alkyne click chemistry);
J⁴ optionally comprises a linked WSG;
each D¹ is independently a pendant light absorbing chromophore linked to J²;
each Z¹ is independently a chemoselective tag linked to J³;
x is 50 mol% or more; and
y+z is 50 mol% or less, where * is a connection to the polymeric backbone of the multichromophore or a terminal group, e.g., as described herein. In certain instances of formula (VD), J², J³ and J⁴ comprise repeating units selected from ethylene oxide, ethylenamino, 2-substituted propyleneoxide and 2-substituted propyleneamino. In some instances of formula (VD), each T is 1,4-substituted 1,2,3-triazole, i.e., the product of azide-alkyne click chemistry conjugation reaction.

In some instances of formula (VD), J², J³ and J⁴ have the following structures: 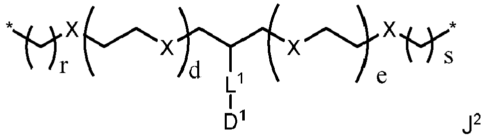 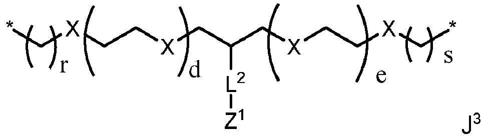 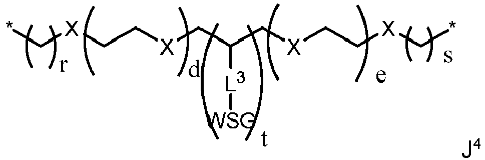 wherein:
each X is independently O or NR³¹ wherein R³¹ is H , alkyl, substituted alkyl, alkanoyl or substituted alkanoyl;
each r and s is independently 1-6 (e.g., 1, 2 or 3);
each d and e is independently 1-12 (e.g., 1-6, such as 1, 2, 3, 4, 5 or 6);
t is 0 or 1;
D¹ is a pendant donor chromophore (e.g., as described herein);
Z¹ is a chemoselective tag (e.g., as described herein);
WSG is a water solubilizing group (e.g., as described herein);
each L¹, L² and L³ is independently a linker; and
* is a connection to a 1,4-substituted 1,2,3-triazole (T).

It is understood that for any of the co-monomers from which the structures of J²-J⁴ described above are derived, either an azide or an alkyne group may be utilized at the terminal of the co-monomer for linking during polymerization. As such, the 1,4-substituted 1,2,3-triazole (T) may be present in one of two possible orientations as follows:

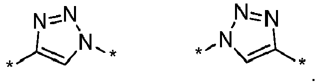

The terminals of the polymeric backbone can include any convenient terminal groups, such as an azide or alkyne group, linker or linked specific binding moiety.

In some instances, the tandem dye includes one of the following structures: 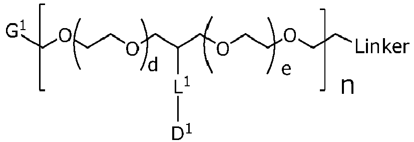 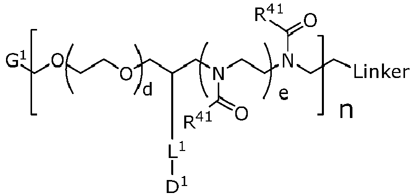 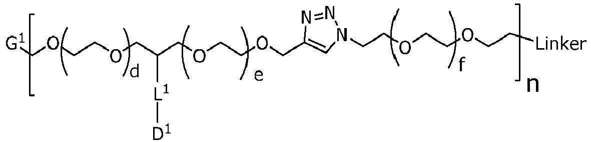 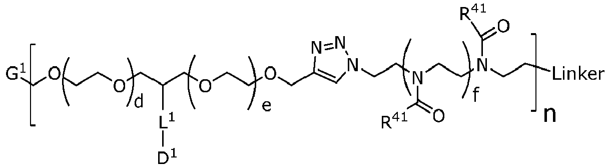 wherein:
G¹ is a terminal group (e.g., as described herein);
L¹ and L² are independently a linker;
D¹ is a donor chromophore (e.g., as described herein);
each d, e and f is independently 1-6;
n is 1-1000 (e.g., 2-1000, 2-500, 2-100 or 2-50);
each R⁴¹ is selected from alkyl, substituted alkyl and WSG; and
"Linker" is a linker including an optional chemoselective functional group, e.g., for conjugation to a co-monomer or a biomolecule. In some instances of formula (VD), L¹ - L³ comprise a linkage to the backbone of the polymer selected from - NHCO-alkyl.

Cyclic carbonate and protected carbonate monomers of interest which can be adapted for use in preparing polymeric backbones of the subject light harvesting chromophores includes, but are not limited to, those described by Barnes et al. in WO2013036532, Cooley et al. (J. Am. Chem. Soc., 131, 45, 1640-3, 2009), and Rothbard et al. in U.S. Patent 7,169,814. Such monomers are utilized in a polymerization reaction using an initiator and a suitable feed ratio of cyclic carbonate monomers to provide a polymeric backbone. Alternative, protected carbonate monomers can be assembled in a step wise synthesis to provide a defined sequence. In some cases of the subject tandem dyes, the backbone has a polycarbonate backbone. As such, the light harvesting chromophore can have formula (VE): 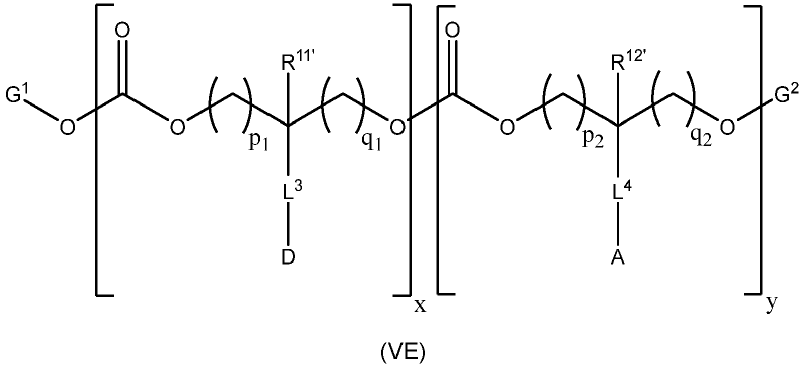 wherein:
each D is independently a pendant donor chromophore group (e.g., as described herein);
each A is independently an acceptor fluorophore;
each L³ and L⁴ is independently a linker;
x is 75 mol% or more;
y is 25 mol% or less; and
G¹ and G² are each independently selected from the group consisting of a terminal group, a polymer segment, a donor chromophore group, an acceptor fluorophore, a linker and a linked specific binding member.

In certain embodiments of the subject tandem dye, the repeat units (e.g., J or J²-J⁴) are arranged in a defined linear sequence. Any convenient co-monomers which can be polymerized in a defined stepwise fashion can be utilized in constructing a subject light harvesting chromophore (e.g., of formulae (VA) or (VB)). Co-monomers can be derived from amino acids, peptoid monomers, or a protected or cyclic carbonate monomer.

In some cases of the subject tandem dye, the backbone is a polypeptide having a defined sequence of α-amino acid residues and/or β-amino acid residues. Two types of β-amino acids and polypeptides can find use in the polymeric backbones of subject light harvesting chromophores: those with the sidechain group next to the amine are called β3-peptides/residues and those with the sidechain group next to the carbonyl group are called β2-peptides/residues. In certain embodiments, the light harvesting chromophore is of the formula (VF): 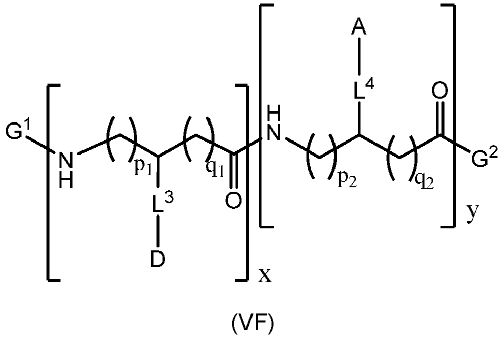 wherein:
each D is independently a pendant light absorbing chromophore group (e.g., as described herein);
each A is an acceptor fluorophore;
each L³ and L⁴ are independently a linker;
p₁ and q₁ are independently 0 or 1 wherein p₁ + q₁ ≤ 1;
p₂ and q₂ are independently 0 or 1 wherein p₁ + q₁ ≤ 1;
x is 75 mol% or more;
y is 25 mol% or less; and
G¹ and G² are each independently selected from a terminal group, a polymer segment, a light absorbing (e.g., donor) chromophore group, an acceptor fluorophore, a linker and a linked specific binding member.

In certain embodiments of the tandem dye of formula (VA) or (VB), each repeat unit J is an amino acid or a peptide unit.

In some embodiments of formula (VA), the polymeric tandem dye is of formula (VA1): 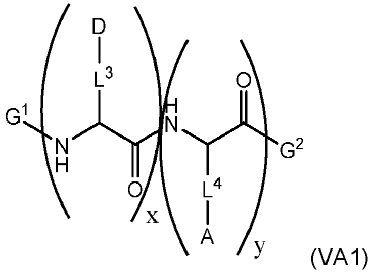 wherein:
each D is independently a pendant donor chromophore group (e.g., as described herein);
each A is independently an acceptor fluorophore;
L³ and L⁴ are each independently a linker;
x is 75 mol% or more;
y is 25 mol% or less; and
G¹ and G² are each independently selected from a terminal group, a polymer segment, a donor chromophore, an acceptor fluorophore, a linker, and a linked specific binding member. It is understood that the chromophores described by formula (VA1) include any convenient arrangements of co-monomers in a defined linear sequence, which have in total the defined mol% ratios of x and y. In some cases, the A containing co-monomers are spaced throughout the sequence of the polymeric backbone and as such are always flanked on both sides by one or more D containing co-monomers.

In certain instances of formula (VB), the polymeric tandem dye includes a segment of formula (VB1): 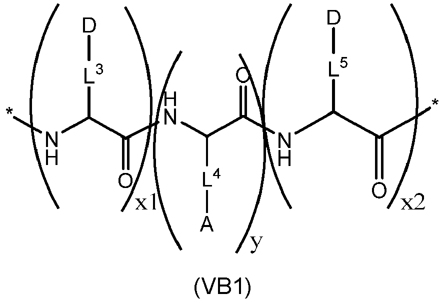 wherein:
each D is independently a pendant donor chromophore group (e.g., as described herein);
each A is independently an acceptor chromophore;
each L³-L⁵ are each independently a linker;
x1 and x2 are each independently an integer from 1 to 20 wherein x1+x2 ≥ 2; and
y is 1 or 2.

In some cases of formula (VB1), x1 and x2 are each independently 1 to 10 such as 2 to 10, 3 to 10 or 3 to 6. In some instances of formula (VB1), x1+x2 is an integer from 2 to 20, such as 3 to 20, 4 to 20, 5 to 20, 5 to 15 or 5 to 12. In certain embodiments of formula (VB1), y is 1.

The subject polymeric tandem dyes can include multiple segments of formula (VB1) where each segment includes one isolated A containing co-monomers flanked by blocks of D containing co-monomers. In some cases, the chromophore includes two or more segments of formula (VB1) located directed adjacent to each other to provide two isolated A containing co-monomers separated by a block of 2-20 D containing co-monomers, such as a block of 3 to 20, 4 to 20, 5 to 20, 5 to 15 or 5 to 12 D containing co-monomers. As such, in certain embodiments, the polymeric tandem dye includes q segments of a block copolymer and is of formula (VB2): 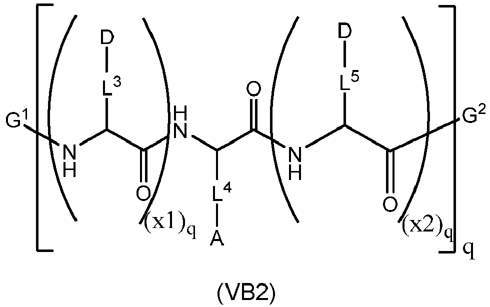 wherein: each (x1)_{q} and each (x2)_{q} is independently an integer from 1 to 20, wherein for each of the q segments (x1)_{q} +(x2)_{q} ≥ 3; and q is an integer from 1 to 100.

Any convenient amino acids can be adapted for use to provide a polymeric backbone to which pendant groups can be covalently linked. The amino acids can be naturally occurring or non-naturally occurring. For example, amino acids such as lysine, ornithine have sidechain amino groups suitable for conjugation with an amino-reactive group such as an activated carboxylic acid. Cysteine includes a sidechain thiol group suitable for conjugation with a thiol-reactive group such as a maleimide or a haloacetyl. Aspartate and glutamate have sidechain carboxylic acid groups which can be conjugated with a nucleophilic group such as an amino group. Methods of preparing the subject multichromophores including peptide synthesis methods are described herein.

In some instances, the polymeric backbone of the multichromophore has one or more of the following polypeptide sequence segments:
XYXX
XXYXX
XXXYXXX
XXXYXXXX
XXXXYXXX
XXXXYXXXX
XXXXXYXXXXX
XXXXXXYXXXXXX
XXXXXXXYXXXXXXX
XXXXXXXXYXXXXXXXX
XXXXXXXXXYXXXXXXXXX
Y(X)nY
XY(X)ₙYX
XXY(X)ₙYXX
XXXY(X)ₙYXXX
XXXXY(X)ₙYXXXX
XXXXXY(X)ₙYXXXXX
where:
each X is a first amino acid residue having a sidechain-linked first chemoselective tag, or a sidechain-linked pendant donor chromophore;
each Y is a second amino acid residue having a sidechain-linked second chemoselective tag, or a sidechain-linked pendant acceptor fluorophore; and
n is an integer of 2 to 20, such as 2 to 10, 3 to 10, 4 to 10 or 5 to 10, e.g., n is 2, 3, 4, 5, 6, 7, 8, 9 or 10.

In certain embodiments, in addition to the one or more polypeptide segments described herein, a third type of amino acid residue (Z) can be incorporated into the polymeric backbone, e.g., between two of the segments. In certain cases, a single isolated third amino acid residue is incorporated between any two of the segments described herein, e.g., (segment 1)-Z-(segment 2). This third amino acid residue can be a spacer residue, or a residue having a chemoselective functional group suitable for selective installation of an additional moiety of interest, such as a second pendant light absorbing chromophore, a chemoselective tag (e.g., a bio-orthogonal click chemistry tag), a linker, a linked biomolecule, a acceptor fluorophore, a WSG, etc. In certain instances the additional moiety of interest is incorporated into the third amino acid residue prior to preparation.

In certain instances, the first amino acid residue (X) includes a sidechain amino group, e.g., lysine or ornithine or a protected version thereof. During SPPS of the polymeric backbone, these sidechain amino groups can remain protected (e.g., with a Cbz or Boc protecting group), and then subsequently be orthogonally deprotected to provide for conjugation of the first residues to a pendant donor chromophore group. In certain cases, the second amino acid residue includes a sidechain thiol group, e.g., cysteine or a protected version thereof. Similarly, during SPPS of the polymeric backbone, these sidechain thiol groups can remain protected, and then subsequently be orthogonally deprotected to provide for conjugation of the second residues to a pendant group of interest, e.g., an acceptor fluorophore.

In some embodiments, the polymeric backbone of the multichromophore has, or is derived from, one or more of the following polypeptide sequence segments:
KCKK (SEQ ID NO: 1)
KKCK (SEQ ID NO: 2)
KKYKK (SEQ ID NO: 3)
KKKYKK (SEQ ID NO: 4)
KKYKKK (SEQ ID NO: 5)
KKKYKKK (SEQ ID NO: 6)
KKKYKKKK (SEQ ID NO: 7)
KKKKYKKK (SEQ ID NO: 8)
KKKKCKKKK (SEQ ID NO: 9)
KKKKKCKKKKK (SEQ ID NO: 10)
KKKKKKCKKKKKK (SEQ ID NO: 11)
KKKKKKKCKKKKKKK (SEQ ID NO: 12)
KKKKKKKKCKKKKKKKK (SEQ ID NO: 13)
KKKKKKKKKCKKKKKKKKK (SEQ ID NO: 14)
KKKKCKKKKKKKKKKCKKKKK (SEQ ID NO: 15)
C(K)ₙC (SEQ ID NO: 16)
KC(K)ₙCK (SEQ ID NO: 17)
KKC(K)ₙCKK (SEQ ID NO: 18)
KKKC(K)ₙCKKK (SEQ ID NO: 19)
KKKKC(K)ₙCKKKK (SEQ ID NO: 20)
KKKKKC(K)ₙCKKKKK (SEQ ID NO: 21)
wherein: each K is a lysine residue, a protected lysine residue, or a lysine residue covalently linked via the sidechain amino group to a pendant donor chromophore group; n is an integer from 2 to 20 (such as 2 to 10, 3 to 10, 4 to 10 or 5 to 10, e.g., n is 2, 3, 4, 5, 6, 7, 8, 9 or 10); and C is a cysteine residue or a protected cysteine residue.

In some embodiments, the polymeric backbone of the multichromophore has, or is derived from, one or more of the following polypeptide sequence segments:
OCOO (SEQ ID NO: 22)
OOCO (SEQ ID NO:23)
OOCOO (SEQ ID NO: 24)
OOOCOO (SEQ ID NO: 25)
OOCOOO (SEQ ID NO: 26)
OOOCOOO (SEQ ID NO: 27)
OOOCOOOO (SEQ ID NO: 28)
OOOOCOOO (SEQ ID NO: 29)
OOOOCOOOO (SEQ ID NO: 30)
OOOOOCOOOOO (SEQ ID NO: 31)
OOOOOOCOOOOOO (SEQ ID NO: 32)
OOOOOOOCOOOOOOO (SEQ ID NO: 33)
OOOOOOOOCOOOOOOOO (SEQ ID NO: 34)
OOOOOOOOOCOOOOOOOOO (SEQ ID NO: 35)
OOOOOOOOOOCOOOOOOOOOO (SEQ ID NO: 36)
C(O)ₙC (SEQ ID NO: 37)
OC(O)ₙCO (SEQ ID NO: 38)
OOC(O)ₙCOO (SEQ ID NO: 39)
OOOC(O)ₙCOOO (SEQ ID NO: 40)
OOOOC(O)ₙCOOOO (SEQ ID NO: 41)
OOOOOC(O)ₙCOOOOO (SEQ ID NO: 42)
wherein:
O is an ornithine residue, a protected ornithine residue, or an ornithine residue covalently linked via the sidechain amino group to a pendant donor chromophore group; n is an integer from 2 to 20 (such as 2 to 10, 3 to 10, 4 to 10 or 5 to 10, e.g., n is 2, 3, 4, 5, 6, 7, 8, 9 or 10); and C is a cysteine residue or a protected cysteine residue.

In some cases of the subject tandem dyes, the polymeric backbone is a peptoid backbone. As such, the chromophore of formula (VA) can have formula (VA2): 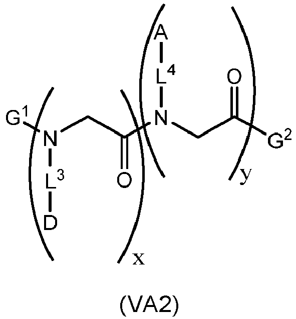 wherein
each D is independently a pendant donor chromophore group (e.g., as described herein);
each A is independently an acceptor fluorophore;
each L³ and L⁴ is independently a linker;
x is 75 mol% or more;
y is 25 mol% or less; and
G¹ and G² are each independently selected from a terminal group, a polymer segment, a light absorbing (e.g., donor) chromophore group, an acceptor fluorophore, a linker and a linked specific binding member. It is understood that the chromophores described by formula (VA2) can include any convenient arrangements of co-monomers in a defined linear sequence, which have in total the defined mol% ratios of x and y. In some cases, the A containing co-monomers are spaced throughout the sequence of the polymeric backbone and as such are always flanked on both sides by one or more D containing co-monomers.

In some instances of formulae (VA2), x is 80 mol% or more, such as 85 mol% or more, 90 mol% or more, 95 mol% or more, 96 mol% or more, 97 mol% or more, 98 mol% or more, or 99 mol% or more. In some instances of formula (VA2), y is 20 mol% or less, such as 15 mol% or less, 10 mol% or less, 5 mol% or less, 4 mol% or less, 3 mol% or less, 2 mol% or less, 1 mol% or less.

As discussed above, the subject tandem dyes provide for a donor chromophore having an absorption maxima of from 555 nm to 585 nm, such as 555 nm to 575 nm, such as 555 to 575 nm, such as 555 to 574 nm, 555 to 573 nm, 555 to 572 nm, 555 to 571 nm, 555 to 570 nm, 555 to 569 nm, 555 to 568 nm, 555 to 567 nm, 555 to 566 nm, 555 to 565 nm, 555 to 564 nm, 555 to 563 nm, or 555 to 562 nm. In certain cases of the tandem dye, the donor chromophore has an absorption maxima of from 558 to 568 nm, such as 558 to 567 nm, 558 to 566 nm, 558 to 565 nm, 558 to 564 nm, 558 to 563 nm, or 558 to 562 nm. In certain cases of the tandem dye, the donor chromophore has an absorption maxima of about 561 nm.

A variety of emission profiles which depend on a variety of factors such as the selected co-monomers, linking groups, substituents and linked acceptor fluorophores of which the tandem dyes are composed. In some embodiments, the chromophore has an emission maximum wavelength in the range of 300 to 900 nm, such as 350 to 850 nm, 350 to 600 nm, 360 to 500 nm, 370 to 500 nm, 380 to 500 nm, 390 to 500 nm or 400 to 500 nm, where specific examples of emission maxima of interest include, but are not limited to: 395 nm ± 5nm, 460nm ± 5nm, 490nm ± 5nm, 550nm ± 5nm, 560nm ± 5nm, 605nm ± 5nm, 650nm ± 5nm, 680nm ± 5nm, 700nm ± 5nm, 805nm ± 5nm. In certain instances, the chromophore has an emission maximum wavelength selected from the group consisting of 395 nm, 460nm, 490nm, 550nm, 560nm, 605nm, 650nm, 680nm, 700nm and 805nm. In certain instances, the chromophore has an emission maximum wavelength of 395 nm ± 5nm. In some instances, the chromophore itself has an emission maximum wavelength in the range of 375 to 900 nm (such as in the range of 380nm to 900nm, 390nm to 900 nm, or 400nm to 900nm).

In certain embodiments of the subject tandem dyes, the ratio of donor chromophores to acceptor fluorophores is selected from 5:1, 6:1, 7:1, 8:1, 9:1, 10:1, and 20:2. In certain cases, the ratio of donor chromophores to acceptor fluorophores is 5:1. In certain cases, the ratio of donor chromophores to acceptor fluorophores is 6:1. In certain cases, the ratio of donor chromophores to acceptor fluorophores is 7:1. In certain cases, the ratio of donor chromophores to acceptor fluorophores is 8:1. In certain cases, the ratio of donor chromophores to acceptor fluorophores is 9:1. In certain cases, the ratio of donor chromophores to acceptor fluorophores is 10:1.

In certain embodiments of the tandem dye, the light harvesting chromophore further comprises a specific binding member. In certain instances of the tandem dye, the specific binding member is selected from an antibody, an antibody fragment or a binding derivative thereof (e.g., as described herein below). In certain instances of the tandem dye, the specific binding member is linked to a terminal group of the light harvesting chromophore. In certain other instances of the tandem dye, the specific binding member is linked to the backbone, at any point between the terminal groups of the light harvesting chromophore. In certain cases, the specific binding member is linked to the backbone at a central point of the light harvesting chromophore.

### End Groups

Any convenient end groups (e.g., G¹ and G²) may be utilized at the terminals of the subject tandem dyes. As used herein, the terms "end group" and "terminal group" are used interchangeably to refer to the groups located at the terminals of the polymeric structure of the light harvesting chromophore, e.g., as described herein. G¹ and G² groups of interest include, but are not limited to a terminal capping group, a π conjugated segment, a linker and a linked specific binding member. In some embodiments, a terminal capping groups is a monovalent group which is conjugated to the backbone of the light harvesting chromophore after polymerization. In certain instances, the terminal capping group is an aryl, a substituted aryl, a heteroaryl, a substituted heteroaryl, an alkyl or a substituted alkyl. In some cases, the terminal co-monomer is directed linked to a chemoselective tag or linker. In certain cases, the terminal capping group is derived from a monomer used in the method of polymerization, e.g., a terminal group such as a halogen (e.g., Br), a boronic acid or a boronic ester, which is capable of undergoing further conjugation. In some instances, G¹ and/or G² is a π conjugated segment. As used herein, a π conjugated segment refers to any convenient segment of a conjugated polymer to which the light harvesting chromophore may be conjugated, i.e., allowing delocalization of pi electron across adjacent units. In certain embodiments, G¹ and/or G² is a linker, such as a linker including a functional group suitable for conjugation to a specific binding moiety. It is understood that linkers located at the G¹ and/or G² positions of the tandem dye may be selected so as to be orthogonal to any other linkers including chemoselective tags (e.g., as described herein) that may be present at a sidechain of the light harvesting chromophore. In certain embodiments, an amino functional group or derivative thereof is included at G¹ and/or G². In certain embodiments, a carboxylic acid functional group or derivative thereof is included at G¹ and/or G².

In some embodiments of the formulae described herein, at least one of G¹ and G² is -L⁶-Z⁴ where L⁶ is a linker (e.g., as described herein) and Z⁴ is a specific binding member (e.g., as described herein). In some embodiments of formulae described herein, at least one of G¹ and G² is -L⁶-Z³ where L⁶ is a linker (e.g., as described herein) and Z³ is a chemoselective tag (e.g., as described herein). Any convenient chemoselective tag and conjugation chemistries can be adapted for use in the subject light harvesting chromophores. Chemoselective tags of interest include, but are not limited to, amine, active ester, maleimide, thiol, sulfur(VI) fluoride exchange chemistry (SuFEX), sulfonyl fluoride, Diers Alder cycloaddition click reagents and click chemistry, tetrazine, transcyclooctene, aldehyde, alkoxylamine, alkynes, cyclooctynes, azide, and the like. In some instances, Z³ is selected from the group consisting of carboxylic acid, active ester (e.g., N-hydroxy succinimidyl ester (NHS) or sulfo-NHS), amino, maleimide, iodoacetyl and thiol. In certain embodiments of formulae described herein, at least one of G¹ and G² is described by the following structure:

^{∗}-Ar-L-Z

where Ar is a π-conjugated aryl group, L is a linker and Z is a chemoselective tag or a specific binding member. In some cases, the L-Z group can be connected directed to a terminal co-monomer. In certain embodiments of formulae described herein, at least one of G¹ and G² is described by the following structure: 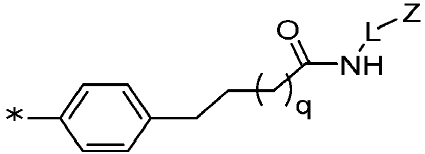 wherein:
q is 0 or an integer from 1-12;
L is an optional linker; and
Z is a chemoselective tag or a specific binding member.

In certain embodiments, Z is a biomolecule. Biomolecules of interest include, but are not limited to, polypeptides, polynucleotides, carbohydrates, fatty acids, steroids, purines, pyrimidines, derivatives, structural analogs thereof and combinations thereof. In certain instances, Z is an antibody. In some instances, Z is an antibody fragment or binding derivative thereof. In some cases, the antibody fragment or binding derivative thereof is selected from the group consisting of a Fab fragment, a F(ab')₂ fragment, a scFv, a diabody and a triabody.

It is understood that for any of the structures and formula depicted herein that in some cases of the subject light harvesting chromophore the end groups depicted may be located at the opposite ends to those shown, e.g., the end groups G¹ and - Ph-L-Z may be switched. In some embodiments of the light harvesting chromophores described herein, at least one end group (e.g., G¹, G²), or sidechain group, is selected from one of the following structures: 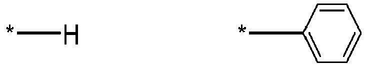 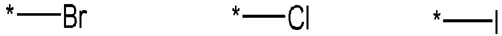 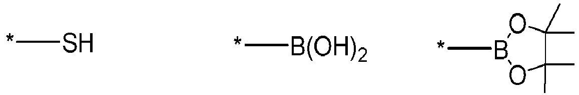 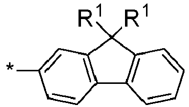 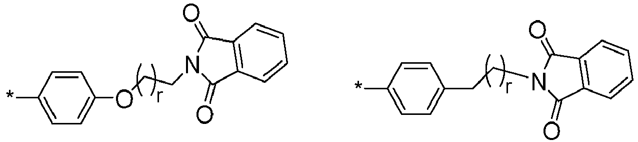 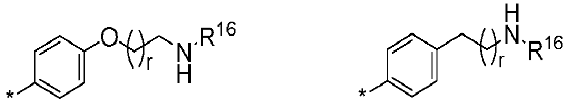 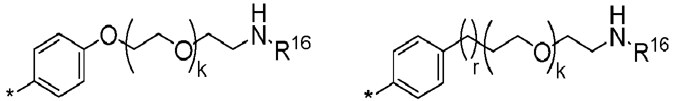 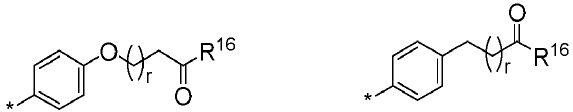 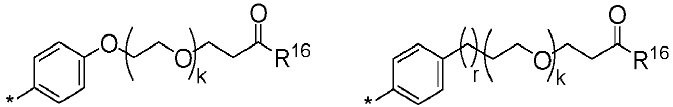 wherein r is 0 or an integer from 1-50; k is 0 or an integer from 1-50 (e.g., 1-20); R¹ is as defined for any of the fluorene co-monomers described herein; and R¹⁶ is selected from H, OH, NH₂, -NH(CH₂)r-NH₂, and -NH(CH₂)ᵣCOOH. In certain instances, r is 1 to 20, such as 3 to 20, 3 to 15, 3 to 12, or 6 to 12. In certain cases, r is 1, 2, 3, 4, 5, 6, 7, 8, 9, 10, 11 or 12. In some cases, r is 3. In some cases, r is 4. In some cases, r is 5. In some cases, r is 6. In some cases, r is 7. In some cases, r is 8. In some cases, r is 9. In some cases, r is 10. In some cases, r is 11.

In some embodiments, the tandem dye includes one or more of the following groups, e.g., as an end group or sidechain group for conjugation to a compatible functional group on another molecule:

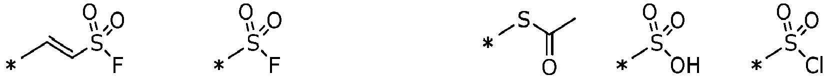

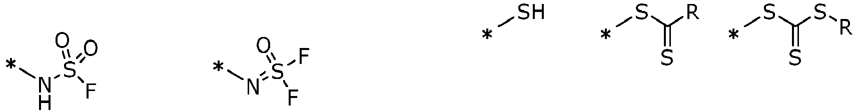

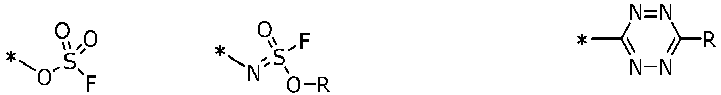

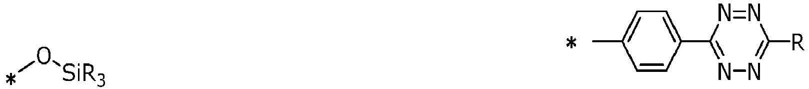

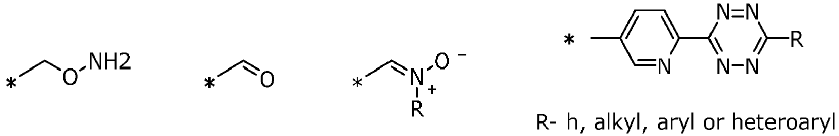

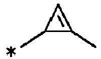

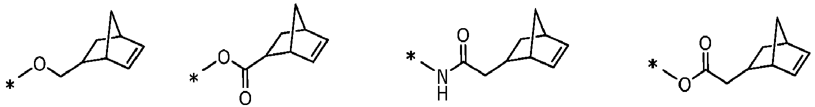

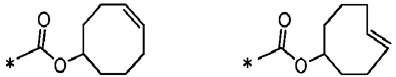

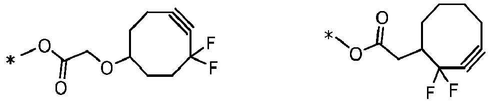

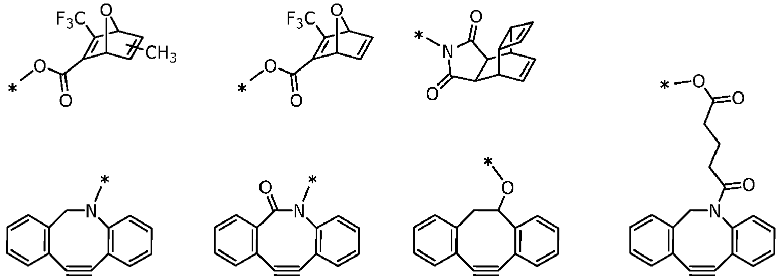

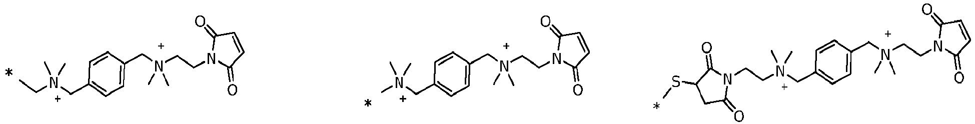

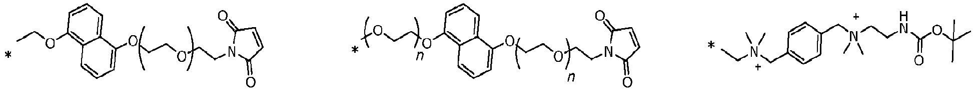

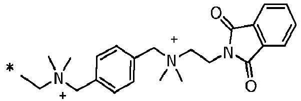

### Labelled Specific Binding Members

Aspects of the present disclosure include labelled specific binding members. A labelled specific binding member is a conjugate of a subject polymeric dye (e.g., as described herein) and a specific binding member. Any of the polymeric dyes or polymeric tandem dyes described herein may be conjugated to a specific binding member. The specific binding member and the light harvesting chromophore can be conjugated (e.g., covalently linked) to each other at any convenient locations of the two molecules, via an optional linker.

In some embodiments, the labelled specific binding member is aggregation resistant. As used herein, by "aggregation-resistant" is meant a labelled specific binding member capable of forming a homogenous aqueous composition without aggregated precipitate at a concentration of 1mg/ml or more in an aqueous buffer of interest, such as 2mg/ml or more, 3mg/ml or more, 4mg/ml or more, 5mg/ml or more, 6mg/ml or more, 7mg/ml or more, 8mg/ml or more, 9mg/ml or more, 10mg/mL or more or even more of the labelled specific binding member. In certain embodiments, the aggregation-resistant labelled specific binding member comprises: a polymeric tandem dye including a light harvesting chromophore (e.g., as described herein) and a acceptor fluorophore linked to the chromophore in energy-receiving proximity therewith; and a specific binding member covalently linked to the chromophore.

As used herein, the term "specific binding member" refers to one member of a pair of molecules which have binding specificity for one another. One member of the pair of molecules may have an area on its surface, or a cavity, which specifically binds to an area on the surface of, or a cavity in, the other member of the pair of molecules. Thus the members of the pair have the property of binding specifically to each other to produce a binding complex. In some embodiments, the affinity between specific binding members in a binding complex is characterized by a K_{d} (dissociation constant) of 10⁻⁶ M or less, such as 10⁻⁷ M or less, including 10⁻⁸ M or less, e.g., 10⁻⁹ M or less, 10⁻¹⁰ M or less, 10⁻¹¹ M or less, 10⁻¹² M or less, 10⁻¹³ M or less, 10⁻¹⁴ M or less, including 10⁻¹⁵ M or less. In some embodiments, the specific binding members specifically bind with high avidity. By high avidity is meant that the binding member specifically binds with an apparent affinity characterized by an apparent K_{d} of 10 × 10⁻⁹ M or less, such as 1× 10⁻⁹ M or less, 3 × 10⁻¹⁰ M or less, 1 × 10⁻¹⁰ M or less, 3 × 10⁻¹¹ M or less, 1 × 10⁻¹¹ M or less, 3 × 10⁻¹² M or less or 1 × 10⁻¹² M or less.

The specific binding member can be proteinaceous. As used herein, the term "proteinaceous" refers to a moiety that is composed of amino acid residues. A proteinaceous moiety can be a polypeptide. In certain cases, the proteinaceous specific binding member is an antibody. In certain embodiments, the proteinaceous specific binding member is an antibody fragment, e.g., a binding fragment of an antibody that specific binds to a polymeric dye. As used herein, the terms "antibody" and "antibody molecule" are used interchangeably and refer to a protein consisting of one or more polypeptides substantially encoded by all or part of the recognized immunoglobulin genes. The recognized immunoglobulin genes, for example in humans, include the kappa (k), lambda (I), and heavy chain genetic loci, which together comprise the myriad variable region genes, and the constant region genes mu (u), delta (d), gamma (g), sigma (e), and alpha (a) which encode the IgM, IgD, IgG, IgE, and IgA isotypes respectively. An immunoglobulin light or heavy chain variable region consists of a "framework" region (FR) interrupted by three hypervariable regions, also called "complementarity determining regions" or "CDRs". The extent of the framework region and CDRs have been precisely defined (see, "Sequences of Proteins of Immunological Interest," E. Kabat et al., U.S. Department of Health and Human Services, (1991)). The numbering of all antibody amino acid sequences discussed herein conforms to the Kabat system. The sequences of the framework regions of different light or heavy chains are relatively conserved within a species. The framework region of an antibody, that is the combined framework regions of the constituent light and heavy chains, serves to position and align the CDRs. The CDRs are primarily responsible for binding to an epitope of an antigen. The term antibody is meant to include full length antibodies and may refer to a natural antibody from any organism, an engineered antibody, or an antibody generated recombinantly for experimental, therapeutic, or other purposes as further defined below.

Antibody fragments of interest include, but are not limited to, Fab, Fab', F(ab')2, Fv, scFv, or other antigen-binding subsequences of antibodies, either produced by the modification of whole antibodies or those synthesized de novo using recombinant DNA technologies. Antibodies may be monoclonal or polyclonal and may have other specific activities on cells (e.g., antagonists, agonists, neutralizing, inhibitory, or stimulatory antibodies). It is understood that the antibodies may have additional conservative amino acid substitutions which have substantially no effect on antigen binding or other antibody functions.

In certain embodiments, the specific binding member is a Fab fragment, a F(ab')₂ fragment, a scFv, a diabody or a triabody. In certain embodiments, the specific binding member is an antibody. In some cases, the specific binding member is a murine antibody or binding fragment thereof. In certain instances, the specific binding member is a recombinant antibody or binding fragment thereof.

### METHODS

As summarized above, aspects of the invention include methods of evaluating a sample for the presence of a target analyte. Aspects of the method include contacting the sample with a polymeric dye conjugate that specifically binds the target analyte to produce a labelling composition contacted sample. As used herein, the terms "polymeric dye conjugate" and "labelled specific binding member" are used interchangeably. As such, the polymeric dye conjugate can include: (i) a water solvated polymeric tandem dye (e.g., as described herein); and (ii) a specific binding member (e.g., as described herein). In some instances, the polymeric tandem dye conjugate includes an acceptor fluorophore (e.g., as described herein) covalently linked to a chromophore of the polymeric dye in energy-receiving proximity therewith.

Any convenient method may be used to contact the sample with a polymeric dye conjugate that specifically binds to the target analyte to produce the labelling composition contacted sample. In some instances, the sample is contacted with the polymeric dye conjugate under conditions in which the specific binding member specifically binds to the target analyte, if present. For specific binding of the specific binding member of the conjugate with the target analyte, an appropriate solution may be used that maintains the biological activity of the components of the sample and the specific binding member. The solution may be a balanced salt solution, e.g., normal saline, PBS, Hank's balanced salt solution, etc., conveniently supplemented with fetal calf serum, human platelet lysate or other factors, in conjunction with an acceptable buffer at low concentration, such as from 5-25 mM. Convenient buffers include HEPES, phosphate buffers, lactate buffers, etc. Various media are commercially available and may be used according to the nature of the target analyte, including dMEM, HBSS, dPBS, RPMI, Iscove's medium, etc., in some cases supplemented with fetal calf serum or human platelet lysate. The final components of the solution may be selected depending on the components of the sample which are included.

The temperature at which specific binding of the specific binding member of the conjugate to the target analyte takes place may vary, and in some instances may range from 5°C to 50°C, such as from 10°C to 40°C, 15°C to 40° C, 20° C to 40°C, e.g., 20°C, 25°C, 30°C, 35°C or 37°C (e.g., as described above). In some instances, the temperature at which specific binding takes place is selected to be compatible with the biological activity of the specific binding member and/or the target analyte. In certain instances, the temperature is 25°C, 30° C, 35° C or 37° C. In certain cases, the specific binding member is an antibody or fragment thereof and the temperature at which specific binding takes place is room temperature (e.g., 25°C), 30° C, 35° C or 37° C. Any convenient incubation time for specific binding may be selected to allow for the formation of a desirable amount of binding complex, and in some instances, may be 1 minute (min) or more, such as 2 min or more, 10 min or more, 30 min or more, 1 hour or more, 2 hours or more, or even 6 hours or more.

Any convenient specific binding members may be utilized in the conjugate. Specific binding members of interest include, but are not limited to, those agents that specifically bind cell surface proteins of a variety of cell types, including but not limited to, stem cells, e.g., pluripotent stem cells, hematopoietic stem cells, T cells, T regulator cells, dendritic cells, B Cells, e.g., memory B cells, antigen specific B cells, granulocytes, leukemia cells, lymphoma cells, virus cells (e.g., HIV cells) NK cells, macrophages, monocytes, fibroblasts, epithelial cells, endothelial cells, and erythroid cells. Target cells of interest include cells that have a convenient cell surface marker or antigen that may be captured by a convenient specific binding member conjugate. In some embodiments, the target cell is selected from HIV containing cell, a Treg cell, an antigen-specific T -cell populations, tumor cells or hematopoetic progenitor cells (CD34+) from whole blood, bone marrow or cord blood. Any convenient cell surface proteins or cell markers may be targeted for specific binding to polymeric dye conjugates in the subject methods. In some embodiments, the target cell includes a cell surface marker selected from a cell receptor and a cell surface antigen. In some cases, the target cell may include a cell surface antigen such as CD11b, CD123, CD14, CD15, CD16, CD19, CD193, CD2, CD25, CD27, CD3, CD335, CD36, CD4, CD43, CD45RO, CD56, CD61, CD7, CD8, CD34, CD1c, CD23, CD304, CD235a, T cell receptor alpha/beta, T cell receptor gamma/delta, CD253, CD95, CD20, CD105, CD117, CD120b, Notch4, Lgr5 (N-Terminal), SSEA-3, TRA-1-60 Antigen, Disialoganglioside GD2 and CD71.

Any convenient targets may be selected for evaluation utilizing the subject methods. Targets of interest include, but are not limited to, a nucleic acid, such as an RNA, DNA, PNA, CNA, HNA, LNA or ANA molecule, a protein, such as a fusion protein, a modified protein, such as a phosphorylated, glycosylated, ubiquitinated, SUMOylated, or acetylated protein, or an antibody, a peptide, an aggregated biomolecule, a cell, a small molecule, a vitamin and a drug molecule. As used herein, the term "a target protein" refers to all members of the target family, and fragments thereof. The target protein may be any protein of interest, such as a therapeutic or diagnostic target, including but not limited to: hormones, growth factors, receptors, enzymes, cytokines, osteoinductive factors, colony stimulating factors and immunoglobulins. The term "target protein" is intended to include recombinant and synthetic molecules, which can be prepared using any convenient recombinant expression methods or using any convenient synthetic methods, or purchased commercially. In some embodiments, the polymeric dye conjugates include an antibody or antibody fragment. Any convenient target analyte that specifically binds an antibody or antibody fragment of interest may be targeted in the subject methods.

In some embodiments, the target analyte is associated with a cell. In certain instances, the target analyte is a cell surface marker of the cell. In certain cases, the cell surface marker is selected from the group consisting of a cell receptor and a cell surface antigen. In some instances, the target analyte is an intracellular target, and the method further includes lysing the cell.

In some embodiments, the sample may include a heterogeneous cell population from which target cells are isolated. In some instances, the sample includes peripheral whole blood, peripheral whole blood in which erythrocytes have been lysed prior to cell isolation, cord blood, bone marrow, density gradient-purified peripheral blood mononuclear cells or homogenized tissue. In some cases, the sample includes hematopoetic progenitor cells (e.g., CD34+ cells) in whole blood, bone marrow or cord blood. In certain embodiments, the sample includes tumor cells in peripheral blood. In certain instances, the sample is a sample including (or suspected of including) viral cells (e.g., HIV).

The labelled specific binding members find use in the subject methods, e.g., for labeling a target cell, particle, target or analyte with a polymeric dye or polymeric tandem dye. For example, labelled specific binding members find use in labeling cells to be processed (e.g., detected, analyzed, and/or sorted) in a flow cytometer. The labelled specific binding members may include antibodies that specifically bind to, e.g., cell surface proteins of a variety of cell types (e.g., as described herein). The labelled specific binding members may be used to investigate a variety of biological (e.g., cellular) properties or processes such as cell cycle, cell proliferation, cell differentiation, DNA repair, T cell signaling, apoptosis, cell surface protein expression and/or presentation, and so forth. Labelled specific binding members may be used in any application that includes (or may include) antibody-mediated labeling of a cell, particle or analyte.

Aspects of the method include assaying the labelling composition contacted sample for the presence of a dye conjugate-target analyte binding complex to evaluate whether the target analyte is present in the sample. Once the sample has been contacted with the dye conjugate, any convenient methods may be utilized in assaying the labelling composition contacted sample that is produced for the presence of a dye conjugate-target analyte binding complex. The dye conjugate-target analyte binding complex is the binding complex that is produced upon specific binding of the specific binding member of the conjugate to the target analyte, if present. Assaying the labelling composition contacted sample can include detecting a fluorescent signal from the binding complex, if present. In some cases, the assaying includes a separating step where the target analyte, if present, is separated from the sample. A variety of methods can be utilized to separate a target analyte from a sample, e.g., via immobilization on a support. Assay methods of interest include, but are not limited to, any convenient methods and assay formats where pairs of specific binding members such as avidin- biotin or hapten-anti-hapten antibodies find use, are of interest. Methods and assay formats of interest that may be adapted for use with the subject compositions include, but are not limited to, flow cytometry methods, in-situ hybridization methods, enzyme-linked immunosorbent assays (ELISAs), western blot analysis, magnetic cell separation assays and fluorochrome purification chromatography.

In certain embodiments, the method further includes contacting the sample with a second specific binding member that specifically binds the target analyte. In certain instances, the second specific binding member is support bound. Any convenient supports may be utilized to immobilize a component of the subject methods (e.g., a second specific binding member). In certain instances, the support is a particle, such as a magnetic particle. In some instances, the second specific binding member and the polymeric dye conjugate produce a sandwich complex that may be isolated and detected, if present, using any convenient methods. In some embodiments, the method further includes flow cytometrically analyzing the polymeric dye conjugate-target analyte binding complex, i.e., a fluorescently labelled target analyte. Assaying for the presence of a polymeric dye conjugate-target analyte binding complex may provide assay results (e.g., qualitative or quantitative assay data) which can be used to evaluate whether the target analyte is present in the sample.

Any convenient supports may be utilized in the subject methods to immobilize any convenient component of the methods, e.g., labelled specific binding member, target, secondary specific binding member, etc. Supports of interest include, but are not limited to: solid substrates, where the substrate can have a variety of configurations, e.g., a sheet, bead, or other structure, such as a plate with wells; beads, polymers, particle, a fibrous mesh, hydrogels, porous matrix, a pin, a microarray surface, a chromatography support, and the like. In some instances, the support is selected from the group consisting of a particle, a planar solid substrate, a fibrous mesh, a hydrogel, a porous matrix, a pin, a microarray surface and a chromatography support. The support may be incorporated into a system that it provides for cell isolation assisted by any convenient methods, such as a manually-operated syringe, a centrifuge or an automated liquid handling system. In some cases, the support finds use in an automated liquid handling system for the high throughput isolation of cells, such as a flow cytometer.

In some embodiments of the method, the separating step includes applying an external magnetic field to immobilize a magnetic particle. Any convenient magnet may be used as a source of the external magnetic field (e.g., magnetic field gradient). In some cases, the external magnetic field is generated by a magnetic source, e.g. by a permanent magnet or electromagnet. In some cases, immobilizing the magnetic particles means the magnetic particles accumulate near the surface closest to the magnetic field gradient source, i.e. the magnet.

The separating may further include one or more optional washing steps to remove unbound material of the sample from the support. Any convenient washing methods may be used, e.g., washing the immobilized support with a biocompatible buffer which preserves the specific binding interaction of the polymeric dye and the specific binding member. Separation and optional washing of unbound material of the sample from the support provides for an enriched population of target cells where undesired cells and material may be removed.

In certain embodiments, the method further includes detecting the labelled target. Detecting the labelled target may include exciting the chromophore with one or more lasers and subsequently detecting fluorescence emission from the polymeric tandem dye using one or more optical detectors. Detection of the labelled target can be performed using any convenient instruments and methods, including but not limited to, flow cytometry, FACS systems, fluorescence microscopy; fluorescence, luminescence, ultraviolet, and/or visible light detection using a plate reader; high performance liquid chromatography (HPLC); and mass spectrometry. When using fluorescently labeled components in the methods and compositions of the present disclosure, it is recognized that different types of fluorescence detection systems can be used to practice the subject methods. In some cases, high throughput screening can be performed, e.g., systems that use 96 well or greater microtiter plates. A variety of methods of performing assays on fluorescent materials can be utilized, such as those methods described in, e.g., Lakowicz, J. R., Principles of Fluorescence Spectroscopy, New York: Plenum Press (1983); Herman, B., Resonance energy transfer microscopy, in: Fluorescence Microscopy of Living Cells in Culture, Part B, Methods in Cell Biology, vol. 30, ed. Taylor, D. L. & Wang, Y.-L., San Diego: Academic Press (1989), pp. 219-243; Turro, N.J., Modern Molecular Photochemistry, Menlo Park: Benjamin/Cummings Publishing Col, Inc. (1978), pp. 296-361.

Fluorescence in a sample can be measured using a fluorimeter. In some cases, excitation radiation, from an excitation source having a first wavelength, passes through excitation optics. The excitation optics cause the excitation radiation to excite the sample. In response, fluorescently labelled targets in the sample emit radiation which has a wavelength that is different from the excitation wavelength. Collection optics then collect the emission from the sample. The device can include a temperature controller to maintain the sample at a specific temperature while it is being scanned. In certain instances, a multi-axis translation stage moves a microtiter plate holding a plurality of samples in order to position different wells to be exposed. The multi-axis translation stage, temperature controller, auto-focusing feature, and electronics associated with imaging and data collection can be managed by an appropriately programmed digital computer. The computer also can transform the data collected during the assay into another format for presentation.

In some embodiments, the method of evaluating a sample for the presence of a target analyte further includes detecting fluorescence in a flow cytometer. In some embodiments, the method of evaluating a sample for the presence of a target analyte further includes imaging the labelling composition contacted sample using fluorescence microscopy. Fluorescence microscopy imaging can be used to identify a polymeric dye conjugate-target analyte binding complex in the contacted sample to evaluate whether the target analyte is present. Microscopy methods of interest that find use in the subject methods include laser scanning confocal microscopy.

Also provided are methods of labelling a target molecule. The subject polymeric tandem dyes find use in a variety of methods of labelling, separation, detection and/or analysis. In some embodiments, the method includes: contacting the target molecule with a polymeric tandem dye (e.g., as described herein) to produce a labelled target molecule, wherein the polymeric tandem dye includes a conjugation tag that covalently links to the target molecule. In some instances of the method, the polymeric dye member includes a chromophore according to any one of formulae (VA)-(VB) (e.g., as described herein), where one of G¹ and G² is a terminal group and the other of G¹ and G² is the conjugation tag.

The term "conjugation tag" refers to a group that includes a chemoselective functional group (e.g., as described herein) that can covalently link with a compatible functional group of a target molecule, after optional activation and/or deprotection. Any convenient conjugation tags may be utilized in the subject polymeric dyes in order to conjugate the dye to a target molecule of interest. In some embodiments, the conjugation tag includes a terminal functional group selected from an amino, a carboxylic acid or a derivative thereof, a thiol, a hydroxyl, a hydrazine, a hydrazide, a azide, an alkyne and a protein reactive group (e.g. amino-reactive, thiol-reactive, hydroxyl-reactive, imidazolyl-reactive or guanidinyl-reactive).

Any convenient methods and reagent may be adapted for use in the subject labelling methods in order to covalently link the conjugation tag to the target molecule. Methods of interest for labelling a target, include but are not limited to, those methods and reagents described by Hermanson, Bioconjugate Techniques, Third edition, Academic Press, 2013. The contacting step may be performed in an aqueous solution. In some instances, the conjugation tag includes an amino functional group and the target molecule includes an activated ester functional group, such as a NHS ester or sulfo-NHS ester, or vice versa. In certain instances, the conjugation tag includes a maleimide functional group and the target molecule includes a thiol functional group, or vice versa. In certain instances, the conjugation tag includes an alkyne (e.g., a cyclooctyne group) functional group and the target molecule includes an azide functional group, or vice versa, which can be conjugated via Click chemistry.

Any convenient target molecules may be selected for labelling utilizing the subject methods. Target molecules of interest include, but are not limited to, a nucleic acid, such as an RNA, DNA, PNA, CNA, HNA, LNA or ANA molecule, a protein, such as a fusion protein, a modified protein, such as a phosphorylated, glycosylated, ubiquitinated, SUMOylated, or acetylated protein, or an antibody, a peptide, an aggregated biomolecule, a cell, a small molecule, a vitamin and a drug molecule. As used herein, the term "a target protein" refers to all members of the target family, and fragments thereof. The target protein may be any protein of interest, such as a therapeutic or diagnostic target, including but not limited to: hormones, growth factors, receptors, enzymes, cytokines, osteoinductive factors, colony stimulating factors and immunoglobulins. The term "target protein" is intended to include recombinant and synthetic molecules, which can be prepared using any convenient recombinant expression methods or using any convenient synthetic methods, or purchased commercially. In some embodiments, the target molecule is a specific binding member (e.g., as described herein). In certain instances, the specific binding member is an antibody. In some instances, the specific binding member is an antibody fragment or binding derivative thereof. In some case, the antibody fragment or binding derivative thereof is selected from the group consisting of a Fab fragment, a F(ab')₂ fragment, a scFv, a diabody and a triabody.

In some cases, the method includes a separating step where the labelled target molecule is separated from the reaction mixture, e.g., excess reagents or unlabeled target. A variety of methods may be utilized to separate a target from a sample, e.g., via immobilization on a support, precipitation, chromatography, and the like.

In some instances, the method further includes detecting and/or analyzing the labelled target molecule. In some instances, the method further includes fluorescently detecting the labelled target molecule. Any convenient methods may be utilized to detect and/or analyze the labelled target molecule in conjunction with the subject methods and compositions. Methods of analyzing a target of interest that find use in the subject methods, include but are not limited to, flow cytometry, fluorescence microscopy, in-situ hybridization, enzyme-linked immunosorbent assays (ELISAs), western blot analysis, magnetic cell separation assays and fluorochrome purification chromatography. Detection methods of interest include but are not limited to fluorescence spectroscopy, fluorescence microscopy, nucleic acid sequencing, fluorescence in-situ hybridization (FISH), protein mass spectroscopy, flow cytometry, and the like.

Detection may be achieved directly via the polymeric tandem dye, or indirectly by a secondary detection system. The latter may be based on any one or a combination of several different principles including, but not limited to, antibody labelled anti-species antibody and other forms of immunological or non-immunological bridging and signal amplification systems (e.g., biotin-streptavidin technology, protein-A and protein-G mediated technology, or nucleic acid probe/anti-nucleic acid probes, and the like). Suitable reporter molecules may be those known in the field of immunocytochemistry, molecular biology, light, fluorescence, and electron microscopy, cell immunophenotyping, cell sorting, flow cytometry, cell visualization, detection, enumeration, and/or signal output quantification. More than one antibody of specific and/or non-specific nature might be labelled and used simultaneously or sequentially to enhance target detection, identification, and/or analysis.

### SYSTEMS

Aspects of the invention further include systems for use in practicing the subject methods and compositions. A sample analysis system can include sample field of view or a flow channel loaded with a sample and a labelled specific binding member. In some embodiments, the system is a flow cytometric system including: a flow cytometer including a flow path; a composition in the flow path, wherein the composition includes: a sample; and a labelled specific binding member (e.g., as described herein).

In some embodiments, the system for analyzing a sample is a fluorescence microscopy system, including: a fluorescence microscope comprising a sample field of view; and a composition disposed in the sample field of view, wherein the composition comprises a sample; and a labelled specific binding member (e.g., as described herein).

In some instances of the systems, the labelled specific binding member includes: a tandem dye (e.g., as described herein) and a specific binding member that specifically binds a target analyte covalently linked to the chromophore. In some instances of the subject systems, the labelled specific binding member, the tandem dye is described by formula (VA) or (VB) (e.g., as described herein), wherein: G¹ and G² are each independently selected from the group consisting of a terminal group, a π conjugated segment, a linker and a linked specific binding member, wherein at least one of G¹ and G² is a linked specific binding member.

In certain embodiments of the systems, the composition further includes a second specific binding member that is support bound and specifically binds the target analyte. In some cases, the support includes a magnetic particle. As such, in certain instances, the system may also include a controllable external paramagnetic field configured for application to an assay region of the flow channel.

The sample may include a cell. In some instances, the sample is a cell-containing biological sample. In some instances, the sample includes a labelled specific binding member specifically bound to a target cell. In certain instances, the target analyte that is specifically bound by the specific binding member is a cell surface marker of the cell. In certain cases, the cell surface marker is selected from a cell receptor and a cell surface antigen.

In certain aspects, the system may also include a light source configured to direct light to an assay region of the flow channel or sample field of view. The system may include a detector configured to receive a signal from an assay region of the flow channel or a sample field of view, wherein the signal is provided by the fluorescent composition. Optionally further, the sample analysis system may include one or more additional detectors and/or light sources for the detection of one or more additional signals.

In certain aspects, the system may further include computer-based systems configured to detect the presence of the fluorescent signal. A "computer-based system" refers to the hardware means, software means, and data storage means used to analyze the information of the present invention. The minimum hardware of the computer-based systems of the present invention includes a central processing unit (CPU), input means, output means, and data storage means. A skilled artisan can readily appreciate that any one of the currently available computer-based system are suitable for use in the subject systems. The data storage means may include any manufacture including a recording of the present information as described above, or a memory access means that can access such a manufacture.

To "record" data, programming or other information on a computer readable medium refers to a process for storing information, using any such methods as known in the art. Any convenient data storage structure may be chosen, based on the means used to access the stored information. A variety of data processor programs and formats can be used for storage, e.g., word processing text file, database format, etc.

A "processor" references any hardware and/or software combination that will perform the functions required of it. For example, any processor herein may be a programmable digital microprocessor such as available in the form of an electronic controller, mainframe, server or personal computer (desktop or portable). Where the processor is programmable, suitable programming can be communicated from a remote location to the processor, or previously saved in a computer program product (such as a portable or fixed computer readable storage medium, whether magnetic, optical or solid state device based). For example, a magnetic medium or optical disk may carry the programming, and can be read by a suitable reader communicating with each processor at its corresponding station.

In addition to the sensor device and signal processing module, e.g., as described above, systems of the invention may include a number of additional components, such as data output devices, e.g., monitors and/or speakers, data input devices, e.g., interface ports, keyboards, etc., fluid handling components, power sources, etc.

In certain aspects, the system includes a flow cytometer. Flow cytometers of interest include, but are not limited, to those devices described in U.S. Patent Nos.: 4,704,891; 4,727,029; 4,745,285; 4,867,908; 5,342,790; 5,620,842; 5,627,037; 5,701,012; 5,895,922; and 6,287,791; the disclosures of which are herein incorporated by reference.

Other systems may find use in practicing the subject methods. In certain aspects, the system may be a fluorimeter or microscope loaded with a sample having a fluorescent composition of any of the embodiments discussed herein. The fluorimeter or microscope may include a light source configured to direct light to the assay region of the flow channel or sample field of view. The fluorimeter or microscope may also include a detector configured to receive a signal from an assay region of the flow channel or field of view, wherein the signal is provided by the fluorescent composition.

### KITS

Aspects of the invention further include kits for use in practicing the subject methods and compositions. The compositions of the invention can be included as reagents in kits either as starting materials or provided for use in, for example, the methodologies described above.

A kit can include a tandem dye including a water-soluble dipyrromethene-based dye (e.g., as described herein) and a container. In other embodiments, a kit can include a water-soluble dipyrromethene-based dye (e.g., as described herein) and a container. Any convenient containers can be utilized, such as tubes, bottles, or wells in a multi-well strip or plate, a box, a bag, an insulated container, and the like. The subject kits can further include one or more components selected from a polymeric tandem dye, a fluorophore, a specific binding member, a specific binding member conjugate, a support bound specific binding member, a cell, a support, a biocompatible aqueous elution buffer, and instructions for use. In some embodiments of the kit, the light harvesting chromophore is covalently linked to a specific binding member. In some instances, the specific binding member is an antibody. In certain instances, the specific binding member is an antibody fragment or binding derivative thereof. In certain cases, the antibody fragment or binding derivative thereof is selected from the group consisting of a Fab fragment, a F(ab')2 fragment, a scFv, a diabody and a triabody.

In certain embodiments, the kit finds use in evaluating a sample for the presence of a target analyte, such as an intracellular target. As such, in some instances, the kit includes one or more components suitable for lysing cells. The one or more additional components of the kit may be provided in separate containers (e.g., separate tubes, bottles, or wells in a multi-well strip or plate).

In certain aspects, the kit further includes reagents for performing a flow cytometric assay. Reagents of interest include, but are not limited to, buffers for reconstitution and dilution, buffers for contacting a cell sample the chromophore, wash buffers, control cells, control beads, fluorescent beads for flow cytometer calibration and combinations thereof. The kit may also include one or more cell fixing reagents such as paraformaldehyde, glutaraldehyde, methanol, acetone, formalin, or any combinations or buffers thereof. Further, the kit may include a cell permeabilizing reagent, such as methanol, acetone or a detergent (e.g., triton, NP-40, saponin, tween 20, digitonin, leucoperm, or any combinations or buffers thereof. Other protein transport inhibitors, cell fixing reagents and cell permeabilizing reagents familiar to the skilled artisan are within the scope of the subject kits.

The compositions of the kit may be provided in a liquid composition, such as any suitable buffer. Alternatively, the compositions of the kit may be provided in a dry composition (e.g., may be lyophilized), and the kit may optionally include one or more buffers for reconstituting the dry composition. In certain aspects, the kit may include aliquots of the compositions provided in separate containers (e.g., separate tubes, bottles, or wells in a multi-well strip or plate).

In addition, one or more components may be combined into a single container, e.g., a glass or plastic vial, tube or bottle. In certain instances, the kit may further include a container (e.g., such as a box, a bag, an insulated container, a bottle, tube, etc.) in which all of the components (and their separate containers) are present. The kit may further include packaging that is separate from or attached to the kit container and upon which is printed information about the kit, the components of the and/or instructions for use of the kit.

In addition to the above components, the subject kits may further include instructions for practicing the subject methods. These instructions may be present in the subject kits in a variety of forms, one or more of which may be present in the kit. One form in which these instructions may be present is as printed information on a suitable medium or substrate, e.g., a piece or pieces of paper on which the information is printed, in the packaging of the kit, in a package insert, etc. Yet another means would be a computer readable medium, e.g., diskette, CD, DVD, portable flash drive, etc., on which the information has been recorded. Yet another means that may be present is a website address which may be used via the Internet to access the information at a removed site. Any convenient means may be present in the kits.

### UTILITY

The water-soluble dipyrromethene-based dyes, tandem dyes, compositions, methods and systems as described herein may find use in a variety of applications, including diagnostic and research applications, in which the labelling, detection and/or analysis of a target of interest is desirable. Such applications include methodologies such as cytometry, microscopy, immunoassays (e.g. competitive or non-competitive), assessment of a free analyte, assessment of receptor bound ligand, and so forth. The compositions, system and methods described herein may be useful in analysis of any of a number of samples, including but not limited to, biological fluids, cell culture samples, and tissue samples. In certain aspects, the compositions, system and methods described herein may find use in methods where analytes are detected in a sample, if present, using fluorescent labels, such as in fluorescent activated cell sorting or analysis, immunoassays, immunostaining, and the like. In certain instances, the compositions and methods find use in applications where the evaluation of a sample for the presence of a target analyte is of interest.

In some cases, the methods and compositions find use in any assay format where the detection and/or analysis of a target from a sample is of interest, including but not limited to, flow cytometry, fluorescence microscopy, in-situ hybridization, enzyme-linked immunosorbent assays (ELISAs), western blot analysis, magnetic cell separation assays and fluorochrome purification chromatography. In certain instances, the methods and compositions find use in any application where the fluorescent labelling of a target molecule is of interest. The subject compositions may be adapted for use in any convenient applications where pairs of specific binding members find use, such as biotin-streptavidin and hapten-anti-hapten antibody.

### EXAMPLES

### Example 1: Synthesis and Characterization of yellow green water-soluble dipyrromethene-based dyes

Example water-soluble dipyrromethene-based dyes can be prepared according to any convenient methods, including those described by Loudet and Burgess ("BODIPY Dyes and their derivative: Synthesis and Spectroscopic properties", Chem. Rev. 2007, 107, 4891-4932). Example yellow green water-soluble dipyrromethene-based dyes are depicted in FIG. 1.

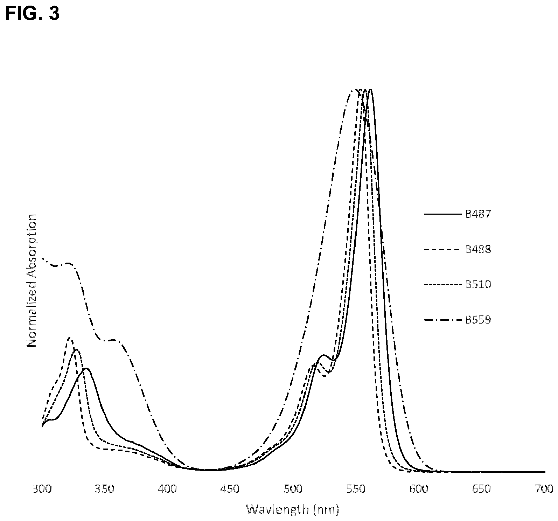

The absorption properties of four example water-soluble dipyrromethene-based dyes (depicted in FIG. 1, and reproduced below) were evaluated, as shown in FIG. 3.

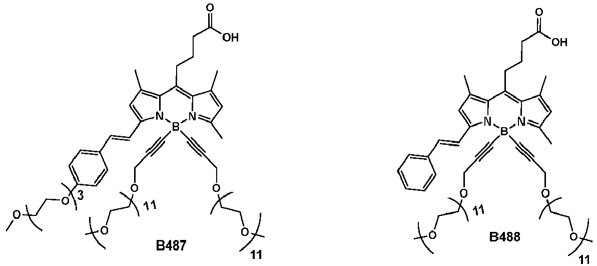

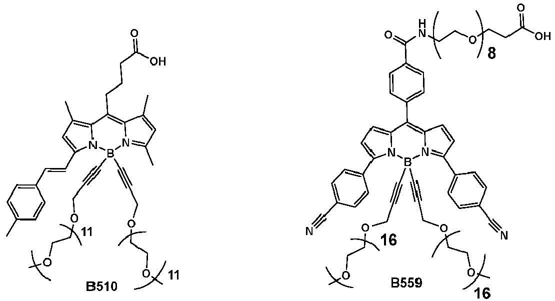

As depicted in FIG. 3, the absorption maxima for each of compounds B487, B488, B510, and B559 is near 561 nm.

The above results are exemplary of the water-soluble dipyrromethene-based dyes that absorb in the yellow green range at 561 nm (e.g., a dye of formulae (I)-(IV)), e.g., as described in the above specification. These dyes have strong absorption at 561 nm while minimizing absorption at other common laser source wavelengths for flow cytometry. Doing so allows for the dye and FRET pairs made from the dye to be excited selectively by a single laser rather than several. In this way, dyes can be made that are excited from the YG laser and emit at a wavelength determined by an attached acceptor. A key advantage of dyes of the invention according to embodiments is the high water solubility imparted to these dyes by attaching polyethylene glycol oligomers. This makes the dye and other moieties attached to it water soluble and increases the quantum yield of the dye in common buffer solutions relative to the non-PEGylated core dye structure.

### Example 2: Preparation of polypeptide light harvesting chromophores

A summary of some of the various methods available for synthesizing the subject polypeptide light harvesting chromophores can be found in Steward et al., in "Solid Phase Peptide Synthesis", W.H. Freeman Co., San Francisco, 1969; Bodanszky et al., in "Peptide Synthesis", John Wiley & Sons, Second Edition, 1976 and Meienhofer, in "Hormonal Proteins and Peptides", Vol. 2, p.46, Academic Press (New York), 1983; and Kent, Ann. Rev. Biochem., 57, 957, 1988, for solid phase peptide synthesis, and Schroder et al., in "The Peptides", Vol. 1, Academic Press (New York), 1965 for solution synthesis. Any convenient protecting group strategies may be used such as, but are not limited to, Fmoc solid-phase peptide synthesis and Boc solid-phase peptide synthesis strategies. In Boc solid-phase peptide synthesis a Boc-amino protecting group is used at the amino terminal and benzyl or benzyl-based or other convenient protecting groups may be used for the protection of sidechain functional groups. In Fmoc solid-phase peptide synthesis a Fmoc-amino protecting group is used at the amino terminal and tert-butyl or benzyl-based or other convenient protecting groups may be used for protection of sidechain functional groups. Convenient protecting groups that may be used in such synthetic methods are described in the above references and by McOmic in "Protective Groups in Organic Chemistry", Plenum Press, New York, 1973; and Greene and Wuts, "Protective Groups in Organic Synthesis", John Wiley & Sons, 4th Edition, 2006.

### Example 3: Preparation of Dyes

Dipyrromethene-based dyes can be prepared according to any convenient methods, including those described by Loudet and Burgess ("BODIPY Dyes and their derivative: Synthesis and Spectroscopic properties", Chem. Rev. 2007, 107, 4891-4932).

Deep learning was used to calculate optical characteristics (e.g. main absorption band) of dyes with BODIPY core structures. Dyes were identified that had calculated absorption maximum values of 562 nm +/- 5 nm, and these candidate dyes were subjected to additional absorption band calculations at a higher degree of refinement. These candidate dyes were experimentally synthesized and their absorption characteristics were measured and compared to predicted values. It was found that the deep learning calculations were surprisingly accurate at predicting the experimentally measured absorption bands of the candidate dyes. The chemical structures of seven candidate dyes are shown below. The candidate dyes were found to have relatively narrow absorption spectra that is well aligned with 562 nm light emitted by a yellow-green laser.

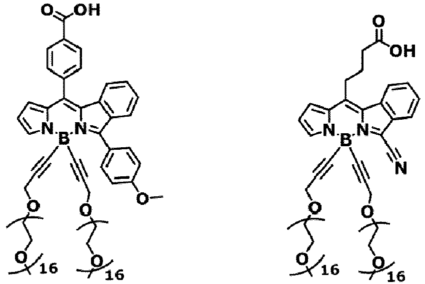

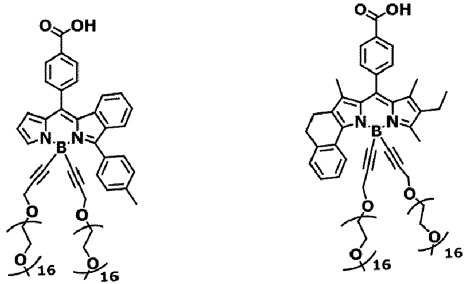

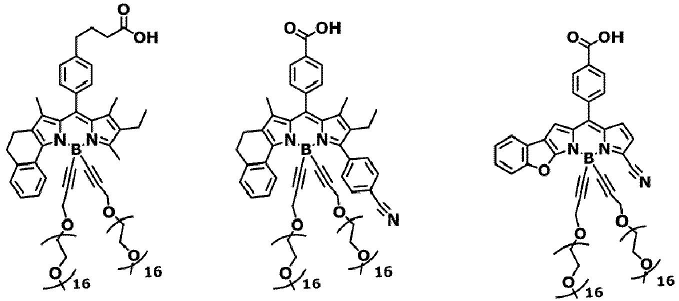

The candidate dyes were also tested for their ability to act as donor chromophores and transfer energy to acceptor chromophores through FRET (Forster resonance energy transfer). The efficiency of energy transfer, as measured by quantum yield, was compared to predicted values for quantum yield. It was found that the predictions were successful in qualitatively predicting the relative quantum yields of the candidate dyes, i.e., the calculated degree of energy transfer showed trends in line with experimental results. Thus, when used as donor chromophores the candidate dyes were found to be part of highly efficient tandem pairs. Calculations suggested that the high quantum yield of the candidate dyes could be caused at least in part by low amounts of ground-state interactions. Additional studies showed that the candidate dyes have good photostability.

Notwithstanding the appended claims, the disclosure is also defined by the following clauses:
1. A water-soluble dipyrromethene-based dye that absorbs in the yellow-green range at 561 nm.
2. The water-soluble dipyrromethene-based dye according to clause 1, wherein the absorption maxima is from 555 to 585 nm.
3. The water-soluble dipyrromethene-based dye of clause 2, wherein the absorption maxima is from 558 to 568 nm.
4. The water-soluble dipyrromethene-based dye of clause 3, wherein the absorption maxima is about 561 nm.
5. The water-soluble dipyrromethene-based dye of any one of clauses 1 to 4, wherein the dye is a dipyrromethene-boron dye.
6. The water-soluble dipyrromethene-based dye of any one of clauses 1 to 5, of formula (I): 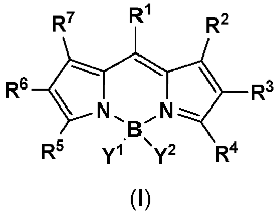 wherein:
   R¹-R⁷ are each independently selected from H, alkyl, halogen, substituted alkyl, alkoxy, substituted alkoxy, amino, substituted amino, amido, substituted amido, thiol, substituted thiol, aryl, substituted aryl, heteroaryl, substituted heteroaryl, heterocycle, substituted heterocycle, alkenyl, substituted alkenyl, alkynyl, substituted alkynyl, water solubilizing group (WSG), and -T¹-J¹, or
   optionally any one or more pairs of substituents selected from R⁶ and R⁷, R² and R³, R⁵ and R⁶, R³ and R⁴, R² and R¹ and R⁷ and R¹, together form a divalent radical and are cyclically linked and together with the carbon atoms to which they are bound provide a 5- or 6-membered fused heterocycle, carbocycle, aryl or heteroaryl ring (e.g., a 5- or 6-membered ring comprising carbon atoms and 0-3 heteroatoms selected from O, S and N), which ring may be unsubstituted or further substituted with a substituent independently selected from alkyl, substituted alkyl, alkoxy, substituted alkoxy, aryl, substituted aryl, heteroaryl, substituted heteroaryl, alkenyl, substituted alkenyl, alkynyl, substituted alkynyl, water solubilizing group (WSG), and -T¹-J¹;
   T¹ is an optional linker;
   J¹ is a repeat unit of a compound of formula (I); and
   Y¹ and Y² are independently selected from F, OH, H, cyano, alkyl, substituted alkyl, aryl, substituted aryl, heteroaryl, substituted heteroaryl, alkoxy, substituted alkoxy, alkenyl, substituted alkenyl, alkynyl, substituted alkynyl, and water solubilizing group (WSG), or
   Y¹ and Y² together with the boron atom to which they are bound form a 5- or 6-membered ring (e.g., a 5- or 6-membered ring comprising a boron atom, carbon atoms and 0-3 heteroatoms selected from O, S and N), which ring may be unsubstituted or further substituted with a substituent independently selected from alkyl, substituted alkyl, alkoxy, substituted alkoxy, aryl, substituted aryl, heteroaryl, substituted heteroaryl, alkenyl, substituted alkenyl, alkynyl, substituted alkynyl, water solubilizing group (WSG), and -T¹-J¹; and
   wherein at least one moiety selected from Y¹, Y² and R¹-R⁷ is a WSG, or at least one moiety selected from Y¹, Y² and R¹-R⁷ is substituted with a WSG, where the WSG is of the formula (la):

      -(L¹)ₙ₁ - (X¹)ₙ₂ - ((L²)ₙ₃-Z¹)ₘ (Ia)

      wherein:
      n1, n2 and n3 are each independently 0 or 1;
      L¹, if present, is a linker;
      L² if present, is a linker;
      X¹, if present, is a branching point;
      Z¹ is a water soluble polymer comprising 6-24 monomeric units; and
      m is an integer from 1 to 3.
7. The water-soluble dipyrromethene-based dye of clause 6, further comprising a WSG of formula (Ib):

   -(L¹)ₙ₁ - (X¹)ₙ₂ - ((L²)ₙ₃-Z²)ₘ (Ib)

   wherein:
   n1, n2 and n3 are each independently 0 or 1;
   L¹, if present, is a linker;
   L² if present, is a linker;
   X¹, if present, is a branching point;
   Z² is a water soluble polymer comprising 1-50 monomeric units; and
   m is an integer from 1 to 3.
8. The water-soluble dipyrromethene-based dye of clause 6 or 7, wherein the water solubilizing group (WSG) comprises a PEG moiety.
9. The water-soluble dipyrromethene-based dye of any one of clauses 6 to 8, wherein one or more water solubilizing groups (WSGs) is independently selected from the following formulae: 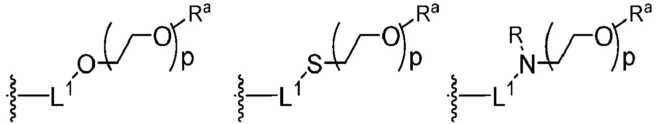 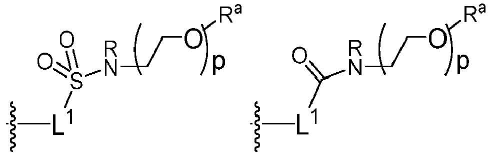 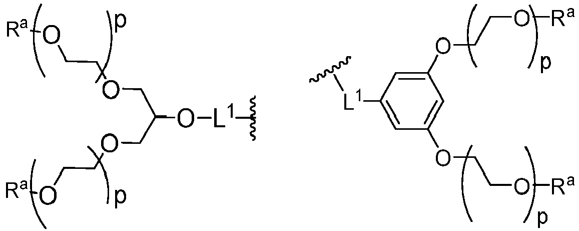 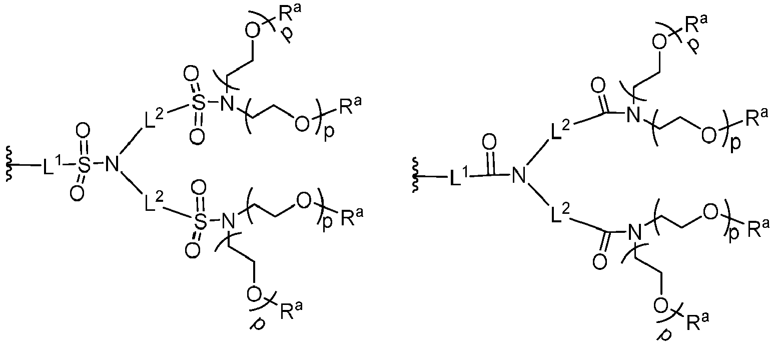 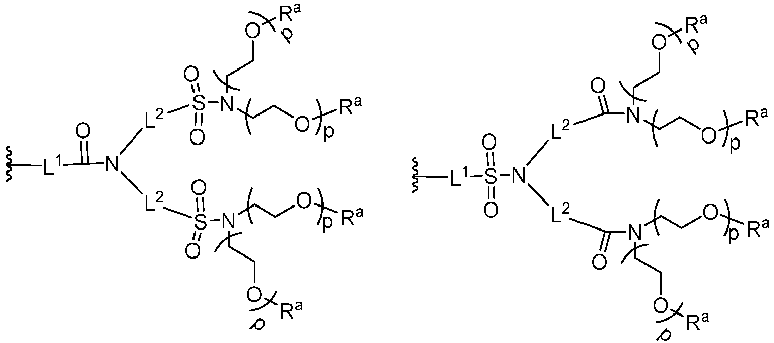 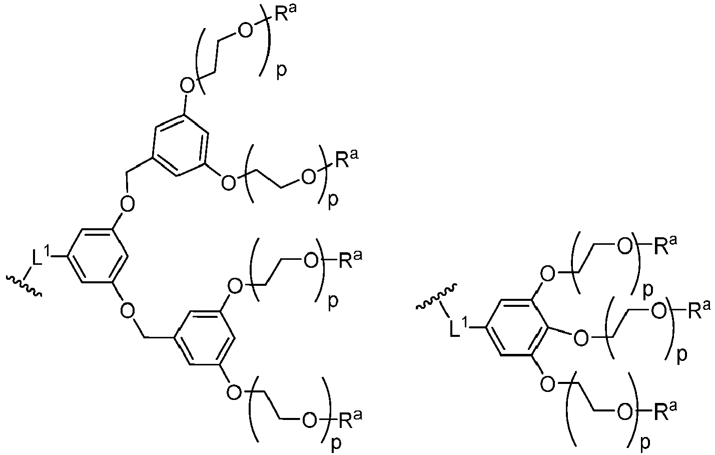 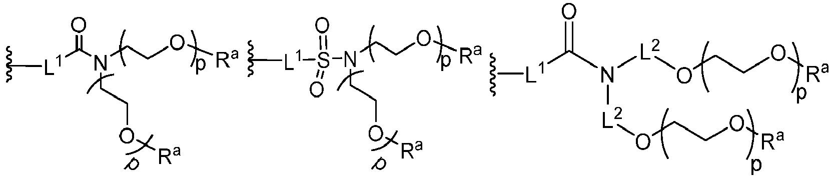 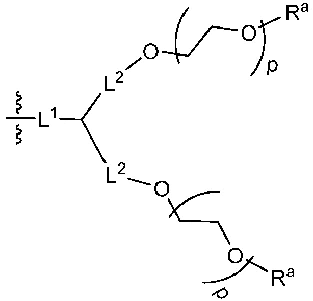 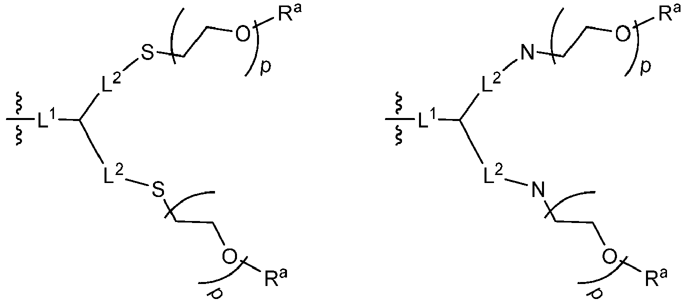 wherein:
   L¹ and L² are each independently an optional linker;
   R¹¹ and R are each independently H, alkyl or substituted alkyl; and
   each p is an integer from 6 to 24.
10. The water-soluble dipyrromethene-based dye of any one of clauses 6 to 9, wherein R¹ is selected from substituted alkyl, and substituted aryl, wherein the substituent comprises a chemoselective tag.
11. The water-soluble dipyrromethene-based dye of any one of clauses 6 to 10, wherein Y¹ and Y² each comprise a water solubilizing group (WSG).
12. The water-soluble dipyrromethene-based dye of any one of clauses 6 to 11, of the formula (II): 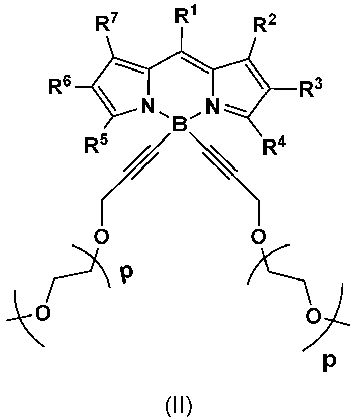 wherein p is an integer from 6-24.
13. The water-soluble dipyrromethene-based dye of any one of clauses 6 to 12, wherein R⁵ is selected from halogen, substituted alkyl, substituted alkenyl, alkenyl aryl, aryl, substituted aryl, heterocycle, substituted heterocycle, spiro heterocycle, substituted spiro heterocycle, heteroaryl, substituted heteroaryl, substituted alkoxy, substituted amino, substituted amido, and substituted thiol.
14. The water-soluble dipyrromethene-based dye of clause 13, wherein R⁵ and R⁴ are the same.
15. The water-soluble dipyrromethene-based dye of any one of clauses 6 to 14, of the formula (IIa): 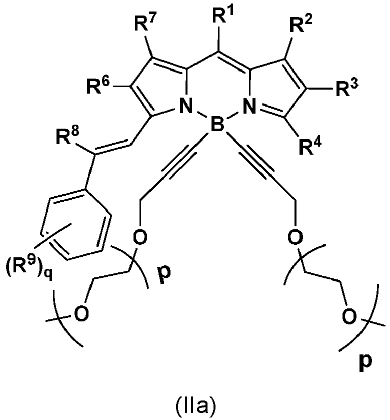 wherein:
   R⁸ is selected from H, halogen, cyano, alkyl, substituted alkyl, aryl, substituted aryl, heteroaryl, substituted heteroaryl, alkoxy, substituted alkoxy, alkenyl, substituted alkenyl, alkynyl, substituted alkynyl, water solubilizing group (WSG), or
   R⁸ and R⁶ together with the atoms to which they are bound form a 5- or 6-membered heterocycle, carbocycle, aryl or heteroaryl ring (e.g., a 5- or 6-membered ring comprising carbon atoms and 0-3 heteroatoms selected from O, S and N), which ring may be unsubstituted or further substituted with a substituent independently selected from alkyl, substituted alkyl, alkoxy, substituted alkoxy, aryl, substituted aryl, heteroaryl, substituted heteroaryl, alkenyl, substituted alkenyl, alkynyl, substituted alkynyl, and water solubilizing group (WSG);
   q is an integer from 0 to 5; and
   each R⁹, if present, is independently selected from alkyl, substituted alkyl, hydroxy, alkoxy, substituted alkoxy, amino, substituted amino, thiol, substituted thiol, cyano, aryl, substituted aryl, heteroaryl, substituted heteroaryl, alkenyl, substituted alkenyl, alkynyl, substituted alkynyl, and water solubilizing group (WSG), or
   optionally two adjacent R⁹ groups, together form a divalent radical and are cyclically linked and together with the carbon atoms to which they are bound provide a 5- or 6-membered fused heterocycle, carbocycle, aryl or heteroaryl ring (e.g., a 5- or 6-membered ring comprising carbon atoms and 0-3 heteroatoms selected from O, S and N), which ring may be unsubstituted or further substituted with a substituent independently selected from alkyl, substituted alkyl, alkoxy, substituted alkoxy, aryl, substituted aryl, heteroaryl, substituted heteroaryl, alkenyl, substituted alkenyl, alkynyl, substituted alkynyl, and water solubilizing group (WSG).
16. The water-soluble dipyrromethene-based dye of clause 15, wherein R⁸ is H.
17. The water-soluble dipyrromethene-based dye of clause 15, wherein R⁸ and R⁶ together with the atoms to which they are bound form a 5- membered carbocycle, which carbocycle is optionally further substituted with a substituent independently selected from alkyl, substituted alkyl, alkoxy, substituted alkoxy, aryl, substituted aryl, heteroaryl, substituted heteroaryl, alkenyl, substituted alkenyl, alkynyl, substituted alkynyl, and water solubilizing group (WSG).
18. The water-soluble dipyrromethene-based dye of any one of clauses 15 to 17, of any one of the following structures: 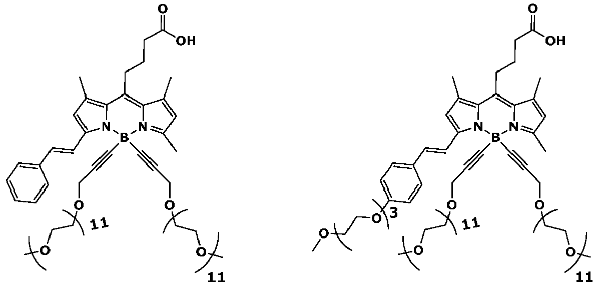 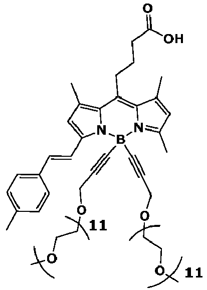 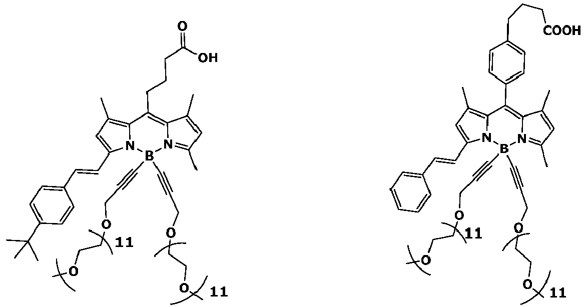 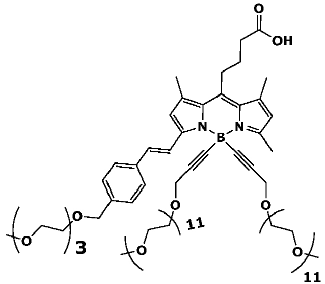 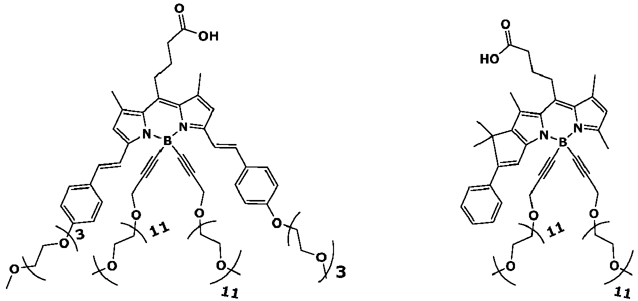 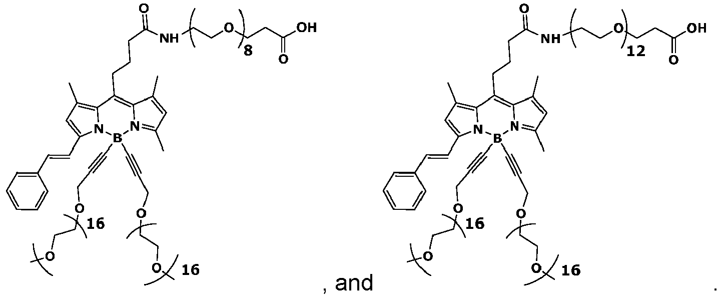
19. The water-soluble dipyrromethene-based dye of any one of clauses 6 to 14, of the formula (IIb): 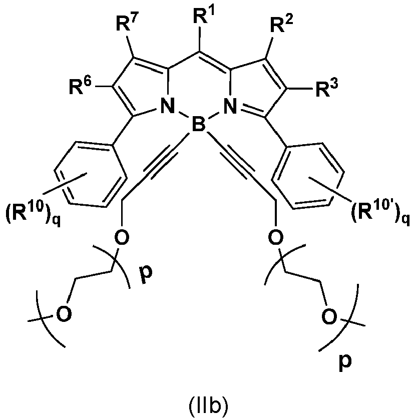 wherein:
   each q is independently an integer from 0 to 5; and
   R¹⁰ and R¹⁰', if present, are each independently selected from halogen, hydroxyl, cyano, carboxamide, substituted carboxamide amino, substituted amino, thiol, substituted thiol, alkyl, substituted alkyl, aryl, substituted aryl, heteroaryl, substituted heteroaryl, alkoxy, substituted alkoxy, alkenyl, substituted alkenyl, alkynyl, substituted alkynyl, and water soluble group (WSG), or
   at least one R¹⁰ group is at an ortho position, and R¹⁰ and R⁶ together with the atoms to which they are bound form a 5- or 6-membered ring (e.g., a 5- or 6-membered ring comprising a boron atom, carbon atoms and 0-3 heteroatoms selected from O, S and N), which ring may be unsubstituted or further substituted with a substituent independently selected from alkyl, substituted alkyl, alkoxy, substituted alkoxy, aryl, substituted aryl, heteroaryl, substituted heteroaryl, alkenyl, substituted alkenyl, alkynyl, substituted alkynyl, and water solubilizing group (WSG); and
   at least one R^{10'} is an ortho position, and R¹⁰ and R³ together with the atoms to which they are bound form a 5- or 6-membered ring (e.g., a 5- or 6-membered ring comprising a boron atom, carbon atoms and 0-3 heteroatoms selected from O, S and N), which ring may be unsubstituted or further substituted with a substituent independently selected from alkyl, substituted alkyl, alkoxy, substituted alkoxy, aryl, substituted aryl, heteroaryl, substituted heteroaryl, alkenyl, substituted alkenyl, alkynyl, substituted alkynyl, and water solubilizing group (WSG).
20. The water-soluble dipyrromethene-based dye of clause 19, wherein each q is 1, and R¹⁰ and R^{10'} are both present in their respective para positions.
21. The water-soluble dipyrromethene-based dye of clause 20, wherein R¹⁰ and R^{10'} are each independently selected from cyano and a PEG moiety.
22. The water-soluble dipyrromethene-based dye of any one of clauses 19 to 21, of any one of the following structures: 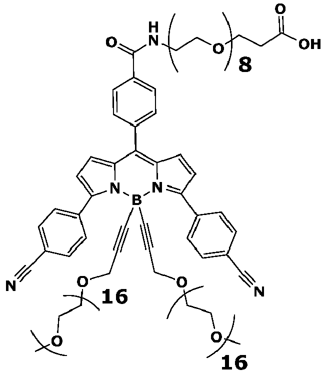 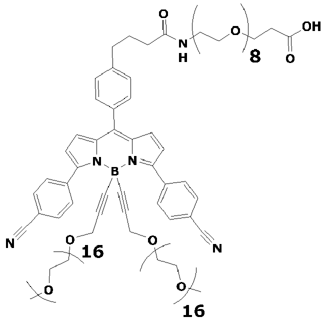 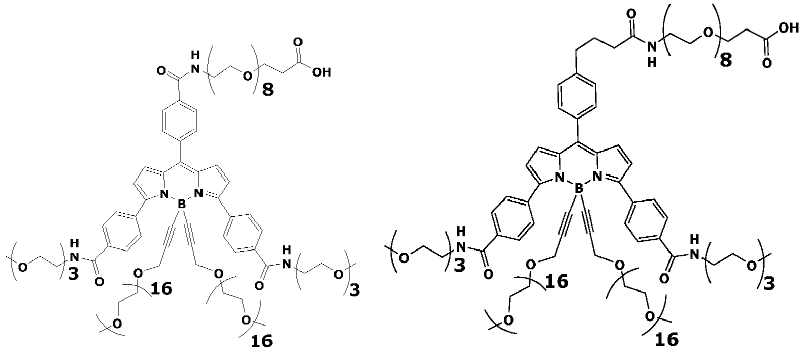 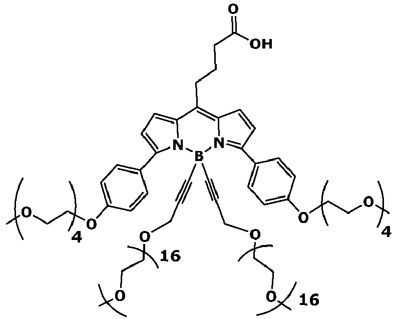 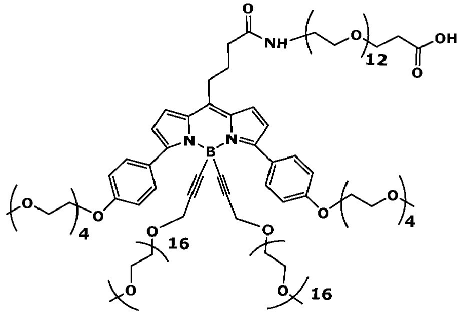 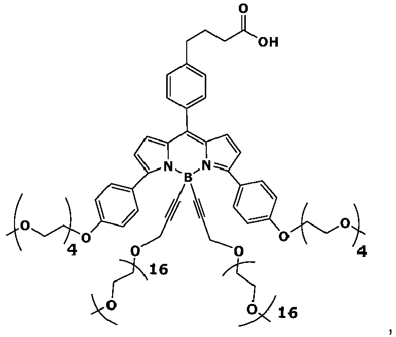 and 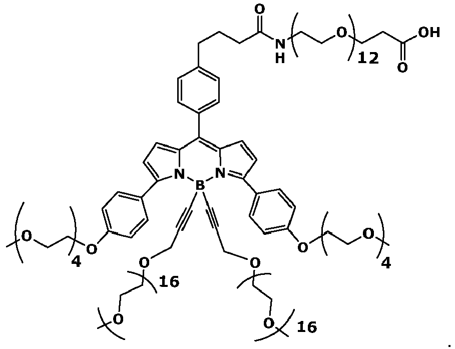
23. The water-soluble dipyrromethene-based dye of any one of clauses 6 to 14, of the formula (IIc): 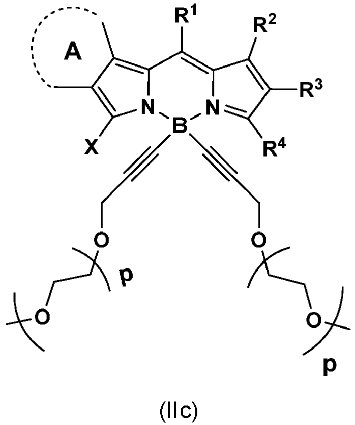 wherein:
   X is a halogen; and
   ring A is a 5- or 6-membered fused heterocycle, carbocycle, aryl or heteroaryl ring (e.g., a 5- or 6-membered ring comprising carbon atoms and 0-3 heteroatoms selected from O, S and N), which ring may be unsubstituted or further substituted with a substituent independently selected from alkyl, substituted alkyl, alkoxy, substituted alkoxy, aryl, substituted aryl, heteroaryl, substituted heteroaryl, alkenyl, substituted alkenyl, alkynyl, substituted alkynyl, and water solubilizing group (WSG).
24. The water-soluble dipyrromethene-based dye of clause 23, wherein the A ring is an aryl ring.
25. The water-soluble dipyrromethene-based dye of clause 23 or 24, of any one of the following compounds: 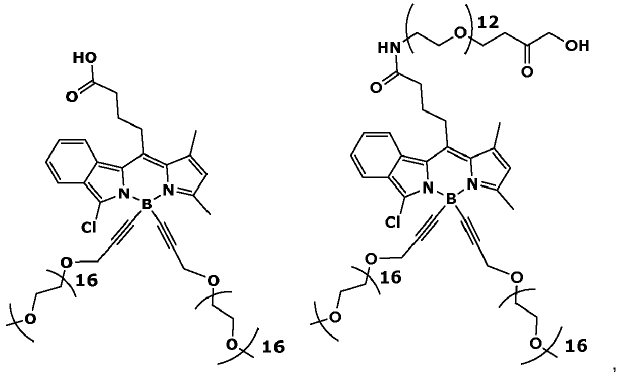 and 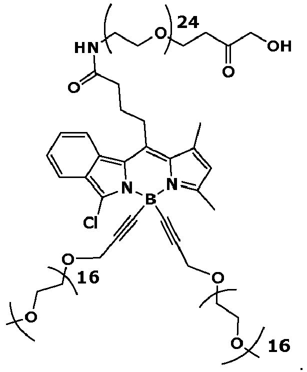
26. The water-soluble dipyrromethene-based dye of any one of clauses 6 to 14, of the formula (IId): 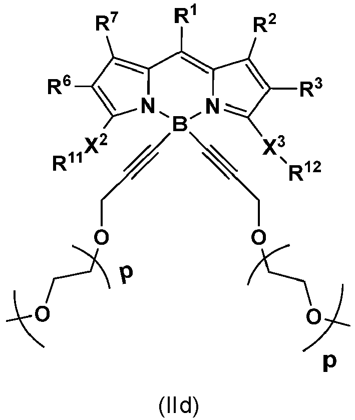 wherein:
   X² and X³ are each independently selected from NR¹³, S and O;
   R¹³ is selected from H, alkyl, and substituted alkyl;
   R¹¹ and R¹² are each independently selected from aryl, substituted aryl, heteroaryl, substituted heteroaryl, and water soluble group (WSG), or
   when X² is NR¹³, optionally R¹³ and R¹¹ together with the nitrogen atom to which they are attached, form a heterocyclic or spiro heterocyclic ring, which ring may be unsubstituted or further substituted with a substituent independently selected from alkyl, substituted alkyl, alkoxy, substituted alkoxy, aryl, substituted aryl, heteroaryl, substituted heteroaryl, alkenyl, substituted alkenyl, alkynyl, substituted alkynyl, and water solubilizing group (WSG), and
   when X³ is NR¹³, optionally R¹³ and R¹² together with the nitrogen atom to which they are attached, form a heterocyclic or spiro heterocyclic ring, which ring may be unsubstituted or further substituted with a substituent independently selected from alkyl, substituted alkyl, alkoxy, substituted alkoxy, aryl, substituted aryl, heteroaryl, substituted heteroaryl, alkenyl, substituted alkenyl, alkynyl, substituted alkynyl, and water solubilizing group (WSG).
27. The water-soluble dipyrromethene-based dye of clause 26, wherein X² and X³ are each NR¹³, wherein R¹³ is H.
28. The water-soluble dipyrromethene-based dye of clause 26, wherein X² is NR¹³, and R¹³ and R¹¹ together with the nitrogen atom to which they are attached form a heterocyclic or a spiro heterocyclic ring; and X³ is NR¹³, and R¹³ and R¹² together with the nitrogen atom to which they are attached form a heterocyclic or a spiro heterocyclic ring.
29. The water-soluble dipyrromethene-based dye of clause 26, wherein X² and X³ are both S.
30. The water-soluble dipyrromethene-based dye of any one of clauses 26 to 29, of any one of the following structures: 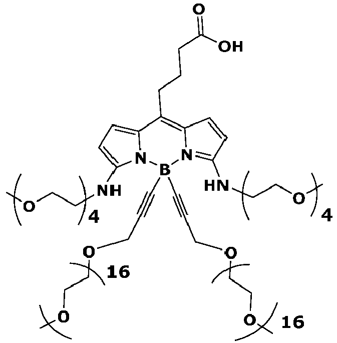 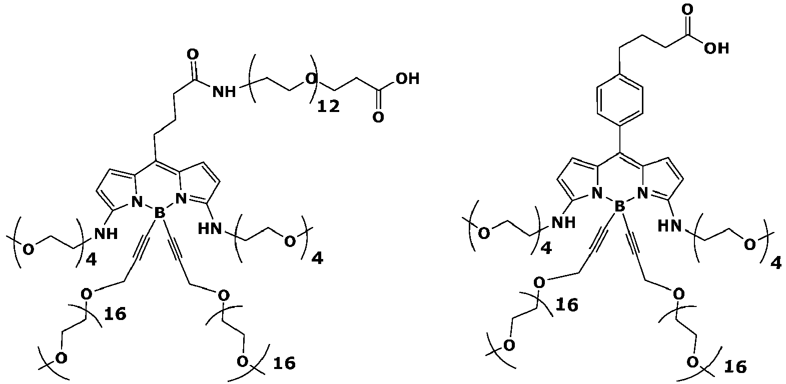 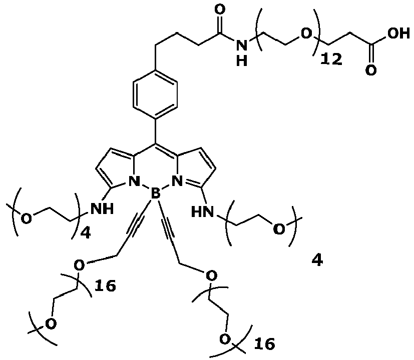 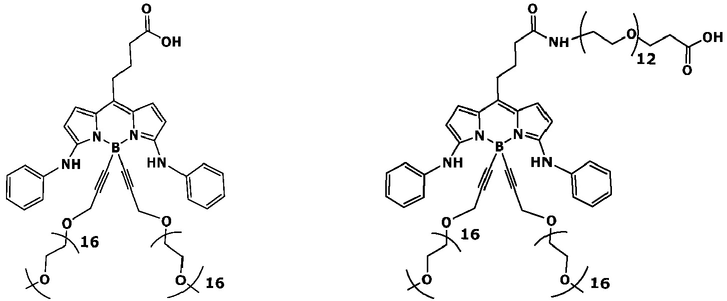 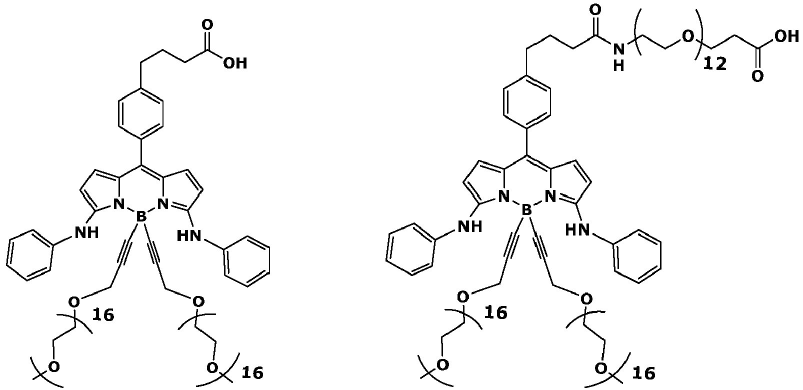 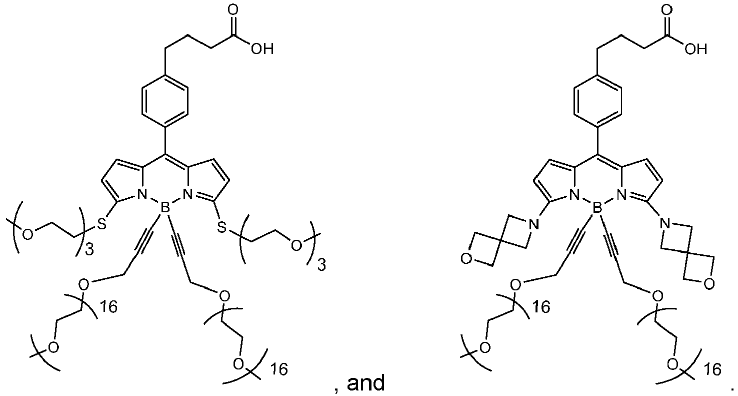
31. The water-soluble dipyrromethene-based dye of any one of clauses 6 to 10, of the formula (III): 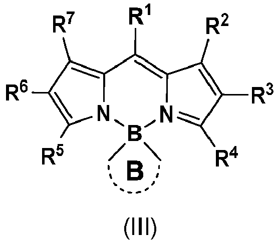 wherein:
   ring B is a 5- or 6-membered cyclic group (e.g., a 5- or 6-membered ring comprising a boron atom, carbon atoms and 0-3 heteroatoms selected from O, S and N), which cyclic group may be unsubstituted or further substituted with one or more substituents independently selected from alkyl, substituted alkyl, alkoxy, substituted alkoxy, aryl, substituted aryl, heteroaryl, substituted heteroaryl, alkenyl, substituted alkenyl, alkynyl, substituted alkynyl, and water solubilizing group (WSG).
32. The water-soluble dipyrromethene-based dye of clause 31, of the formula (IIIa): 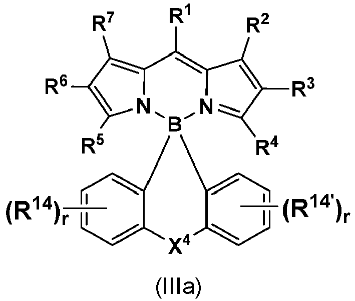 wherein:
   X⁴ is selected from O, S, NR, and CR₂;
   each R is independently selected from H, alkyl, and substituted alkyl;
   each r is independently an integer from 0 to 4; and
   each R¹⁴ and R^{14'}, if present, is independently selected from halogen, hydroxyl, cyano, amino, substituted amino, thiol, substituted thiol, amido, substituted amido, acyl, substituted acyl, alkyl, substituted alkyl, aryl, substituted aryl, heteroaryl, substituted heteroaryl, alkoxy, substituted alkoxy, alkenyl, substituted alkenyl, alkynyl, substituted alkynyl, and water solubilizing group (WSG).
33. The water-soluble dipyrromethene-based dye of clause 31 or 32, wherein at least one of R⁵ and R⁶ and R⁴ and R³ together form a divalent radical and are cyclically linked and together with the carbon atoms to which they are bound provide a 5- or 6-membered fused heterocycle, carbocycle, aryl or heteroaryl ring (e.g., a 5- or 6-membered ring comprising carbon atoms and 0-3 heteroatoms selected from O, S and N), which ring may be unsubstituted or further substituted with a substituent independently selected from alkyl, substituted alkyl, alkoxy, substituted alkoxy, aryl, substituted aryl, heteroaryl, substituted heteroaryl, alkenyl, substituted alkenyl, alkynyl, substituted alkynyl, and water solubilizing group (WSG).
34. The water-soluble dipyrromethene-based dye of clause 33, of the formula (IIIb) or (IIIc): 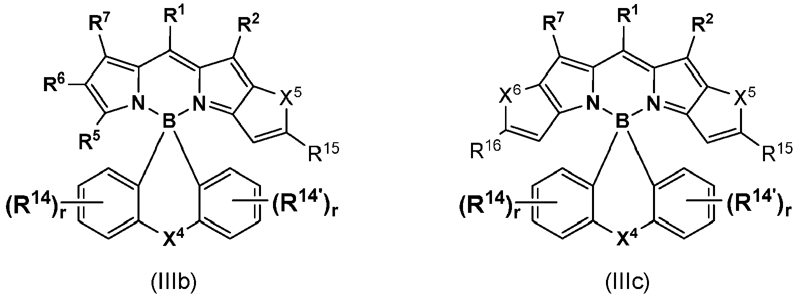 wherein:
   X⁵ and X⁶ are each independently selected from O, S, NR, and CR₂;
   each R is independently selected from H, alkyl, and substituted alkyl; and
   R¹⁵ and R¹⁶, are each independently selected from hydrogen, halogen, hydroxyl, cyano, amino, substituted amino, thiol, substituted thiol, amido, substituted amido, acyl, substituted acyl, alkyl, substituted alkyl, aryl, substituted aryl, heteroaryl, substituted heteroaryl, alkoxy, substituted alkoxy, alkenyl, substituted alkenyl, alkynyl, substituted alkynyl, and water solubilizing group (WSG).
35. The water-soluble dipyrromethene-based dye of clause 34, wherein X⁵ and X⁶ are O.
36. The water-soluble dipyrromethene-based dye of any one of clauses 31 to 35, of any one of the following structures: 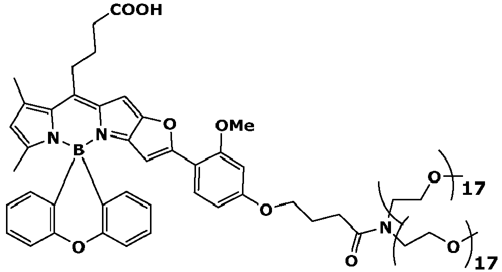 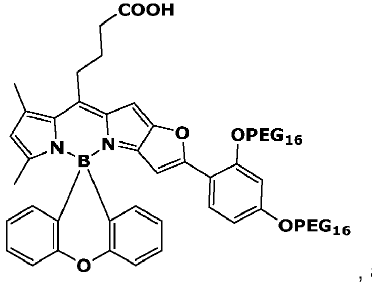 and 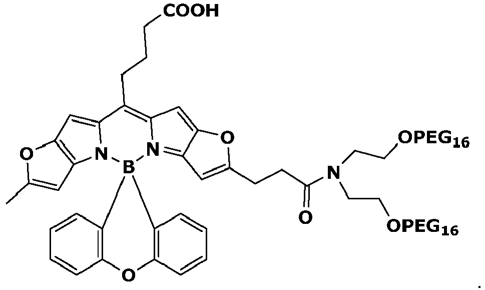
37. The water-soluble dipyrromethene-based dye of any one of clauses 6 to 12, wherein R³ is -L¹-J¹.
38. The water-soluble dipyrromethene-based dye of clause 37, of one of the following structures: 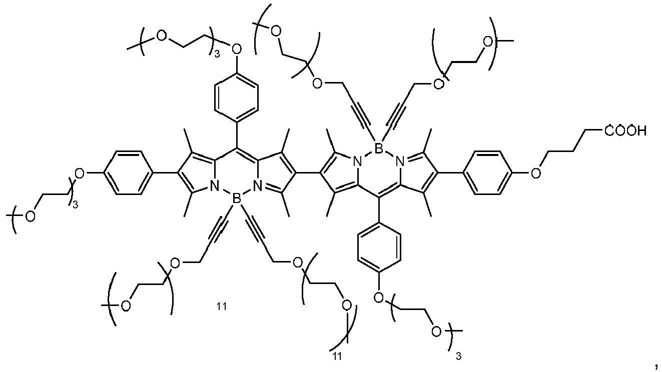 and 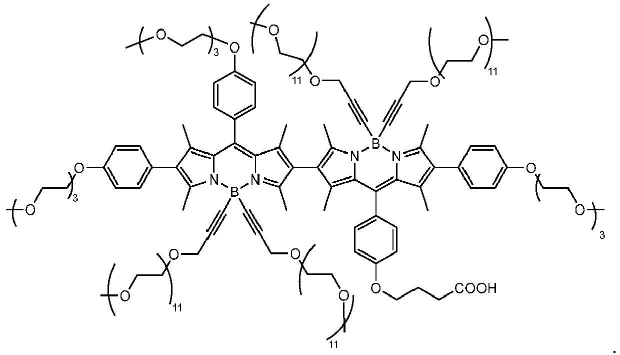
39. The water-soluble dipyrromethene-based dye of any one of clauses 1 to 4, of formula (IV): 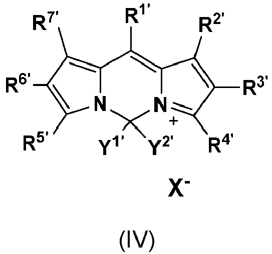 wherein:
   X⁻ is a counterion;
   R^{1'}-R^{7'} are each independently selected from H, alkyl, halogen, substituted alkyl, alkoxy, substituted alkoxy, amino, substituted amino, amido, substituted amido, thiol, substituted thiol, aryl, substituted aryl, heteroaryl, substituted heteroaryl, heterocycle, substituted heterocycle, alkenyl, substituted alkenyl, alkynyl, substituted alkynyl, water solubilizing group (WSG), and -T¹-Z¹, or
   optionally any one or more pairs of substituents selected from R^{6'} and R^{7'}, R^{2'} and R^{3'}, R^{5'} and R^{6'}, R^{3'} and R^{4'}, R^{2'} and R^{1'} and R^{7'} and R^{1'}, together form a divalent radical and are cyclically linked and together with the carbon atoms to which they are bound provide a 5- or 6-membered fused heterocycle, carbocycle, aryl or heteroaryl ring (e.g., a 5- or 6-membered ring comprising carbon atoms and 0-3 heteroatoms selected from O, S and N), which ring may be unsubstituted or further substituted with a substituent independently selected from alkyl, substituted alkyl, alkoxy, substituted alkoxy, aryl, substituted aryl, heteroaryl, substituted heteroaryl, alkenyl, substituted alkenyl, alkynyl, substituted alkynyl, water solubilizing group (WSG), and -T¹-Z¹;
   T¹ is an optional linker;
   Z¹ is a repeat unit of a compound of formula (I); and
   Y^{1'} and Y^{2'} are independently selected from F, OH, H, cyano, alkyl, substituted alkyl, aryl, substituted aryl, heteroaryl, substituted heteroaryl, alkoxy, substituted alkoxy, alkenyl, substituted alkenyl, alkynyl, substituted alkynyl, and WSG, or
   Y^{1'} and Y^{2'} together with the boron atom to which they are bound form a 5- or 6-membered ring (e.g., a 5- or 6-membered ring comprising a boron atom, carbon atoms and 0-3 heteroatoms selected from O, S and N), which ring may be unsubstituted or further substituted with a substituent independently selected from alkyl, substituted alkyl, alkoxy, substituted alkoxy, aryl, substituted aryl, heteroaryl, substituted heteroaryl, alkenyl, substituted alkenyl, alkynyl, substituted alkynyl, water solubilizing group (WSG), and -T¹-Z¹; and
   wherein at least one moiety selected from Y^{1'}, Y^{2'} and R^{1'}-R^{7'} is a WSG, or at least one moiety selected from Y^{1'}, Y^{2'} and R^{1'}-R^{7'} is substituted with a WSG, where the WSG is of the formula (la):

      -(L¹)ₙ₁-(X¹)ₙ₂-((L²)ₙ₃-Z¹)ₘ (Ia)

      wherein:
      n1, n2 and n3 are each independently 0 or 1;
      L¹, if present, is a linker;
      L² if present, is a linker;
      X¹, if present, is a branching point;
      Z¹ is a water soluble polymer comprising 6-24 monomeric units; and
      m is an integer from 1 to 3.
40. The water-soluble dipyrromethene-based dye of clause 39, further comprising a WSG of formula (Ib):

   -(L¹)ₙ₁ - (X¹)ₙ₂ - ((L²)ₙ₃-Z²)ₘ (Ib)

   wherein:
   n1, n2 and n3 are each independently 0 or 1;
   L¹, if present, is a linker;
   L² if present, is a linker;
   X¹, if present, is a branching point;
   Z² is a water soluble polymer comprising 1-50 monomeric units; and
   m is an integer from 1 to 3.
41. The water-soluble dipyrromethene-based dye of clause 39 or 40, wherein the WSG comprises a PEG moiety.
42. The water-soluble dipyrromethene-based dye of any one of clauses 39 to 41, wherein one or more water solubilizing groups (WSGs) is independently selected from the following formulae: 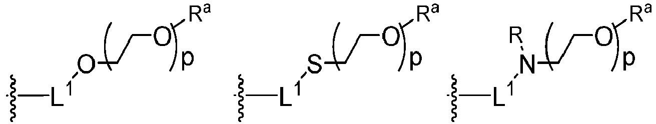 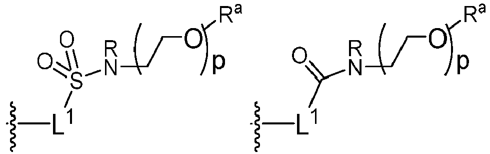 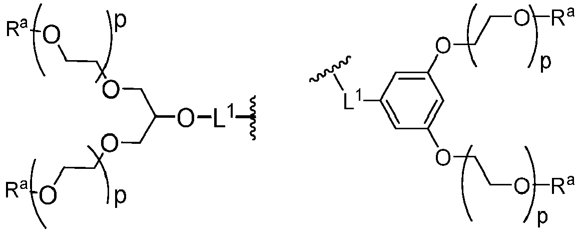 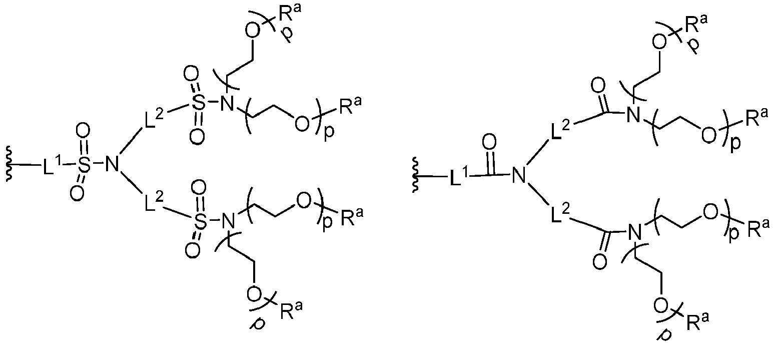 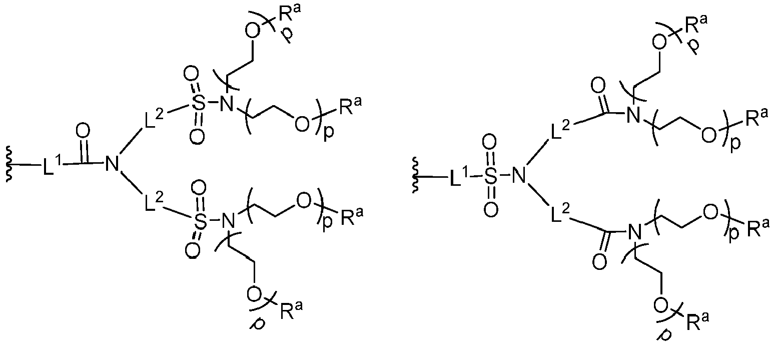 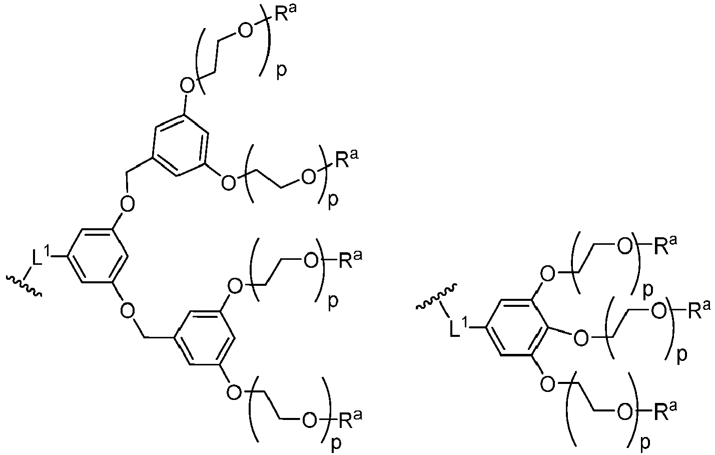 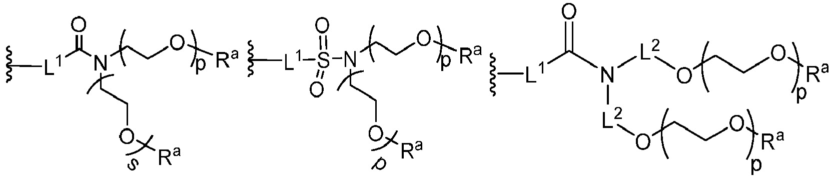 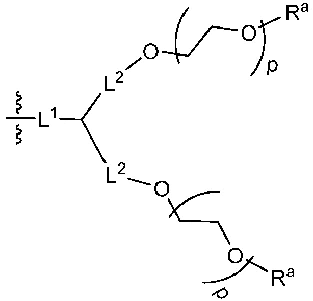 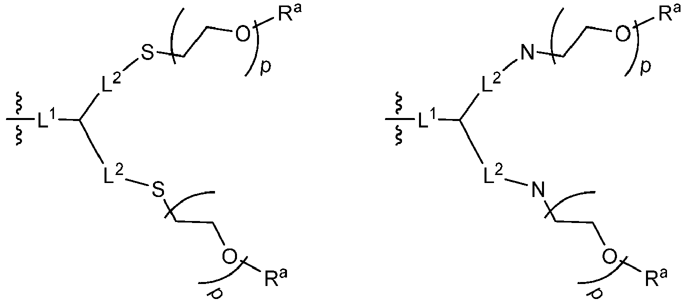 wherein:
   L¹ and L² are each independently an optional linker;
   R¹¹ and R are each independently H, alkyl or substituted alkyl; and
   each p is an integer from 6 to 24.
43. The water-soluble dipyrromethene-based dye of any one of clauses 39 to 42, wherein R¹ is selected from substituted alkyl, and substituted aryl, wherein the substituent comprises a chemoselective tag.
44. The water-soluble dipyrromethene-based dye of any one of clauses 39 to 43, wherein R^{5'} and R^{4'} both comprise a water solubilizing group (WSG).
45. The water-soluble dipyrromethene-based dye of any one of clauses 39 to 44, of one of the following structures: 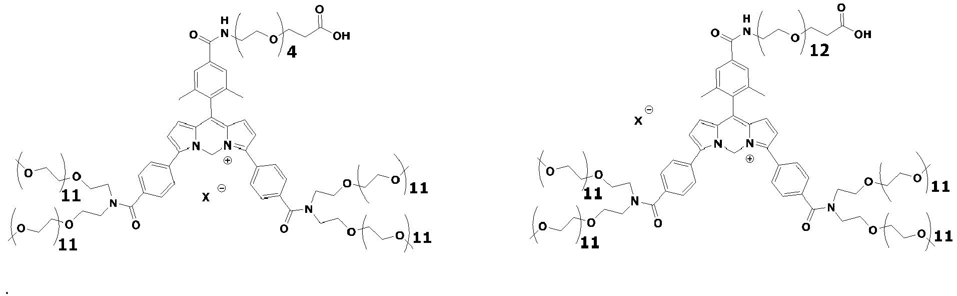
46. A yellow green dipyrromethene-based dye of formula (II): 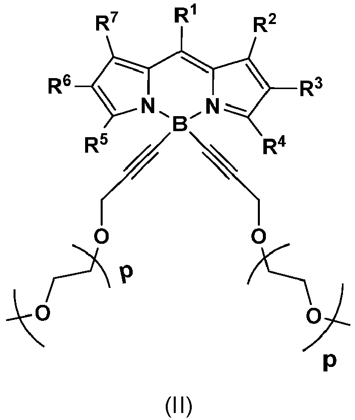 wherein:
   R¹-R⁷ are each independently selected from H, alkyl, halogen, substituted alkyl, alkoxy, substituted alkoxy, amino, substituted amino, amido, substituted amido, thiol, substituted thiol, aryl, substituted aryl, heteroaryl, substituted heteroaryl, heterocycle, substituted heterocycle, alkenyl, substituted alkenyl, alkynyl, substituted alkynyl, water solubilizing group (WSG), and -T¹-Z¹, or
   optionally any one or more pairs of substituents selected from R⁶ and R⁷, R² and R³, R⁵ and R⁶, R³ and R⁴, R² and R¹ and R⁷ and R¹, together form a divalent radical and are cyclically linked and together with the carbon atoms to which they are bound provide a 5- or 6-membered fused heterocycle, carbocycle, aryl or heteroaryl ring (e.g., a 5- or 6-membered ring comprising carbon atoms and 0-3 heteroatoms selected from O, S and N), which ring may be unsubstituted or further substituted with a substituent independently selected from alkyl, substituted alkyl, alkoxy, substituted alkoxy, aryl, substituted aryl, heteroaryl, substituted heteroaryl, alkenyl, substituted alkenyl, alkynyl, substituted alkynyl, water solubilizing group (WSG), and -T¹-Z¹;
   T¹ is an optional linker;
   Z¹ is a repeat unit of a compound of formula (I); and
   p is an integer from 6-24.
47. The yellow green dipyrromethene-based dye of clause 46, further comprising one or more water solubilizing groups (WSG) of formula (Ib):

   -(L¹)ₙ₁ - (X¹)ₙ₂ - ((L²)ₙ₃-Z²)ₘ (Ib)

   wherein:
   n1, n2 and n3 are each independently 0 or 1;
   L¹, if present, is a linker;
   L² if present, is a linker;
   X¹, if present, is a branching point;
   Z² is a water soluble polymer comprising 1-50 monomeric units; and
   m is an integer from 1 to 3.
48. The yellow green dipyrromethene-based dye of clause 47, wherein the WSG comprises a PEG moiety.
49. The yellow green dipyrromethene-based dye of clause 48, wherein one or more water solubilizing groups (WSGs) is independently selected from the following formulae: 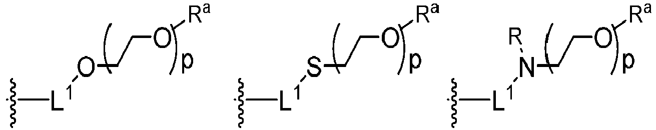 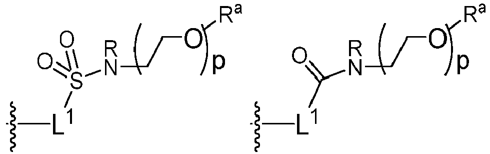 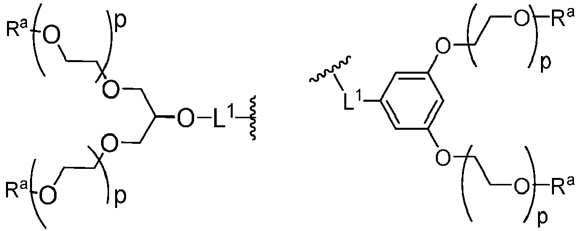 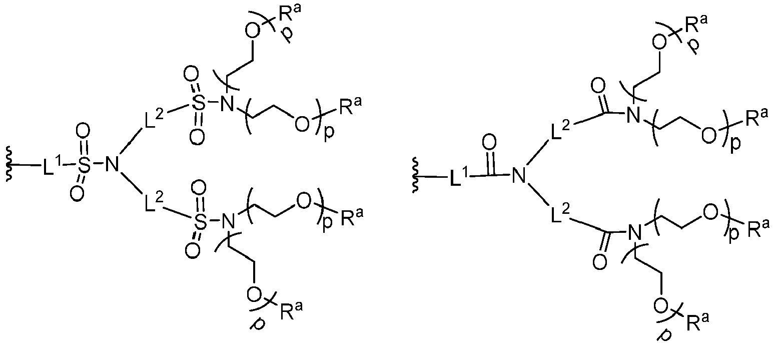 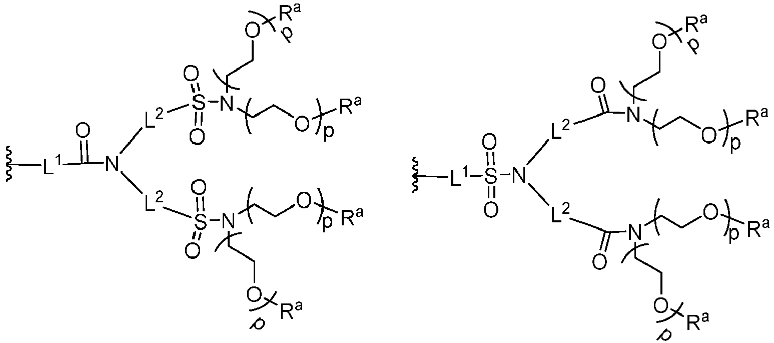 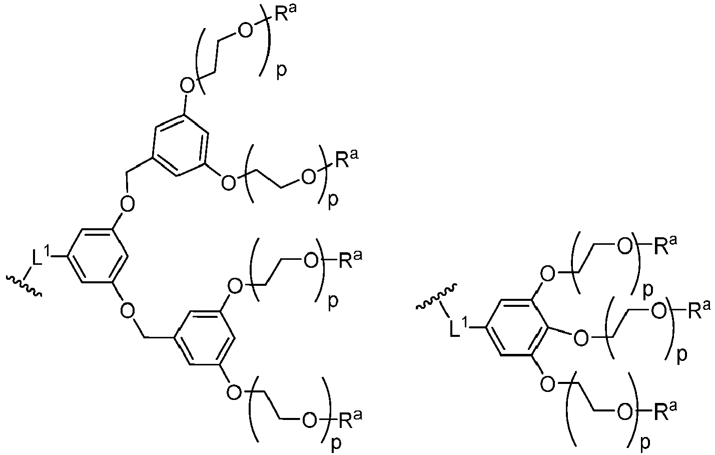 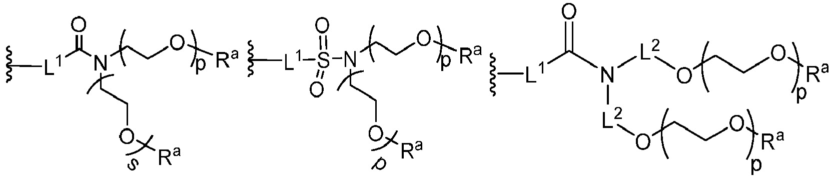 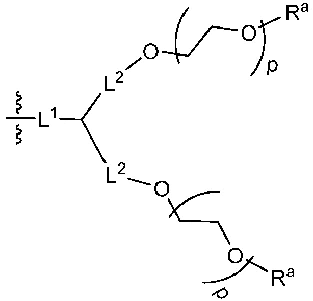 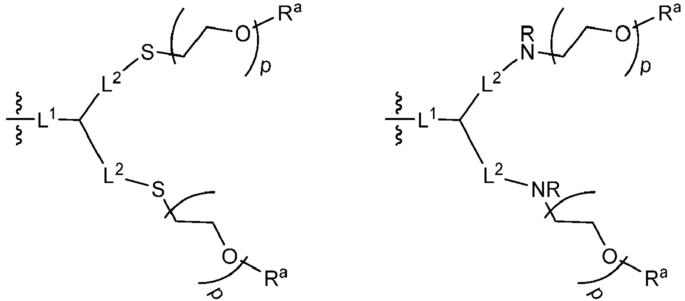 wherein:
   L¹ and L² are each independently an optional linker;
   R¹¹ and R are each independently H, alkyl or substituted alkyl; and
   each p is an integer from 6 to 24.
50. The yellow green dipyrromethene-based dye of any one of clauses 46 to 49, wherein R¹ is selected from substituted alkyl, and substituted aryl, wherein the substituent comprises a chemoselective tag.
51. The yellow green dipyrromethene-based dye of any one of clauses 46 to 50, wherein R⁵ is selected from halogen, substituted alkyl, substituted alkenyl, alkenyl aryl, aryl, substituted aryl, heterocycle, substituted heterocycle, spiro heterocycle, substituted spiro heterocycle, heteroaryl, substituted heteroaryl, substituted amino, substituted amido, and substituted thiol.
52. The yellow green dipyrromethene-based dye of clause 51, wherein R⁵ and R⁴ are the same.
53. The yellow green dipyrromethene-based dye of any one of clauses 46 to 52, of the formula (IIa): 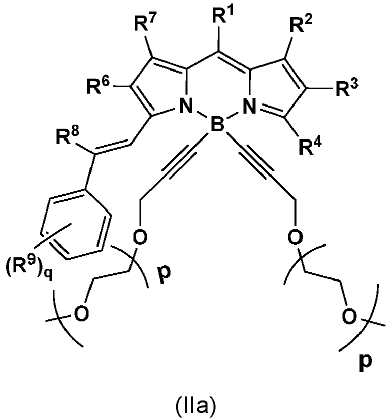 wherein:
   R⁸ is selected from H, halogen, cyano, alkyl, substituted alkyl, aryl, substituted aryl, heteroaryl, substituted heteroaryl, alkoxy, substituted alkoxy, alkenyl, substituted alkenyl, alkynyl, substituted alkynyl, and water solubilizing group (WSG), or
   R⁸ and R⁶ together with the atoms to which they are bound form a 5- or 6-membered heterocycle, carbocycle, aryl or heteroaryl ring (e.g., a 5- or 6-membered ring comprising carbon atoms and 0-3 heteroatoms selected from O, S and N), which ring may be unsubstituted or further substituted with a substituent independently selected from alkyl, substituted alkyl, alkoxy, substituted alkoxy, aryl, substituted aryl, heteroaryl, substituted heteroaryl, alkenyl, substituted alkenyl, alkynyl, substituted alkynyl, and water solubilizing group (WSG);
   q is an integer from 0 to 5; and
   each R⁹, if present, is independently selected from alkyl, substituted alkyl, hydroxy, alkoxy, substituted alkoxy, amino, substituted amino, thiol, substituted thiol, cyano, aryl, substituted aryl, heteroaryl, substituted heteroaryl, alkenyl, substituted alkenyl, alkynyl, substituted alkynyl, and water solubilizing group (WSG), or
   optionally two adjacent R⁹ groups, together form a divalent radical and are cyclically linked and together with the carbon atoms to which they are bound provide a 5- or 6-membered fused heterocycle, carbocycle, aryl or heteroaryl ring (e.g., a 5- or 6-membered ring comprising carbon atoms and 0-3 heteroatoms selected from O, S and N), which ring may be unsubstituted or further substituted with a substituent independently selected from alkyl, substituted alkyl, alkoxy, substituted alkoxy, aryl, substituted aryl, heteroaryl, substituted heteroaryl, alkenyl, substituted alkenyl, alkynyl, substituted alkynyl, and water solubilizing group (WSG).
54. The yellow green dipyrromethene-based dye of clause 53, wherein R⁸ is H.
55. The yellow green dipyrromethene-based dye of clause 53, wherein R⁸ and R⁶ together with the atoms to which they are bound form a 5- membered carbocycle, which carbocycle is optionally further substituted with a substituent independently selected from alkyl, substituted alkyl, alkoxy, substituted alkoxy, aryl, substituted aryl, heteroaryl, substituted heteroaryl, alkenyl, substituted alkenyl, alkynyl, substituted alkynyl, and water solubilizing group (WSG).
56. The yellow green dipyrromethene-based dye of any one of clauses 46 to 55, of any one of the following structures: 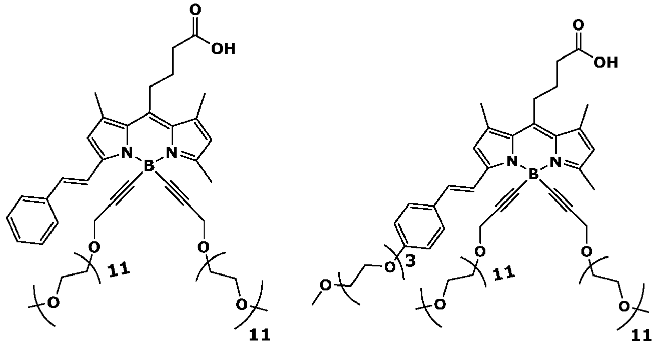 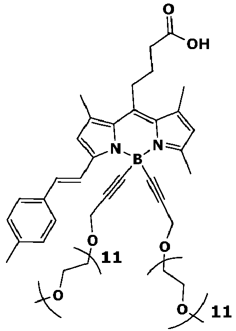 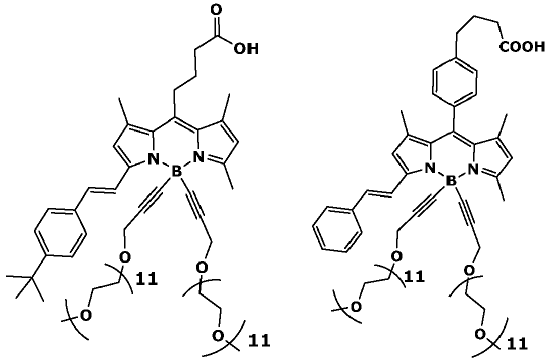 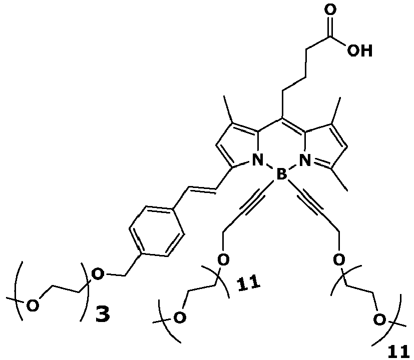 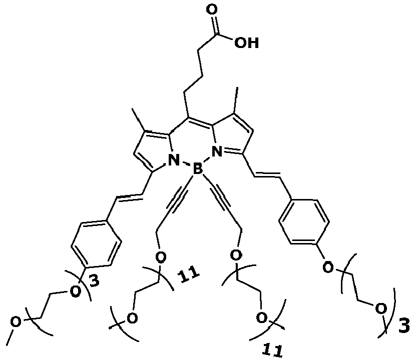 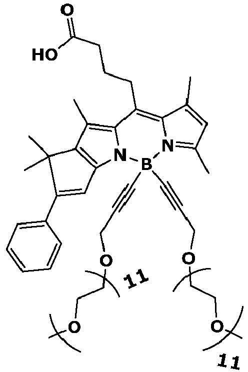 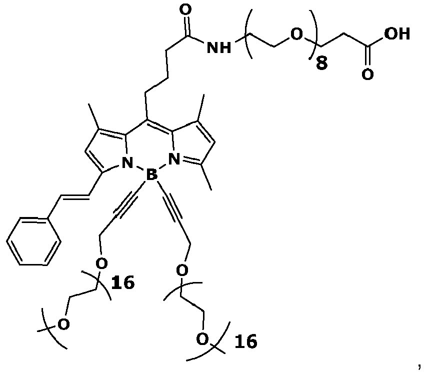 and 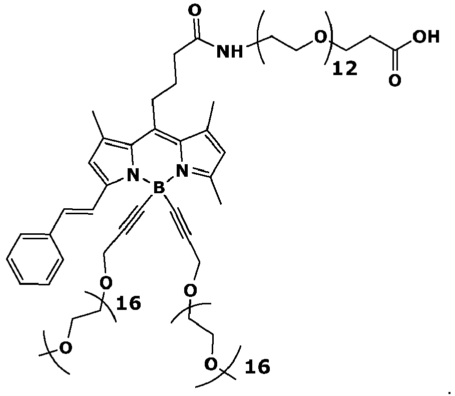
57. The yellow green dipyrromethene-based dye of any one of clauses 46 to 52, of the formula (IIb): 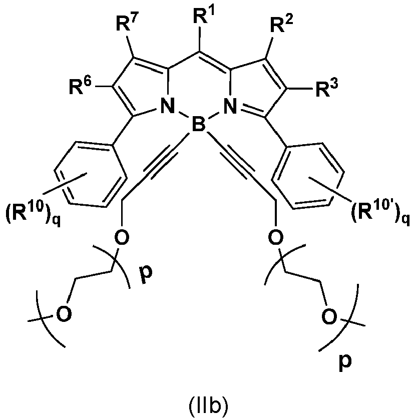 wherein:
   each q is independently an integer from 0 to 5; and
   R¹⁰ and R¹⁰', if present, are each independently selected from halogen, hydroxyl, cyano, amino, substituted amino, thiol, substituted thiol, alkyl, substituted alkyl, aryl, substituted aryl, heteroaryl, substituted heteroaryl, alkoxy, substituted alkoxy, alkenyl, substituted alkenyl, alkynyl, substituted alkynyl, and water soluble group (WSG), or
   at least one R¹⁰ group is at an ortho position, and R¹⁰ and R⁶ together with the atoms to which they are bound form a 5- or 6-membered ring (e.g., a 5- or 6-membered ring comprising a boron atom, carbon atoms and 0-3 heteroatoms selected from O, S and N), which ring may be unsubstituted or further substituted with a substituent independently selected from alkyl, substituted alkyl, alkoxy, substituted alkoxy, aryl, substituted aryl, heteroaryl, substituted heteroaryl, alkenyl, substituted alkenyl, alkynyl, substituted alkynyl, and water solubilizing group (WSG); and
   at least one R^{10'} is an ortho position, and R¹⁰ and R³ together with the atoms to which they are bound form a 5- or 6-membered ring (e.g., a 5- or 6-membered ring comprising a boron atom, carbon atoms and 0-3 heteroatoms selected from O, S and N), which ring may be unsubstituted or further substituted with a substituent independently selected from alkyl, substituted alkyl, alkoxy, substituted alkoxy, aryl, substituted aryl, heteroaryl, substituted heteroaryl, alkenyl, substituted alkenyl, alkynyl, substituted alkynyl, and water solubilizing group (WSG).
58. The yellow green dipyrromethene-based dye of clause 57, wherein each q is 1, and R¹⁰ and R^{10'} are both present in their respective para positions.
59. The yellow green dipyrromethene-based dye of clause 58, wherein R¹⁰ and R^{10'} are each independently selected from cyano and a PEG moiety.
60. The yellow green dipyrromethene-based dye of clause 59, wherein the PEG moiety is selected from one of the following formulae: 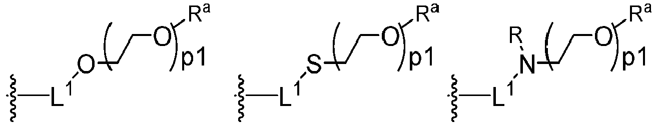 wherein:
   L¹ is an optional linker;
   R^{a} and R are each independently H, alkyl or substituted alkyl; and
   each p1 is an integer from 1 to 50.
61. The yellow green dipyrromethene-based dye of any one of clauses 57 to 60, of any one of the following structures: 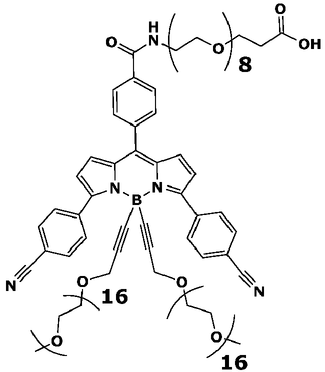 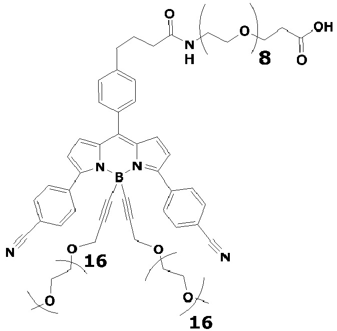 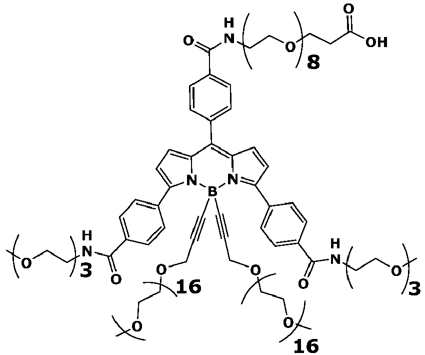 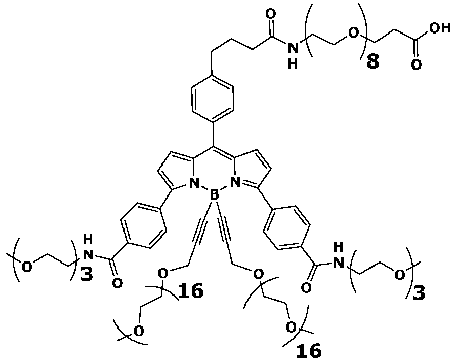 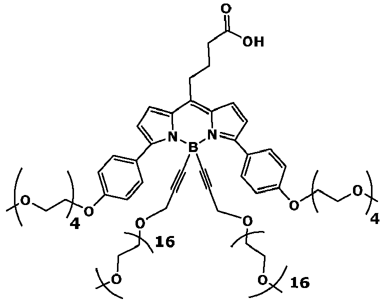 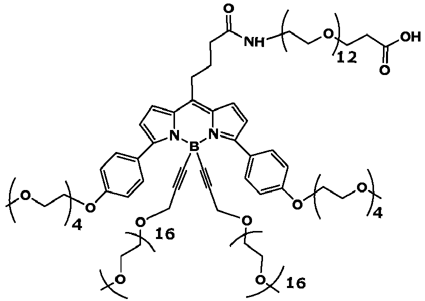 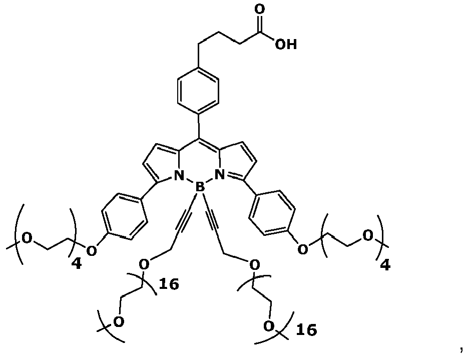 and 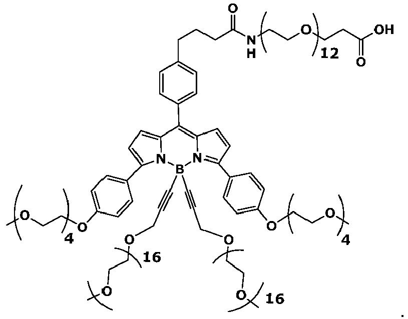
62. The yellow green dipyrromethene-based dye of any one of clauses 46 to 52, of the formula (IIc): 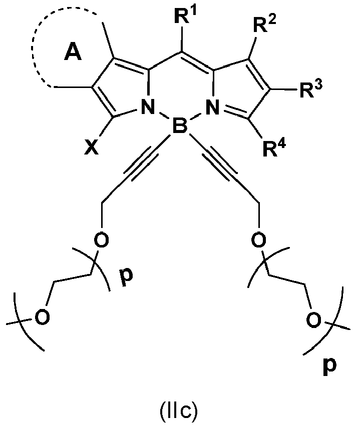 wherein:
   X is a halogen; and
   ring A is a 5- or 6-membered fused heterocycle, carbocycle, aryl or heteroaryl ring (e.g., a 5- or 6-membered ring comprising carbon atoms and 0-3 heteroatoms selected from O, S and N), which ring may be unsubstituted or further substituted with a substituent independently selected from alkyl, substituted alkyl, alkoxy, substituted alkoxy, aryl, substituted aryl, heteroaryl, substituted heteroaryl, alkenyl, substituted alkenyl, alkynyl, substituted alkynyl, and water solubilizing group (WSG).
63. The yellow green dipyrromethene-based dye of clause 62, wherein the A ring is an aryl ring.
64. The yellow green dipyrromethene-based dye of clause 62 or 63, wherein X is chloride.
65. The yellow green dipyrromethene-based dye of any one of clauses 62 to 64, of any one of the following compounds: 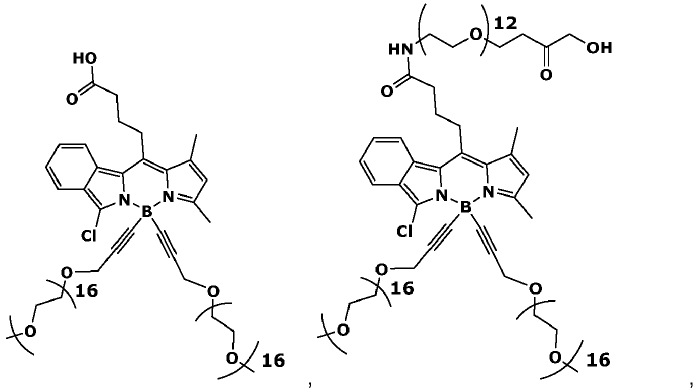 and 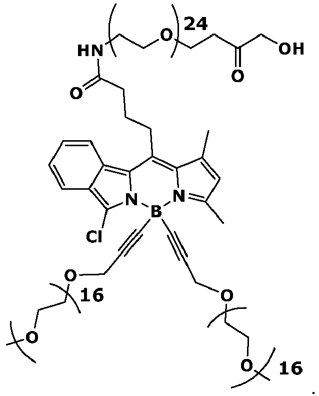
66. The yellow green dipyrromethene-based dye of any one of clauses 46 to 52, of the formula (IId): 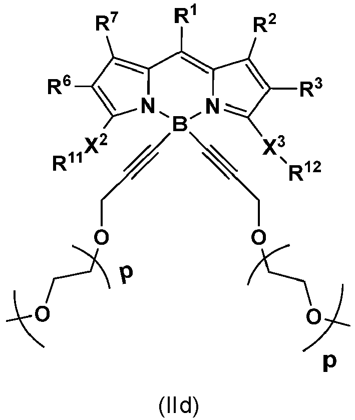 wherein:
   X² and X³ are each independently selected from NR¹³, S and O;
   R¹³ is selected from H, alkyl, and substituted alkyl;
   R¹¹ and R¹² are each independently selected from aryl, substituted aryl, heteroaryl, substituted heteroaryl, and a water soluble group (WSG), or
   when X² is NR¹³, optionally R¹³ and R¹¹ together with the nitrogen atom to which they are attached, form a heterocyclic or spiro heterocyclic ring, which ring may be unsubstituted or further substituted with a substituent independently selected from alkyl, substituted alkyl, alkoxy, substituted alkoxy, aryl, substituted aryl, heteroaryl, substituted heteroaryl, alkenyl, substituted alkenyl, alkynyl, substituted alkynyl, and a water solubilizing group (WSG); and
   when X³ is NR¹³, optionally R¹³ and R¹² together with the nitrogen atom to which they are attached, form a heterocyclic or spiro heterocyclic ring, which ring may be unsubstituted or further substituted with a substituent independently selected from alkyl, substituted alkyl, alkoxy, substituted alkoxy, aryl, substituted aryl, heteroaryl, substituted heteroaryl, alkenyl, substituted alkenyl, alkynyl, substituted alkynyl, and a water solubilizing group (WSG).
67. The yellow green dipyrromethene-based dye of clause 66, wherein X² and X³ are each NR¹³, wherein R¹³ is H.
68. The yellow green dipyrromethene-based dye of clause 66, wherein X² is NR¹³, and R¹³ and R¹¹ together with the nitrogen atom to which they are attached form a heterocyclic or a spiro heterocyclic ring; and X³ is NR¹³, and R¹³ and R¹² together with the nitrogen atom to which they are attached form a heterocyclic or a spiro heterocyclic ring.
69. The yellow green dipyrromethene-based dye of clause 66, wherein X² and X³ are both S.
70. The yellow green dipyrromethene-based dye of any one of clauses 66 to 69, of any one of the following structures: 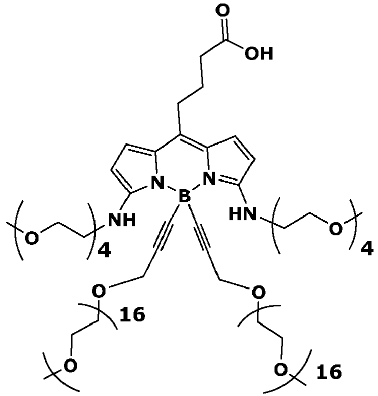 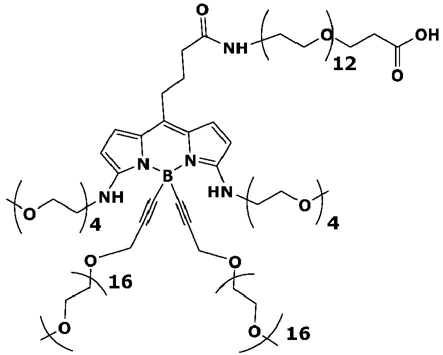 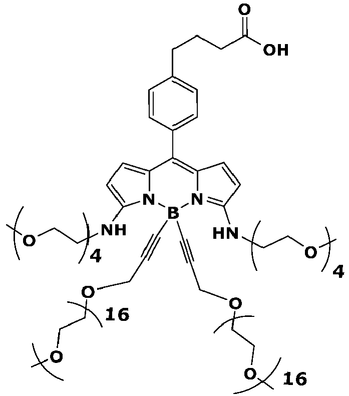 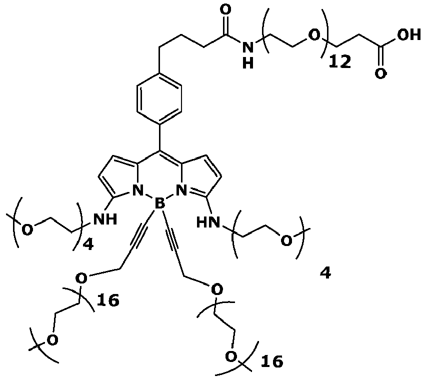 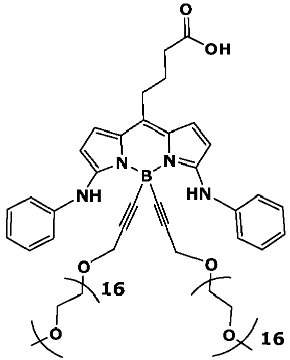 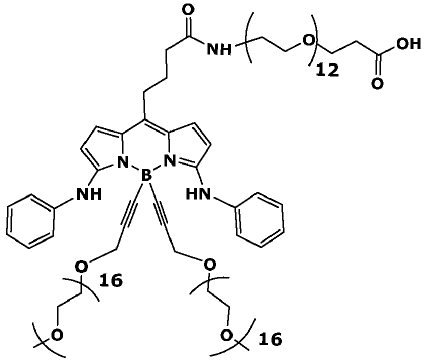 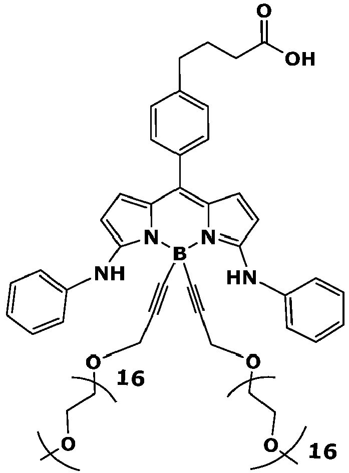 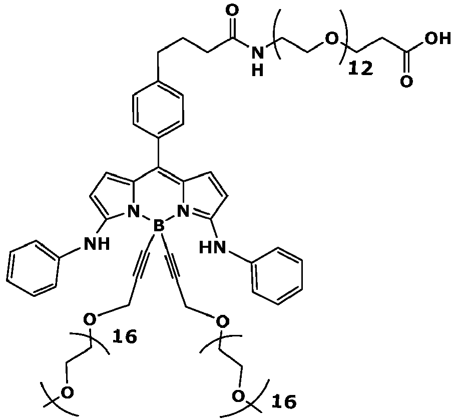 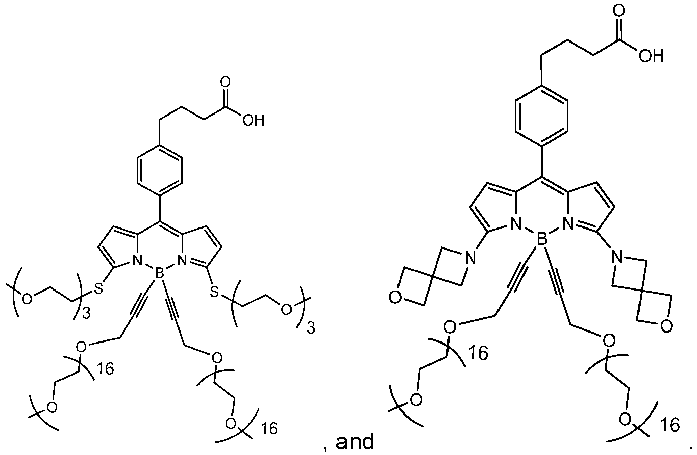
71. The yellow green dipyrromethene-based dye of any one of clauses 46 to 52, wherein R³ is -L¹-Z¹.
72. The yellow green dipyrromethene-based dye of clause 71, wherein R⁶ is selected from halogen, substituted alkyl, substituted alkenyl, alkenyl aryl, aryl, substituted aryl, heterocycle, substituted heterocycle, spiro heterocycle, substituted spiro heterocycle, heteroaryl, substituted heteroaryl, substituted amino, substituted amido, and substituted thiol.
73. The yellow green dipyrromethene-based dye of clause 72, wherein R⁶ comprises a PEG moiety.
74. The yellow green dipyrromethene-based dye of clause 73, wherein the PEG moiety is selected from one of the following formulae: 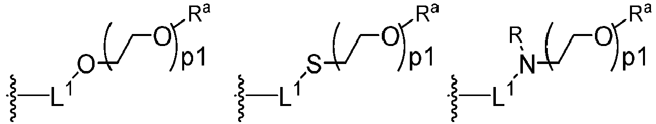 wherein:
   L¹ and L² are each independently an optional linker;
   R is H, alkyl or substituted alkyl; and
   each p1 is an integer from 1 to 50.
75. The yellow green dipyrromethene-based dye of any one of clauses 71 to 74, of one of the following structures: 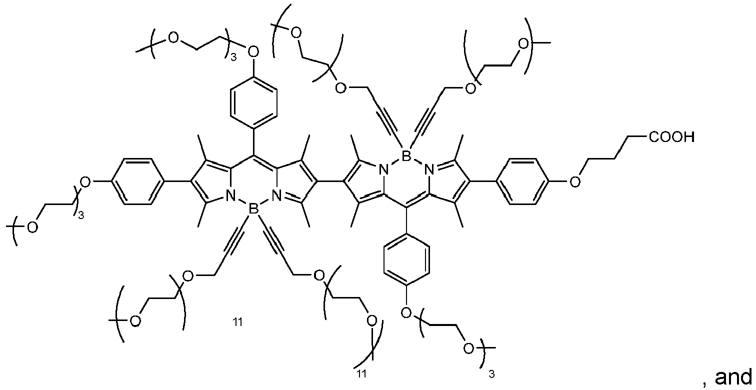 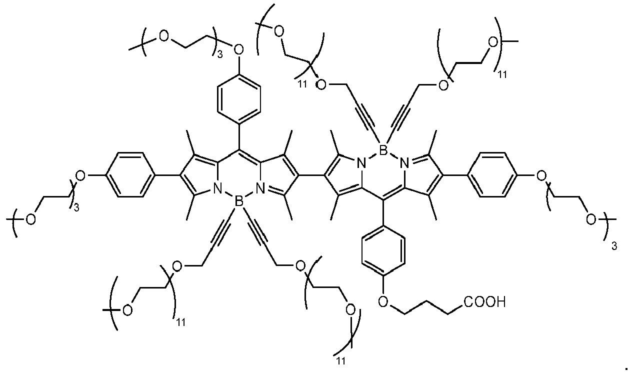
76. A yellow green dipyrromethene-based dye of formula (III): 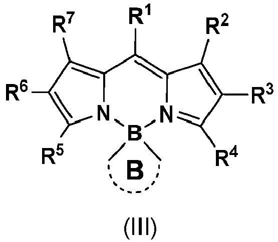 wherein:
   R¹-R⁷ are each independently selected from H, alkyl, halogen, substituted alkyl, alkoxy, substituted alkoxy, amino, substituted amino, amido, substituted amido, thiol, substituted thiol, aryl, substituted aryl, heteroaryl, substituted heteroaryl, heterocycle, substituted heterocycle, alkenyl, substituted alkenyl, alkynyl, substituted alkynyl, water solubilizing group (WSG), and -T¹-Z¹, or
   optionally any one or more pairs of substituents selected from R⁶ and R⁷, R² and R³, R⁵ and R⁶, R³ and R⁴, R² and R¹ and R⁷ and R¹, together form a divalent radical and are cyclically linked and together with the carbon atoms to which they are bound provide a 5- or 6-membered fused heterocycle, carbocycle, aryl or heteroaryl ring (e.g., a 5- or 6-membered ring comprising carbon atoms and 0-3 heteroatoms selected from O, S and N), which ring may be unsubstituted or further substituted with a substituent independently selected from alkyl, substituted alkyl, alkoxy, substituted alkoxy, aryl, substituted aryl, heteroaryl, substituted heteroaryl, alkenyl, substituted alkenyl, alkynyl, substituted alkynyl, water solubilizing group (WSG), and -T¹-Z¹;
   T¹ is an optional linker;
   Z¹ is a repeat unit of a compound of formula (I); and
   ring B is a 5- or 6-membered cyclic group (e.g., a 5- or 6-membered ring comprising a boron atom, carbon atoms and 0-3 heteroatoms selected from O, S and N), which cyclic group may be unsubstituted or further substituted with one or more substituents independently selected from alkyl, substituted alkyl, alkoxy, substituted alkoxy, aryl, substituted aryl, heteroaryl, substituted heteroaryl, alkenyl, substituted alkenyl, alkynyl, substituted alkynyl, and water solubilizing group (WSG); and
   wherein at least one moiety selected from Y¹, Y² and R¹-R⁷ is a WSG, or at least one moiety selected from Y¹, Y² and R¹-R⁷ is substituted with a WSG, where the WSG is of the formula (la):

      -(L¹)ₙ₁ - (X¹)ₙ₂ - ((L²)ₙ₃-Z¹)ₘ (Ia)

      wherein:
      n1, n2 and n3 are each independently 0 or 1;
      L¹, if present, is a linker;
      L² if present, is a linker;
      X¹, if present, is a branching point;
      Z¹ is a water soluble polymer comprising 6-24 monomeric units; and
      m is an integer from 1 to 3.
77. The yellow green dipyrromethene-based dye of clause 76, further comprising a WSG of formula (Ib):

   -(L¹)ₙ₁-(X¹)ₙ₂-((L²)ₙ₃-Z²)ₘ (Ib)

   wherein:
   n1, n2 and n3 are each independently 0 or 1;
   L¹, if present, is a linker;
   L² if present, is a linker;
   X¹, if present, is a branching point;
   Z² is a water soluble polymer comprising 1-50 monomeric units; and
   m is an integer from 1 to 3.
78. The yellow green dipyrromethene-based dye of clause 76 or 77, wherein the WSG comprises a PEG moiety.
79. The yellow green dipyrromethene-based dye of any one of clauses 76 to 78, wherein one or more water solubilizing groups (WSGs) is independently selected from the following formulae: 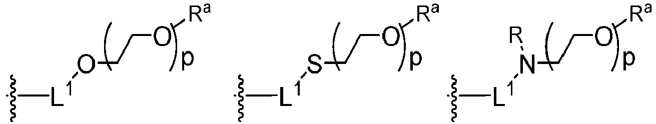 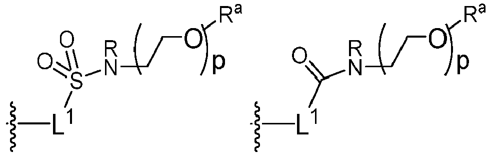 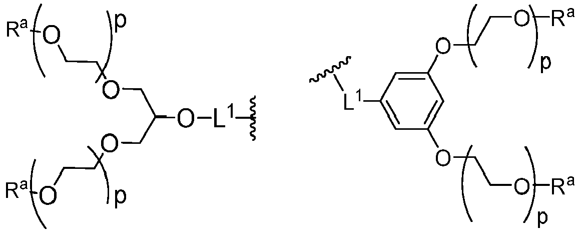 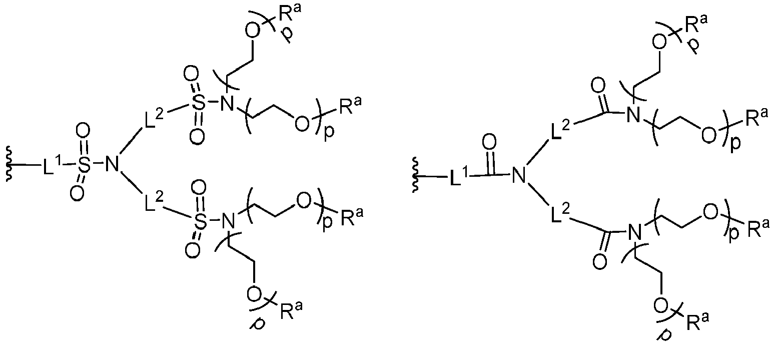 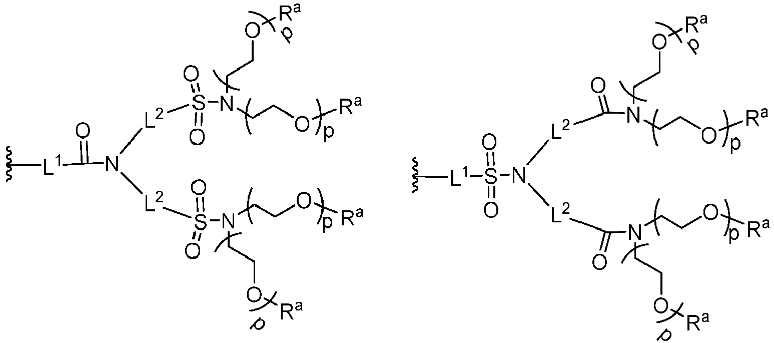 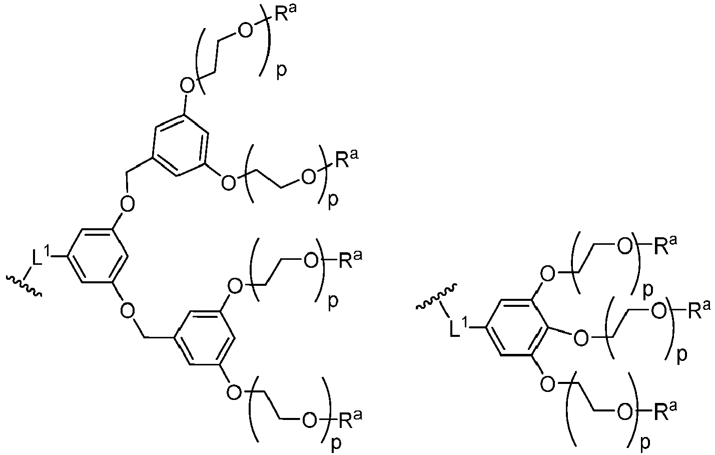 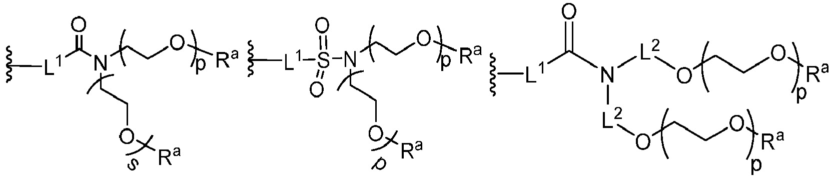 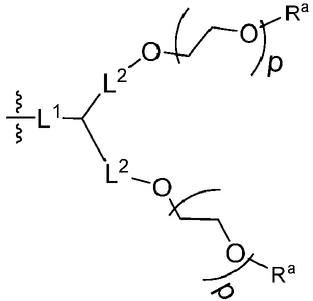 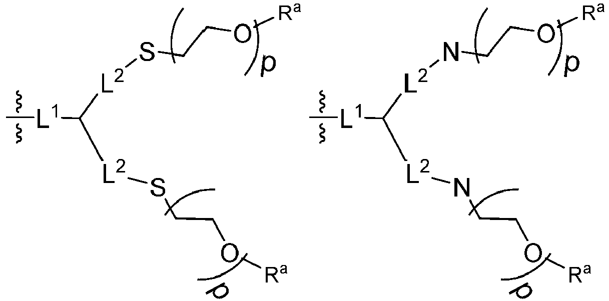 wherein:
   L¹ and L² are each independently an optional linker;
   R¹¹ and R are each independently H, alkyl or substituted alkyl; and each p is an integer from 6 to 24.
80. The yellow green dipyrromethene-based dye of any one of clauses 76 to 79, wherein R¹ is selected from, substituted alkyl, and substituted aryl, wherein the substituent comprises a chemoselective tag.
81. The yellow green dipyrromethene-based dye of any one of clauses 76 to 80, of the formula (IIIa): 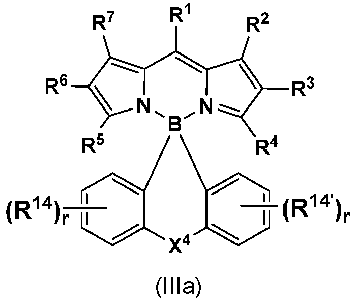 wherein:
   X⁴ is selected from O, S, NR, and CR₂;
   each R is independently selected from H, alkyl, and substituted alkyl;
   each r is independently an integer from 0 to 4; and
   each R¹⁴ and R^{14'}, if present, is independently selected from halogen, hydroxyl, cyano, amino, substituted amino, thiol, substituted thiol, amido, substituted amido, acyl, substituted acyl, alkyl, substituted alkyl, aryl, substituted aryl, heteroaryl, substituted heteroaryl, alkoxy, substituted alkoxy, alkenyl, substituted alkenyl, alkynyl, substituted alkynyl, and water soluble group (WSG).
82. The yellow green dipyrromethene-based dye of any one of clauses 76 to 81, wherein at least one of R⁵ and R⁶ and R⁴ and R³ together form a divalent radical and are cyclically linked and together with the carbon atoms to which they are bound provide a 5- or 6-membered fused heterocycle, carbocycle, aryl or heteroaryl ring (e.g., a 5- or 6-membered ring comprising carbon atoms and 0-3 heteroatoms selected from O, S and N), which ring may be unsubstituted or further substituted with a substituent independently selected from alkyl, substituted alkyl, alkoxy, substituted alkoxy, aryl, substituted aryl, heteroaryl, substituted heteroaryl, alkenyl, substituted alkenyl, alkynyl, substituted alkynyl, and water solubilizing group (WSG).
83. The yellow green dipyrromethene-based dye of clause 82, of the formula (IIIb) or (IIIc): 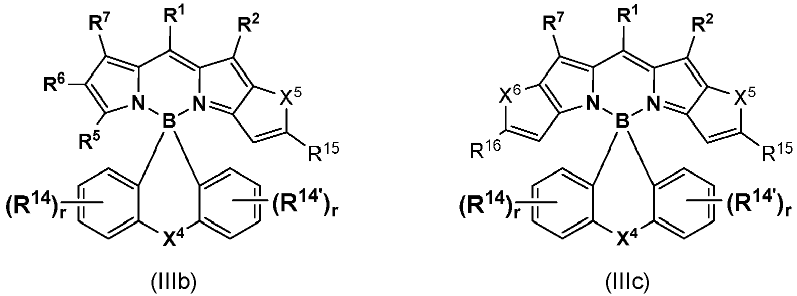 wherein:
   X⁵ and X⁶ are each independently selected from O, S, NR, and CR₂;
   each R is independently selected from H, alkyl, and substituted alkyl; and
   R¹⁵ and R¹⁶, are each independently selected from hydrogen, halogen, hydroxyl, cyano, amino, substituted amino, thiol, substituted thiol, amido, substituted amido, acyl, substituted acyl, alkyl, substituted alkyl, aryl, substituted aryl, heteroaryl, substituted heteroaryl, alkoxy, substituted alkoxy, alkenyl, substituted alkenyl, alkynyl, substituted alkynyl, and water soluble group (WSG).
84. The yellow green dipyrromethene-based dye of clause 83, wherein X⁵ and X⁶ are O.
85. The yellow green dipyrromethene-based dye of any one of clauses 76 to 84, of any one of the following structures: 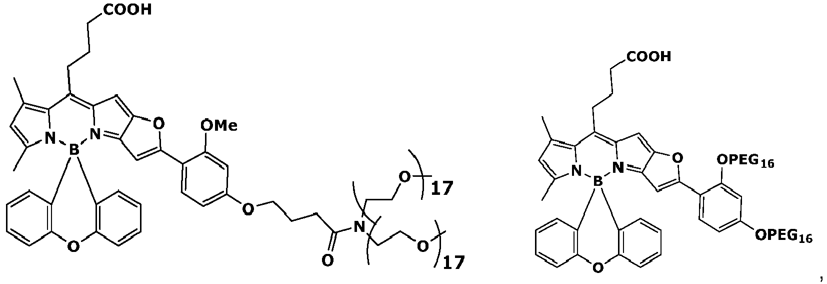 and 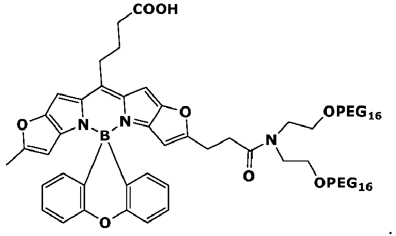
86. A tandem dye comprising:
   a light harvesting chromophore comprising:
   a backbone;
   a donor chromophore linked to the backbone, wherein the donor chromophore is a water-soluble dipyrromethene-based dye that absorbs in the yellow-green range at 561 nm; and
   an energy acceptor comprising a pendant acceptor fluorophore linked to the backbone, and configured in energy-receiving proximity to the donor chromophore.
87. The tandem dye of clause 86, wherein the donor chromophore has an absorption maxima from 555 to 585 nm.
88. The tandem dye of clause 87, wherein the donor chromophore has an absorption maxima from 558 to 568 nm.
89. The tandem dye of clause 88, wherein the donor chromophore has an absorption maxima of about 561 nm.
90. The tandem dye of any one of clauses 86 to 89, wherein the donor chromophore is a water-soluble dipyrromethene-boron dye.
91. The tandem dye of any one of clauses 86 to 90, wherein the donor chromophore is of formula (I): 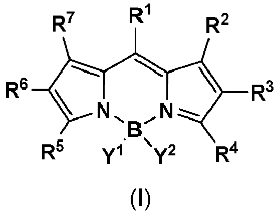 wherein:
   R¹-R⁷ are each independently selected from H, alkyl, halogen, substituted alkyl, alkoxy, substituted alkoxy, amino, substituted amino, amido, substituted amido, thiol, substituted thiol, aryl, substituted aryl, heteroaryl, substituted heteroaryl, heterocycle, substituted heterocycle, alkenyl, substituted alkenyl, alkynyl, substituted alkynyl, water solubilizing group (WSG), and -T¹-Z¹, or
   optionally any one or more pairs of substituents selected from R⁶ and R⁷, R² and R³, R⁵ and R⁶, R³ and R⁴, R² and R¹ and R⁷ and R¹, together form a divalent radical and are cyclically linked and together with the carbon atoms to which they are bound provide a 5- or 6-membered fused heterocycle, carbocycle, aryl or heteroaryl ring (e.g., a 5- or 6-membered ring comprising carbon atoms and 0-3 heteroatoms selected from O, S and N), which ring may be unsubstituted or further substituted with a substituent independently selected from alkyl, substituted alkyl, alkoxy, substituted alkoxy, aryl, substituted aryl, heteroaryl, substituted heteroaryl, alkenyl, substituted alkenyl, alkynyl, substituted alkynyl, water solubilizing group (WSG), and -T¹-Z¹;
   T¹ is an optional linker;
   Z¹ is a repeat unit of a compound of formula (I); and
   Y¹ and Y² are independently selected from F, OH, H, cyano, alkyl, substituted alkyl, aryl, substituted aryl, heteroaryl, substituted heteroaryl, alkoxy, substituted alkoxy, alkenyl, substituted alkenyl, alkynyl, substituted alkynyl, and a water solubilizing group (WSG), or
   Y¹ and Y² together with the boron atom to which they are bound form a 5- or 6-membered ring (e.g., a 5- or 6-membered ring comprising a boron atom, carbon atoms and 0-3 heteroatoms selected from O, S and N), which ring may be unsubstituted or further substituted with a substituent independently selected from alkyl, substituted alkyl, alkoxy, substituted alkoxy, aryl, substituted aryl, heteroaryl, substituted heteroaryl, alkenyl, substituted alkenyl, alkynyl, substituted alkynyl, water solubilizing group (WSG), and -T¹-Z¹; and
   wherein at least one moiety selected from Y¹, Y² and R¹-R⁷ is a WSG, or at least one moiety selected from Y¹, Y² and R¹-R⁷ is substituted with a WSG, where the WSG is of the formula (Ia):

      -(L¹)ₙ₁-(X¹)ₙ₂-((L²)ₙ₃-Z¹)ₘ (Ia)

      wherein:
      n1, n2 and n3 are each independently 0 or 1;
      L¹, if present, is a linker;
      L² if present, is a linker;
      X¹, if present, is a branching point;
      Z¹ is a water soluble polymer comprising 6-24 monomeric units; and
      m is an integer from 1 to 3.
92. The tandem dye of clause 91, wherein the donor chromophore of formula (I) further comprises a WSG of formula (Ib):

   -(L¹)ₙ₁-(X¹)ₙ₂-((L²)ₙ₃-Z²)ₘ (Ib)

   wherein:
   n1, n2 and n3 are each independently 0 or 1;
   L¹, if present, is a linker;
   L² if present, is a linker;
   X¹, if present, is a branching point;
   Z² is a water soluble polymer comprising 1-50 monomeric units; and
   m is an integer from 1 to 3.
93. The tandem dye of clause 91 or 92, wherein one or more water solubilizing groups (WSGs) comprises a PEG moiety.
94. The tandem dye of clause 93, wherein one or more water solubilizing groups (WSGs) is independently selected from the following formulae: 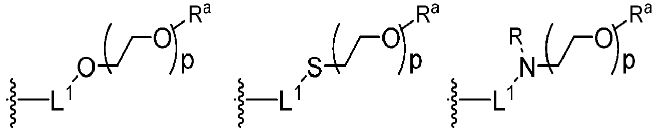 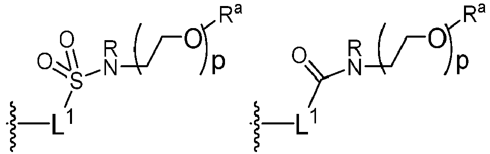 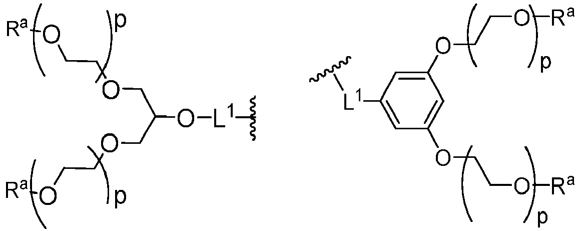 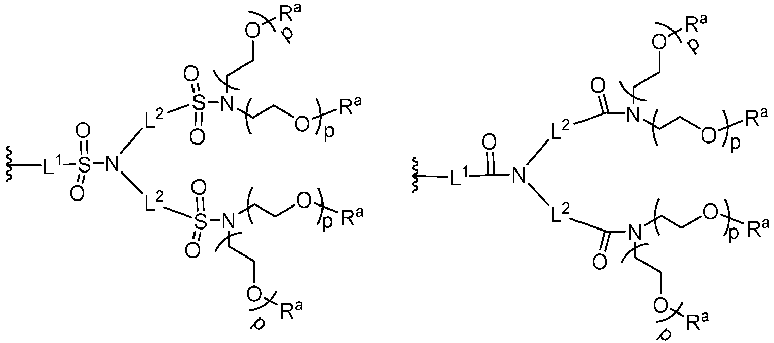 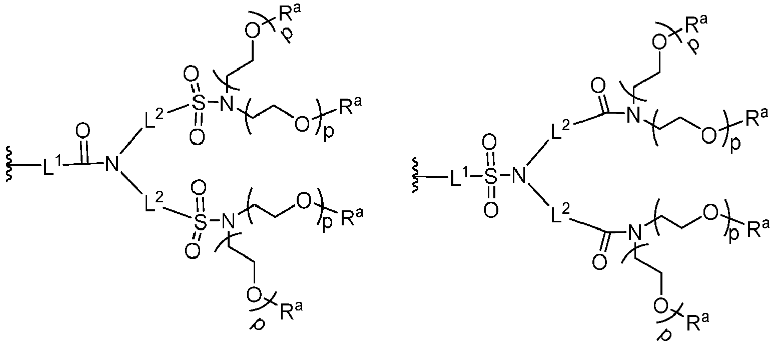 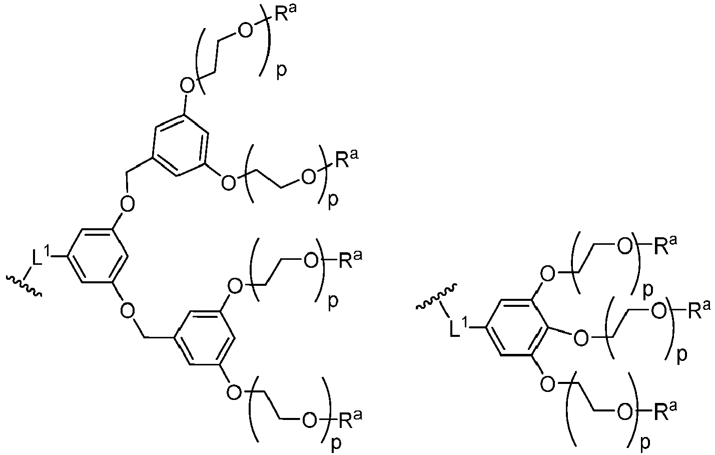 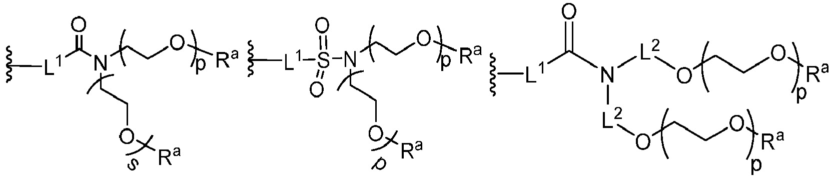 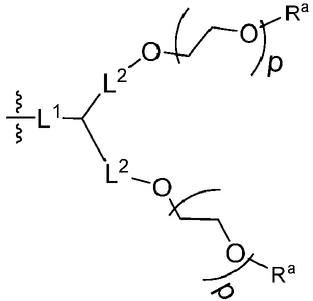 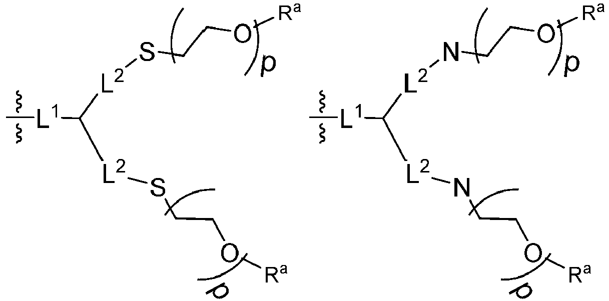 wherein:
   L¹ and L² are each independently an optional linker;
   R¹¹ and R are each independently H, alkyl or substituted alkyl; and
   each p is an integer from 6 to 24.
95. The tandem dye of clause any one of clauses 91 to 94, wherein R¹ is selected from substituted alkyl, and substituted aryl, wherein the substituent comprises a chemoselective tag.
96. The tandem dye of any one of clauses 91 to 95, wherein Y¹ and Y² each comprise a water solubilizing group (WSG).
97. The tandem dye of any one of clauses 91 to 96, wherein the donor chromophore is of the formula (II): 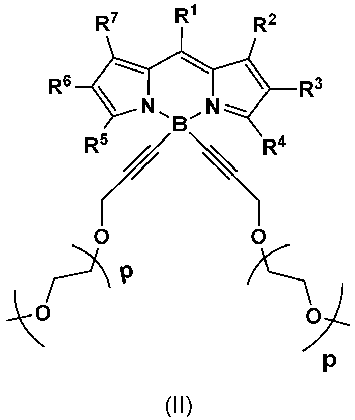 wherein p is an integer from 6-24.
98. The tandem dye of any one of clauses 91 to 97, wherein R⁵ is selected from halogen, substituted alkyl, substituted alkenyl, alkenyl aryl, aryl, substituted aryl, heterocycle, substituted heterocycle, spiro heterocycle, substituted spiro heterocycle, heteroaryl, substituted heteroaryl, substituted amino, substituted amido, and substituted thiol.
99. The tandem dye of clause 98, wherein R⁵ and R⁴ are the same.
100. The tandem dye of any one of clauses 91 to 99, wherein the donor chromophore is of the formula (IIa): 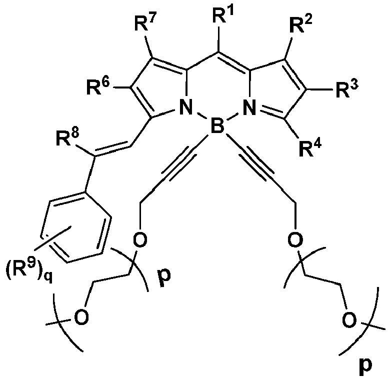 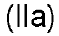 wherein:
   R⁸ is selected from H, halogen, cyano, alkyl, substituted alkyl, aryl, substituted aryl, heteroaryl, substituted heteroaryl, alkoxy, substituted alkoxy, alkenyl, substituted alkenyl, alkynyl, substituted alkynyl, and water solubilizing group (WSG), or
   R⁸ and R⁶ together with the atoms to which they are bound form a 5- or 6-membered heterocycle, carbocycle, aryl or heteroaryl ring (e.g., a 5- or 6-membered ring comprising carbon atoms and 0-3 heteroatoms selected from O, S and N), which ring may be unsubstituted or further substituted with a substituent independently selected from alkyl, substituted alkyl, alkoxy, substituted alkoxy, aryl, substituted aryl, heteroaryl, substituted heteroaryl, alkenyl, substituted alkenyl, alkynyl, substituted alkynyl, and water solubilizing group (WSG);
   q is an integer from 0 to 5; and
   each R⁹, if present, is independently selected from alkyl, substituted alkyl, hydroxy, alkoxy, substituted alkoxy, amino, substituted amino, thiol, substituted thiol, cyano, aryl, substituted aryl, heteroaryl, substituted heteroaryl, alkenyl, substituted alkenyl, alkynyl, substituted alkynyl, and water solubilizing group (WSG), or
   optionally two adjacent R⁹ groups, together form a divalent radical and are cyclically linked and together with the carbon atoms to which they are bound provide a 5- or 6-membered fused heterocycle, carbocycle, aryl or heteroaryl ring (e.g., a 5- or 6-membered ring comprising carbon atoms and 0-3 heteroatoms selected from O, S and N), which ring may be unsubstituted or further substituted with a substituent independently selected from alkyl, substituted alkyl, alkoxy, substituted alkoxy, aryl, substituted aryl, heteroaryl, substituted heteroaryl, alkenyl, substituted alkenyl, alkynyl, substituted alkynyl, and water solubilizing group (WSG).
101. The tandem dye of clause 100, wherein R⁸ is H.
102. The tandem dye of clause 100, wherein R⁸ and R⁶ together with the atoms to which they are bound form a 5- membered carbocycle, which carbocycle is optionally further substituted with a substituent independently selected from alkyl, substituted alkyl, alkoxy, substituted alkoxy, aryl, substituted aryl, heteroaryl, substituted heteroaryl, alkenyl, substituted alkenyl, alkynyl, substituted alkynyl, and water solubilizing group (WSG).
103. The tandem dye of any one of clauses 100 to 102, wherein the donor chromophore is of any one of the following structures: 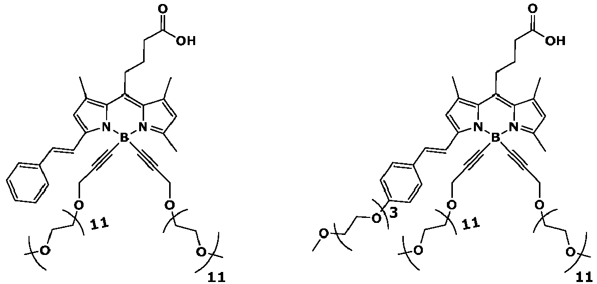 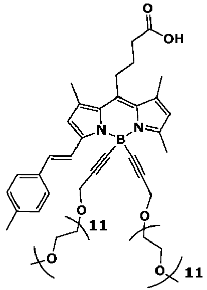 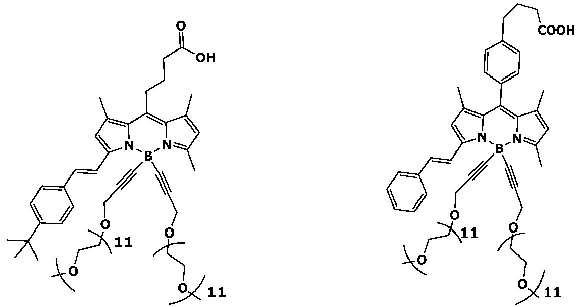 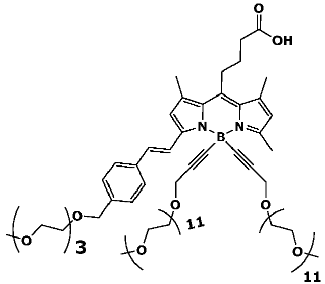 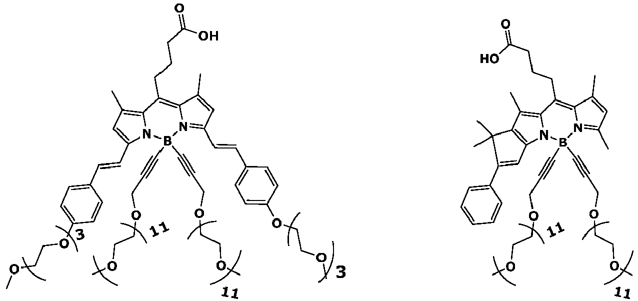 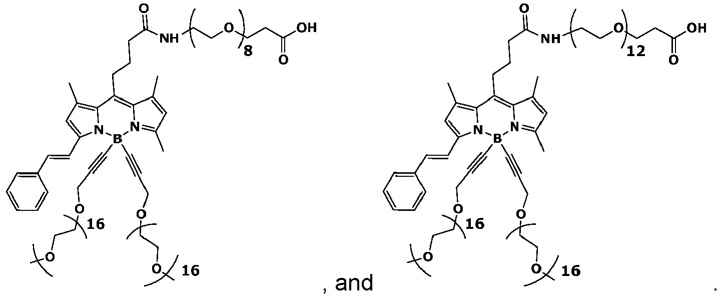
104. The tandem dye of any one of clauses 91 to 99, wherein the donor chromophore is of the formula (IIb): 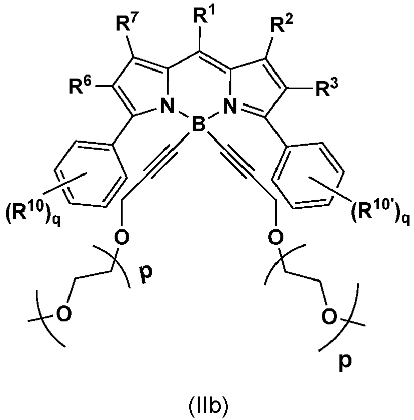 wherein:
   each q is independently an integer from 0 to 5; and
   R¹⁰ and R^{10'}, if present, are each independently selected from halogen, hydroxyl, cyano, amino, substituted amino, thiol, substituted thiol, alkyl, substituted alkyl, aryl, substituted aryl, heteroaryl, substituted heteroaryl, alkoxy, substituted alkoxy, alkenyl, substituted alkenyl, alkynyl, substituted alkynyl, and water soluble group (WSG), or
   at least one R¹⁰ group is at an ortho position, and R¹⁰ and R⁶ together with the atoms to which they are bound form a 5- or 6-membered ring (e.g., a 5- or 6-membered ring comprising a boron atom, carbon atoms and 0-3 heteroatoms selected from O, S and N), which ring may be unsubstituted or further substituted with a substituent independently selected from alkyl, substituted alkyl, alkoxy, substituted alkoxy, aryl, substituted aryl, heteroaryl, substituted heteroaryl, alkenyl, substituted alkenyl, alkynyl, substituted alkynyl, and water solubilizing group (WSG); and
   at least one R^{10'} is an ortho position, and R¹⁰ and R³ together with the atoms to which they are bound form a 5- or 6-membered ring (e.g., a 5- or 6-membered ring comprising a boron atom, carbon atoms and 0-3 heteroatoms selected from O, S and N), which ring may be unsubstituted or further substituted with a substituent independently selected from alkyl, substituted alkyl, alkoxy, substituted alkoxy, aryl, substituted aryl, heteroaryl, substituted heteroaryl, alkenyl, substituted alkenyl, alkynyl, substituted alkynyl, and water solubilizing group (WSG).
105. The tandem dye of clause 104, wherein each q is 1, and R¹⁰ and R^{10'} are both present in their respective para positions.
106. The tandem dye of clause 105, wherein R¹⁰ and R^{10'} are each independently selected from cyano and a PEG moiety.
107. The tandem dye of any one of clauses 104 to 106, wherein the donor chromophore is of any one of the following structures: 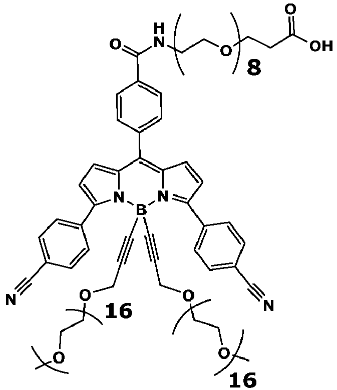 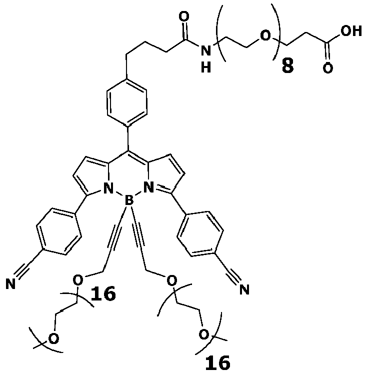 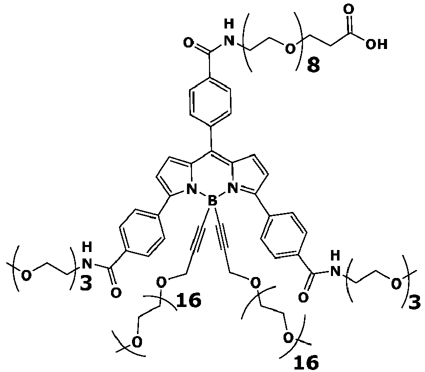 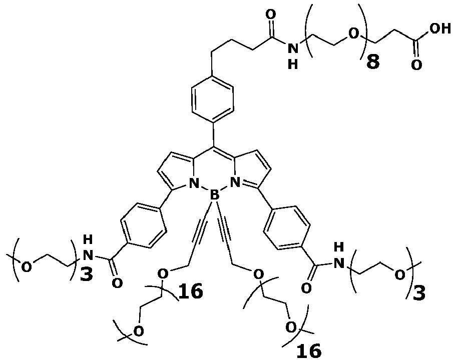 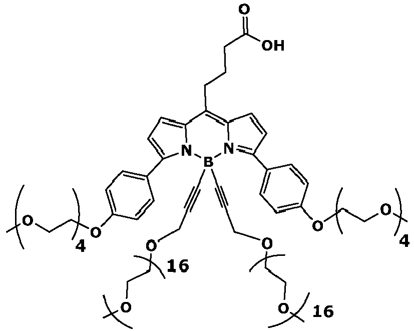 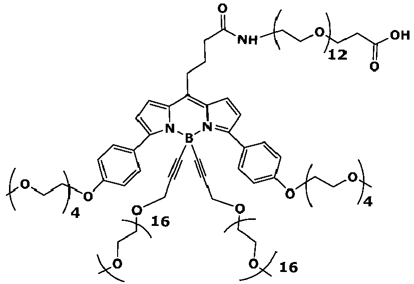 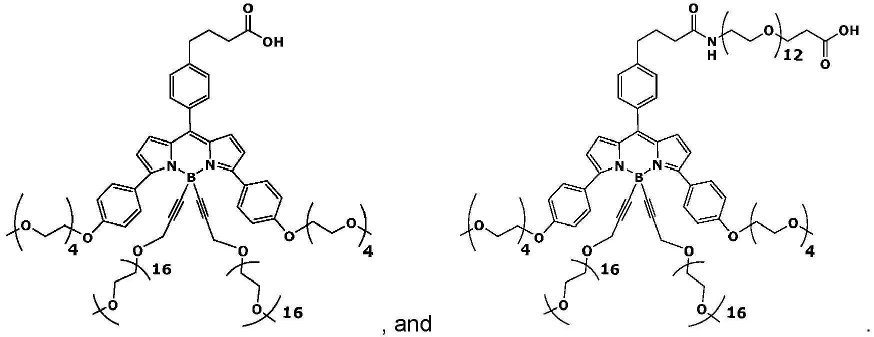
108. The tandem dye of any one of clauses 91 to 99, wherein the donor chromophore is of the formula (IIc): 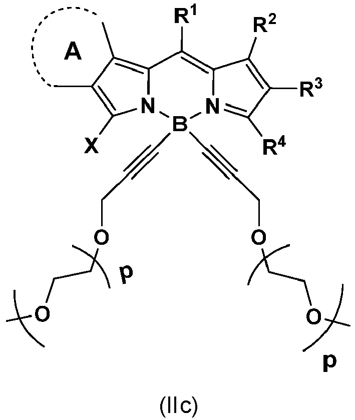 wherein:
   X is a halogen; and
   ring A is a 5- or 6-membered fused heterocycle, carbocycle, aryl or heteroaryl ring (e.g., a 5- or 6-membered ring comprising carbon atoms and 0-3 heteroatoms selected from O, S and N), which ring may be unsubstituted or further substituted with a substituent independently selected from alkyl, substituted alkyl, alkoxy, substituted alkoxy, aryl, substituted aryl, heteroaryl, substituted heteroaryl, alkenyl, substituted alkenyl, alkynyl, substituted alkynyl, and a water solubilizing group (WSG).
109. The tandem dye of clause 108, wherein the A ring is an aryl ring.
110. The tandem dye of clause 108 or 109, wherein the donor chromophore is of any one of the following compounds: 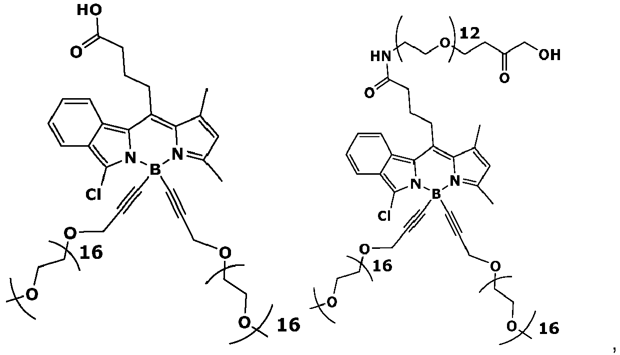 and 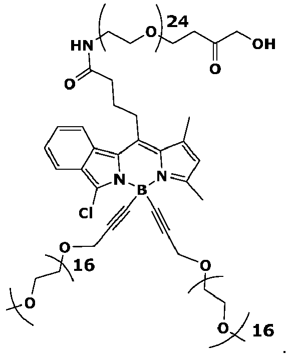
111. The tandem dye of any one of clauses 91 to 99, wherein the donor chromophore is of the formula (IId): 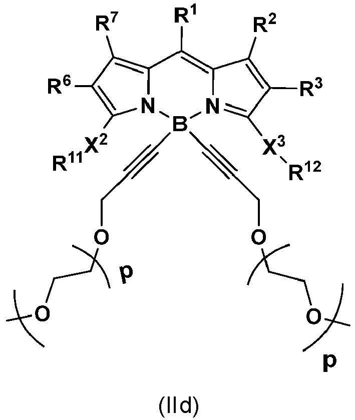 wherein:
   X² and X³ are each independently selected from NR¹³, S and O;
   R¹³ is selected from H, alkyl, and substituted alkyl;
   R¹¹ and R¹² are each independently selected from aryl, substituted aryl, heteroaryl, substituted heteroaryl, and water soluble group (WSG), or
   when X² is NR¹³, optionally R¹³ and R¹¹ together with the nitrogen atom to which they are attached, form a heterocyclic or spiro heterocyclic ring, which ring may be unsubstituted or further substituted with a substituent independently selected from alkyl, substituted alkyl, alkoxy, substituted alkoxy, aryl, substituted aryl, heteroaryl, substituted heteroaryl, alkenyl, substituted alkenyl, alkynyl, substituted alkynyl, and a water solubilizing group (WSG), and
   when X³ is NR¹³, optionally R¹³ and R¹² together with the nitrogen atom to which they are attached, form a heterocyclic or spiro heterocyclic ring, which ring may be unsubstituted or further substituted with a substituent independently selected from alkyl, substituted alkyl, alkoxy, substituted alkoxy, aryl, substituted aryl, heteroaryl, substituted heteroaryl, alkenyl, substituted alkenyl, alkynyl, substituted alkynyl, and water solubilizing group (WSG).
112. The tandem dye of clause 111, wherein X² and X³ are each NR¹³, wherein R¹³ is H.
113. The tandem dye of clause 111, wherein X² is NR¹³, and R¹³ and R¹¹ together with the nitrogen atom to which they are attached form a heterocyclic or a spiro heterocyclic ring; and X³ is NR¹³, and R¹³ and R¹² together with the nitrogen atom to which they are attached form a heterocyclic or a spiro heterocyclic ring.
114. The polymeric tandem dye of clause 111, wherein X² and X³ are both S.
115. The tandem dye of any one of clauses 111 to 114, wherein the donor chromophore is of any one of the following structures: 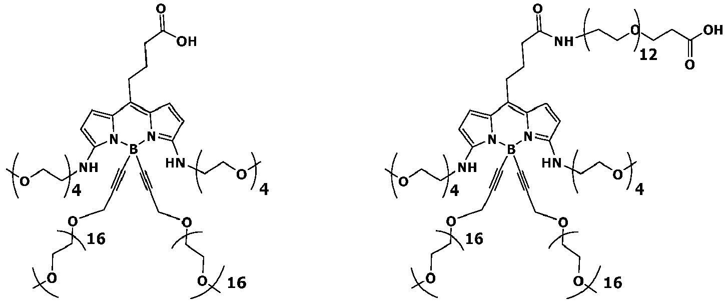 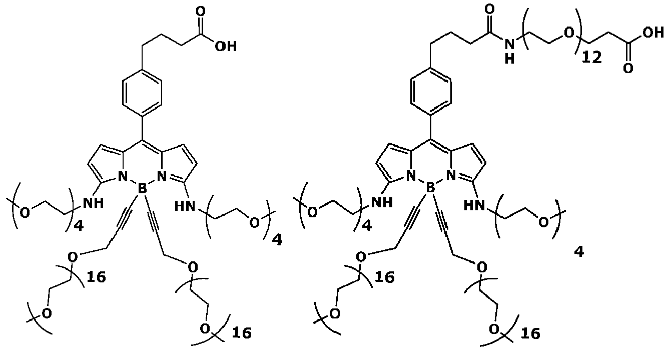 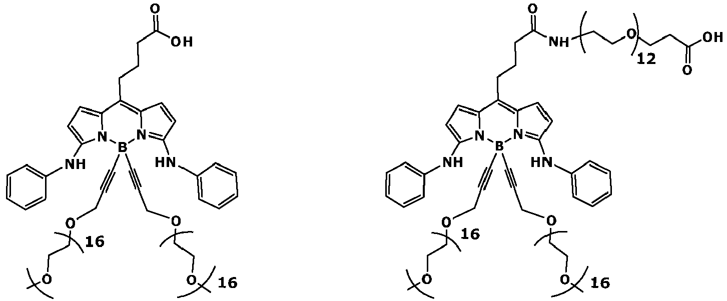 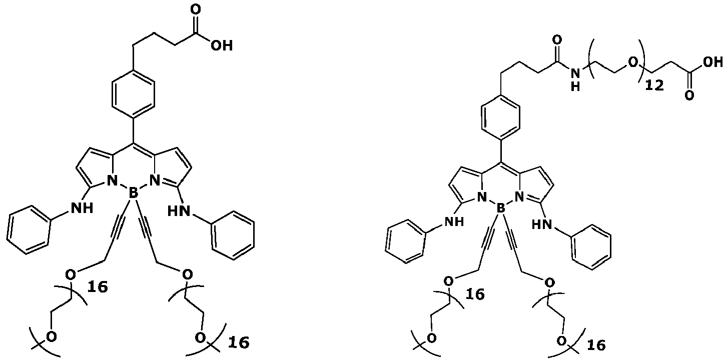 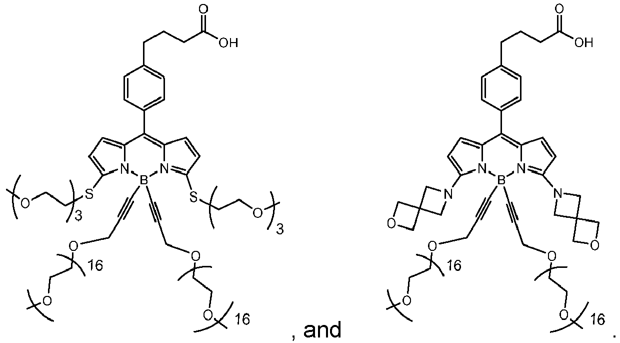
116. The tandem dye of any one of clauses 91 to 95, wherein the donor chromophore is of the formula (III): 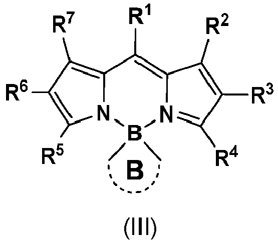 wherein:
   ring B is a 5- or 6-membered cyclic group (e.g., a 5- or 6-membered ring comprising a boron atom, carbon atoms and 0-3 heteroatoms selected from O, S and N), which cyclic group may be unsubstituted or further substituted with one or more substituents independently selected from alkyl, substituted alkyl, alkoxy, substituted alkoxy, aryl, substituted aryl, heteroaryl, substituted heteroaryl, alkenyl, substituted alkenyl, alkynyl, substituted alkynyl, and water solubilizing group (WSG).
117. The tandem dye of clause 116, wherein the donor chromophore is of the formula (IIIa): 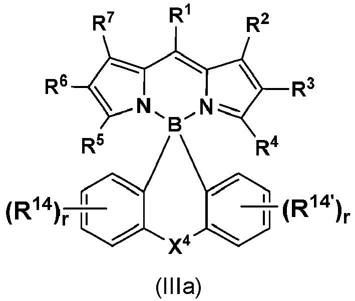 wherein:
   X⁴ is selected from O, S, NR, and CR₂;
   each R is independently selected from H, alkyl, and substituted alkyl;
   each r is independently an integer from 0 to 4; and
   each R¹⁴ and R^{14'}, if present, is independently selected from halogen, hydroxyl, cyano, amino, substituted amino, thiol, substituted thiol, amido, substituted amido, acyl, substituted acyl, alkyl, substituted alkyl, aryl, substituted aryl, heteroaryl, substituted heteroaryl, alkoxy, substituted alkoxy, alkenyl, substituted alkenyl, alkynyl, substituted alkynyl, and water solubilizing group (WSG).
118. The tandem dye of clause 116 or 117, wherein at least one of R⁵ and R⁶ and R⁴ and R³ together form a divalent radical and are cyclically linked and together with the carbon atoms to which they are bound provide a 5- or 6-membered fused heterocycle, carbocycle, aryl or heteroaryl ring (e.g., a 5- or 6-membered ring comprising carbon atoms and 0-3 heteroatoms selected from O, S and N), which ring may be unsubstituted or further substituted with a substituent independently selected from alkyl, substituted alkyl, alkoxy, substituted alkoxy, aryl, substituted aryl, heteroaryl, substituted heteroaryl, alkenyl, substituted alkenyl, alkynyl, substituted alkynyl, and water solubilizing group (WSG).
119. The tandem dye of clause 118, wherein the donor chromophore is of the formula (IIIb) or (IIIc): 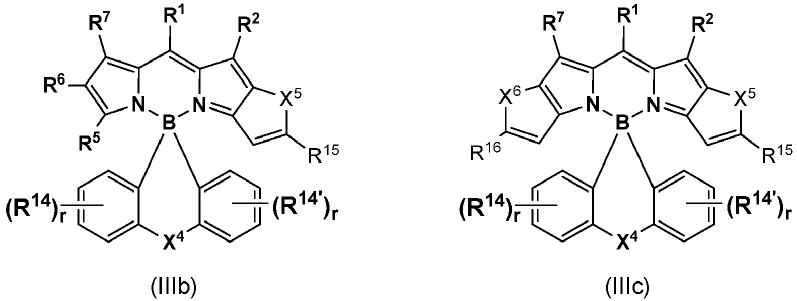 wherein:
   X⁵ and X⁶ are each independently selected from O, S, NR, and CR₂;
   each R is independently selected from H, alkyl, and substituted alkyl; and
   R¹⁵ and R¹⁶, are each independently selected from hydrogen, halogen, hydroxyl, cyano, amino, substituted amino, thiol, substituted thiol, amido, substituted amido, acyl, substituted acyl, alkyl, substituted alkyl, aryl, substituted aryl, heteroaryl, substituted heteroaryl, alkoxy, substituted alkoxy, alkenyl, substituted alkenyl, alkynyl, substituted alkynyl, and water solubilizing group (WSG).
120. The tandem dye of clause 119, wherein X⁵ and X⁶ are O.
121. The tandem dye of any one of clauses 116 to 120, wherein the donor chromophore is of any one of the following structures: 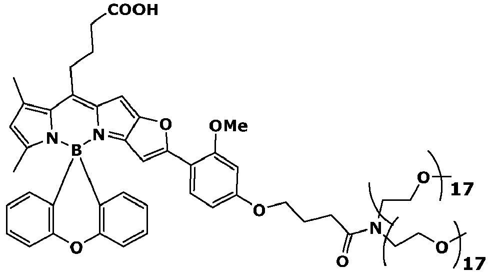 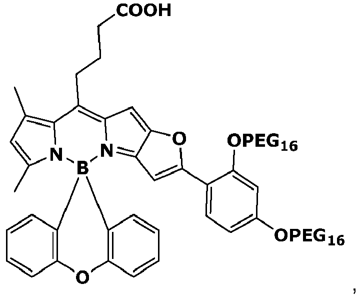 and 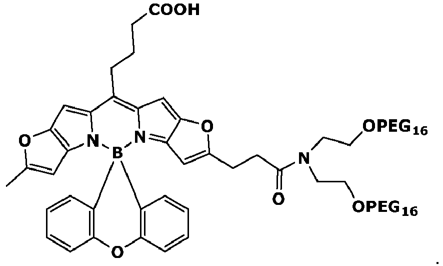
122. The tandem dye of any one of clauses 91 to 97, wherein R³ is -L¹-Z¹.
123. The tandem dye of clause 122, wherein the donor chromophore is of one of the following structures: 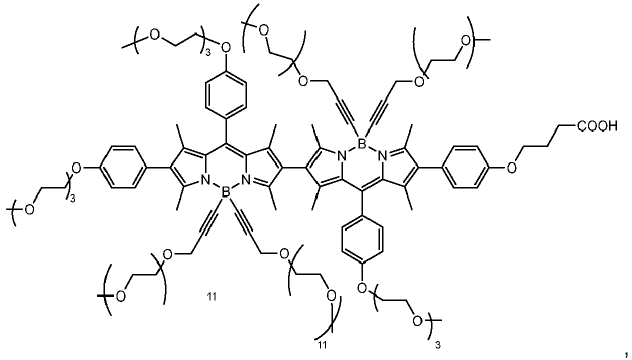 and 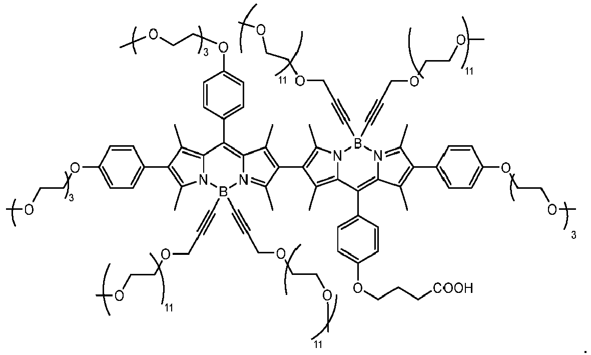
124. The tandem dye of any one of clauses 86 to 89, of formula (IV): 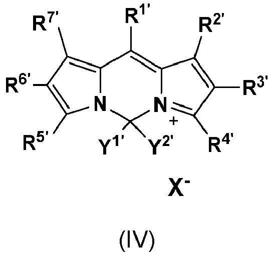 wherein:
   X⁻ is a counterion;
   R^{1'}-R^{7'} are each independently selected from H, alkyl, halogen, substituted alkyl, alkoxy, substituted alkoxy, amino, substituted amino, amido, substituted amido, thiol, substituted thiol, aryl, substituted aryl, heteroaryl, substituted heteroaryl, heterocycle, substituted heterocycle, alkenyl, substituted alkenyl, alkynyl, substituted alkynyl, water solubilizing group (WSG), and -T¹-Z¹, or
   optionally any one or more pairs of substituents selected from R^{6'} and R^{7'}, R^{2'} and R^{3'}, R^{5'} and R^{6'}, R^{3'} and R^{4'}, R^{2'} and R^{1'} and R^{7'} and R^{1'}, together form a divalent radical and are cyclically linked and together with the carbon atoms to which they are bound provide a 5- or 6-membered fused heterocycle, carbocycle, aryl or heteroaryl ring (e.g., a 5- or 6-membered ring comprising carbon atoms and 0-3 heteroatoms selected from O, S and N), which ring may be unsubstituted or further substituted with a substituent independently selected from alkyl, substituted alkyl, alkoxy, substituted alkoxy, aryl, substituted aryl, heteroaryl, substituted heteroaryl, alkenyl, substituted alkenyl, alkynyl, substituted alkynyl, water solubilizing group (WSG), and -T¹-Z¹;
   T¹ is an optional linker;
   Z¹ is a repeat unit of a compound of formula (I); and
   Y^{1'} and Y^{2'} are independently selected from F, OH, H, cyano, alkyl, substituted alkyl, aryl, substituted aryl, heteroaryl, substituted heteroaryl, alkoxy, substituted alkoxy, alkenyl, substituted alkenyl, alkynyl, substituted alkynyl, and WSG, or
   Y^{1'} and Y^{2'} together with the boron atom to which they are bound form a 5- or 6-membered ring (e.g., a 5- or 6-membered ring comprising a boron atom, carbon atoms and 0-3 heteroatoms selected from O, S and N), which ring may be unsubstituted or further substituted with a substituent independently selected from alkyl, substituted alkyl, alkoxy, substituted alkoxy, aryl, substituted aryl, heteroaryl, substituted heteroaryl, alkenyl, substituted alkenyl, alkynyl, substituted alkynyl, water solubilizing group (WSG), and -T¹-Z¹; and
   wherein at least one moiety selected from Y^{1'}, Y^{2'} and R^{1'}-R^{7'} is a WSG, or at least one moiety selected from Y^{1'}, Y^{2'} and R^{1'}-R^{7'} is substituted with a WSG, where the WSG is of the formula (la):

      -(L¹)ₙ₁ - (X¹)ₙ₂ - ((L²)ₙ₃-Z¹)ₘ (Ia)

      wherein:
      n1, n2 and n3 are each independently 0 or 1;
      L¹, if present, is a linker;
      L² if present, is a linker;
      X¹, if present, is a branching point;
      Z¹ is a water soluble polymer comprising 6-24 monomeric units; and
      m is an integer from 1 to 3.
125. The tandem dye of clause 124, further comprising a WSG of formula (Ib):

   -(L¹)ₙ₁ - (X¹)ₙ₂ - ((L²)ₙ₃-Z²)ₘ (Ib)

   wherein:
   n1, n2 and n3 are each independently 0 or 1;
   L¹, if present, is a linker;
   L² if present, is a linker;
   X¹, if present, is a branching point;
   Z² is a water soluble polymer comprising 1-50 monomeric units; and
   m is an integer from 1 to 3.
126. The tandem dye of clause 124 or 125, wherein the WSG comprises a PEG moiety.
127. The tandem dye of any one of clauses 124 to 126, wherein one or more water solubilizing groups (WSGs) is independently selected from the following formulae: 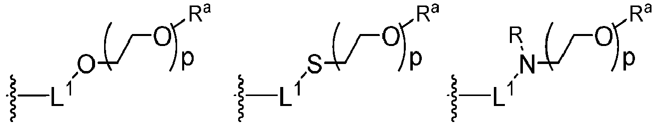 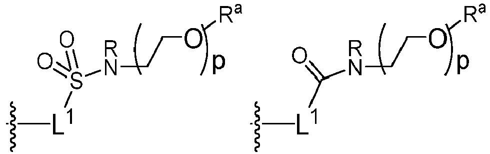 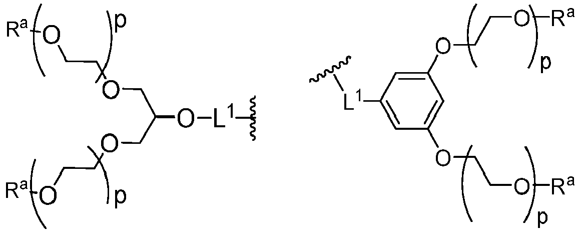 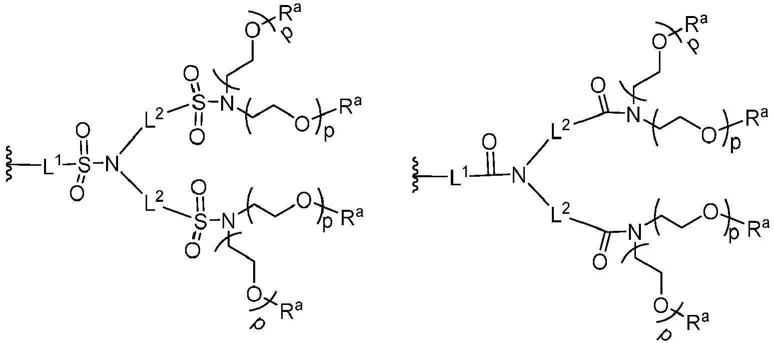 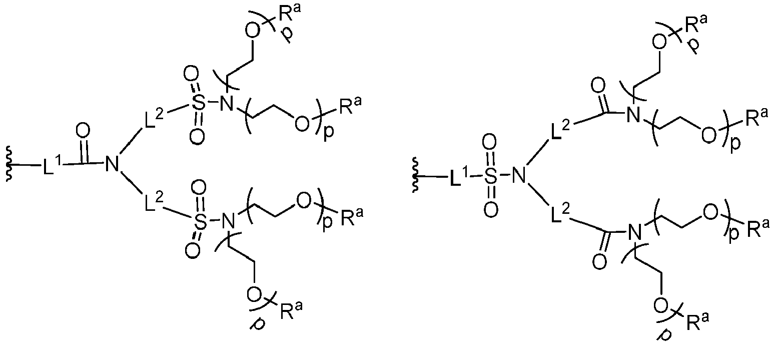 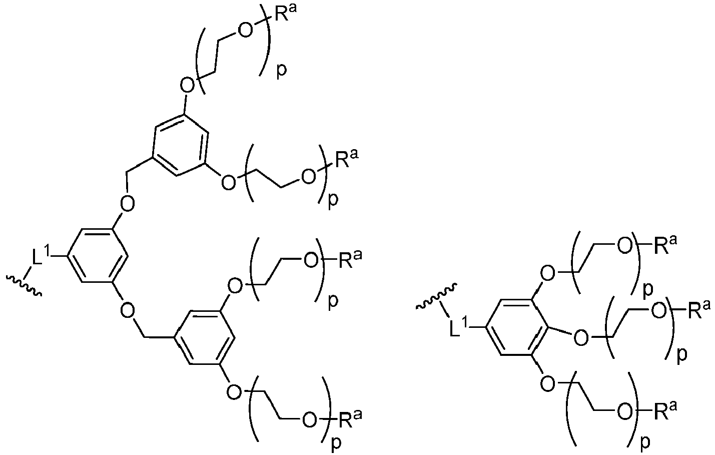 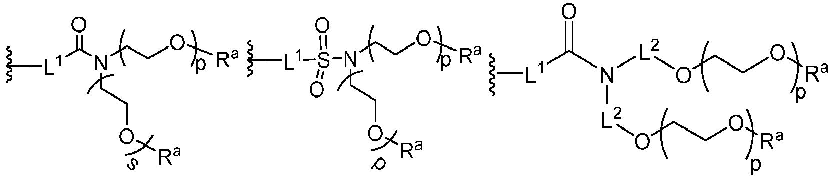 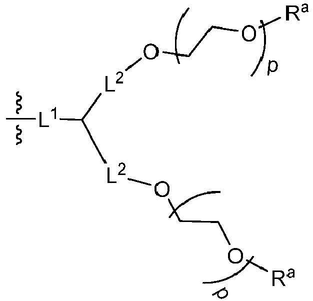 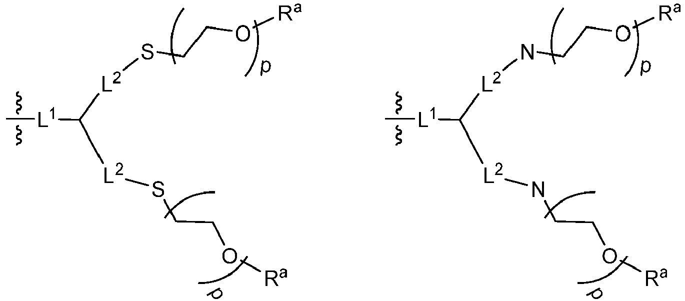 wherein:
   L¹ and L² are each independently an optional linker;
   R¹¹ and R are each independently H, alkyl or substituted alkyl; and
   each p is an integer from 6 to 24.
128. The tandem dye of any one of clauses 124 to 127, wherein R¹ is selected from substituted alkyl, and substituted aryl, wherein the substituent comprises a chemoselective tag.
129. The tandem dye of any one of clauses 124 to 128, wherein R^{5'} and R^{4'} both comprise a water solubilizing group (WSG).
130. The tandem dye of any one of clauses 124 to 129, of one of the following structures: 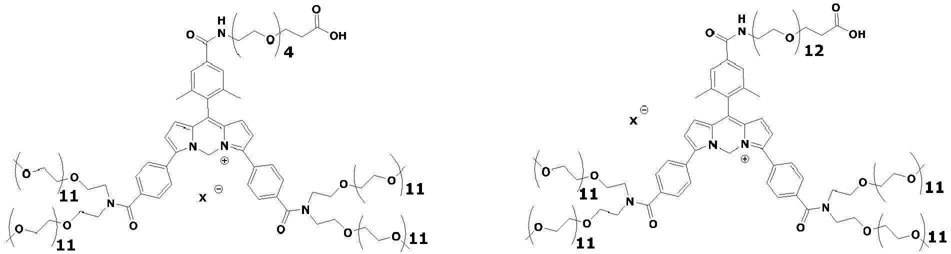
131. The tandem dye according to any one of clauses 86 to 130, wherein the backbone is a linear polymer.
132. The tandem dye according to clause 131, wherein the linear polymer is selected from a peptide, a peptoid, a hydrocarbon polymer, and a PEG polymer.
133. The tandem dye according to clause 132, wherein the linear polymer is a peptide.
134. The tandem dye according to clause 133, wherein the peptide is from 2 to 100 amino acids in length.
135. The tandem dye according to clause 134, wherein the peptide is from 5 to 30 amino acids in length.
136. The tandem dye according to any one of clauses 131 to 133, wherein the linear polymer comprises 2 to 1,000 repeating monomeric units.
137. The tandem dye according to clause 136, wherein the linear polymer comprises 2 to 500 repeating monomeric units.
138. The tandem dye according to clause 137, wherein the linear polymer comprises 2 to 100 repeating monomeric units.
139. The tandem dye according to any one of clauses 86 to 138, wherein a plurality of pendant donor chromophore groups are present, and are configured in energy-transferring proximity to the pendant acceptor fluorophore.
140. The tandem dye according to any one of clauses 86 to 139, wherein the pendant acceptor fluorophore is a small molecule fluorophore.
141. The tandem dye according to any one of clauses 86 to 140, wherein the pendant acceptor fluorophore is selected from the group consisting of a cyanine dye, a rhodamine dye, a perylene diimide dye, a xanthene dye, a coumarin dye, a polymethine, a pyrene, a dipyrromethene borondifluoride, a napthalimide, a thiazine dye and an acridine dye.
142. The tandem dye according to any one of clauses 86 to 141, wherein the pendant acceptor fluorophore is selected from fluorescein, 6-FAM, rhodamine, Texas Red, California Red, iFluor594, tetramethylrhodamine, a carboxyrhodamine, carboxyrhodamine 6G, carboxyrhodol, carboxyrhodamine 110, Cascade Blue, Cascade Yellow, coumarin, Cy2^{®}, Cy3^{®}, Cy3.5^{®}, Cy5^{®}, Cy5.5^{®}, Cy7^{®}, Cy-Chrome, DyLight 350, DyLight 405, DyLight 488, DyLight 549, DyLight 594, DyLight 633, DyLight 649, DyLight 680, DyLight 750, DyLight 800, phycoerythrin, PerCP (peridinin chlorophyll-a Protein), PerCP-Cy5.5, JOE (6-carboxy-4',5'-dichloro-2',7'-dimelhoxyfluorescein), NED, ROX (5-(and -6)-carboxy-X-rhodamine), HEX, Lucifer Yellow, Marina Blue, Oregon Green 488, Oregon Green 500, Oregon Green 514, Alexa Fluor^{®} 350, Alexa Fluor^{®} 430, Alexa Fluor^{®} 488, Alexa Fluor^{®} 532, Alexa Fluor^{®} 546, Alexa Fluor^{®} 568, Alexa Fluor^{®} 594, Alexa Fluor^{®} 633, Alexa Fluor^{®} 647, Alexa Fluor^{®} 660, Alexa Fluor^{®} 680, 7-amino-4-methylcoumarin-3-acetic acid, BODIPY^{®} FL, BODIPY^{®} FL-Br2, BODIPY^{®} 530/550, BODIPY^{®} 558/568, BODIPY^{®} 564/570, BODIPY^{®} 576/589, BODIPY^{®} 581/591, BODIPY^{®} 630/650, BODIPY^{®} 650/665, BODIPY^{®} R6G, BODIPY^{®} TMR, BODIPY^{®} TR, conjugates thereof and combinations thereof.
143. The tandem dye according to any one of clauses 86 to 142, wherein the ratio of donor chromophores to acceptor fluorophores is selected from 5:1, 6:1, 7:1, 8:1, 9:1, 10:1, and 20:2.
144. The tandem dye according to any one of clauses 86 to 143, wherein the light harvesting chromophore further comprises a specific binding member.
145. The tandem dye according to clause 144, wherein the specific binding member is linked to a terminal group of the light harvesting chromophore.
146. The tandem dye according to clause 144, wherein the specific binding member is linked to the backbone, at any point between the terminal groups of the light harvesting chromophore.
147. The tandem dye according to clause 146, wherein the specific binding member is linked to the backbone at a central point of the light harvesting chromophore.
148. The tandem dye according to any one of clauses 144 to 147, wherein the specific binding member is selected from an antibody, an antibody fragment or a binding derivative thereof.
149. The tandem dye according to any one of clauses 86 to 148, wherein the light harvesting chromophore is of formula (VA) or (VB): 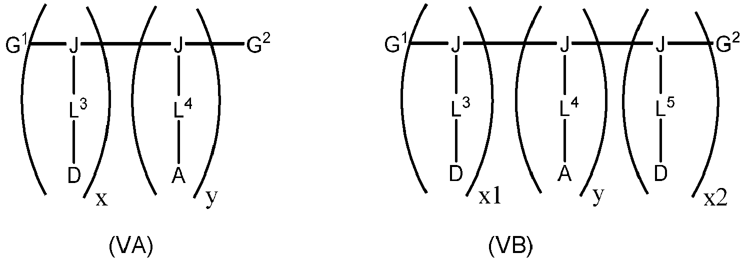 wherein:
   each J are repeat units of the backbone;
   L³-L⁵ are each independently a linker;
   each D is a donor chromophore;
   each A is an acceptor fluorophore;
   G¹ and G² are each independently selected from the group consisting of terminal group, a polymer segment, a donor chromophore, an acceptor fluorophore, a linker, and a linked specific binding member;
   x is 75 mol% or more;
   x1 + X2 is 75% or more; and
   y is 25 mol% or less.
150. The tandem dye according to clause 149, wherein the light harvesting chromophore is of formula (VA), and x is 80 mol% or more; or
   the light harvesting chromophore is of formula (VB), and x1 + X2 is 80 mol% or more.
151. The tandem dye according to clause 150, wherein the light harvesting chromophore is of formula (VA), and x is 90 mol% or more; or
   the light harvesting chromophore is of formula (VB), and x1 + X2 is 90 mol% or more.
152. The tandem dye according to clause 149, wherein the light harvesting chromophore is of formula (VA), and y is 20 mol% or less; or
   the light harvesting chromophore is of formula (VB), and y is 20 mol% or less.
153. The tandem dye according to clause 152, wherein the light harvesting chromophore is of formula (VA), and y is 10 mol% or less; or
   the light harvesting chromophore is of formula (VB), and y is 10 mol% or less.
154. The tandem dye according to any one of clauses 149 to 153, wherein G1 or G2 is a linked specific binding member.
155. The tandem dye according to any one of clauses 149 to 153, wherein any of J, L³-L⁵, D or A comprise a linked specific binding member.
156. The tandem dye according to any one of clauses 149 to 155, wherein each J is an amino acid or a peptide unit.
157. A method of evaluating a sample for the presence of a target analyte, the method comprising:
   (a) contacting the sample with an aggregation-resistant dye conjugate that specifically binds the target analyte to produce a labelling composition contacted sample, wherein the dye conjugate comprises:
      (i) a tandem dye according to any one of clauses 86 to 156; and
      (ii) a specific binding member linked to the tandem dye; and
   (b) assaying the labelling composition contacted sample for the presence of a dye conjugate-target analyte binding complex to evaluate whether the target analyte is present in the sample.
158. The method according to clause 157, further comprising contacting the sample with a second specific binding member that is support bound and specifically binds the target analyte.
159. The method according to clause 158, wherein the support comprises a magnetic particle.
160. The method according to any one of clauses 157 to 159, wherein the target analyte is associated with a cell.
161. The method according to clause 160, wherein the target analyte is a cell surface marker of the cell.
162. The method according to clause 161, wherein the cell surface marker is selected from the group consisting of a cell receptor and a cell surface antigen.
163. The method according to clause 160, wherein the target analyte is an intracellular target, and the method further comprises lysing the cell.
164. The method according to any one of clauses 157 to 163, wherein the method further comprises flow cytometrically analyzing the fluorescently labelled target analyte.
165. A method of labelling a target molecule, the method comprising:
   contacting the target molecule with a tandem dye to produce a labelled target molecule, wherein:
   the tandem dye is a dye according to any one of clauses 86-156, and comprises a conjugation tag that covalently links to the target molecule.
166. The method according to clause 165, further comprising fluorescently detecting the labelled target molecule.
167. The method according to clause 165 or 166, wherein the conjugation tag comprises a terminal functional group selected from an amino, a thiol, a hydroxyl, a hydrazine, a hydrazide, a azide, an alkyne, maleimide, iodoacetyl, amine, an active ester and a protein reactive group.
168. The method according to any one of clauses 165 to 167, wherein the target molecule is a specific binding member.
169. The method according to clause 168, wherein the specific binding member is an antibody.
170. The method according to clause 168, wherein the specific binding member is an antibody fragment or binding derivative thereof.
171. The method according to clause 170, wherein the antibody fragment or binding derivative thereof is selected from the group consisting of a Fab fragment, a F(ab')₂ fragment, a scFv, a diabody and a triabody.
172. A kit comprising:
   a water-soluble dipyrromethene-based dye of any one of clauses 1 to 45, or a tandem dye according to any one of clauses 86 to 156; and
   a container.
173. The kit according to clause 172, further comprising one or more components selected from the group consisting of a fluorophore, a specific binding member, a specific binding member conjugate, a cell, a support, a biocompatible aqueous elution buffer and instructions for use.
174. The kit according to clause 172 or 173, wherein the tandem dye is covalently linked to a specific binding member.
175. The kit according to clause 174, wherein the specific binding member is an antibody.
176. The kit according to clause 174, wherein the specific binding member is an antibody fragment or binding derivative thereof.
177. The kit according to clause 176, wherein the antibody fragment or binding derivative thereof is selected from the group consisting of a Fab fragment, a F(ab')₂ fragment, a scFv, a diabody and a triabody.

Notwithstanding the appended claims, the disclosure is also defined by the following clauses:
1. A dipyrromethene-based dye that efficiently transfers energy to an acceptor chromophore while a second dipyrromethene-based dye of the same or different chemical structure is within energy-receiving proximity therewith.
2. The dye of clause 1, wherein the efficient energy transfer occurs after excitation of the dye by light with a wavelength ranging from 540 nm to 590 nm.
3. The dye of any one of clauses 1-2, wherein the energy transfer is Forster resonance energy transfer (FRET).
4. The dye of any one of clauses 1-3, wherein the efficient energy transfer to the acceptor chromophore involves the transfer of 5% or more of the energy harvested by the dipyrromethene-based dye.
5. The dye of any one of clauses 1-4, where the dye has formula (I): 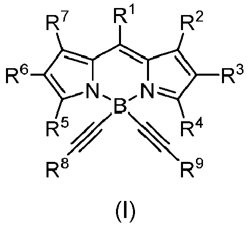 wherein:
   R¹-R⁷ are each independently selected from H, alkyl, substituted alkyl, alkenyl, alkynyl, substituted alkynyl, aryl, substituted aryl, heteroaryl, substituted heteroaryl, heterocycle, substituted heterocycle, alkoxy, substituted alkoxy, amino, amido, substituted amido, thiol, halogen, hydroxyl, cyano, water solubilizing group (WSG), -T¹-J¹, wherein T¹ is an optional linker and J¹ has formula (I),
   optionally one or more pairs of substituents selected from R¹ and R², R² and R³, R³ and R⁴, R⁵ and R⁶, R⁶ and R⁷, and R⁷ and R¹ together form a fused ring, and optionally a pair of further substituents on the fused ring together form a further fused ring;
   R⁸ and R⁹ are each independently a poly(alkylene oxide) group.
6. The dye of clause 5, wherein R¹ is aryl, substituted aryl, heteroaryl, or substituted heteroaryl.
7. The dye of clause 6, wherein R¹ has the formula: 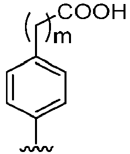 wherein m ranges from 0 to 10.
8. The dye of clause 7, wherein m is 0.
9. The dye of clause 5, wherein R¹ is alkyl or substituted alkyl.
10. The dye of clause 9, wherein R¹ has the formula: 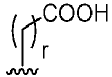 wherein r ranges from 1 to 10.
11. The dye of clause 10, wherein r ranges from 1 to 4.
12. The dye of any one of clauses 5-11, wherein at least one of R²-R⁷ comprises a π bond that is π-conjugated to π electrons of a pyrrole ring of the formula (I) structure.
13. The dye according clause 12, wherein R² and R³ together form a fused aromatic ring.
14. The dye of clause 13, wherein the fused aromatic ring formed by R² and R³ is an aryl ring or a substituted aryl ring.
15. The dye of any one of clauses 5-14, wherein R⁴ is selected from the group consisting of: alkyl, aryl, substituted aryl, heteroaryl, substituted heteroaryl, and cyano.
16. The dye of clause 15, wherein R⁴ is methyl, ethyl, aryl, substituted aryl, or cyano.
17. The dye of any one of clauses 5-16, wherein R⁵ and R⁶ together form a fused ring.
18. The dye of clause 14, wherein the fused ring formed by R⁵ and R⁶ is substituted with a pair of further substituents that provide a further fused ring.
19. The dye of clause 18, wherein R⁵ and R⁶ together form a group of formula 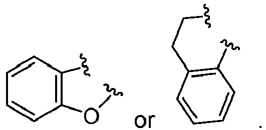
20. The dye of any one of clauses 5-19, wherein R⁸ and R⁹ are each independently a poly(ethylene glycol) group.
21. The dye of clause 20, wherein R⁸ and R⁹ each independently have the formula: 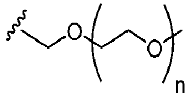 wherein n ranges from 6 to 24.
22. The dye of clause 21, wherein n ranges from 10 to 20.
23. The dye of clause 22, wherein n ranges from 12 to 16.
24. The dye of any one of clauses 5-23, wherein if R⁴ is substituted aryl then R⁵ is not substituted aryl.
25. The dye of any one of clauses 5-24, wherein if R⁴ is aryl then R⁵ is not alkyl.
26. The dye of any one of clauses 5-25, wherein if R², R⁴, and R⁵ are methyl then R⁷ is not methyl.
27. The dye of any one of clauses 5-26, wherein if R² and R³ or R⁵ and R⁶ together form a fused ring, then R³ and R⁶ are not hydrogen.
28. A polymeric tandem dye comprising:
   a polymeric backbone comprising non-conjugated repeat units;
   a dipyrromethene-based donor chromophore linked to a non-conjugated repeat unit of the polymeric backbone; and
   an acceptor chromophore linked to a non-conjugated repeat unit of the polymeric backbone and located in energy-receiving proximity to the donor chromophore,
   wherein the donor chromophore efficiently transfers energy to the acceptor chromophore while a second dipyrromethene-based donor chromophore of the same or different chemical structure is within energy-receiving proximity therewith.
29. The polymeric tandem dye of clause 28, wherein the dipyrromethene-based donor chromophore is a dipyrromethene-based dye of any one of clauses 1-27.
30. The polymeric tandem dye of any one of clauses 28-29, wherein a second donor chromophore is linked to a non-conjugated repeat unit of the polymeric backbone.
31. The polymeric tandem dye of any one of clauses 28-30, wherein the acceptor chromophore is selected from the group consisting of cyanine dyes, rhodamine dyes, xanthene dyes, coumarin dyes, polymethines, pyrenes, dipyrromethene borondifluorides, napthalimides, thiazine dyes, and acridine dyes.
32. A labeled specific binding member, comprising:
   a dye of any one of clauses 1-27 or a polymeric tandem dye of any one of clauses 28-31; and
   a specific binding member linked to the dye or polymeric tandem dye.
33. The labeled specific binding member of clause 32, wherein the labeled specific binding member comprises a dye of any one of clauses 1-27.
34. The labeled specific binding member of clause 32, wherein the labeled specific binding member comprises a polymeric tandem dye of any one of clauses 28-31.
35. The labeled specific binding member of any one of clauses 32-34, wherein the specific binding member is an antibody.
36. The labeled specific binding member of any one of clauses 32-35, wherein the specific binding member is an antibody fragment or binding derivative thereof.
37. The labeled specific binding member of any one of clauses 32-36, wherein the antibody fragment or binding derivative thereof is selected from the group consisting of a Fab fragment, a F(ab')₂ fragment, a scFv, a diabody and a triabody.
38. The labeled specific binding member of any one of clauses 32-37, wherein the acceptor chromophore is selected from a cyanine dye, a rhodamine dye, a xanthene dye, a coumarin dye, a polymethine, a pyrene, a dipyrromethene borondifluoride, a napthalimide, a thiazine dye and an acridine dye.
39. A method of evaluating a sample for presence of a target analyte, the method comprising:
   (a) contacting the sample with a labelled specific binding member that specifically binds the target analyte to produce a labelling composition contacted sample, wherein the labelled specific binding member comprises:
      (i) a dye of any one of clauses 1-27 or a polymeric tandem dye of any one of clauses 28-31; and
      (ii) a specific binding member linked to the dye or polymeric tandem dye; and
   (b) assaying the labelling composition contacted sample for the presence of a labelled specific binding member-target analyte binding complex to evaluate whether the target analyte is present in the sample.
40. The method of clause 39, wherein the labeled specific binding member comprises a dye of any one of clauses 1-27.
41. The method of clause 39, wherein the labeled specific binding member comprises a polymeric tandem dye of any one of clauses 28-31.
42. The method of any one of clauses 39-41, further comprising contacting the sample with a second specific binding member that is support bound and specifically binds the target analyte.
43. The method of clause 42, wherein the support comprises a magnetic particle.
44. The method of any one of clauses 39-43, wherein the target analyte is associated with a cell.
45. The method of clause 44, wherein the target analyte is a cell surface marker of the cell.
46. The method of clause 45, wherein the cell surface marker is selected from the group consisting of a cell receptor and a cell surface antigen.
47. The method of any one of clauses 39-44, wherein the target analyte is an intracellular target, and the method further comprises lysing the cell.
48. The method of any one of clauses 39-47, wherein the method further comprises flow cytometrically analyzing the fluorescently labelled target analyte.
49. A method of labelling a target molecule, the method comprising:
   contacting the target molecule with a dye of any one of clauses 1-27 or a polymeric tandem dye of any one of clauses 28-31 to produce a labelled target molecule, wherein the dye or polymeric tandem dye further comprises a conjugation tag that covalently links to the target molecule.
50. The method of clause 49, wherein the method comprises contacting the target molecule with a dye of any one of clauses 1-27.
51. The method of clause 49, wherein the method comprises contacting the target molecule with a polymeric tandem dye of any one of clauses 28-31.
52. A kit comprising:
   a dye of any one of clauses 1-27, a polymeric tandem dye of any one of clauses 28-31, or a combination thereof; and
   a container.
53. The kit of clause 52, wherein the kit comprises a dye of any one of clauses 1-27.
54. The kit of clause 52, wherein the kit comprises a polymeric tandem dye of any one of clauses 28-31.
55. The kit of any one of clauses 52-54, further comprising one or more components selected from the group consisting of a fluorophore, a specific binding member, a specific binding member conjugate, a cell, a support, a biocompatible aqueous elution buffer and instructions for use.
56. The kit of clause 55, further comprising a specific binding member.
57. The kit of clause 56, wherein the specific binding member is covalently linked to the dye or polymeric tandem dye.
58. The kit of clause 57, wherein the specific binding member is an antibody.
59. The kit of clause 57, wherein the specific binding member is an antibody fragment or binding derivative thereof.
60. The kit of clause 59, wherein the antibody fragment or binding derivative thereof is selected from the group consisting of a Fab fragment, a F(ab')₂ fragment, a scFv, a diabody and a triabody.
61. The kit any one of clauses 56-60, wherein the specific binding member is a support-bound specific binding member.

Aspects, including embodiments, of the subject matter described herein may be beneficial alone or in combination, with one or more other aspects or embodiments. Without limiting the description, certain non-limiting aspects of the disclosure provided below. As will be apparent to those of skill in the art upon reading this disclosure, each of the individually numbered aspects may be used or combined with any of the preceding or following individually numbered aspects. This is intended to provide support for all such combinations of aspects and is not limited to combinations of aspects explicitly provided below:
In at least some of the previously described embodiments, one or more elements used in an embodiment can interchangeably be used in another embodiment unless such a replacement is not technically feasible. It will be appreciated by those skilled in the art that various other omissions, additions and modifications may be made to the methods and structures described above without departing from the scope of the claimed subject matter. All such modifications and changes are intended to fall within the scope of the subject matter, as defined by the appended claims.

It will be understood by those within the art that, in general, terms used herein, and especially in the appended claims (e.g., bodies of the appended claims) are generally intended as "open" terms (e.g., the term "including" should be interpreted as "including but not limited to," the term "having" should be interpreted as "having at least," the term "includes" should be interpreted as "includes but is not limited to," etc.). It will be further understood by those within the art that if a specific number of an introduced claim recitation is intended, such an intent will be explicitly recited in the claim, and in the absence of such recitation no such intent is present. For example, as an aid to understanding, the following appended claims may contain usage of the introductory phrases "at least one" and "one or more" to introduce claim recitations. However, the use of such phrases should not be construed to imply that the introduction of a claim recitation by the indefinite articles "a" or "an" limits any particular claim containing such introduced claim recitation to embodiments containing only one such recitation, even when the same claim includes the introductory phrases "one or more" or "at least one" and indefinite articles such as "a" or "an" (e.g., "a" and/or "an" should be interpreted to mean "at least one" or "one or more"); the same holds true for the use of definite articles used to introduce claim recitations. In addition, even if a specific number of an introduced claim recitation is explicitly recited, those skilled in the art will recognize that such recitation should be interpreted to mean at least the recited number (e.g., the bare recitation of "two recitations," without other modifiers, means at least two recitations, or two or more recitations). Furthermore, in those instances where a convention analogous to "at least one of A, B, and C, etc." is used, in general such a construction is intended in the sense one having skill in the art would understand the convention (e.g., " a system having at least one of A, B, and C" would include but not be limited to systems that have A alone, B alone, C alone, A and B together, A and C together, B and C together, and/or A, B, and C together, etc.). In those instances where a convention analogous to "at least one of A, B, or C, etc." is used, in general such a construction is intended in the sense one having skill in the art would understand the convention (e.g., " a system having at least one of A, B, or C" would include but not be limited to systems that have A alone, B alone, C alone, A and B together, A and C together, B and C together, and/or A, B, and C together, etc.). It will be further understood by those within the art that virtually any disjunctive word and/or phrase presenting two or more alternative terms, whether in the description, claims, or drawings, should be understood to contemplate the possibilities of including one of the terms, either of the terms, or both terms. For example, the phrase "A or B" will be understood to include the possibilities of "A" or "B" or "A and B."

In addition, where features or aspects of the disclosure are described in terms of Markush groups, those skilled in the art will recognize that the disclosure is also thereby described in terms of any individual member or subgroup of members of the Markush group.

As will be understood by one skilled in the art, for any and all purposes, such as in terms of providing a written description, all ranges disclosed herein also encompass any and all possible sub-ranges and combinations of sub-ranges thereof. Any listed range can be easily recognized as sufficiently describing and enabling the same range being broken down into at least equal halves, thirds, quarters, fifths, tenths, etc. As a non-limiting example, each range discussed herein can be readily broken down into a lower third, middle third and upper third, etc. As will also be understood by one skilled in the art all language such as "up to," "at least," "greater than," "less than," and the like include the number recited and refer to ranges which can be subsequently broken down into sub-ranges as discussed above. Finally, as will be understood by one skilled in the art, a range includes each individual member. Thus, for example, a group having 1-3 articles refers to groups having 1, 2, or 3 articles. Similarly, a group having 1-5 articles refers to groups having 1, 2, 3, 4, or 5 articles, and so forth.

Although the foregoing invention has been described in some detail by way of illustration and example for purposes of clarity of understanding, it is readily apparent to those of ordinary skill in the art in light of the teachings of this invention that certain changes and modifications may be made thereto without departing from the spirit or scope of the appended claims.

Accordingly, the preceding merely illustrates the principles of the invention. It will be appreciated that those skilled in the art will be able to devise various arrangements which, although not explicitly described or shown herein, embody the principles of the invention and are included within its spirit and scope. Furthermore, all examples and conditional language recited herein are principally intended to aid the reader in understanding the principles of the invention and the concepts contributed by the inventors to furthering the art, and are to be construed as being without limitation to such specifically recited examples and conditions. Moreover, all statements herein reciting principles, aspects, and embodiments of the invention as well as specific examples thereof, are intended to encompass both structural and functional equivalents thereof. Additionally, it is intended that such equivalents include both currently known equivalents and equivalents developed in the future, i.e., any elements developed that perform the same function, regardless of structure. Moreover, nothing disclosed herein is intended to be dedicated to the public regardless of whether such disclosure is explicitly recited in the claims.

The scope of the present invention, therefore, is not intended to be limited to the exemplary embodiments shown and described herein. Rather, the scope and spirit of present invention is embodied by the appended claims. In the claims, 35 U.S.C. §112(f) or 35 U.S.C. §112(6) is expressly defined as being invoked for a limitation in the claim only when the exact phrase "means for" or the exact phrase "step for" is recited at the beginning of such limitation in the claim; if such exact phrase is not used in a limitation in the claim, then 35 U.S.C. § 112 (f) or 35 U.S.C. §112(6) is not invoked.

Notwithstanding the appended claims, the disclosure is also defined by the following clauses:
1. A water-soluble dipyrromethene-based dye that absorbs in the yellow-green range at 561 nm.
2. The water-soluble dipyrromethene-based dye according to clause, wherein the absorption maxima is from 555 to 585 nm.
3. The water-soluble dipyrromethene-based dye of clause 2, wherein the absorption maxima is from 558 to 568 nm.
4. The water-soluble dipyrromethene-based dye of clause 3, wherein the absorption maxima is about 561 nm.
5. The water-soluble dipyrromethene-based dye of any one of clauses 1 to 4, wherein the dye is a dipyrromethene-boron dye.
6. The water-soluble dipyrromethene-based dye of any one of clauses 1 to 5, of formula (I): 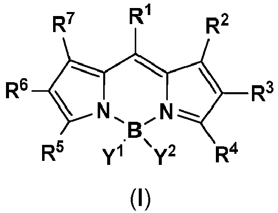 wherein:
   R¹-R⁷ are each independently selected from H, alkyl, halogen, substituted alkyl, alkoxy, substituted alkoxy, amino, substituted amino, amido, substituted amido, thiol, substituted thiol, aryl, substituted aryl, heteroaryl, substituted heteroaryl, heterocycle, substituted heterocycle, alkenyl, substituted alkenyl, alkynyl, substituted alkynyl, water solubilizing group (WSG), and -T¹-J¹, or
   optionally any one or more pairs of substituents selected from R⁶ and R⁷, R² and R³, R⁵ and R⁶, R³ and R⁴, R² and R¹ and R⁷ and R¹, together form a divalent radical and are cyclically linked and together with the carbon atoms to which they are bound provide a 5- or 6-membered fused heterocycle, carbocycle, aryl or heteroaryl ring (e.g., a 5- or 6-membered ring comprising carbon atoms and 0-3 heteroatoms selected from O, S and N), which ring may be unsubstituted or further substituted with a substituent independently selected from alkyl, substituted alkyl, alkoxy, substituted alkoxy, aryl, substituted aryl, heteroaryl, substituted heteroaryl, alkenyl, substituted alkenyl, alkynyl, substituted alkynyl, water solubilizing group (WSG), and -T¹-J¹;
   T¹ is an optional linker;
   J¹ is a repeat unit of a compound of formula (I); and
   Y¹ and Y² are independently selected from F, OH, H, cyano, alkyl, substituted alkyl, aryl, substituted aryl, heteroaryl, substituted heteroaryl, alkoxy, substituted alkoxy, alkenyl, substituted alkenyl, alkynyl, substituted alkynyl, and water solubilizing group (WSG), or
   Y¹ and Y² together with the boron atom to which they are bound form a 5- or 6-membered ring (e.g., a 5- or 6-membered ring comprising a boron atom, carbon atoms and 0-3 heteroatoms selected from O, S and N), which ring may be unsubstituted or further substituted with a substituent independently selected from alkyl, substituted alkyl, alkoxy, substituted alkoxy, aryl, substituted aryl, heteroaryl, substituted heteroaryl, alkenyl, substituted alkenyl, alkynyl, substituted alkynyl, water solubilizing group (WSG), and -T¹-J¹; and
   wherein at least one moiety selected from Y¹, Y² and R¹-R⁷ is a WSG, or at least one moiety selected from Y¹, Y² and R¹-R⁷ is substituted with a WSG, where the WSG is of the formula (la):

      -(L¹)ₙ₁ - (X¹)ₙ₂ - ((L²)ₙ₃-Z¹)ₘ (Ia)

      wherein:
      n1, n2 and n3 are each independently 0 or 1;
      L¹, if present, is a linker;
      L² if present, is a linker;
      X¹, if present, is a branching point;
      Z¹ is a water soluble polymer comprising 6-24 monomeric units; and
      m is an integer from 1 to 3.
7. The water-soluble dipyrromethene-based dye of clause 6, further comprising a WSG of formula (lb):

   -(L¹)ₙ₁ - (X¹)ₙ₂ - ((L²)ₙ₃-Z²)ₘ (Ib)

   wherein:
   n1, n2 and n3 are each independently 0 or 1;
   L¹, if present, is a linker;
   L² if present, is a linker;
   X¹, if present, is a branching point;
   Z² is a water soluble polymer comprising 1-50 monomeric units; and
   m is an integer from 1 to 3.
8. The water-soluble dipyrromethene-based dye of clause 6 or 7, wherein the water solubilizing group (WSG) comprises a PEG moiety.
9. The water-soluble dipyrromethene-based dye of any one of clauses 6 to 8, wherein one or more water solubilizing groups (WSGs) is independently selected from the following formulae: 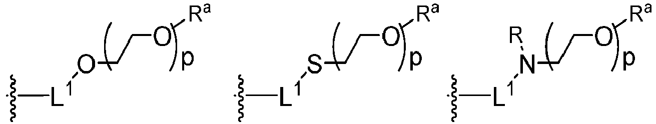 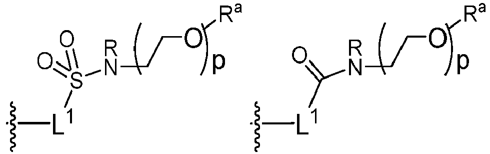 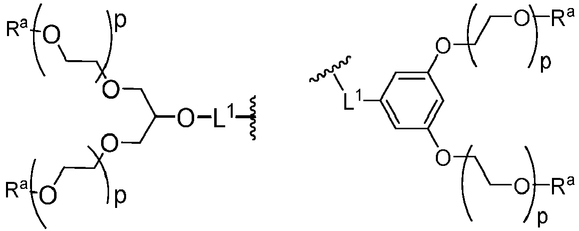 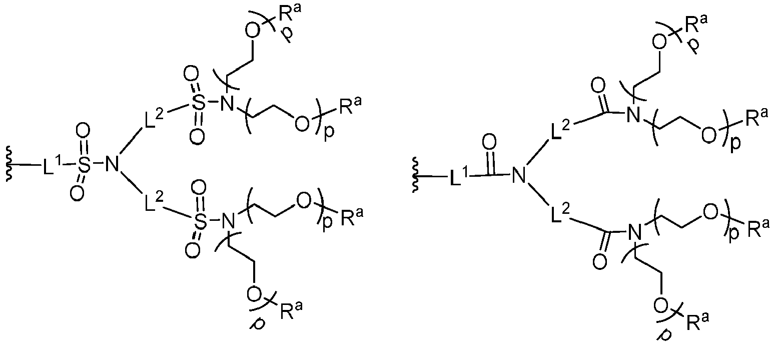 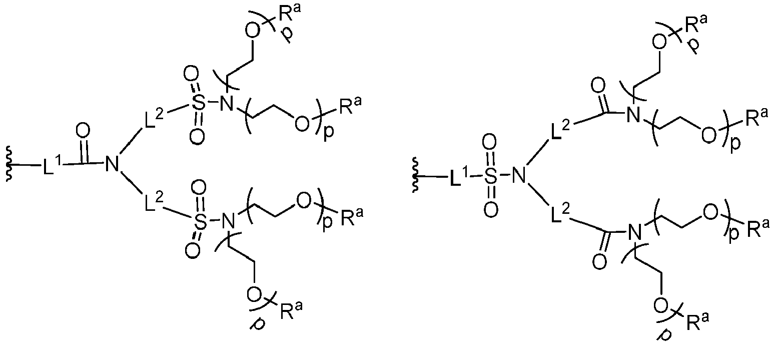 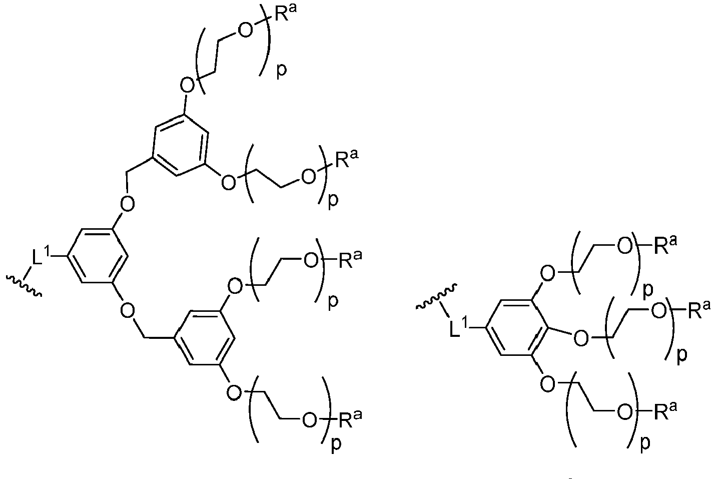 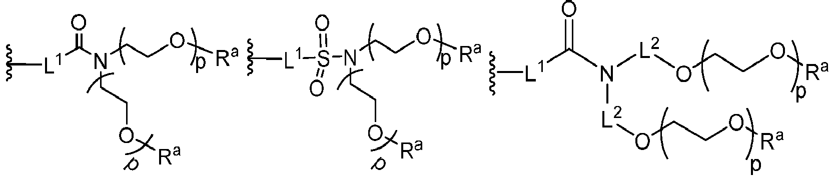 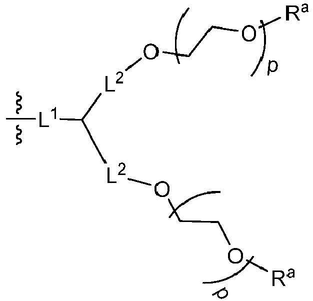 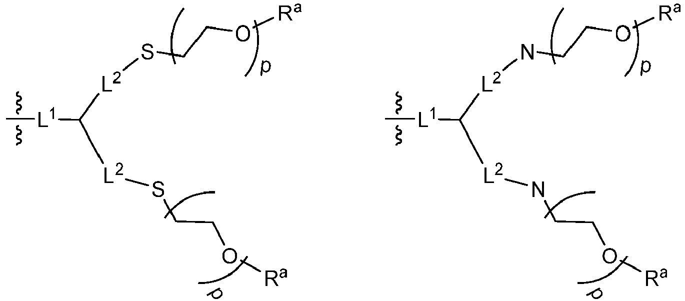 wherein:
   L¹ and L² are each independently an optional linker;
   R¹¹ and R are each independently H, alkyl or substituted alkyl; and
   each p is an integer from 6 to 24.
10. The water-soluble dipyrromethene-based dye of any one of clauses 6 to 9, wherein R¹ is selected from substituted alkyl, and substituted aryl, wherein the substituent comprises a chemoselective tag.
11. The water-soluble dipyrromethene-based dye of any one of clauses 6 to 10, wherein Y¹ and Y² each comprise a water solubilizing group (WSG).
12. The water-soluble dipyrromethene-based dye of any one of clauses 6 to 11, of the formula (II): 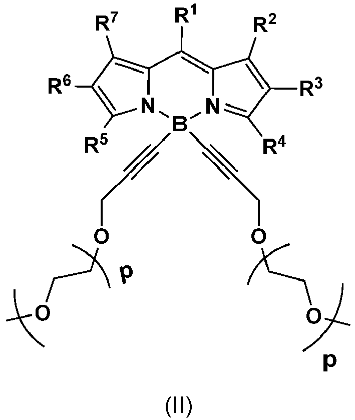 wherein p is an integer from 6-24.
13. The water-soluble dipyrromethene-based dye of any one of clauses 6 to 12, wherein R⁵ is selected from halogen, substituted alkyl, substituted alkenyl, alkenyl aryl, aryl, substituted aryl, heterocycle, substituted heterocycle, spiro heterocycle, substituted spiro heterocycle, heteroaryl, substituted heteroaryl, substituted alkoxy, substituted amino, substituted amido, and substituted thiol.
14. The water-soluble dipyrromethene-based dye of clause 13, wherein R⁵ and R⁴ are the same.
15. The water-soluble dipyrromethene-based dye of any one of clauses 6 to 14, of the formula (IIa): 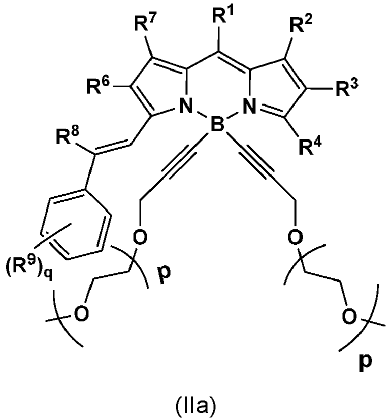 wherein:
   R⁸ is selected from H, halogen, cyano, alkyl, substituted alkyl, aryl, substituted aryl, heteroaryl, substituted heteroaryl, alkoxy, substituted alkoxy, alkenyl, substituted alkenyl, alkynyl, substituted alkynyl, water solubilizing group (WSG), or
   R⁸ and R⁶ together with the atoms to which they are bound form a 5- or 6-membered heterocycle, carbocycle, aryl or heteroaryl ring (e.g., a 5- or 6-membered ring comprising carbon atoms and 0-3 heteroatoms selected from O, S and N), which ring may be unsubstituted or further substituted with a substituent independently selected from alkyl, substituted alkyl, alkoxy, substituted alkoxy, aryl, substituted aryl, heteroaryl, substituted heteroaryl, alkenyl, substituted alkenyl, alkynyl, substituted alkynyl, and water solubilizing group (WSG);
   q is an integer from 0 to 5; and
   each R⁹, if present, is independently selected from alkyl, substituted alkyl, hydroxy, alkoxy, substituted alkoxy, amino, substituted amino, thiol, substituted thiol, cyano, aryl, substituted aryl, heteroaryl, substituted heteroaryl, alkenyl, substituted alkenyl, alkynyl, substituted alkynyl, and water solubilizing group (WSG), or
   optionally two adjacent R⁹ groups, together form a divalent radical and are cyclically linked and together with the carbon atoms to which they are bound provide a 5- or 6-membered fused heterocycle, carbocycle, aryl or heteroaryl ring (e.g., a 5- or 6-membered ring comprising carbon atoms and 0-3 heteroatoms selected from O, S and N), which ring may be unsubstituted or further substituted with a substituent independently selected from alkyl, substituted alkyl, alkoxy, substituted alkoxy, aryl, substituted aryl, heteroaryl, substituted heteroaryl, alkenyl, substituted alkenyl, alkynyl, substituted alkynyl, and water solubilizing group (WSG).
16. The water-soluble dipyrromethene-based dye of clause 15, wherein R⁸ is H.
17. The water-soluble dipyrromethene-based dye of clause 15, wherein R⁸ and R⁶ together with the atoms to which they are bound form a 5- membered carbocycle, which carbocycle is optionally further substituted with a substituent independently selected from alkyl, substituted alkyl, alkoxy, substituted alkoxy, aryl, substituted aryl, heteroaryl, substituted heteroaryl, alkenyl, substituted alkenyl, alkynyl, substituted alkynyl, and water solubilizing group (WSG).
18. The water-soluble dipyrromethene-based dye of any one of clauses 15 to 17, of any one of the following structures: 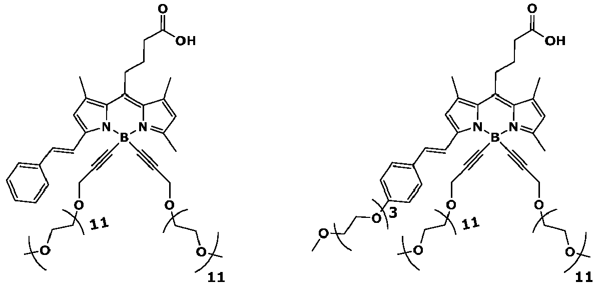 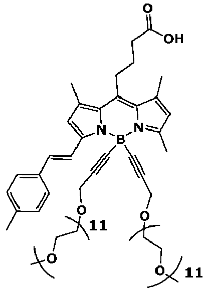 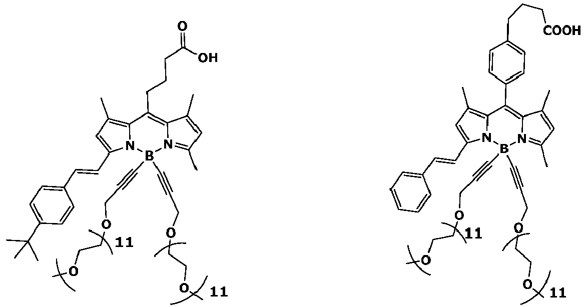 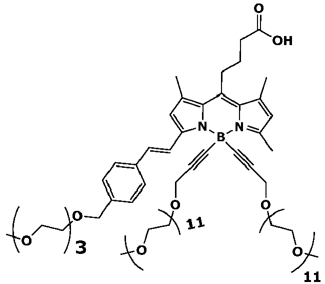 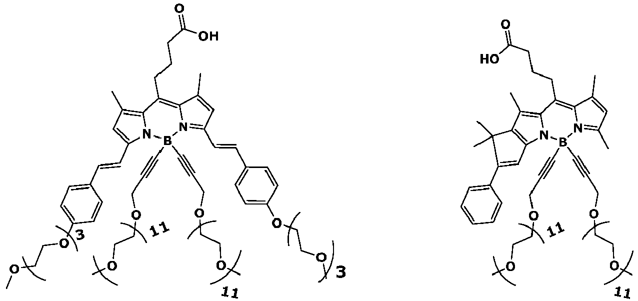 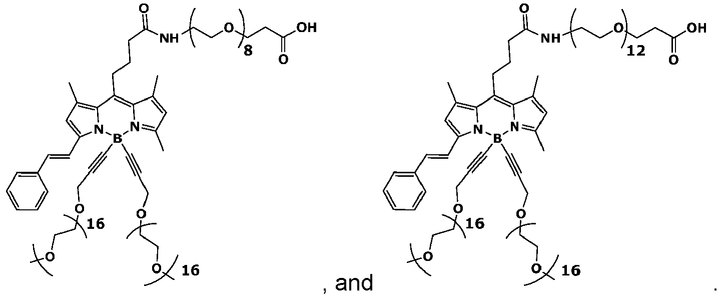
19. The water-soluble dipyrromethene-based dye of any one of clauses 6 to 14, of the formula (IIb): 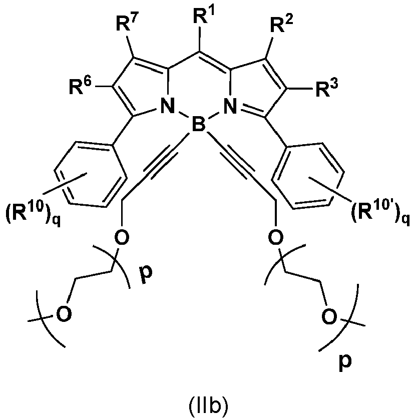 wherein:
   each q is independently an integer from 0 to 5; and
   R¹⁰ and R^{10'}, if present, are each independently selected from halogen, hydroxyl, cyano, carboxamide, substituted carboxamide amino, substituted amino, thiol, substituted thiol, alkyl, substituted alkyl, aryl, substituted aryl, heteroaryl, substituted heteroaryl, alkoxy, substituted alkoxy, alkenyl, substituted alkenyl, alkynyl, substituted alkynyl, and water soluble group (WSG), or
   at least one R¹⁰ group is at an ortho position, and R¹⁰ and R⁶ together with the atoms to which they are bound form a 5- or 6-membered ring (e.g., a 5- or 6-membered ring comprising a boron atom, carbon atoms and 0-3 heteroatoms selected from O, S and N), which ring may be unsubstituted or further substituted with a substituent independently selected from alkyl, substituted alkyl, alkoxy, substituted alkoxy, aryl, substituted aryl, heteroaryl, substituted heteroaryl, alkenyl, substituted alkenyl, alkynyl, substituted alkynyl, and water solubilizing group (WSG); and
   at least one R^{10'} is an ortho position, and R¹⁰ and R³ together with the atoms to which they are bound form a 5- or 6-membered ring (e.g., a 5- or 6-membered ring comprising a boron atom, carbon atoms and 0-3 heteroatoms selected from O, S and N), which ring may be unsubstituted or further substituted with a substituent independently selected from alkyl, substituted alkyl, alkoxy, substituted alkoxy, aryl, substituted aryl, heteroaryl, substituted heteroaryl, alkenyl, substituted alkenyl, alkynyl, substituted alkynyl, and water solubilizing group (WSG).
20. The water-soluble dipyrromethene-based dye of clause 19, wherein each q is 1, and R¹⁰ and R^{10'} are both present in their respective para positions.
21. The water-soluble dipyrromethene-based dye of clause 20, wherein R¹⁰ and R^{10'} are each independently selected from cyano and a PEG moiety.
22. The water-soluble dipyrromethene-based dye of any one of clauses 19 to 21, of any one of the following structures: 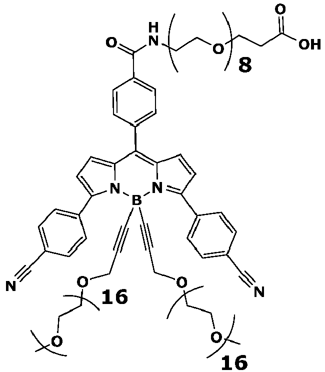 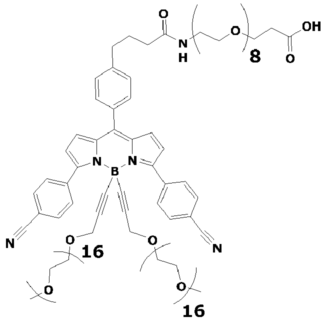 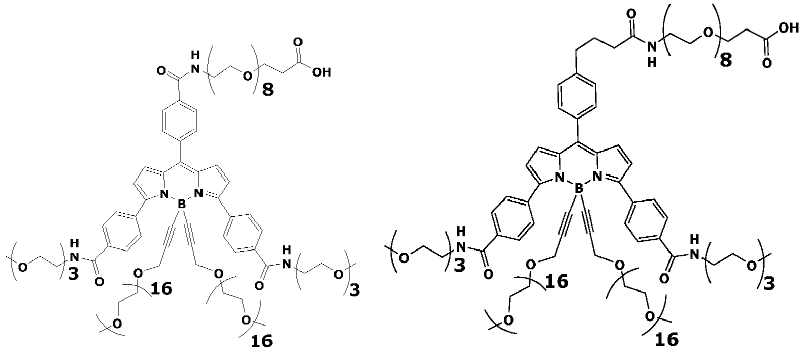 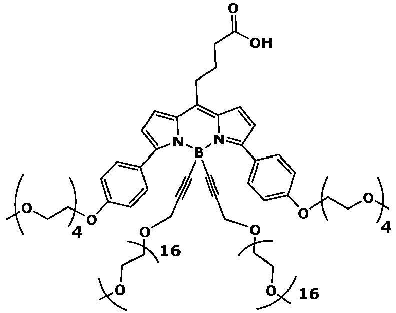 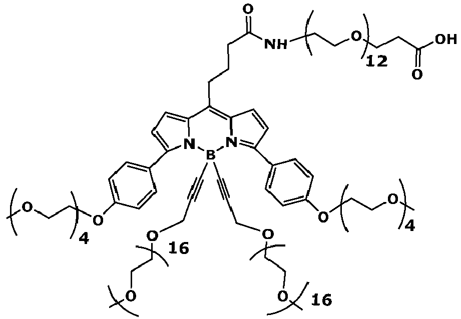 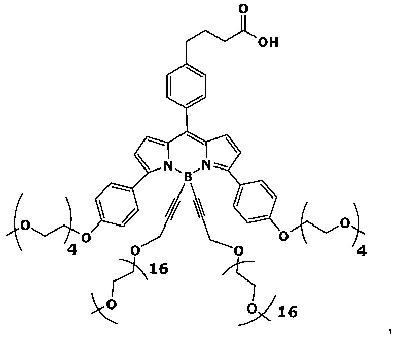 and 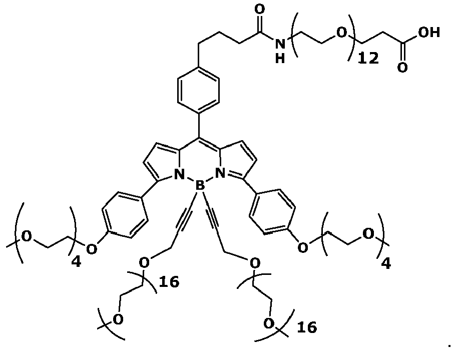
23. The water-soluble dipyrromethene-based dye of any one of clauses 6 to 14, of the formula (IIc): 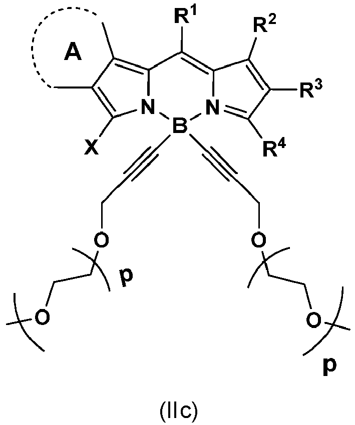 wherein:
   X is a halogen; and
   ring A is a 5- or 6-membered fused heterocycle, carbocycle, aryl or heteroaryl ring (e.g., a 5- or 6-membered ring comprising carbon atoms and 0-3 heteroatoms selected from O, S and N), which ring may be unsubstituted or further substituted with a substituent independently selected from alkyl, substituted alkyl, alkoxy, substituted alkoxy, aryl, substituted aryl, heteroaryl, substituted heteroaryl, alkenyl, substituted alkenyl, alkynyl, substituted alkynyl, and water solubilizing group (WSG).
24. The water-soluble dipyrromethene-based dye of clause 23, wherein the A ring is an aryl ring.
25. The water-soluble dipyrromethene-based dye of clause 23 or 24, of any one of the following compounds: 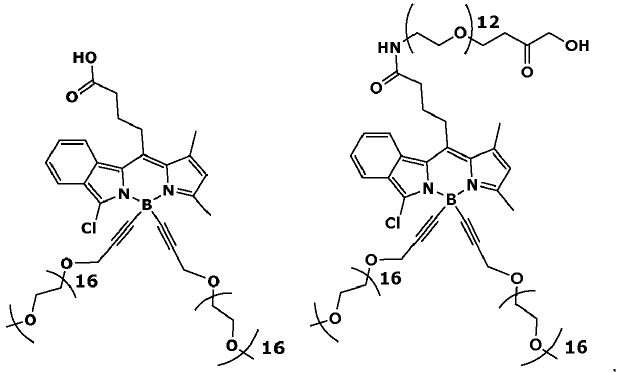 and 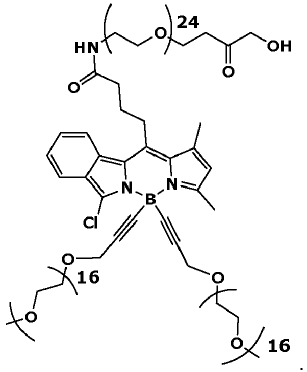
26. The water-soluble dipyrromethene-based dye of any one of clauses 6 to 14, of the formula (IId): 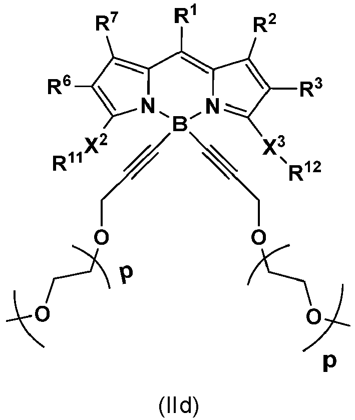 wherein:
   X² and X³ are each independently selected from NR¹³, S and O;
   R¹³ is selected from H, alkyl, and substituted alkyl;
   R¹¹ and R¹² are each independently selected from aryl, substituted aryl, heteroaryl, substituted heteroaryl, and water soluble group (WSG), or
   when X² is NR¹³, optionally R¹³ and R¹¹ together with the nitrogen atom to which they are attached, form a heterocyclic or spiro heterocyclic ring, which ring may be unsubstituted or further substituted with a substituent independently selected from alkyl, substituted alkyl, alkoxy, substituted alkoxy, aryl, substituted aryl, heteroaryl, substituted heteroaryl, alkenyl, substituted alkenyl, alkynyl, substituted alkynyl, and water solubilizing group (WSG), and
   when X³ is NR¹³, optionally R¹³ and R¹² together with the nitrogen atom to which they are attached, form a heterocyclic or spiro heterocyclic ring, which ring may be unsubstituted or further substituted with a substituent independently selected from alkyl, substituted alkyl, alkoxy, substituted alkoxy, aryl, substituted aryl, heteroaryl, substituted heteroaryl, alkenyl, substituted alkenyl, alkynyl, substituted alkynyl, and water solubilizing group (WSG).
27. The water-soluble dipyrromethene-based dye of clause 26, wherein X² and X³ are each NR¹³, wherein R¹³ is H.
28. The water-soluble dipyrromethene-based dye of clause 26, wherein X² is NR¹³, and R¹³ and R¹¹ together with the nitrogen atom to which they are attached form a heterocyclic or a spiro heterocyclic ring; and X³ is NR¹³, and R¹³ and R¹² together with the nitrogen atom to which they are attached form a heterocyclic or a spiro heterocyclic ring.
29. The water-soluble dipyrromethene-based dye of clause 26, wherein X² and X³ are both S.
30. The water-soluble dipyrromethene-based dye of any one of clauses 26 to 29, of any one of the following structures: 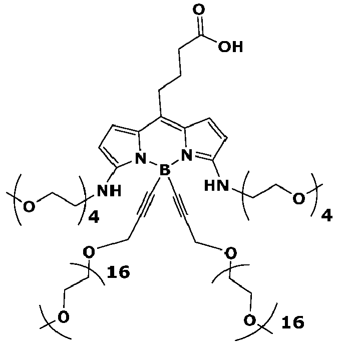 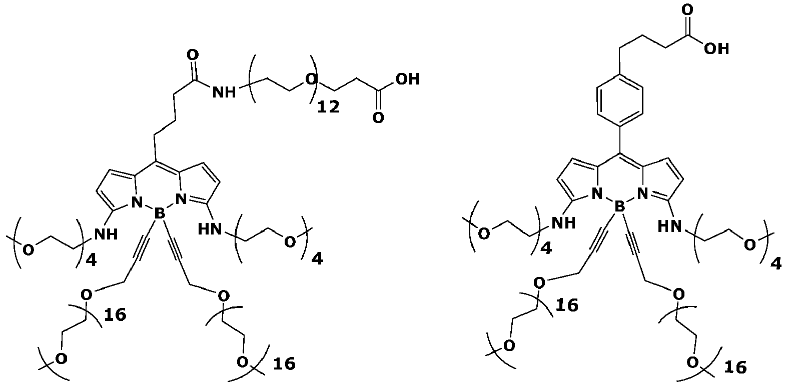 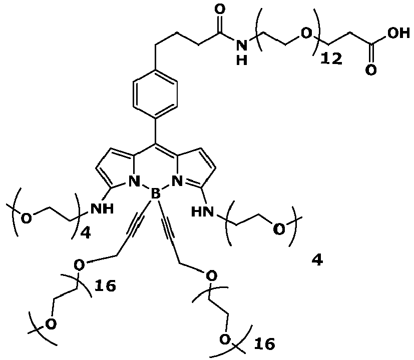 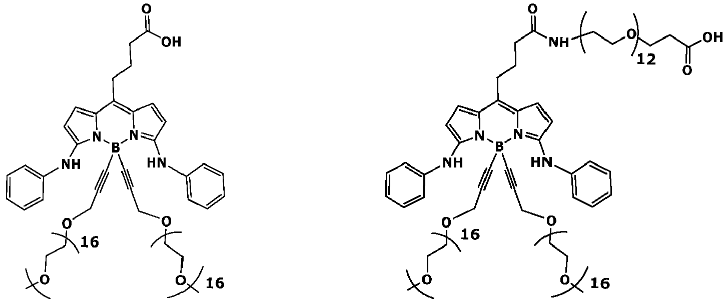 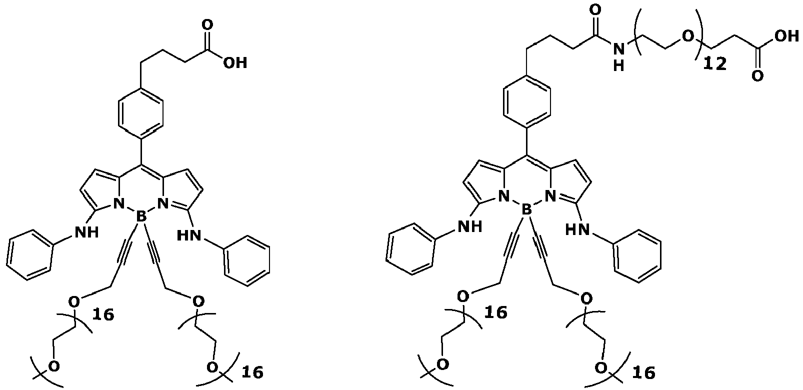 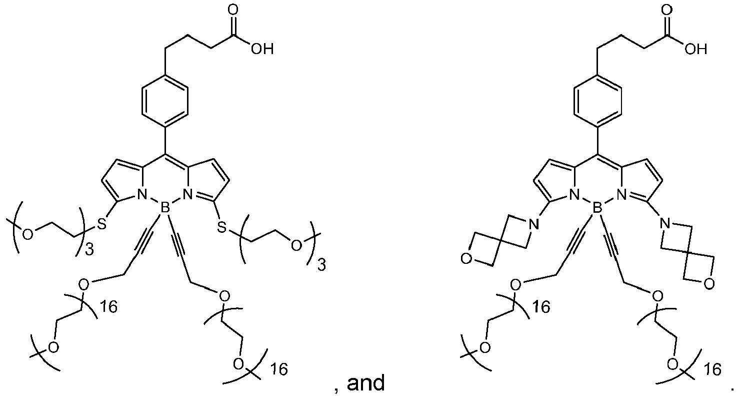
31. The water-soluble dipyrromethene-based dye of any one of clauses 6 to 10, of the formula (III): 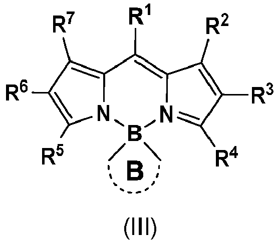 wherein:
   ring B is a 5- or 6-membered cyclic group (e.g., a 5- or 6-membered ring comprising a boron atom, carbon atoms and 0-3 heteroatoms selected from O, S and N), which cyclic group may be unsubstituted or further substituted with one or more substituents independently selected from alkyl, substituted alkyl, alkoxy, substituted alkoxy, aryl, substituted aryl, heteroaryl, substituted heteroaryl, alkenyl, substituted alkenyl, alkynyl, substituted alkynyl, and water solubilizing group (WSG).
32. The water-soluble dipyrromethene-based dye of clause 31, of the formula (IIIa): 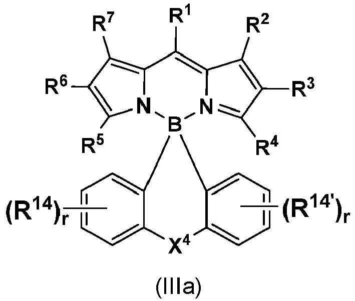 wherein:
   X⁴ is selected from O, S, NR, and CR₂;
   each R is independently selected from H, alkyl, and substituted alkyl;
   each r is independently an integer from 0 to 4; and
   each R¹⁴ and R^{14'}, if present, is independently selected from halogen, hydroxyl, cyano, amino, substituted amino, thiol, substituted thiol, amido, substituted amido, acyl, substituted acyl, alkyl, substituted alkyl, aryl, substituted aryl, heteroaryl, substituted heteroaryl, alkoxy, substituted alkoxy, alkenyl, substituted alkenyl, alkynyl, substituted alkynyl, and water solubilizing group (WSG).
33. The water-soluble dipyrromethene-based dye of clause 31 or 32, wherein at least one of R⁵ and R⁶ and R⁴ and R³ together form a divalent radical and are cyclically linked and together with the carbon atoms to which they are bound provide a 5- or 6-membered fused heterocycle, carbocycle, aryl or heteroaryl ring (e.g., a 5- or 6-membered ring comprising carbon atoms and 0-3 heteroatoms selected from O, S and N), which ring may be unsubstituted or further substituted with a substituent independently selected from alkyl, substituted alkyl, alkoxy, substituted alkoxy, aryl, substituted aryl, heteroaryl, substituted heteroaryl, alkenyl, substituted alkenyl, alkynyl, substituted alkynyl, and water solubilizing group (WSG).
34. The water-soluble dipyrromethene-based dye of clause 33, of the formula (IIIb) or (IIIc): 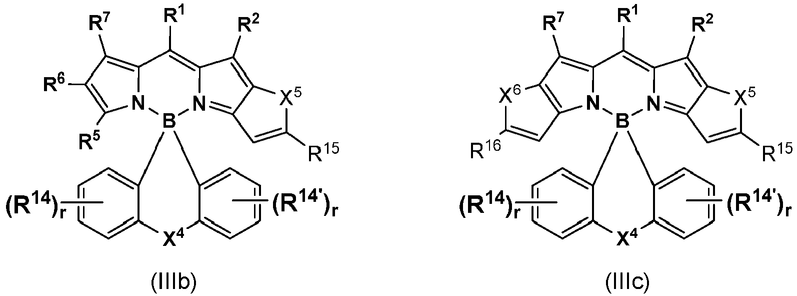 wherein:
   X⁵ and X⁶ are each independently selected from O, S, NR, and CR₂;
   each R is independently selected from H, alkyl, and substituted alkyl; and
   R¹⁵ and R¹⁶, are each independently selected from hydrogen, halogen, hydroxyl, cyano, amino, substituted amino, thiol, substituted thiol, amido, substituted amido, acyl, substituted acyl, alkyl, substituted alkyl, aryl, substituted aryl, heteroaryl, substituted heteroaryl, alkoxy, substituted alkoxy, alkenyl, substituted alkenyl, alkynyl, substituted alkynyl, and water solubilizing group (WSG).
35. The water-soluble dipyrromethene-based dye of clause 34, wherein X⁵ and X⁶ are O.
36. The water-soluble dipyrromethene-based dye of any one of clauses 31 to 35, of any one of the following structures: 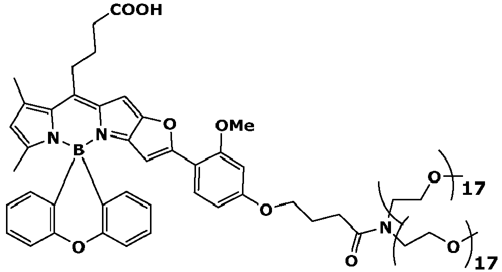 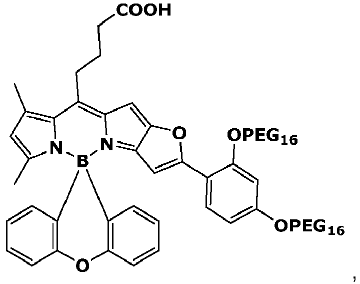 and 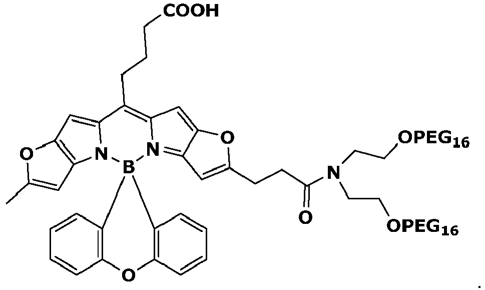
37. The water-soluble dipyrromethene-based dye of any one of clauses 6 to 12, wherein R³ is -L¹-J¹.
38. The water-soluble dipyrromethene-based dye of clause 37, of one of the following structures: 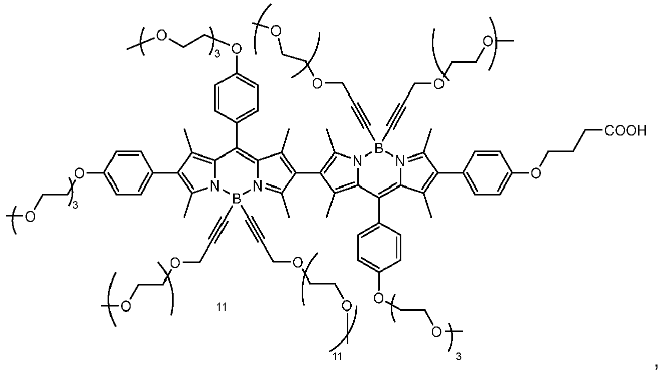 and 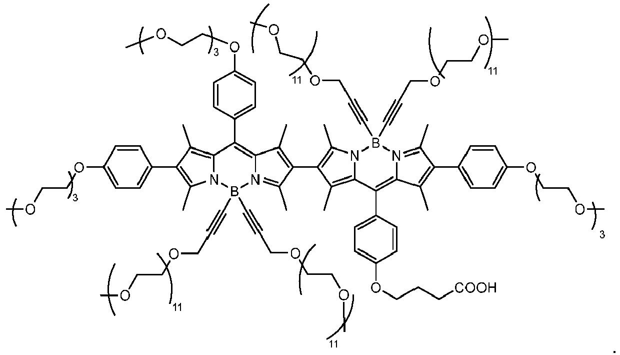
39. The water-soluble dipyrromethene-based dye of any one of clauses 1 to 4, of formula (IV): 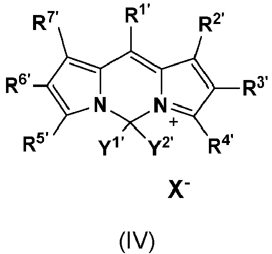 wherein:
   X⁻ is a counterion;
   R^{1'}-R^{7'} are each independently selected from H, alkyl, halogen, substituted alkyl, alkoxy, substituted alkoxy, amino, substituted amino, amido, substituted amido, thiol, substituted thiol, aryl, substituted aryl, heteroaryl, substituted heteroaryl, heterocycle, substituted heterocycle, alkenyl, substituted alkenyl, alkynyl, substituted alkynyl, water solubilizing group (WSG), and -T¹-Z¹, or
   optionally any one or more pairs of substituents selected from R^{6'} and R^{7'}, R^{2'} and R^{3'}, R^{5'} and R^{6'}, R^{3'} and R^{4'}, R^{2'} and R^{1'} and R^{7'} and R^{1'}, together form a divalent radical and are cyclically linked and together with the carbon atoms to which they are bound provide a 5- or 6-membered fused heterocycle, carbocycle, aryl or heteroaryl ring (e.g., a 5- or 6-membered ring comprising carbon atoms and 0-3 heteroatoms selected from O, S and N), which ring may be unsubstituted or further substituted with a substituent independently selected from alkyl, substituted alkyl, alkoxy, substituted alkoxy, aryl, substituted aryl, heteroaryl, substituted heteroaryl, alkenyl, substituted alkenyl, alkynyl, substituted alkynyl, water solubilizing group (WSG), and -T¹-Z¹;
   T¹ is an optional linker;
   Z¹ is a repeat unit of a compound of formula (I); and
   Y^{1'} and Y^{2'} are independently selected from F, OH, H, cyano, alkyl, substituted alkyl, aryl, substituted aryl, heteroaryl, substituted heteroaryl, alkoxy, substituted alkoxy, alkenyl, substituted alkenyl, alkynyl, substituted alkynyl, and WSG, or
   Y^{1'} and Y^{2'} together with the boron atom to which they are bound form a 5- or 6-membered ring (e.g., a 5- or 6-membered ring comprising a boron atom, carbon atoms and 0-3 heteroatoms selected from O, S and N), which ring may be unsubstituted or further substituted with a substituent independently selected from alkyl, substituted alkyl, alkoxy, substituted alkoxy, aryl, substituted aryl, heteroaryl, substituted heteroaryl, alkenyl, substituted alkenyl, alkynyl, substituted alkynyl, water solubilizing group (WSG), and -T¹-Z¹; and
   wherein at least one moiety selected from Y^{1'}, Y^{2'} and R^{1'}-R^{7'} is a WSG, or at least one moiety selected from Y^{1'}, Y^{2'} and R^{1'}-R^{7'} is substituted with a WSG, where the WSG is of the formula (la):

      -(L¹)ₙ₁ - (X¹)ₙ₂ - ((L²)ₙ₃-Z¹)ₘ (Ia)

      wherein:
      n1, n2 and n3 are each independently 0 or 1;
      L¹, if present, is a linker;
      L² if present, is a linker;
      X¹, if present, is a branching point;
      Z¹ is a water soluble polymer comprising 6-24 monomeric units; and
      m is an integer from 1 to 3.
40. The water-soluble dipyrromethene-based dye of clause 39, further comprising a WSG of formula (lb):

   -(L¹)ₙ₁ - (X¹)ₙ₂ - ((L²)ₙ₃-Z²)ₘ (Ib)

   wherein:
   n1, n2 and n3 are each independently 0 or 1;
   L¹, if present, is a linker;
   L² if present, is a linker;
   X¹, if present, is a branching point;
   Z² is a water soluble polymer comprising 1-50 monomeric units; and
   m is an integer from 1 to 3.
41. The water-soluble dipyrromethene-based dye of clause 39 or 40, wherein the WSG comprises a PEG moiety.
42. The water-soluble dipyrromethene-based dye of any one of clauses 39 to 41, wherein one or more water solubilizing groups (WSGs) is independently selected from the following formulae: 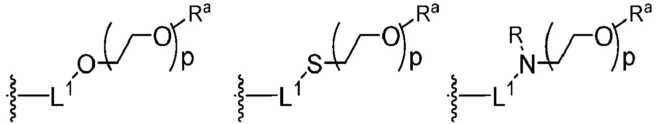 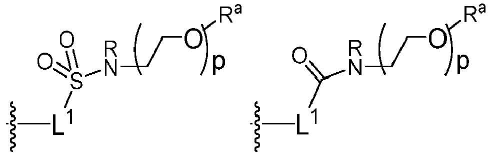 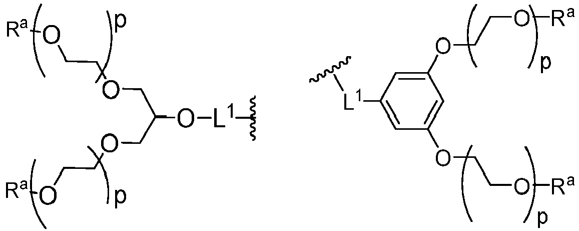 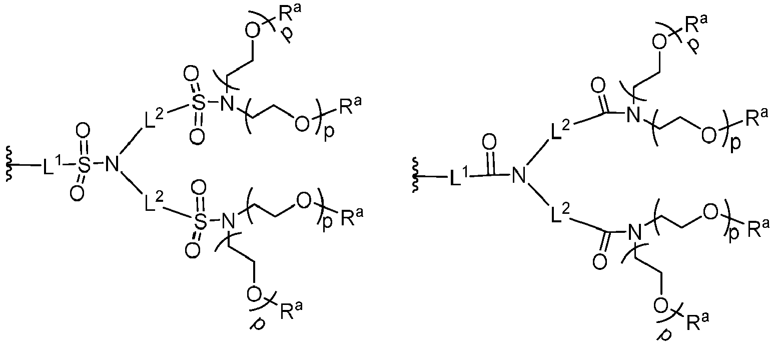 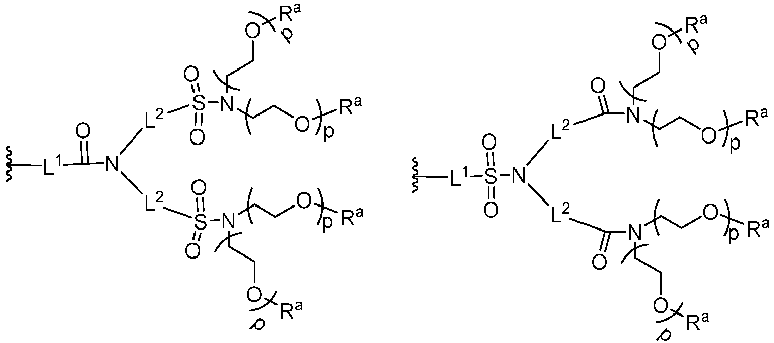 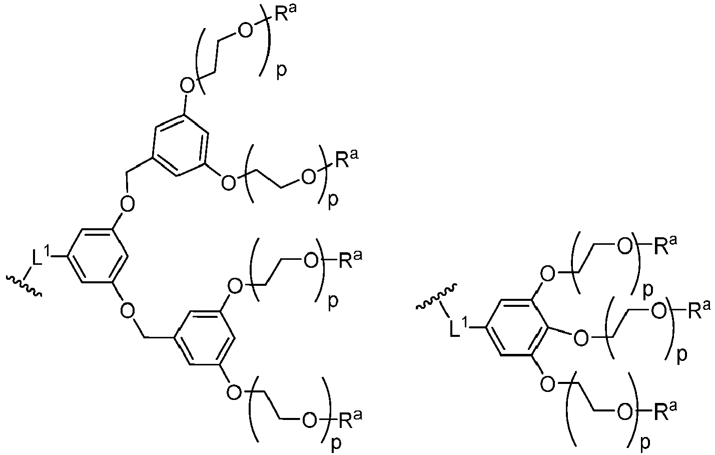 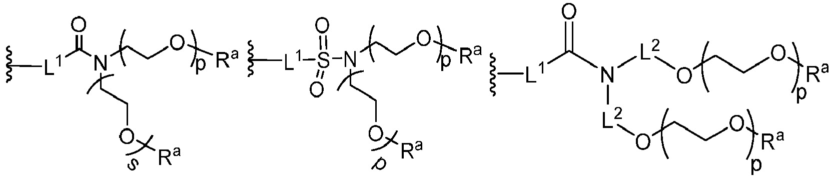 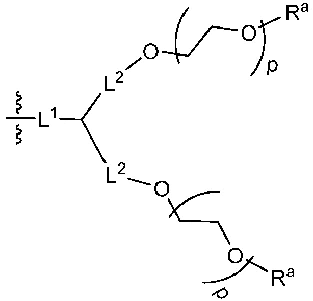 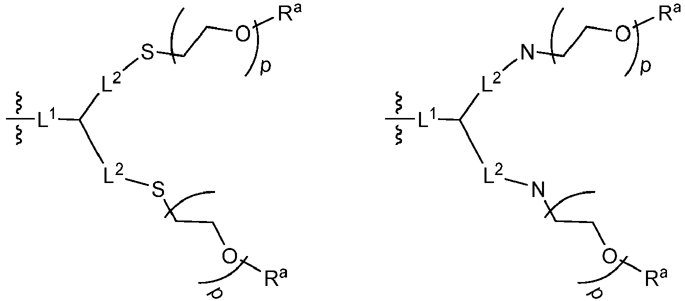 wherein:
   L¹ and L² are each independently an optional linker;
   R¹¹ and R are each independently H, alkyl or substituted alkyl; and
   each p is an integer from 6 to 24.
43. The water-soluble dipyrromethene-based dye of any one of clauses 39 to 42, wherein R¹ is selected from substituted alkyl, and substituted aryl, wherein the substituent comprises a chemoselective tag.
44. The water-soluble dipyrromethene-based dye of any one of clauses 39 to 43, wherein R^{5'} and R^{4'} both comprise a water solubilizing group (WSG).
45. The water-soluble dipyrromethene-based dye of any one of clauses 39 to 44, of one of the following structures: 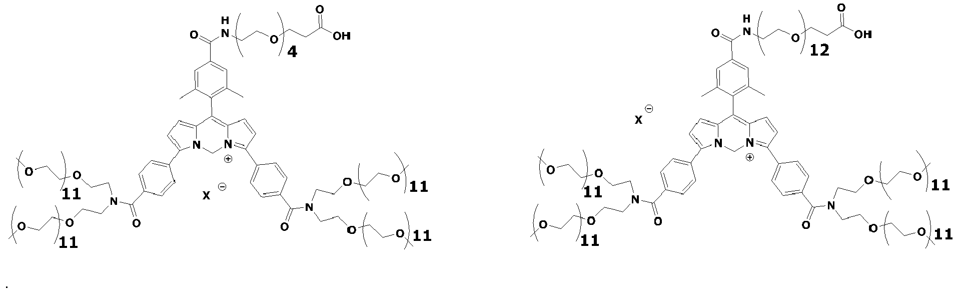
46. A yellow green dipyrromethene-based dye of formula (II): 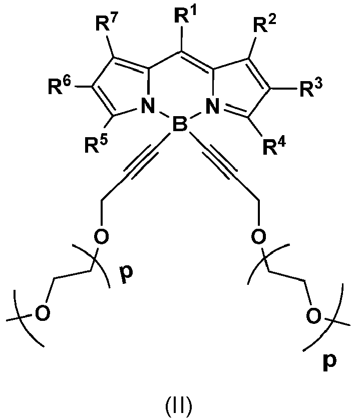 wherein:
   R¹-R⁷ are each independently selected from H, alkyl, halogen, substituted alkyl, alkoxy, substituted alkoxy, amino, substituted amino, amido, substituted amido, thiol, substituted thiol, aryl, substituted aryl, heteroaryl, substituted heteroaryl, heterocycle, substituted heterocycle, alkenyl, substituted alkenyl, alkynyl, substituted alkynyl, water solubilizing group (WSG), and -T¹-Z¹, or
   optionally any one or more pairs of substituents selected from R⁶ and R⁷, R² and R³, R⁵ and R⁶, R³ and R⁴, R² and R¹ and R⁷ and R¹, together form a divalent radical and are cyclically linked and together with the carbon atoms to which they are bound provide a 5- or 6-membered fused heterocycle, carbocycle, aryl or heteroaryl ring (e.g., a 5- or 6-membered ring comprising carbon atoms and 0-3 heteroatoms selected from O, S and N), which ring may be unsubstituted or further substituted with a substituent independently selected from alkyl, substituted alkyl, alkoxy, substituted alkoxy, aryl, substituted aryl, heteroaryl, substituted heteroaryl, alkenyl, substituted alkenyl, alkynyl, substituted alkynyl, water solubilizing group (WSG), and -T¹-Z¹;
   T¹ is an optional linker;
   Z¹ is a repeat unit of a compound of formula (I); and
   p is an integer from 6-24.
47. The yellow green dipyrromethene-based dye of clause 46, further comprising one or more water solubilizing groups (WSG) of formula (lb):

   -(L¹)ₙ₁ - (X¹)ₙ₂ - ((L²)ₙ₃-Z²)ₘ (Ib)

   wherein:
   n1, n2 and n3 are each independently 0 or 1;
   L¹, if present, is a linker;
   L² if present, is a linker;
   X¹, if present, is a branching point;
   Z² is a water soluble polymer comprising 1-50 monomeric units; and m is an integer from 1 to 3.
48. The yellow green dipyrromethene-based dye of clause 47, wherein the WSG comprises a PEG moiety.
49. The yellow green dipyrromethene-based dye of clause 48, wherein one or more water solubilizing groups (WSGs) is independently selected from the following formulae: 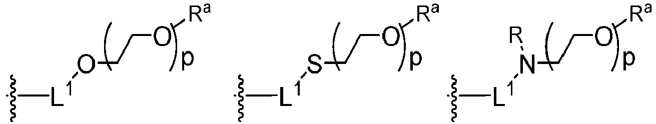 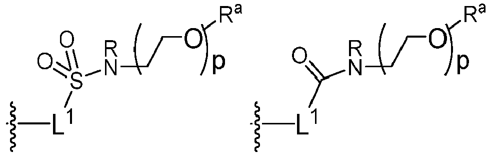 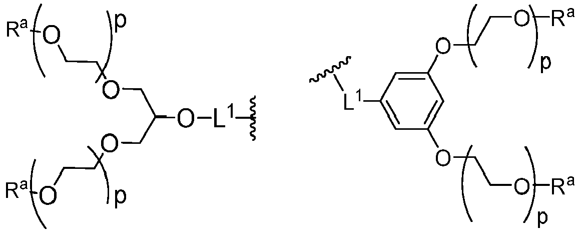 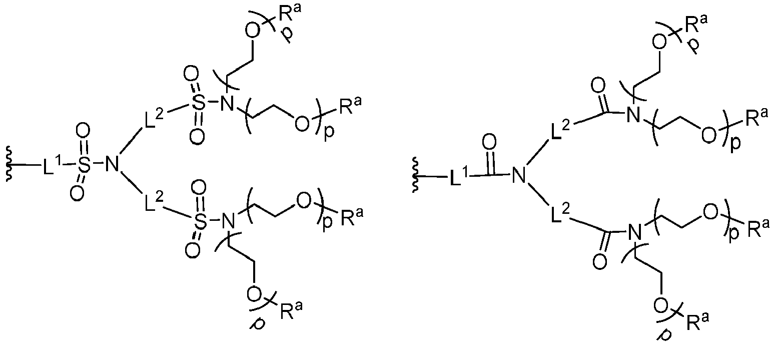 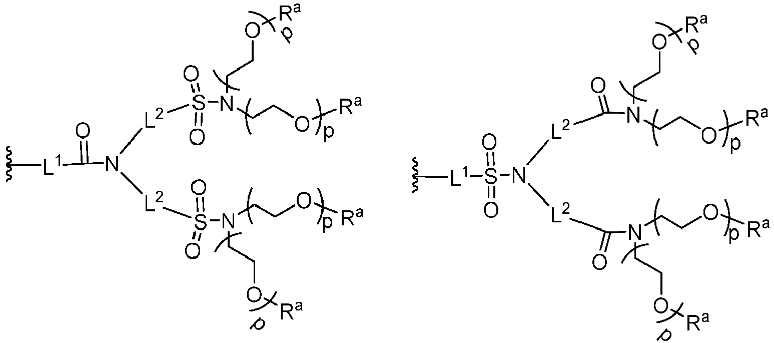 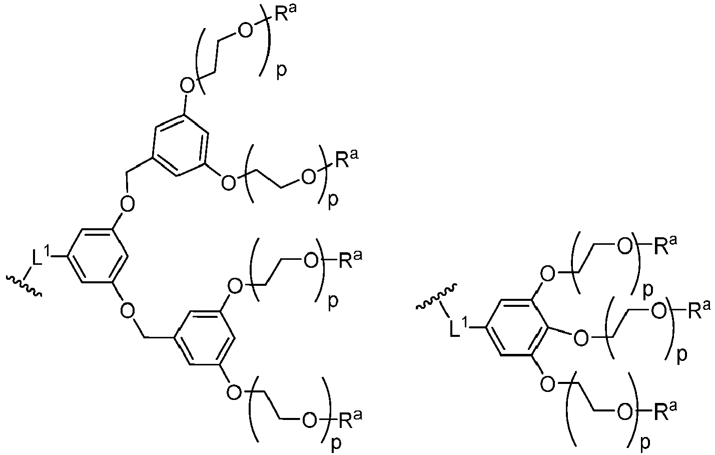 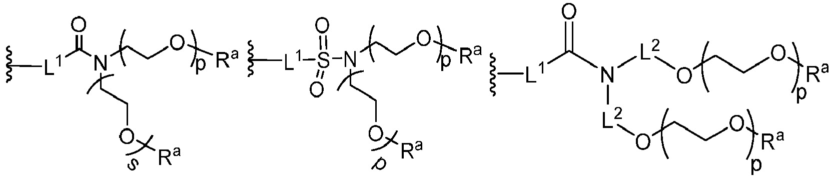 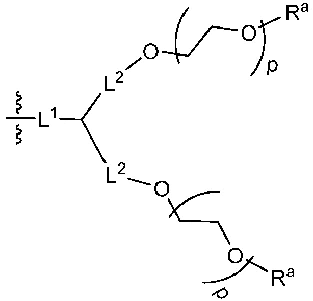 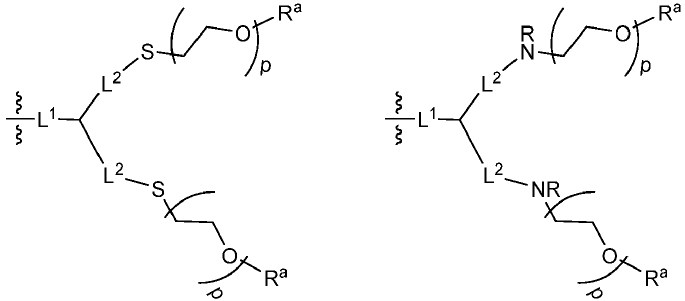 wherein:
   L¹ and L² are each independently an optional linker;
   R¹¹ and R are each independently H, alkyl or substituted alkyl; and
   each p is an integer from 6 to 24.
50. The yellow green dipyrromethene-based dye of any one of clauses 46 to 49, wherein R¹ is selected from substituted alkyl, and substituted aryl, wherein the substituent comprises a chemoselective tag.
51. The yellow green dipyrromethene-based dye of any one of clauses 46 to 50, wherein R⁵ is selected from halogen, substituted alkyl, substituted alkenyl, alkenyl aryl, aryl, substituted aryl, heterocycle, substituted heterocycle, spiro heterocycle, substituted spiro heterocycle, heteroaryl, substituted heteroaryl, substituted amino, substituted amido, and substituted thiol.
52. The yellow green dipyrromethene-based dye of clause 51, wherein R⁵ and R⁴ are the same.
53. The yellow green dipyrromethene-based dye of any one of clauses 46 to 52, of the formula (IIa): 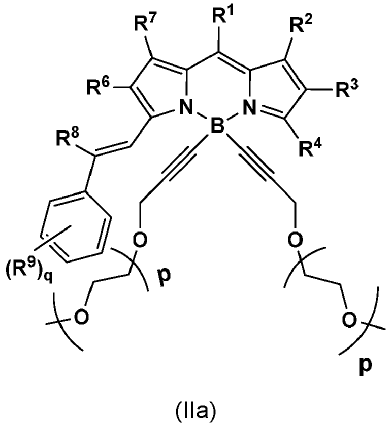 wherein:
   R⁸ is selected from H, halogen, cyano, alkyl, substituted alkyl, aryl, substituted aryl, heteroaryl, substituted heteroaryl, alkoxy, substituted alkoxy, alkenyl, substituted alkenyl, alkynyl, substituted alkynyl, and water solubilizing group (WSG), or
   R⁸ and R⁶ together with the atoms to which they are bound form a 5- or 6-membered heterocycle, carbocycle, aryl or heteroaryl ring (e.g., a 5- or 6-membered ring comprising carbon atoms and 0-3 heteroatoms selected from O, S and N), which ring may be unsubstituted or further substituted with a substituent independently selected from alkyl, substituted alkyl, alkoxy, substituted alkoxy, aryl, substituted aryl, heteroaryl, substituted heteroaryl, alkenyl, substituted alkenyl, alkynyl, substituted alkynyl, and water solubilizing group (WSG);
   q is an integer from 0 to 5; and
   each R⁹, if present, is independently selected from alkyl, substituted alkyl, hydroxy, alkoxy, substituted alkoxy, amino, substituted amino, thiol, substituted thiol, cyano, aryl, substituted aryl, heteroaryl, substituted heteroaryl, alkenyl, substituted alkenyl, alkynyl, substituted alkynyl, and water solubilizing group (WSG), or
   optionally two adjacent R⁹ groups, together form a divalent radical and are cyclically linked and together with the carbon atoms to which they are bound provide a 5- or 6-membered fused heterocycle, carbocycle, aryl or heteroaryl ring (e.g., a 5- or 6-membered ring comprising carbon atoms and 0-3 heteroatoms selected from O, S and N), which ring may be unsubstituted or further substituted with a substituent independently selected from alkyl, substituted alkyl, alkoxy, substituted alkoxy, aryl, substituted aryl, heteroaryl, substituted heteroaryl, alkenyl, substituted alkenyl, alkynyl, substituted alkynyl, and water solubilizing group (WSG).
54. The yellow green dipyrromethene-based dye of clause 53, wherein R⁸ is H.
55. The yellow green dipyrromethene-based dye of clause 53, wherein R⁸ and R⁶ together with the atoms to which they are bound form a 5- membered carbocycle, which carbocycle is optionally further substituted with a substituent independently selected from alkyl, substituted alkyl, alkoxy, substituted alkoxy, aryl, substituted aryl, heteroaryl, substituted heteroaryl, alkenyl, substituted alkenyl, alkynyl, substituted alkynyl, and water solubilizing group (WSG).
56. The yellow green dipyrromethene-based dye of any one of clauses 46 to 55, of any one of the following structures: 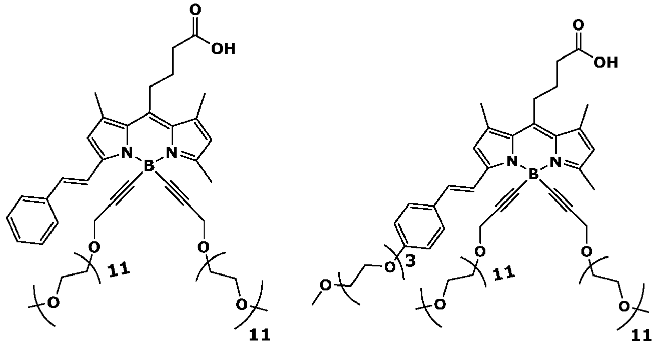 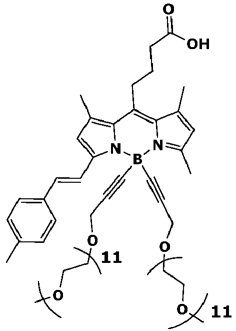 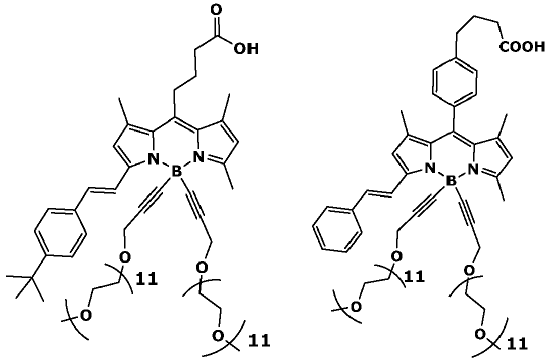 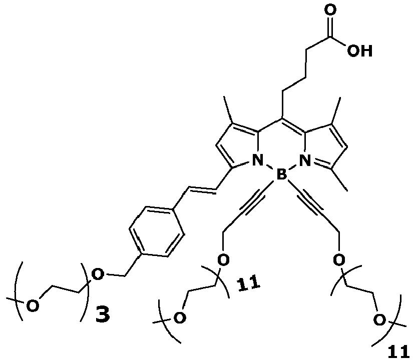 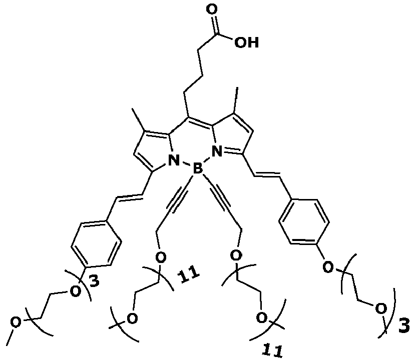 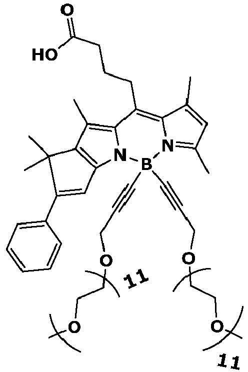 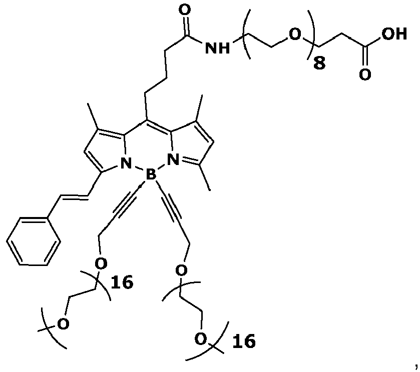 and 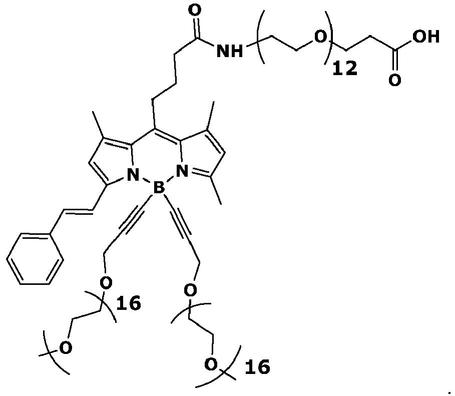
57. The yellow green dipyrromethene-based dye of any one of clauses 46 to 52, of the formula (IIb): 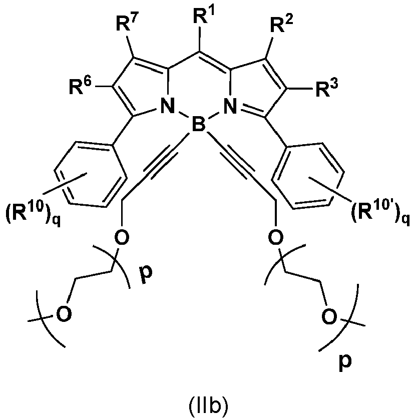 wherein:
   each q is independently an integer from 0 to 5; and
   R¹⁰ and R^{10'}, if present, are each independently selected from halogen, hydroxyl, cyano, amino, substituted amino, thiol, substituted thiol, alkyl, substituted alkyl, aryl, substituted aryl, heteroaryl, substituted heteroaryl, alkoxy, substituted alkoxy, alkenyl, substituted alkenyl, alkynyl, substituted alkynyl, and water soluble group (WSG), or
   at least one R¹⁰ group is at an ortho position, and R¹⁰ and R⁶ together with the atoms to which they are bound form a 5- or 6-membered ring (e.g., a 5- or 6-membered ring comprising a boron atom, carbon atoms and 0-3 heteroatoms selected from O, S and N), which ring may be unsubstituted or further substituted with a substituent independently selected from alkyl, substituted alkyl, alkoxy, substituted alkoxy, aryl, substituted aryl, heteroaryl, substituted heteroaryl, alkenyl, substituted alkenyl, alkynyl, substituted alkynyl, and water solubilizing group (WSG); and
   at least one R¹⁰' is an ortho position, and R¹⁰ and R³ together with the atoms to which they are bound form a 5- or 6-membered ring (e.g., a 5- or 6-membered ring comprising a boron atom, carbon atoms and 0-3 heteroatoms selected from O, S and N), which ring may be unsubstituted or further substituted with a substituent independently selected from alkyl, substituted alkyl, alkoxy, substituted alkoxy, aryl, substituted aryl, heteroaryl, substituted heteroaryl, alkenyl, substituted alkenyl, alkynyl, substituted alkynyl, and water solubilizing group (WSG).
58. The yellow green dipyrromethene-based dye of clause 57, wherein each q is 1, and R¹⁰ and R^{10'} are both present in their respective para positions.
59. The yellow green dipyrromethene-based dye of clause 58, wherein R¹⁰ and R^{10'} are each independently selected from cyano and a PEG moiety.
60. The yellow green dipyrromethene-based dye of clause 59, wherein the PEG moiety is selected from one of the following formulae: 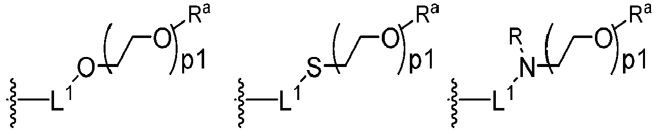 wherein:
   L¹ is an optional linker;
   R^{a} and R are each independently H, alkyl or substituted alkyl; and
   each p1 is an integer from 1 to 50.
61. The yellow green dipyrromethene-based dye of any one of clauses 57 to 60, of any one of the following structures: 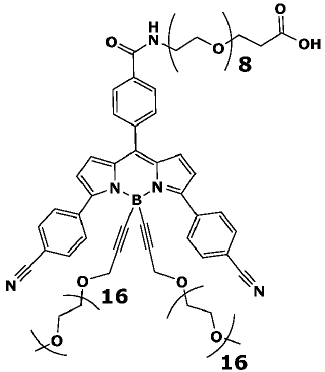 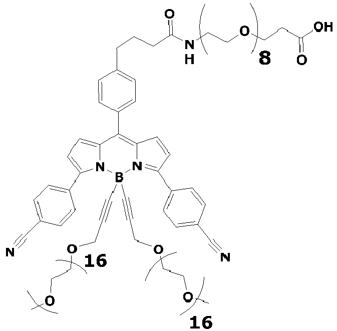 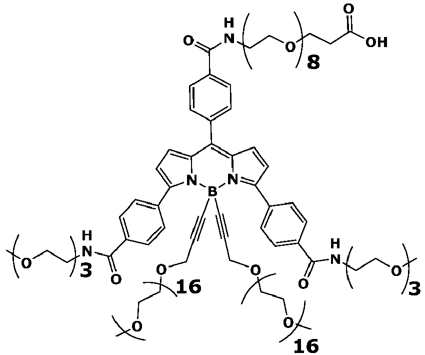 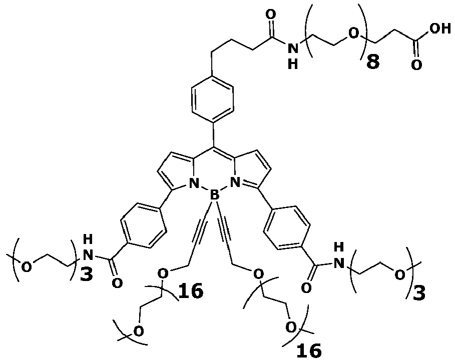 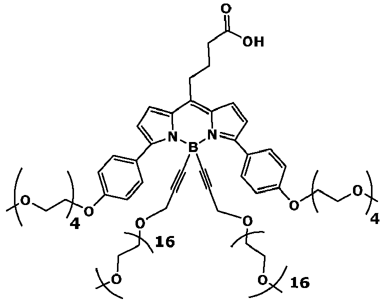 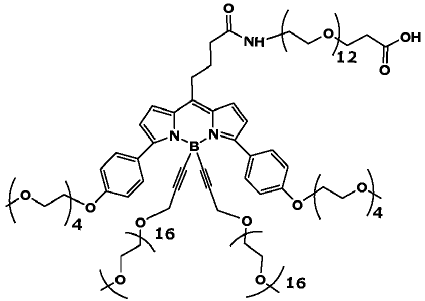 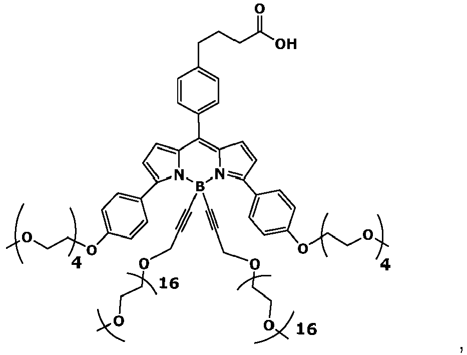 and 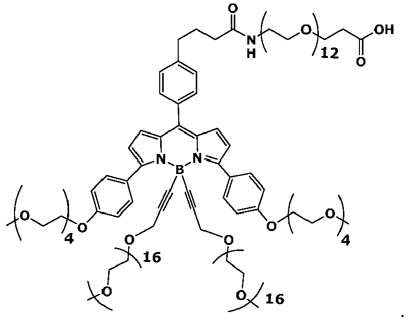
62. The yellow green dipyrromethene-based dye of any one of clauses 46 to 52, of the formula (IIc): 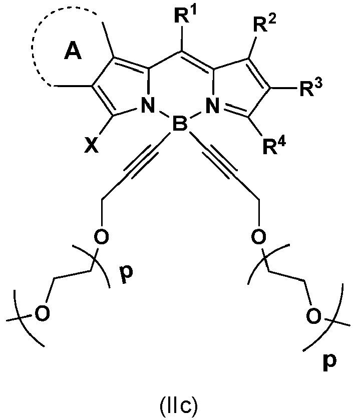 wherein:
   X is a halogen; and
   ring A is a 5- or 6-membered fused heterocycle, carbocycle, aryl or heteroaryl ring (e.g., a 5- or 6-membered ring comprising carbon atoms and 0-3 heteroatoms selected from O, S and N), which ring may be unsubstituted or further substituted with a substituent independently selected from alkyl, substituted alkyl, alkoxy, substituted alkoxy, aryl, substituted aryl, heteroaryl, substituted heteroaryl, alkenyl, substituted alkenyl, alkynyl, substituted alkynyl, and water solubilizing group (WSG).
63. The yellow green dipyrromethene-based dye of clause 62, wherein the A ring is an aryl ring.
64. The yellow green dipyrromethene-based dye of clause 62 or 63, wherein X is chloride.
65. The yellow green dipyrromethene-based dye of any one of clauses 62 to 64, of any one of the following compounds: 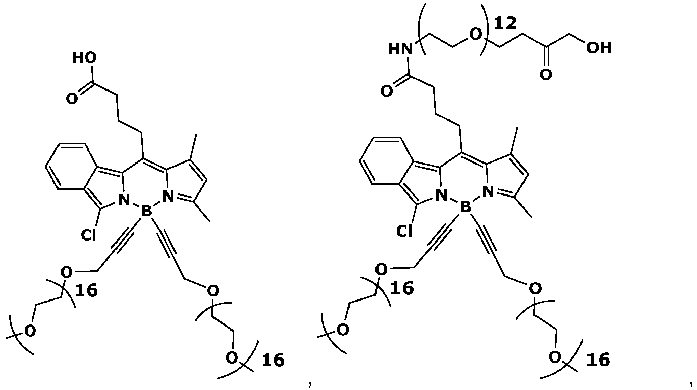 and 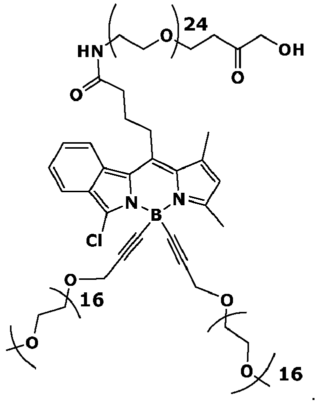
66. The yellow green dipyrromethene-based dye of any one of clauses 46 to 52, of the formula (IId): 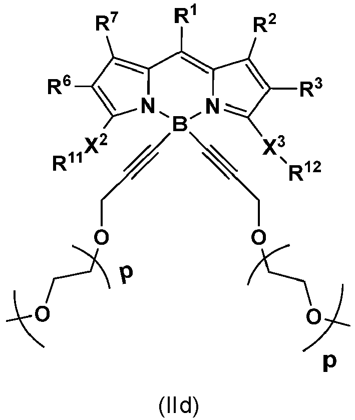 wherein:
   X² and X³ are each independently selected from NR¹³, S and O;
   R¹³ is selected from H, alkyl, and substituted alkyl;
   R¹¹ and R¹² are each independently selected from aryl, substituted aryl, heteroaryl, substituted heteroaryl, and a water soluble group (WSG), or
   when X² is NR¹³, optionally R¹³ and R¹¹ together with the nitrogen atom to which they are attached, form a heterocyclic or spiro heterocyclic ring, which ring may be unsubstituted or further substituted with a substituent independently selected from alkyl, substituted alkyl, alkoxy, substituted alkoxy, aryl, substituted aryl, heteroaryl, substituted heteroaryl, alkenyl, substituted alkenyl, alkynyl, substituted alkynyl, and a water solubilizing group (WSG); and
   when X³ is NR¹³, optionally R¹³ and R¹² together with the nitrogen atom to which they are attached, form a heterocyclic or spiro heterocyclic ring, which ring may be unsubstituted or further substituted with a substituent independently selected from alkyl, substituted alkyl, alkoxy, substituted alkoxy, aryl, substituted aryl, heteroaryl, substituted heteroaryl, alkenyl, substituted alkenyl, alkynyl, substituted alkynyl, and a water solubilizing group (WSG).
67. The yellow green dipyrromethene-based dye of clause 66, wherein X² and X³ are each NR¹³, wherein R¹³ is H.
68. The yellow green dipyrromethene-based dye of clause 66, wherein X² is NR¹³, and R¹³ and R¹¹ together with the nitrogen atom to which they are attached form a heterocyclic or a spiro heterocyclic ring; and X³ is NR¹³, and R¹³ and R¹² together with the nitrogen atom to which they are attached form a heterocyclic or a spiro heterocyclic ring.
69. The yellow green dipyrromethene-based dye of clause 66, wherein X² and X³ are both S.
70. The yellow green dipyrromethene-based dye of any one of clauses 66 to 69, of any one of the following structures: 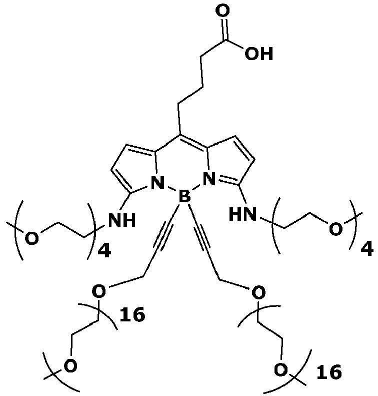 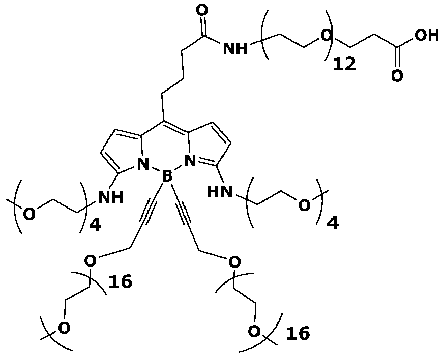 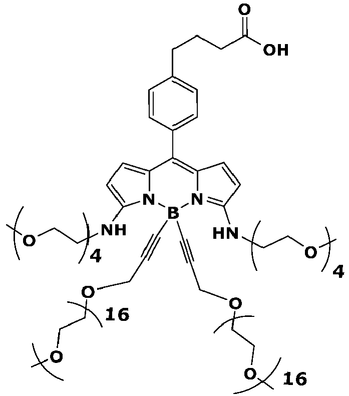 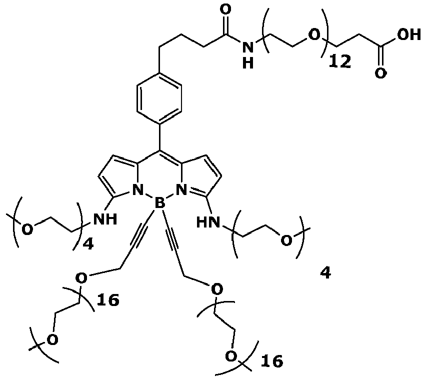 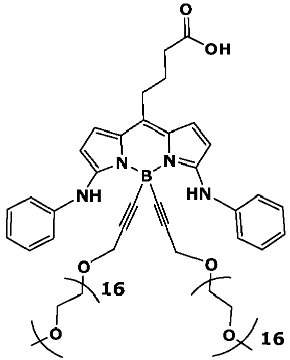 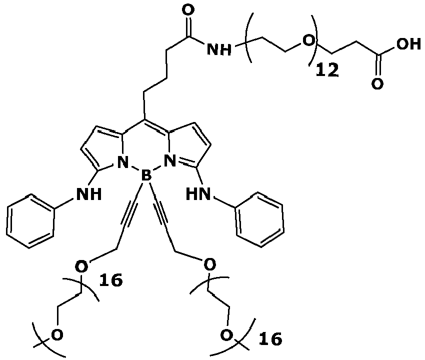 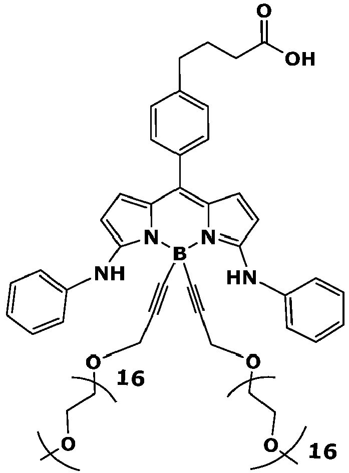 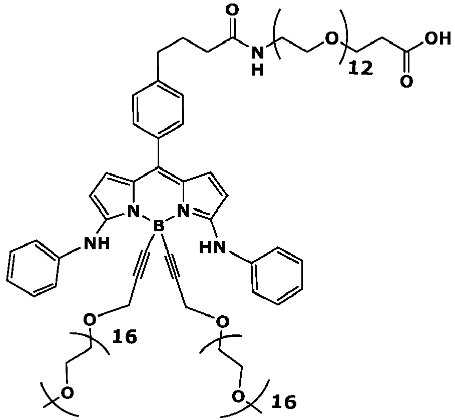 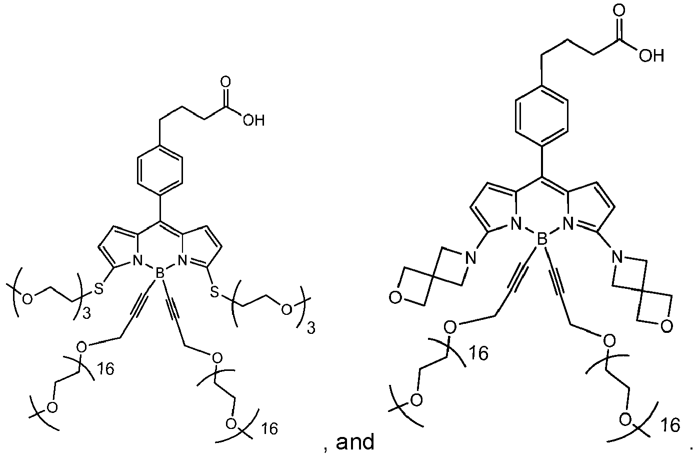
71. The yellow green dipyrromethene-based dye of any one of clauses 46 to 52, wherein R³ is -L¹-Z¹.
72. The yellow green dipyrromethene-based dye of clause 71, wherein R⁶ is selected from halogen, substituted alkyl, substituted alkenyl, alkenyl aryl, aryl, substituted aryl, heterocycle, substituted heterocycle, spiro heterocycle, substituted spiro heterocycle, heteroaryl, substituted heteroaryl, substituted amino, substituted amido, and substituted thiol.
73. The yellow green dipyrromethene-based dye of clause 72, wherein R⁶ comprises a PEG moiety.
74. The yellow green dipyrromethene-based dye of clause 73, wherein the PEG moiety is selected from one of the following formulae: 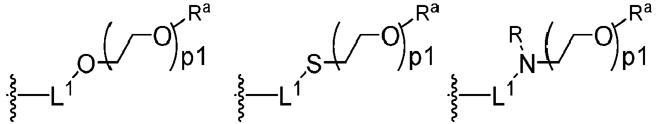 wherein:
   L¹ and L² are each independently an optional linker;
   R is H, alkyl or substituted alkyl; and
   each p1 is an integer from 1 to 50.
75. The yellow green dipyrromethene-based dye of any one of clauses 71 to 74, of one of the following structures: 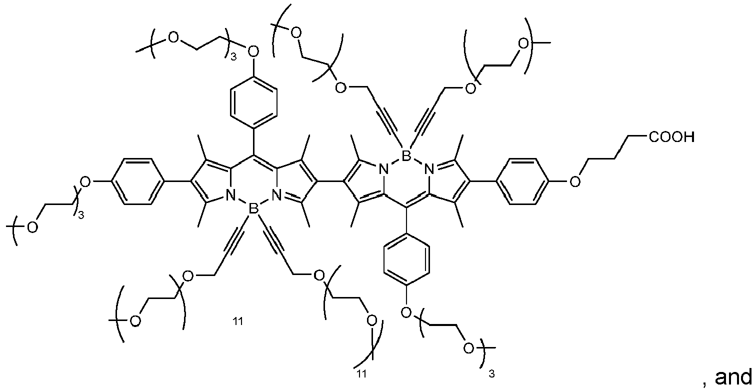 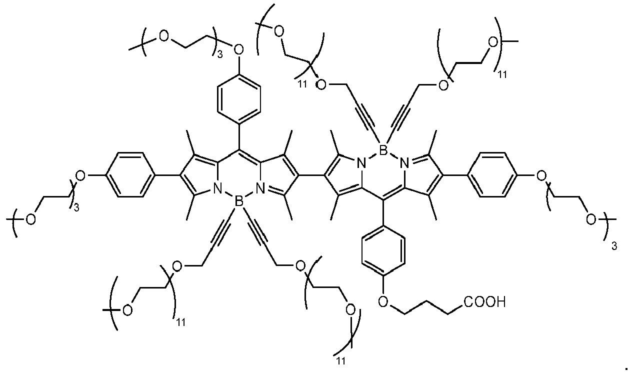
76. A yellow green dipyrromethene-based dye of formula (III): 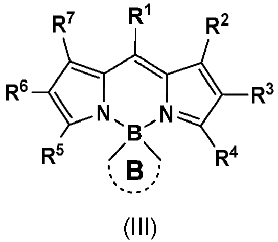 wherein:
   R¹-R⁷ are each independently selected from H, alkyl, halogen, substituted alkyl, alkoxy, substituted alkoxy, amino, substituted amino, amido, substituted amido, thiol, substituted thiol, aryl, substituted aryl, heteroaryl, substituted heteroaryl, heterocycle, substituted heterocycle, alkenyl, substituted alkenyl, alkynyl, substituted alkynyl, water solubilizing group (WSG), and -T¹-Z¹, or
   optionally any one or more pairs of substituents selected from R⁶ and R⁷, R² and R³, R⁵ and R⁶, R³ and R⁴, R² and R¹ and R⁷ and R¹, together form a divalent radical and are cyclically linked and together with the carbon atoms to which they are bound provide a 5- or 6-membered fused heterocycle, carbocycle, aryl or heteroaryl ring (e.g., a 5- or 6-membered ring comprising carbon atoms and 0-3 heteroatoms selected from O, S and N), which ring may be unsubstituted or further substituted with a substituent independently selected from alkyl, substituted alkyl, alkoxy, substituted alkoxy, aryl, substituted aryl, heteroaryl, substituted heteroaryl, alkenyl, substituted alkenyl, alkynyl, substituted alkynyl, water solubilizing group (WSG), and -T¹-Z¹;
   T¹ is an optional linker;
   Z¹ is a repeat unit of a compound of formula (I); and
   ring B is a 5- or 6-membered cyclic group (e.g., a 5- or 6-membered ring comprising a boron atom, carbon atoms and 0-3 heteroatoms selected from O, S and N), which cyclic group may be unsubstituted or further substituted with one or more substituents independently selected from alkyl, substituted alkyl, alkoxy, substituted alkoxy, aryl, substituted aryl, heteroaryl, substituted heteroaryl, alkenyl, substituted alkenyl, alkynyl, substituted alkynyl, and water solubilizing group (WSG); and
   wherein at least one moiety selected from Y¹, Y² and R¹-R⁷ is a WSG, or at least one moiety selected from Y¹, Y² and R¹-R⁷ is substituted with a WSG, where the WSG is of the formula (Ia):

      -(L¹)ₙ₁ - (X¹)ₙ₂ - ((L²)ₙ₃-Z¹)ₘ (Ia)

      wherein:
      n1, n2 and n3 are each independently 0 or 1;
      L¹, if present, is a linker;
      L² if present, is a linker;
      X¹, if present, is a branching point;
      Z¹ is a water soluble polymer comprising 6-24 monomeric units; and
      m is an integer from 1 to 3.
77. The yellow green dipyrromethene-based dye of clause 76, further comprising a WSG of formula (Ib):

   -(L1)ₙ₁ - (X¹)ₙ₂ - ((L²)ₙ₃-Z²)ₘ (Ib)

   wherein:
   n1, n2 and n3 are each independently 0 or 1;
   L¹, if present, is a linker;
   L² if present, is a linker;
   X¹, if present, is a branching point;
   Z² is a water soluble polymer comprising 1-50 monomeric units; and
   m is an integer from 1 to 3.
78. The yellow green dipyrromethene-based dye of clause 76 or 77, wherein the WSG comprises a PEG moiety.
79. The yellow green dipyrromethene-based dye of any one of clauses 76 to 78, wherein one or more water solubilizing groups (WSGs) is independently selected from the following formulae: 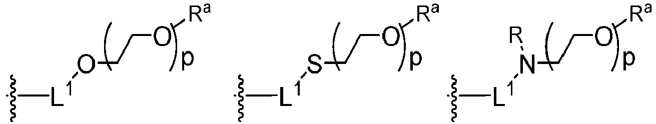 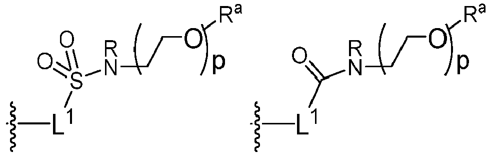 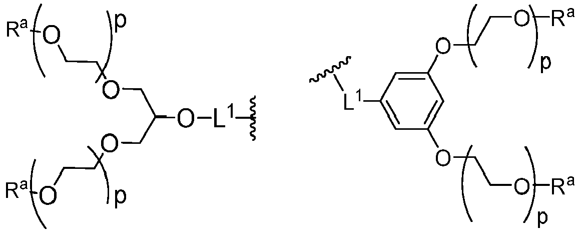 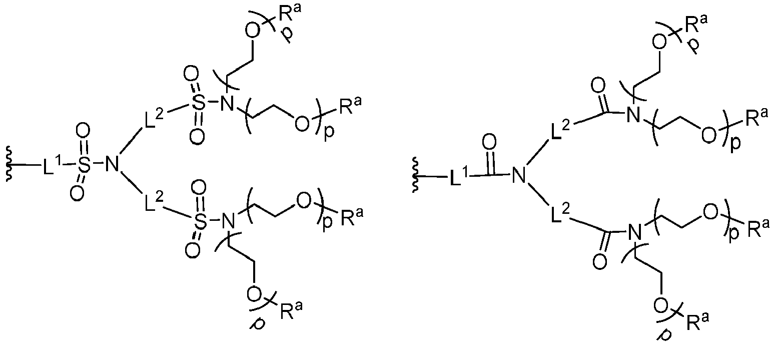 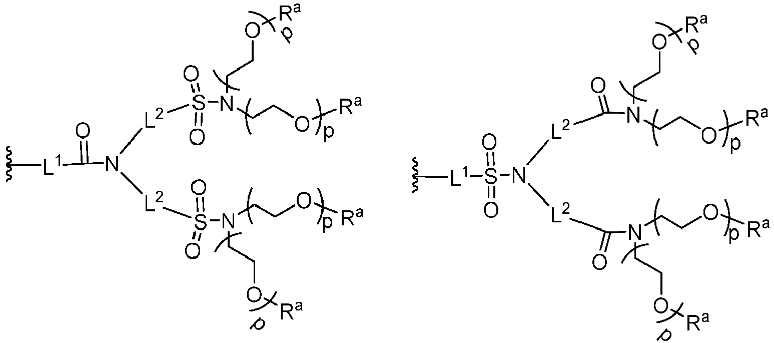 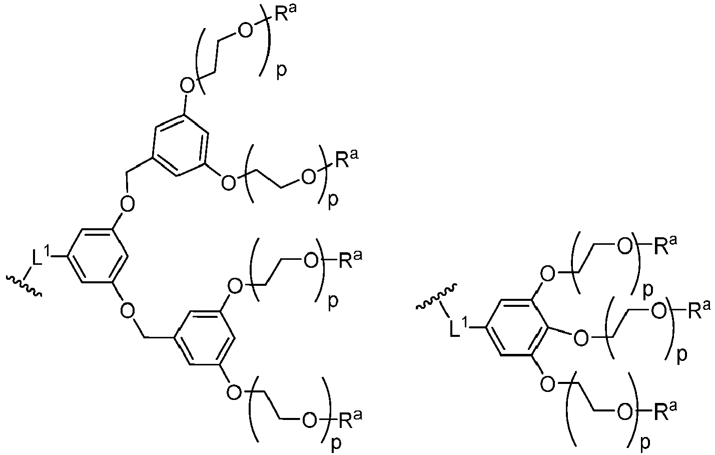 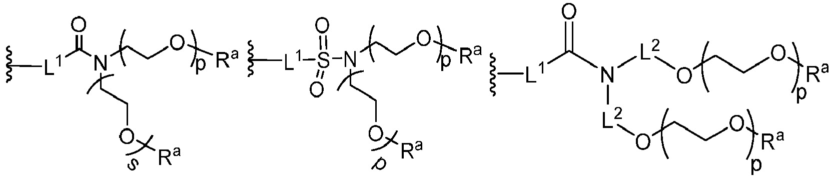 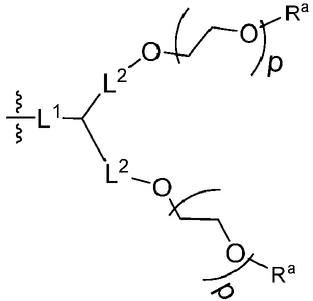 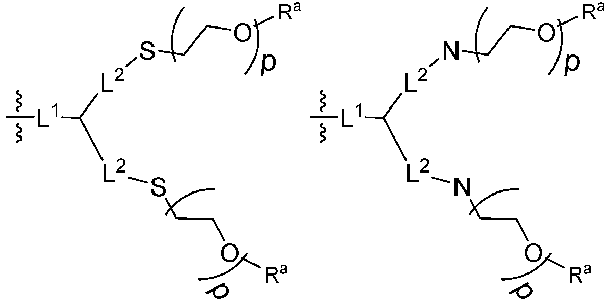 wherein:
   L¹ and L² are each independently an optional linker;
   R¹¹ and R are each independently H, alkyl or substituted alkyl; and
   each p is an integer from 6 to 24.
80. The yellow green dipyrromethene-based dye of any one of clauses 76 to 79, wherein R¹ is selected from, substituted alkyl, and substituted aryl, wherein the substituent comprises a chemoselective tag.
81. The yellow green dipyrromethene-based dye of any one of clauses 76 to 80, of the formula (IIIa): 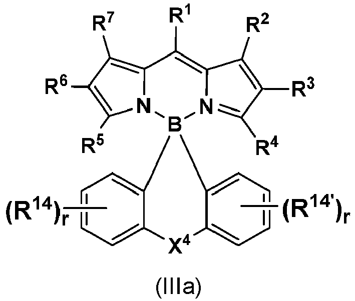 wherein:
   X⁴ is selected from O, S, NR, and CR₂;
   each R is independently selected from H, alkyl, and substituted alkyl;
   each r is independently an integer from 0 to 4; and
   each R¹⁴ and R^{14'}, if present, is independently selected from halogen, hydroxyl, cyano, amino, substituted amino, thiol, substituted thiol, amido, substituted amido, acyl, substituted acyl, alkyl, substituted alkyl, aryl, substituted aryl, heteroaryl, substituted heteroaryl, alkoxy, substituted alkoxy, alkenyl, substituted alkenyl, alkynyl, substituted alkynyl, and water soluble group (WSG).
82. The yellow green dipyrromethene-based dye of any one of clauses 76 to 81, wherein at least one of R⁵ and R⁶ and R⁴ and R³ together form a divalent radical and are cyclically linked and together with the carbon atoms to which they are bound provide a 5- or 6-membered fused heterocycle, carbocycle, aryl or heteroaryl ring (e.g., a 5- or 6-membered ring comprising carbon atoms and 0-3 heteroatoms selected from O, S and N), which ring may be unsubstituted or further substituted with a substituent independently selected from alkyl, substituted alkyl, alkoxy, substituted alkoxy, aryl, substituted aryl, heteroaryl, substituted heteroaryl, alkenyl, substituted alkenyl, alkynyl, substituted alkynyl, and water solubilizing group (WSG).
83. The yellow green dipyrromethene-based dye of clause 82, of the formula (IIIb) or (IIIc): 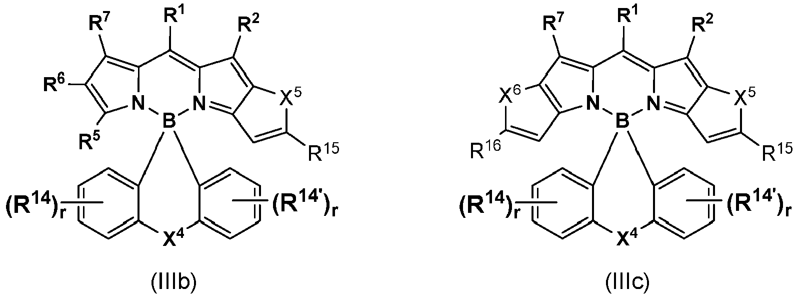 wherein:
   X⁵ and X⁶ are each independently selected from O, S, NR, and CR₂;
   each R is independently selected from H, alkyl, and substituted alkyl; and
   R¹⁵ and R¹⁶, are each independently selected from hydrogen, halogen, hydroxyl, cyano, amino, substituted amino, thiol, substituted thiol, amido, substituted amido, acyl, substituted acyl, alkyl, substituted alkyl, aryl, substituted aryl, heteroaryl, substituted heteroaryl, alkoxy, substituted alkoxy, alkenyl, substituted alkenyl, alkynyl, substituted alkynyl, and water soluble group (WSG).
84. The yellow green dipyrromethene-based dye of clause 83, wherein X⁵ and X⁶ are O.
85. The yellow green dipyrromethene-based dye of any one of clauses 76 to 84, of any one of the following structures: 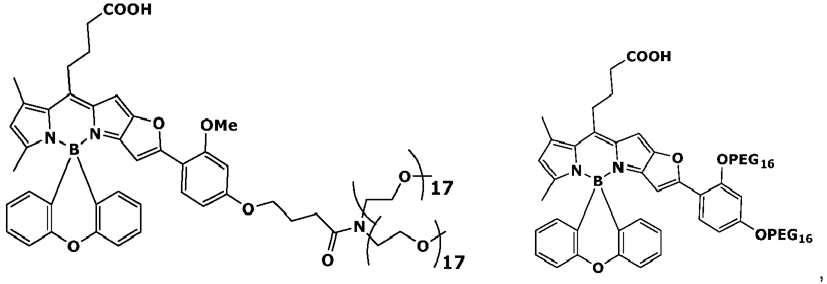 and 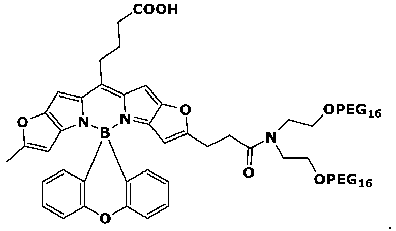
86. A tandem dye comprising:
   a light harvesting chromophore comprising:
   a backbone;
   a donor chromophore linked to the backbone, wherein the donor chromophore is a water-soluble dipyrromethene-based dye that absorbs in the yellow-green range at 561 nm; and
   an energy acceptor comprising a pendant acceptor fluorophore linked to the backbone, and configured in energy-receiving proximity to the donor chromophore.
87. The tandem dye of clause 86, wherein the donor chromophore has an absorption maxima from 555 to 585 nm.
88. The tandem dye of clause 87, wherein the donor chromophore has an absorption maxima from 558 to 568 nm.
89. The tandem dye of clause 88, wherein the donor chromophore has an absorption maxima of about 561 nm.
90. The tandem dye of any one of clauses 86 to 89, wherein the donor chromophore is a water-soluble dipyrromethene-boron dye.
91. The tandem dye of any one of clauses 86 to 90, wherein the donor chromophore is of formula (I): 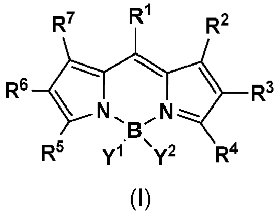 wherein:
   R¹-R⁷ are each independently selected from H, alkyl, halogen, substituted alkyl, alkoxy, substituted alkoxy, amino, substituted amino, amido, substituted amido, thiol, substituted thiol, aryl, substituted aryl, heteroaryl, substituted heteroaryl, heterocycle, substituted heterocycle, alkenyl, substituted alkenyl, alkynyl, substituted alkynyl, water solubilizing group (WSG), and -T¹-Z¹, or
   optionally any one or more pairs of substituents selected from R⁶ and R⁷, R² and R³, R⁵ and R⁶, R³ and R⁴, R² and R¹ and R⁷ and R¹, together form a divalent radical and are cyclically linked and together with the carbon atoms to which they are bound provide a 5- or 6-membered fused heterocycle, carbocycle, aryl or heteroaryl ring (e.g., a 5- or 6-membered ring comprising carbon atoms and 0-3 heteroatoms selected from O, S and N), which ring may be unsubstituted or further substituted with a substituent independently selected from alkyl, substituted alkyl, alkoxy, substituted alkoxy, aryl, substituted aryl, heteroaryl, substituted heteroaryl, alkenyl, substituted alkenyl, alkynyl, substituted alkynyl, water solubilizing group (WSG), and -T¹-Z¹;
   T¹ is an optional linker;
   Z¹ is a repeat unit of a compound of formula (I); and
   Y¹ and Y² are independently selected from F, OH, H, cyano, alkyl, substituted alkyl, aryl, substituted aryl, heteroaryl, substituted heteroaryl, alkoxy, substituted alkoxy, alkenyl, substituted alkenyl, alkynyl, substituted alkynyl, and a water solubilizing group (WSG), or
   Y¹ and Y² together with the boron atom to which they are bound form a 5- or 6-membered ring (e.g., a 5- or 6-membered ring comprising a boron atom, carbon atoms and 0-3 heteroatoms selected from O, S and N), which ring may be unsubstituted or further substituted with a substituent independently selected from alkyl, substituted alkyl, alkoxy, substituted alkoxy, aryl, substituted aryl, heteroaryl, substituted heteroaryl, alkenyl, substituted alkenyl, alkynyl, substituted alkynyl, water solubilizing group (WSG), and -T¹-Z¹; and
   wherein at least one moiety selected from Y¹, Y² and R¹-R⁷ is a WSG, or at least one moiety selected from Y¹, Y² and R¹-R⁷ is substituted with a WSG, where the WSG is of the formula (Ia):

      -(L¹)ₙ₁ - (X¹)ₙ₂ - ((L²)ₙ₃-Z¹)ₘ (Ia)

      wherein:
      n1, n2 and n3 are each independently 0 or 1;
      L¹, if present, is a linker;
      L² if present, is a linker;
      X¹, if present, is a branching point;
      Z¹ is a water soluble polymer comprising 6-24 monomeric units; and
      m is an integer from 1 to 3.
92. The tandem dye of clause 91, wherein the donor chromophore of formula (I) further comprises a WSG of formula (Ib):

   -(L1)ₙ₁ - (X¹)ₙ₂ - ((L²)ₙ₃-Z²)ₘ (Ib)

   wherein:
   n1, n2 and n3 are each independently 0 or 1;
   L¹, if present, is a linker;
   L² if present, is a linker;
   X¹, if present, is a branching point;
   Z² is a water soluble polymer comprising 1-50 monomeric units; and
   m is an integer from 1 to 3.
93. The tandem dye of clause 91 or 92, wherein one or more water solubilizing groups (WSGs) comprises a PEG moiety.
94. The tandem dye of clause 93, wherein one or more water solubilizing groups (WSGs) is independently selected from the following formulae: 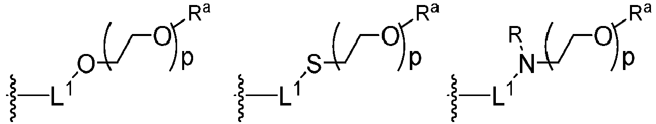 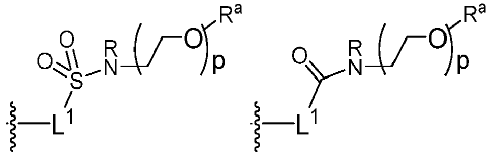 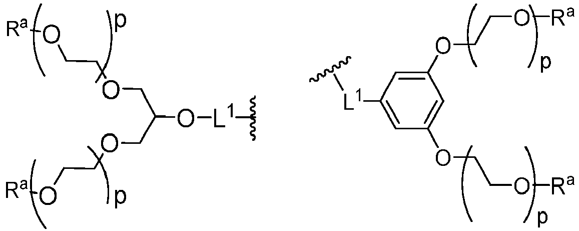 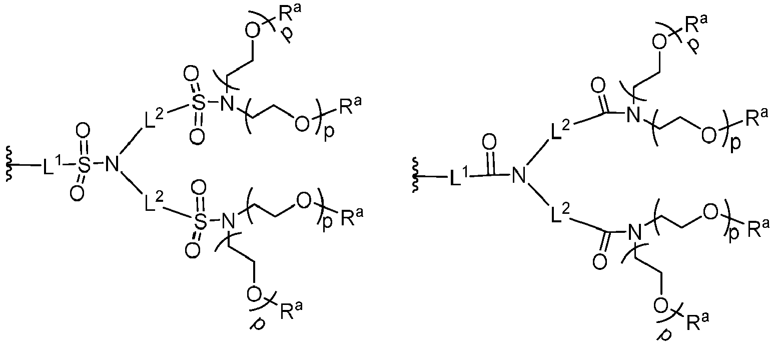 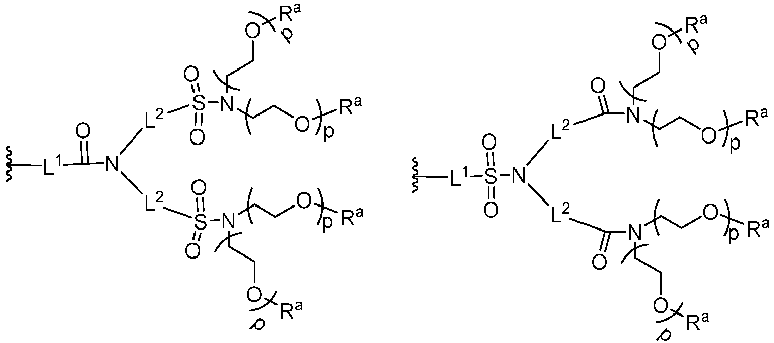 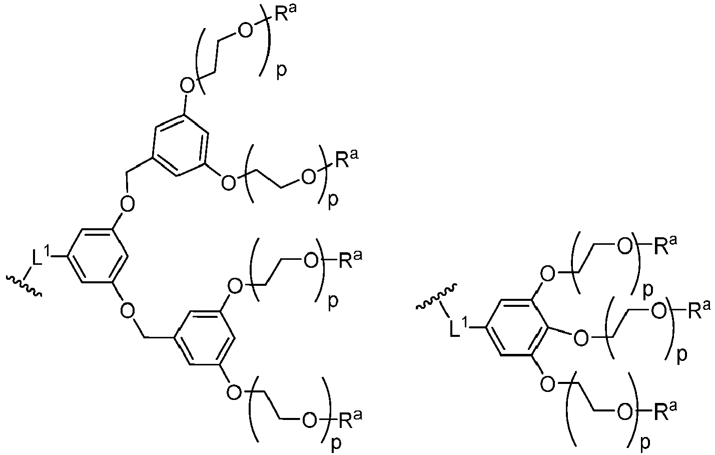 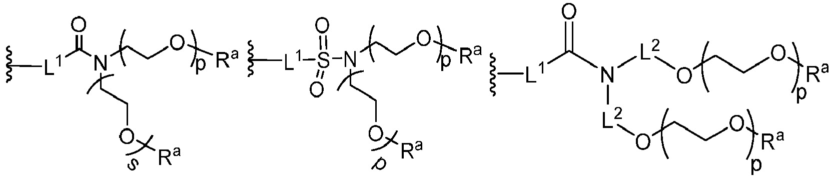 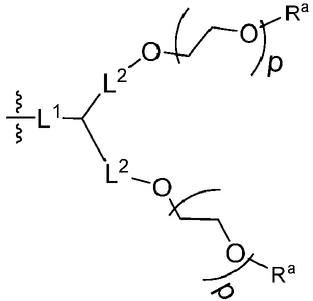 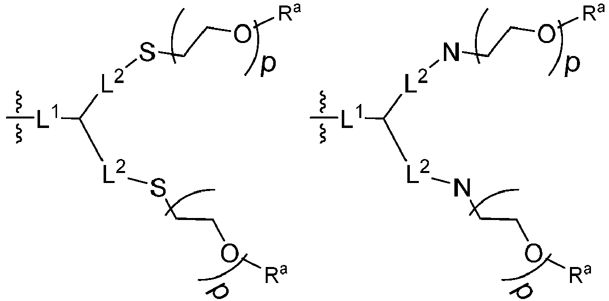 wherein:
   L¹ and L² are each independently an optional linker;
   R¹¹ and R are each independently H, alkyl or substituted alkyl; and
   each p is an integer from 6 to 24.
95. The tandem dye of any one of clauses 91 to 94, wherein R¹ is selected from substituted alkyl, and substituted aryl, wherein the substituent comprises a chemoselective tag.
96. The tandem dye of any one of clauses 91 to 95, wherein Y¹ and Y² each comprise a water solubilizing group (WSG).
97. The tandem dye of any one of clauses 91 to 96, wherein the donor chromophore is of the formula (II): 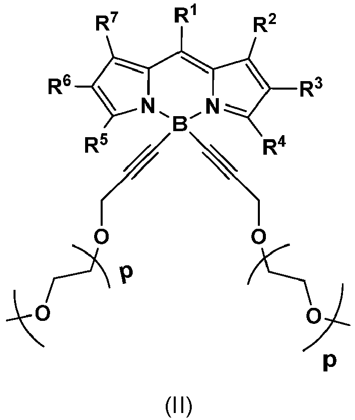 wherein p is an integer from 6-24.
98. The tandem dye of any one of clauses 91 to 97, wherein R⁵ is selected from halogen, substituted alkyl, substituted alkenyl, alkenyl aryl, aryl, substituted aryl, heterocycle, substituted heterocycle, spiro heterocycle, substituted spiro heterocycle, heteroaryl, substituted heteroaryl, substituted amino, substituted amido, and substituted thiol.
99. The tandem dye of clause 98, wherein R⁵ and R⁴ are the same.
100. The tandem dye of any one of clauses 91 to 99, wherein the donor chromophore is of the formula (III): 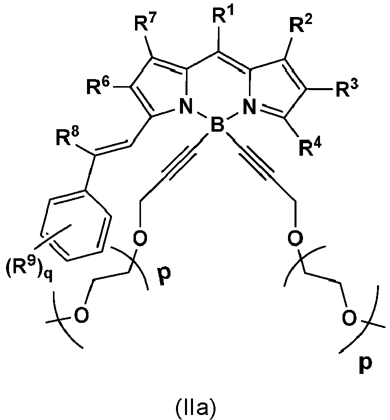 wherein:
   R⁸ is selected from H, halogen, cyano, alkyl, substituted alkyl, aryl, substituted aryl, heteroaryl, substituted heteroaryl, alkoxy, substituted alkoxy, alkenyl, substituted alkenyl, alkynyl, substituted alkynyl, and water solubilizing group (WSG), or
   R⁸ and R⁶ together with the atoms to which they are bound form a 5- or 6-membered heterocycle, carbocycle, aryl or heteroaryl ring (e.g., a 5- or 6-membered ring comprising carbon atoms and 0-3 heteroatoms selected from O, S and N), which ring may be unsubstituted or further substituted with a substituent independently selected from alkyl, substituted alkyl, alkoxy, substituted alkoxy, aryl, substituted aryl, heteroaryl, substituted heteroaryl, alkenyl, substituted alkenyl, alkynyl, substituted alkynyl, and water solubilizing group (WSG);
   q is an integer from 0 to 5; and
   each R⁹, if present, is independently selected from alkyl, substituted alkyl, hydroxy, alkoxy, substituted alkoxy, amino, substituted amino, thiol, substituted thiol, cyano, aryl, substituted aryl, heteroaryl, substituted heteroaryl, alkenyl, substituted alkenyl, alkynyl, substituted alkynyl, and water solubilizing group (WSG), or
   optionally two adjacent R⁹ groups, together form a divalent radical and are cyclically linked and together with the carbon atoms to which they are bound provide a 5- or 6-membered fused heterocycle, carbocycle, aryl or heteroaryl ring (e.g., a 5- or 6-membered ring comprising carbon atoms and 0-3 heteroatoms selected from O, S and N), which ring may be unsubstituted or further substituted with a substituent independently selected from alkyl, substituted alkyl, alkoxy, substituted alkoxy, aryl, substituted aryl, heteroaryl, substituted heteroaryl, alkenyl, substituted alkenyl, alkynyl, substituted alkynyl, and water solubilizing group (WSG).
101. The tandem dye of clause 100, wherein R⁸ is H.
102. The tandem dye of clause 100, wherein R⁸ and R⁶ together with the atoms to which they are bound form a 5- membered carbocycle, which carbocycle is optionally further substituted with a substituent independently selected from alkyl, substituted alkyl, alkoxy, substituted alkoxy, aryl, substituted aryl, heteroaryl, substituted heteroaryl, alkenyl, substituted alkenyl, alkynyl, substituted alkynyl, and water solubilizing group (WSG).
103. The tandem dye of any one of clauses 100 to 102, wherein the donor chromophore is of any one of the following structures: 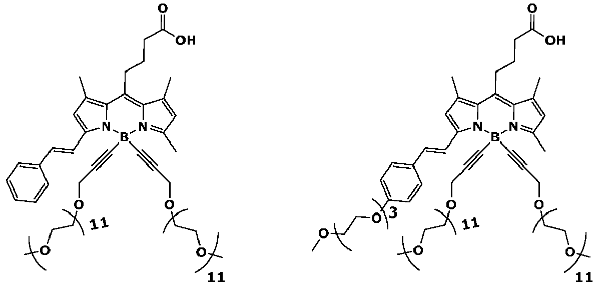 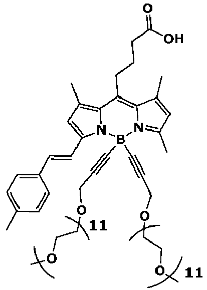 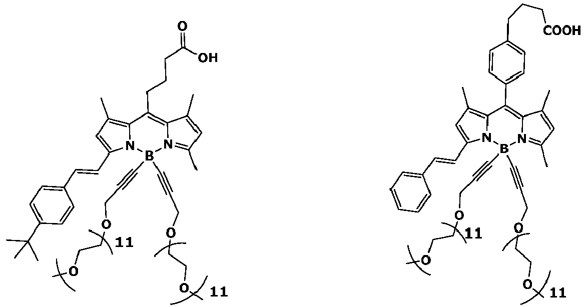 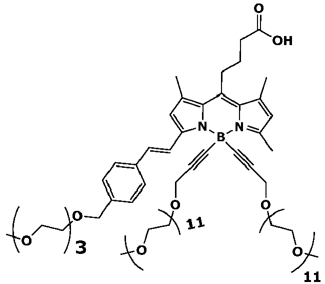 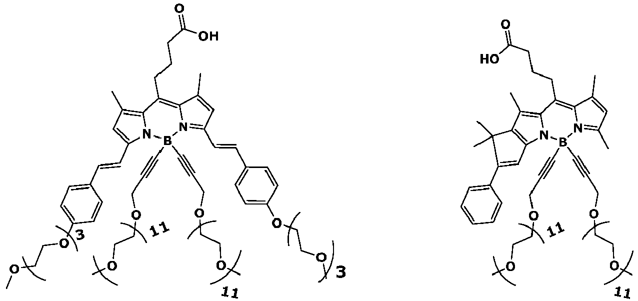 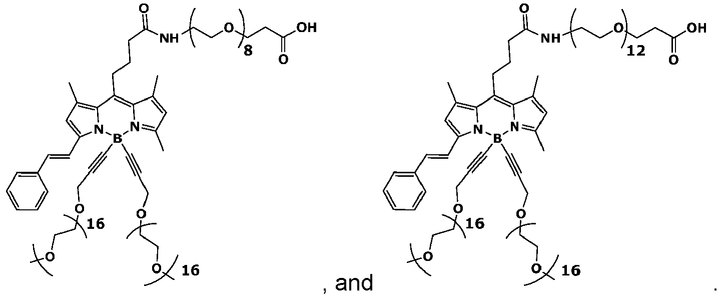
104. The tandem dye of any one of clauses 91 to 99, wherein the donor chromophore is of the formula (IIb): 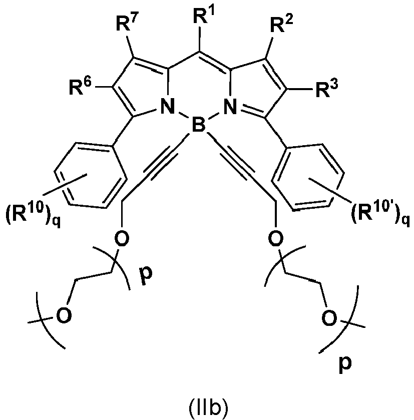 wherein:
   each q is independently an integer from 0 to 5; and
   R¹⁰ and R^{10'}, if present, are each independently selected from halogen, hydroxyl, cyano, amino, substituted amino, thiol, substituted thiol, alkyl, substituted alkyl, aryl, substituted aryl, heteroaryl, substituted heteroaryl, alkoxy, substituted alkoxy, alkenyl, substituted alkenyl, alkynyl, substituted alkynyl, and water soluble group (WSG), or
   at least one R¹⁰ group is at an ortho position, and R¹⁰ and R⁶ together with the atoms to which they are bound form a 5- or 6-membered ring (e.g., a 5- or 6-membered ring comprising a boron atom, carbon atoms and 0-3 heteroatoms selected from O, S and N), which ring may be unsubstituted or further substituted with a substituent independently selected from alkyl, substituted alkyl, alkoxy, substituted alkoxy, aryl, substituted aryl, heteroaryl, substituted heteroaryl, alkenyl, substituted alkenyl, alkynyl, substituted alkynyl, and water solubilizing group (WSG); and
   at least one R^{10'} is an ortho position, and R¹⁰ and R³ together with the atoms to which they are bound form a 5- or 6-membered ring (e.g., a 5- or 6-membered ring comprising a boron atom, carbon atoms and 0-3 heteroatoms selected from O, S and N), which ring may be unsubstituted or further substituted with a substituent independently selected from alkyl, substituted alkyl, alkoxy, substituted alkoxy, aryl, substituted aryl, heteroaryl, substituted heteroaryl, alkenyl, substituted alkenyl, alkynyl, substituted alkynyl, and water solubilizing group (WSG).
105. The tandem dye of clause 104, wherein each q is 1, and R¹⁰ and R^{10'} are both present in their respective para positions.
106. The tandem dye of clause 105, wherein R¹⁰ and R^{10'} are each independently selected from cyano and a PEG moiety.
107. The tandem dye of any one of clauses 104 to 106, wherein the donor chromophore is of any one of the following structures: 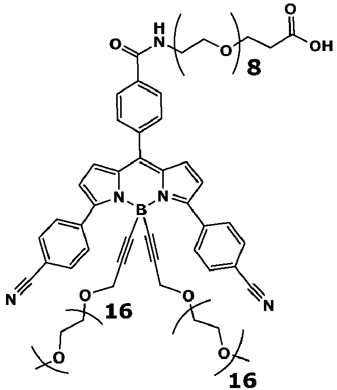 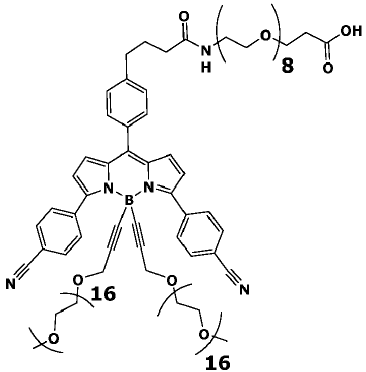 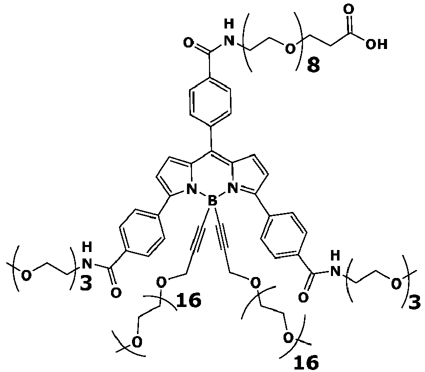 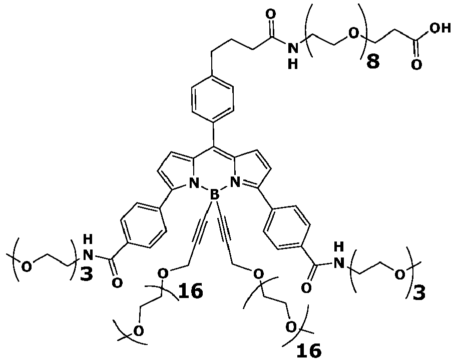 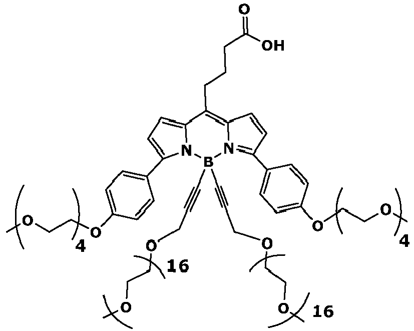 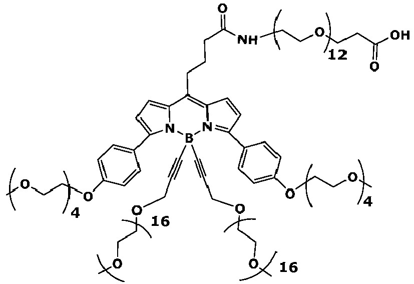 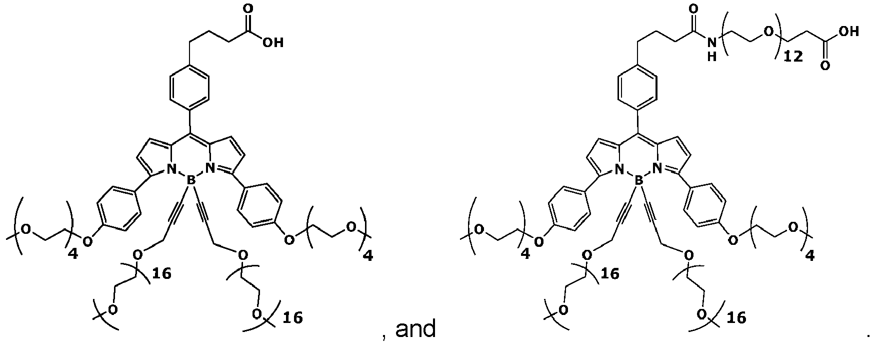
108. The tandem dye of any one of clauses 91 to 99, wherein the donor chromophore is of the formula (IIc): 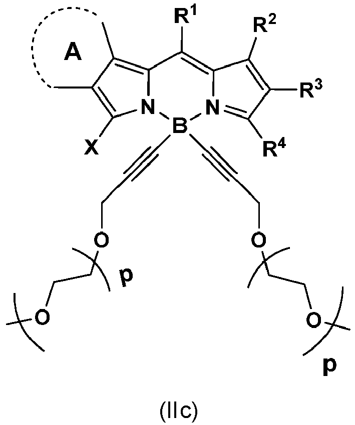 wherein:
   X is a halogen; and
   ring A is a 5- or 6-membered fused heterocycle, carbocycle, aryl or heteroaryl ring (e.g., a 5- or 6-membered ring comprising carbon atoms and 0-3 heteroatoms selected from O, S and N), which ring may be unsubstituted or further substituted with a substituent independently selected from alkyl, substituted alkyl, alkoxy, substituted alkoxy, aryl, substituted aryl, heteroaryl, substituted heteroaryl, alkenyl, substituted alkenyl, alkynyl, substituted alkynyl, and a water solubilizing group (WSG).
109. The tandem dye of clause 108, wherein the A ring is an aryl ring.
110. The tandem dye of clause 108 or 109, wherein the donor chromophore is of any one of the following compounds: 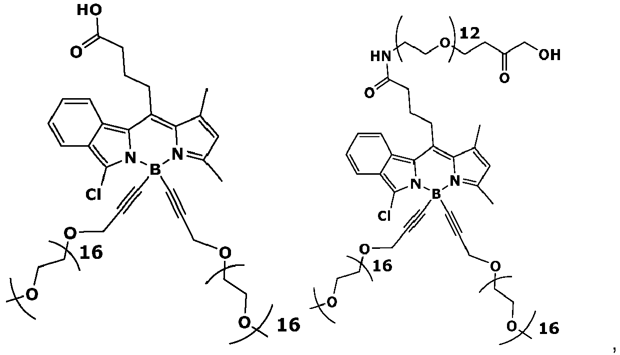 and 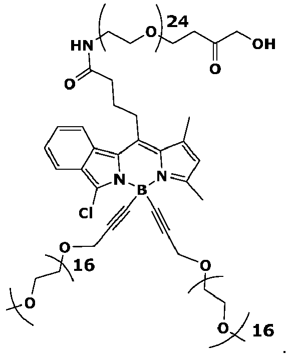
111. The tandem dye of any one of clauses 91 to 99, wherein the donor chromophore is of the formula (IId): 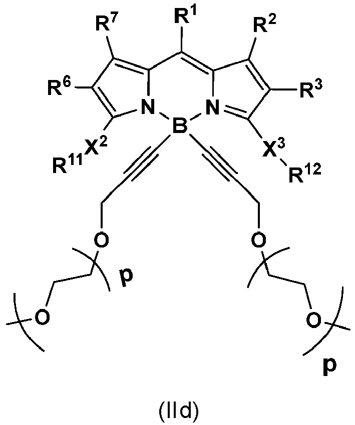 wherein:
   X² and X³ are each independently selected from NR¹³, S and O;
   R¹³ is selected from H, alkyl, and substituted alkyl;
   R¹¹ and R¹² are each independently selected from aryl, substituted aryl, heteroaryl, substituted heteroaryl, and water soluble group (WSG), or
   when X² is NR¹³, optionally R¹³ and R¹¹ together with the nitrogen atom to which they are attached, form a heterocyclic or spiro heterocyclic ring, which ring may be unsubstituted or further substituted with a substituent independently selected from alkyl, substituted alkyl, alkoxy, substituted alkoxy, aryl, substituted aryl, heteroaryl, substituted heteroaryl, alkenyl, substituted alkenyl, alkynyl, substituted alkynyl, and a water solubilizing group (WSG), and
   when X³ is NR¹³, optionally R¹³ and R¹² together with the nitrogen atom to which they are attached, form a heterocyclic or spiro heterocyclic ring, which ring may be unsubstituted or further substituted with a substituent independently selected from alkyl, substituted alkyl, alkoxy, substituted alkoxy, aryl, substituted aryl, heteroaryl, substituted heteroaryl, alkenyl, substituted alkenyl, alkynyl, substituted alkynyl, and water solubilizing group (WSG).
112. The tandem dye of clause 111, wherein X² and X³ are each NR¹³, wherein R¹³ is H.
113. The tandem dye of clause 111, wherein X² is NR¹³, and R¹³ and R¹¹ together with the nitrogen atom to which they are attached form a heterocyclic or a spiro heterocyclic ring; and X³ is NR¹³, and R¹³ and R¹² together with the nitrogen atom to which they are attached form a heterocyclic or a spiro heterocyclic ring.
114. The polymeric tandem dye of clause 111, wherein X² and X³ are both S.
115. The tandem dye of any one of clauses 111 to 114, wherein the donor chromophore is of any one of the following structures: 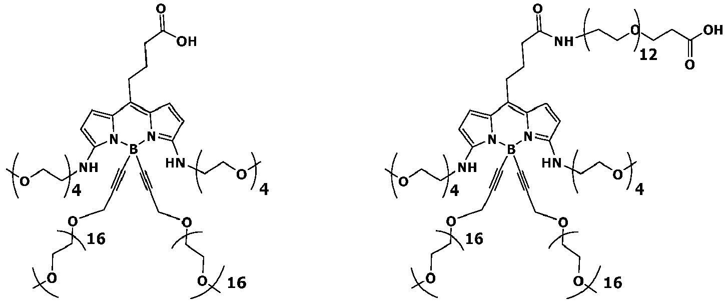 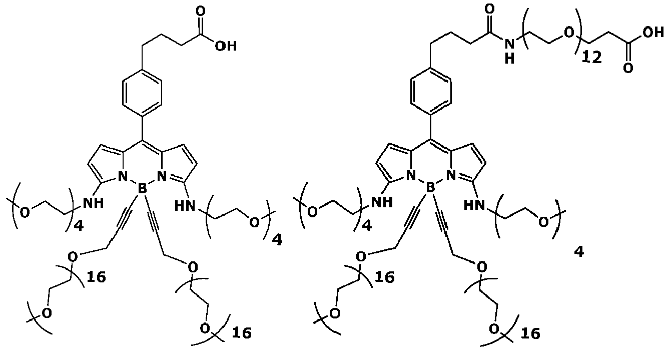 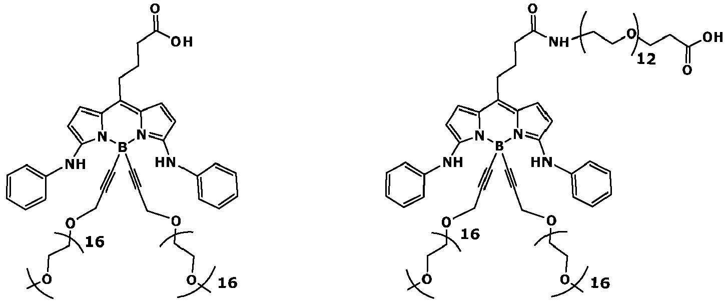 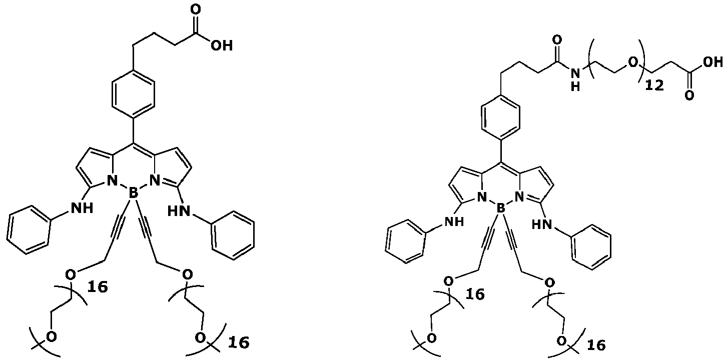 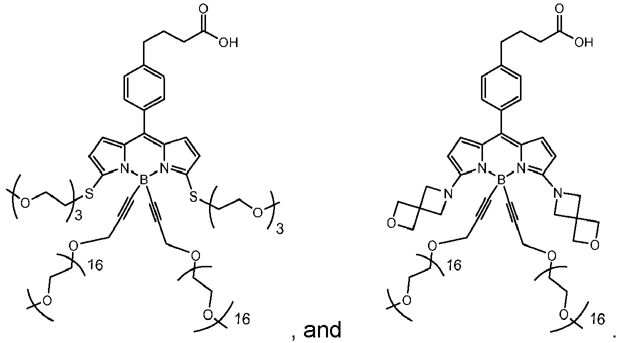
116. The tandem dye of any one of clauses 91 to 95, wherein the donor chromophore is of the formula (III): 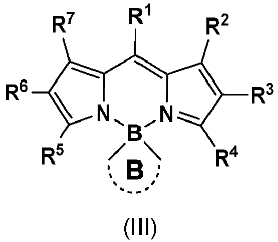 wherein:
   ring B is a 5- or 6-membered cyclic group (e.g., a 5- or 6-membered ring comprising a boron atom, carbon atoms and 0-3 heteroatoms selected from O, S and N), which cyclic group may be unsubstituted or further substituted with one or more substituents independently selected from alkyl, substituted alkyl, alkoxy, substituted alkoxy, aryl, substituted aryl, heteroaryl, substituted heteroaryl, alkenyl, substituted alkenyl, alkynyl, substituted alkynyl, and water solubilizing group (WSG).
117. The tandem dye of clause 116, wherein the donor chromophore is of the formula (IIIa): 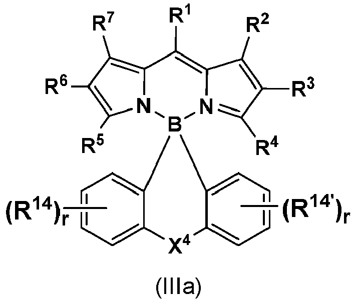 wherein:
   X⁴ is selected from O, S, NR, and CR₂;
   each R is independently selected from H, alkyl, and substituted alkyl;
   each r is independently an integer from 0 to 4; and
   each R¹⁴ and R^{14'}, if present, is independently selected from halogen, hydroxyl, cyano, amino, substituted amino, thiol, substituted thiol, amido, substituted amido, acyl, substituted acyl, alkyl, substituted alkyl, aryl, substituted aryl, heteroaryl, substituted heteroaryl, alkoxy, substituted alkoxy, alkenyl, substituted alkenyl, alkynyl, substituted alkynyl, and water solubilizing group (WSG).
118. The tandem dye of clause 116 or 117, wherein at least one of R⁵ and R⁶ and R⁴ and R³ together form a divalent radical and are cyclically linked and together with the carbon atoms to which they are bound provide a 5- or 6-membered fused heterocycle, carbocycle, aryl or heteroaryl ring (e.g., a 5- or 6-membered ring comprising carbon atoms and 0-3 heteroatoms selected from O, S and N), which ring may be unsubstituted or further substituted with a substituent independently selected from alkyl, substituted alkyl, alkoxy, substituted alkoxy, aryl, substituted aryl, heteroaryl, substituted heteroaryl, alkenyl, substituted alkenyl, alkynyl, substituted alkynyl, and water solubilizing group (WSG).
119. The tandem dye of clause 118, wherein the donor chromophore is of the formula (IIIb) or (IIIc): 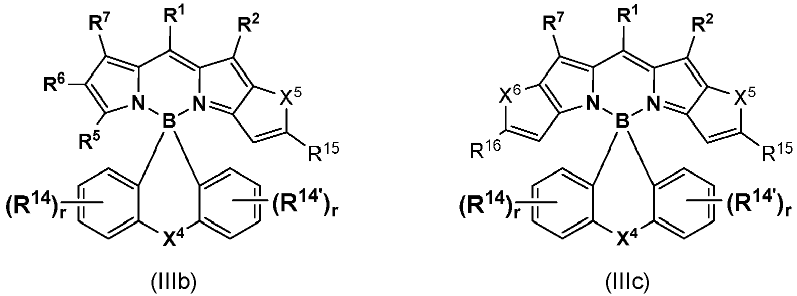 wherein:
   X⁵ and X⁶ are each independently selected from O, S, NR, and CR₂;
   each R is independently selected from H, alkyl, and substituted alkyl; and
   R¹⁵ and R¹⁶, are each independently selected from hydrogen, halogen, hydroxyl, cyano, amino, substituted amino, thiol, substituted thiol, amido, substituted amido, acyl, substituted acyl, alkyl, substituted alkyl, aryl, substituted aryl, heteroaryl, substituted heteroaryl, alkoxy, substituted alkoxy, alkenyl, substituted alkenyl, alkynyl, substituted alkynyl, and water solubilizing group (WSG).
120. The tandem dye of clause 119, wherein X⁵ and X⁶ are O.
121. The tandem dye of any one of clauses 116 to 120, wherein the donor chromophore is of any one of the following structures: 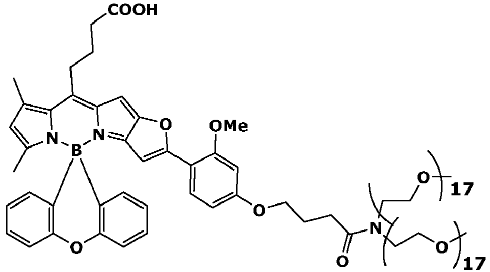 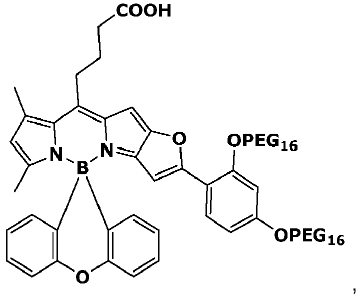 and 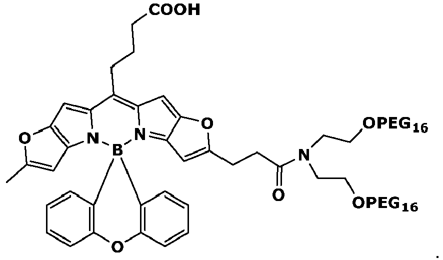
122. The tandem dye of any one of clauses 91 to 97, wherein R³ is -L¹-Z¹.
123. The tandem dye of clause 122, wherein the donor chromophore is of one of the following structures: 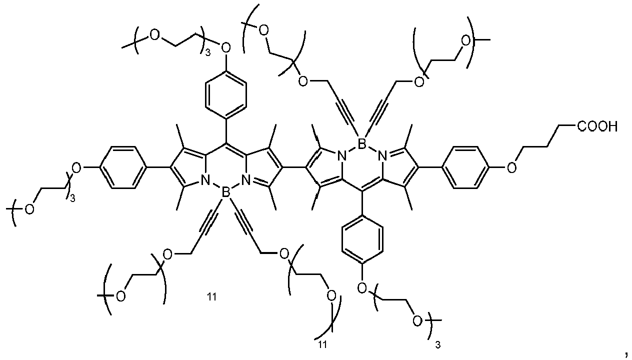 and 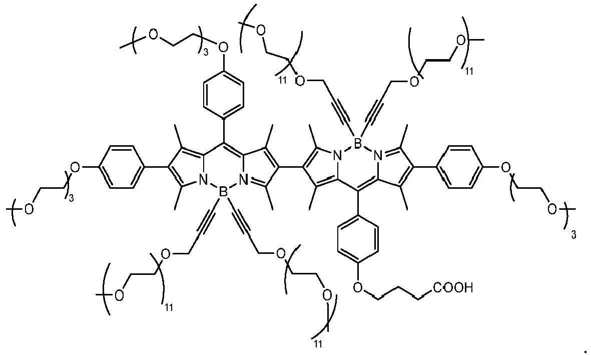
124. The tandem dye of any one of clauses 86 to 89, of formula (IV): 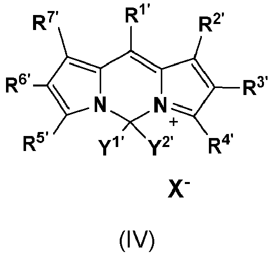 wherein:
   X⁻ is a counterion;
   R^{1'}-R^{7'} are each independently selected from H, alkyl, halogen, substituted alkyl, alkoxy, substituted alkoxy, amino, substituted amino, amido, substituted amido, thiol, substituted thiol, aryl, substituted aryl, heteroaryl, substituted heteroaryl, heterocycle, substituted heterocycle, alkenyl, substituted alkenyl, alkynyl, substituted alkynyl, water solubilizing group (WSG), and -T¹-Z¹, or
   optionally any one or more pairs of substituents selected from R^{6'} and R^{7'}, R^{2'} and R^{3'}, R^{5'} and R^{6'}, R^{3'} and R^{4'}, R^{2'} and R^{1'} and R^{7'} and R^{1'}, together form a divalent radical and are cyclically linked and together with the carbon atoms to which they are bound provide a 5- or 6-membered fused heterocycle, carbocycle, aryl or heteroaryl ring (e.g., a 5- or 6-membered ring comprising carbon atoms and 0-3 heteroatoms selected from O, S and N), which ring may be unsubstituted or further substituted with a substituent independently selected from alkyl, substituted alkyl, alkoxy, substituted alkoxy, aryl, substituted aryl, heteroaryl, substituted heteroaryl, alkenyl, substituted alkenyl, alkynyl, substituted alkynyl, water solubilizing group (WSG), and -T¹-Z¹;
   T¹ is an optional linker;
   Z¹ is a repeat unit of a compound of formula (I); and
   Y^{1'} and Y^{2'} are independently selected from F, OH, H, cyano, alkyl, substituted alkyl, aryl, substituted aryl, heteroaryl, substituted heteroaryl, alkoxy, substituted alkoxy, alkenyl, substituted alkenyl, alkynyl, substituted alkynyl, and WSG, or
   Y^{1'} and Y^{2'} together with the boron atom to which they are bound form a 5- or 6-membered ring (e.g., a 5- or 6-membered ring comprising a boron atom, carbon atoms and 0-3 heteroatoms selected from O, S and N), which ring may be unsubstituted or further substituted with a substituent independently selected from alkyl, substituted alkyl, alkoxy, substituted alkoxy, aryl, substituted aryl, heteroaryl, substituted heteroaryl, alkenyl, substituted alkenyl, alkynyl, substituted alkynyl, water solubilizing group (WSG), and -T¹-Z¹; and
   wherein at least one moiety selected from Y^{1'}, Y^{2'} and R^{1'}-R^{7'} is a WSG, or at least one moiety selected from Y^{1'}, Y^{2'} and R^{1'}-R^{7'} is substituted with a WSG, where the WSG is of the formula (Ia):

      -(L¹)ₙ₁ - (X¹)ₙ₂ - ((L²)ₙ₃-Z¹)ₘ (Ia)

      wherein:
      n1, n2 and n3 are each independently 0 or 1;
      L¹, if present, is a linker;
      L² if present, is a linker;
      X¹, if present, is a branching point;
      Z¹ is a water soluble polymer comprising 6-24 monomeric units; and
      m is an integer from 1 to 3.
125. The tandem dye of clause 124, further comprising a WSG of formula (Ib):

   -(L¹)ₙ₁ - (X¹)ₙ₂ - ((L²)ₙ₃-Z²)ₘ (Ib)

   wherein:
   n1, n2 and n3 are each independently 0 or 1;
   L¹, if present, is a linker;
   L² if present, is a linker;
   X¹, if present, is a branching point;
   Z² is a water soluble polymer comprising 1-50 monomeric units; and
   m is an integer from 1 to 3.
126. The tandem dye of clause 124 or 125, wherein the WSG comprises a PEG moiety.
127. The tandem dye of any one of clauses 124 to 126, wherein one or more water solubilizing groups (WSGs) is independently selected from the following formulae: 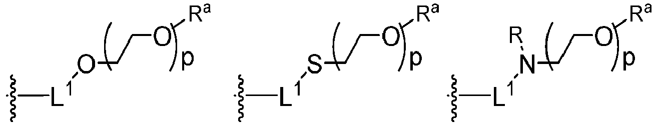 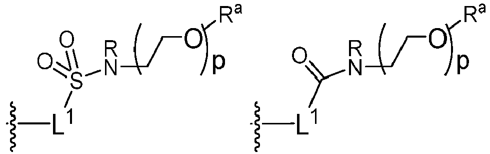 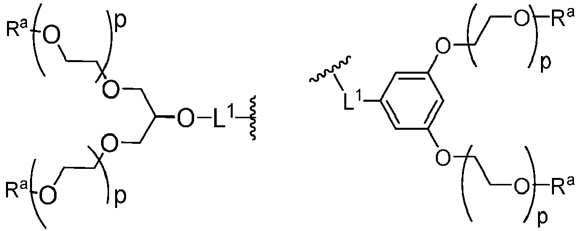 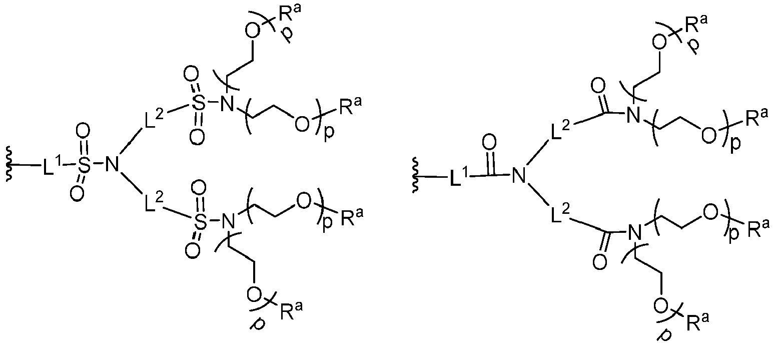 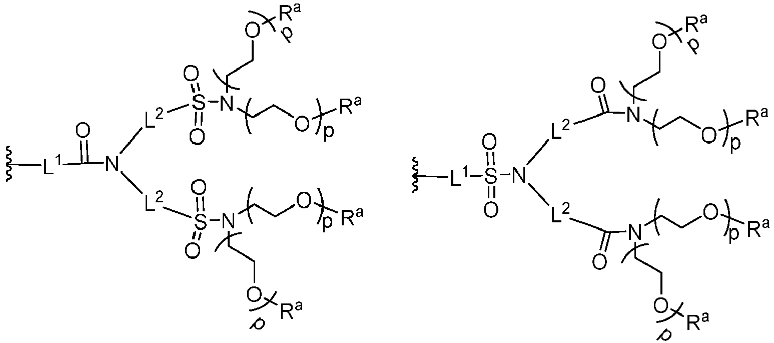 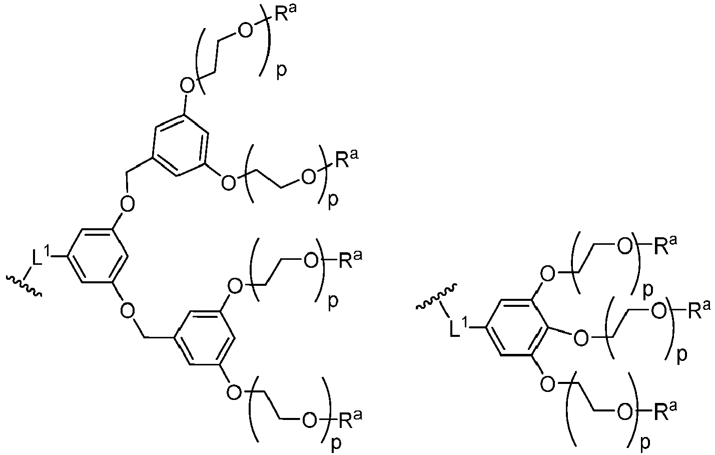 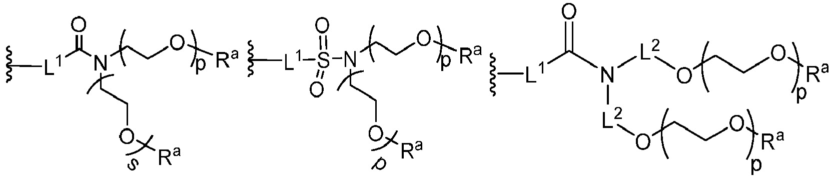 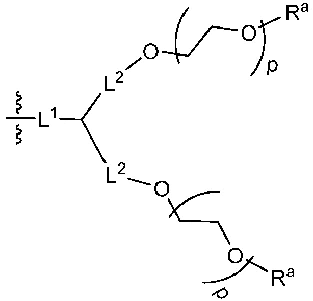 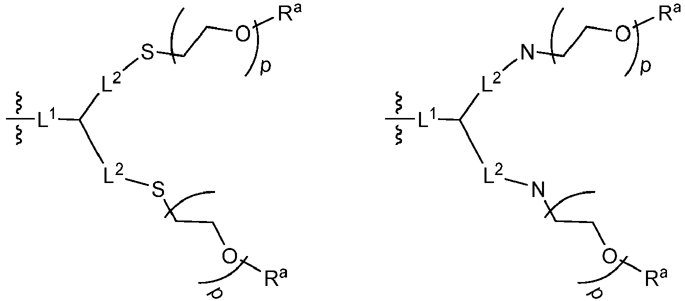 wherein:
   L¹ and L² are each independently an optional linker;
   R¹¹ and R are each independently H, alkyl or substituted alkyl; and each p is an integer from 6 to 24.
128. The tandem dye of any one of clauses 124 to 127, wherein R¹ is selected from substituted alkyl, and substituted aryl, wherein the substituent comprises a chemoselective tag.
129. The tandem dye of any one of clauses 124 to 128, wherein R^{5'} and R^{4'} both comprise a water solubilizing group (WSG).
130. The tandem dye of any one of clauses 124 to 129, of one of the following structures: 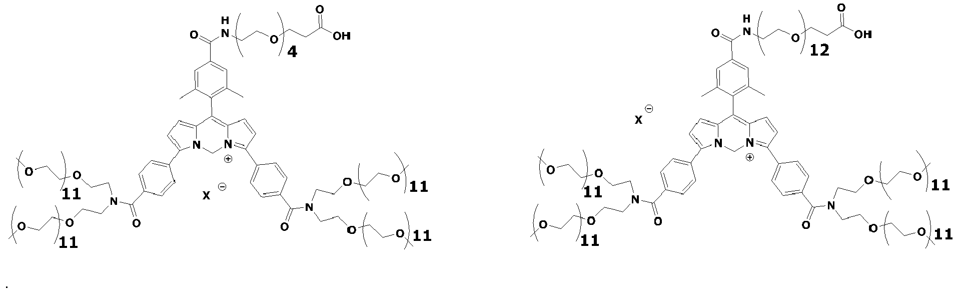
131. The tandem dye according to any one of clauses 86 to 130, wherein the backbone is a linear polymer.
132. The tandem dye according to clause 131, wherein the linear polymer is selected from a peptide, a peptoid, a hydrocarbon polymer, and a PEG polymer.
133. The tandem dye according to clause 132, wherein the linear polymer is a peptide.
134. The tandem dye according to clause 133, wherein the peptide is from 2 to 100 amino acids in length.
135. The tandem dye according to clause 134, wherein the peptide is from 5 to 30 amino acids in length.
136. The tandem dye according to any one of clauses 131 to 133, wherein the linear polymer comprises 2 to 1,000 repeating monomeric units.
137. The tandem dye according to clause 136, wherein the linear polymer comprises 2 to 500 repeating monomeric units.
138. The tandem dye according to clause 137, wherein the linear polymer comprises 2 to 100 repeating monomeric units.
139. The tandem dye according to any one of clauses 86 to 138, wherein a plurality of pendant donor chromophore groups are present, and are configured in energy-transferring proximity to the pendant acceptor fluorophore.
140. The tandem dye according to any one of clauses 86 to 139, wherein the pendant acceptor fluorophore is a small molecule fluorophore.
141. The tandem dye according to any one of clauses 86 to 140, wherein the pendant acceptor fluorophore is selected from the group consisting of a cyanine dye, a rhodamine dye, a perylene diimide dye, a xanthene dye, a coumarin dye, a polymethine, a pyrene, a dipyrromethene borondifluoride, a napthalimide, a thiazine dye and an acridine dye.
142. The tandem dye according to any one of clauses 86 to 141, wherein the pendant acceptor fluorophore is selected from fluorescein, 6-FAM, rhodamine, Texas Red, California Red, iFluor594, tetramethylrhodamine, a carboxyrhodamine, carboxyrhodamine 6G, carboxyrhodol, carboxyrhodamine 110, Cascade Blue, Cascade Yellow, coumarin, Cy2^{®}, Cy3^{®}, Cy3.5^{®}, Cy5^{®}, Cy5.5^{®}, Cy7^{®}, Cy-Chrome, DyLight 350, DyLight 405, DyLight 488, DyLight 549, DyLight 594, DyLight 633, DyLight 649, DyLight 680, DyLight 750, DyLight 800, phycoerythrin, PerCP (peridinin chlorophyll-a Protein), PerCP-Cy5.5, JOE (6-carboxy-4',5'-dichloro-2',7'-dimelhoxyfluorescein), NED, ROX (5-(and -6)-carboxy-X-rhodamine), HEX, Lucifer Yellow, Marina Blue, Oregon Green 488, Oregon Green 500, Oregon Green 514, Alexa Fluor^{®} 350, Alexa Fluor^{®} 430, Alexa Fluor^{®} 488, Alexa Fluor^{®} 532, Alexa Fluor^{®} 546, Alexa Fluor^{®} 568, Alexa Fluor^{®} 594, Alexa Fluor^{®} 633, Alexa Fluor^{®} 647, Alexa Fluor^{®} 660, Alexa Fluor^{®} 680, 7-amino-4-methylcoumarin-3-acetic acid, BODIPY^{®} FL, BODIPY^{®} FL-Br2, BODIPY^{®} 530/550, BODIPY^{®} 558/568, BODIPY^{®} 564/570, BODIPY^{®} 576/589, BODIPY^{®} 581/591, BODIPY^{®} 630/650, BODIPY^{®} 650/665, BODIPY^{®} R6G, BODIPY^{®} TMR, BODIPY^{®} TR, conjugates thereof and combinations thereof.
143. The tandem dye according to any one of clauses 86 to 142, wherein the ratio of donor chromophores to acceptor fluorophores is selected from 5:1, 6:1, 7:1, 8:1, 9:1, 10:1, and 20:2.
144. The tandem dye according to any one of clauses 86 to 143, wherein the light harvesting chromophore further comprises a specific binding member.
145. The tandem dye according to clause 144, wherein the specific binding member is linked to a terminal group of the light harvesting chromophore.
146. The tandem dye according to clause 144, wherein the specific binding member is linked to the backbone, at any point between the terminal groups of the light harvesting chromophore.
147. The tandem dye according to clause 146, wherein the specific binding member is linked to the backbone at a central point of the light harvesting chromophore.
148. The tandem dye according to any one of clauses 144 to 147, wherein the specific binding member is selected from an antibody, an antibody fragment or a binding derivative thereof.
149. The tandem dye according to any one of clauses 86 to 148, wherein the light harvesting chromophore is of formula (VA) or (VB): 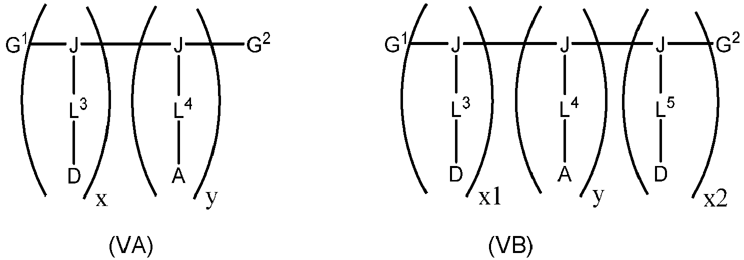 wherein:
   each J are repeat units of the backbone;
   L³-L⁵ are each independently a linker;
   each D is a donor chromophore;
   each A is an acceptor fluorophore;
   G¹ and G² are each independently selected from the group consisting of terminal group, a polymer segment, a donor chromophore, an acceptor fluorophore, a linker, and a linked specific binding member;
   x is 75 mol% or more;
   x1 + X2 is 75% or more; and
   y is 25 mol% or less.
150. The tandem dye according to clause 149, wherein the light harvesting chromophore is of formula (VA), and x is 80 mol% or more; or
   the light harvesting chromophore is of formula (VB), and x1 + X2 is 80 mol% or more.
151. The tandem dye according to clause 150, wherein the light harvesting chromophore is of formula (VA), and x is 90 mol% or more; or
   the light harvesting chromophore is of formula (VB), and x1 + X2 is 90 mol% or more.
152. The tandem dye according to clause 149, wherein the light harvesting chromophore is of formula (VA), and y is 20 mol% or less; or
   the light harvesting chromophore is of formula (VB), and y is 20 mol% or less.
153. The tandem dye according to clause 152, wherein the light harvesting chromophore is of formula (VA), and y is 10 mol% or less; or
   the light harvesting chromophore is of formula (VB), and y is 10 mol% or less.
154. The tandem dye according to any one of clauses 149 to 153, wherein G1 or G2 is a linked specific binding member.
155. The tandem dye according to any one of clauses 149 to 153, wherein any of J, L³-L⁵, D or A comprise a linked specific binding member.
156. The tandem dye according to any one of clauses 149 to 155, wherein each J is an amino acid or a peptide unit.
157. A method of evaluating a sample for the presence of a target analyte, the method comprising:
   (a) contacting the sample with an aggregation-resistant dye conjugate that specifically binds the target analyte to produce a labelling composition contacted sample, wherein the dye conjugate comprises:
      (i) a tandem dye according to any one of clauses 86 to 156; and
      (ii) a specific binding member linked to the tandem dye; and
   (b) assaying the labelling composition contacted sample for the presence of a dye conjugate-target analyte binding complex to evaluate whether the target analyte is present in the sample.
158. The method according to clause 157, further comprising contacting the sample with a second specific binding member that is support bound and specifically binds the target analyte.
159. The method according to clause 158, wherein the support comprises a magnetic particle.
160. The method according to any one of clauses 157 to 159, wherein the target analyte is associated with a cell.
161. The method according to clause 160, wherein the target analyte is a cell surface marker of the cell.
162. The method according to clause 161, wherein the cell surface marker is selected from the group consisting of a cell receptor and a cell surface antigen.
163. The method according to clause 160, wherein the target analyte is an intracellular target, and the method further comprises lysing the cell.
164. The method according to any one of clauses 157 to 163, wherein the method further comprises flow cytometrically analyzing the fluorescently labelled target analyte.
165. A method of labelling a target molecule, the method comprising:
   contacting the target molecule with a tandem dye to produce a labelled target molecule, wherein:
   the tandem dye is a dye according to any one of clauses 86-156, and comprises a conjugation tag that covalently links to the target molecule.
166. The method according to clause 165, further comprising fluorescently detecting the labelled target molecule.
167. The method according to clause 165 or 166, wherein the conjugation tag comprises a terminal functional group selected from an amino, a thiol, a hydroxyl, a hydrazine, a hydrazide, a azide, an alkyne, maleimide, iodoacetyl, amine, an active ester and a protein reactive group.
168. The method according to any one of clauses 165 to 167, wherein the target molecule is a specific binding member.
169. The method according to clause 168, wherein the specific binding member is an antibody.
170. The method according to clause 168, wherein the specific binding member is an antibody fragment or binding derivative thereof.
171. The method according to clause 170, wherein the antibody fragment or binding derivative thereof is selected from the group consisting of a Fab fragment, a F(ab')₂ fragment, a scFv, a diabody and a triabody.
172. A kit comprising:
   a water-soluble dipyrromethene-based dye of any one of clauses 1 to 45, or a tandem dye according to any one of clauses 86 to 156; and
   a container.
173. The kit according to clause 172, further comprising one or more components selected from the group consisting of a fluorophore, a specific binding member, a specific binding member conjugate, a cell, a support, a biocompatible aqueous elution buffer and instructions for use.
174. The kit according to clause 172 or 173, wherein the tandem dye is covalently linked to a specific binding member.
175. The kit according to clause 174, wherein the specific binding member is an antibody.
176. The kit according to clause 174, wherein the specific binding member is an antibody fragment or binding derivative thereof.
177. The kit according to clause 176, wherein the antibody fragment or binding derivative thereof is selected from the group consisting of a Fab fragment, a F(ab')₂ fragment, a scFv, a diabody and a triabody.

## Claims

1. A water-soluble dipyrromethene-based dye that absorbs in the yellow-green range at 561 nm, wherein the dye has a structure according to formula (I): 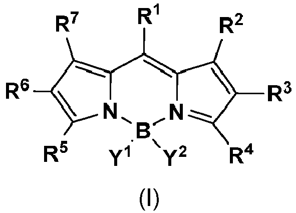 wherein:
R¹-R⁷ are each independently selected from H, alkyl, halogen, substituted alkyl, alkoxy, substituted alkoxy, amino, substituted amino, amido, substituted amido, thiol, substituted thiol, aryl, substituted aryl, heteroaryl, substituted heteroaryl, heterocycle, substituted heterocycle, alkenyl, substituted alkenyl, alkynyl, substituted alkynyl, water solubilizing group (WSG), and -T¹-J¹, or
optionally any one or more pairs of substituents selected from R⁶ and R⁷, R² and R³, R⁵ and R⁶, R³ and R⁴, R² and R¹ and R⁷ and R¹, together form a divalent radical and are cyclically linked and together with the carbon atoms to which they are bound provide a 5- or 6-membered fused heterocycle, carbocycle, aryl or heteroaryl ring (e.g., a 5- or 6-membered ring comprising carbon atoms and 0-3 heteroatoms selected from O, S and N), which ring may be unsubstituted or further substituted with a substituent independently selected from alkyl, substituted alkyl, alkoxy, substituted alkoxy, aryl, substituted aryl, heteroaryl, substituted heteroaryl, alkenyl, substituted alkenyl, alkynyl, substituted alkynyl, water solubilizing group (WSG), and -T¹-J¹;
T¹ is an optional linker;
J¹ is a repeat unit of a compound of formula (I); and
Y¹ and Y² are independently selected from F, OH, H, cyano, alkyl, substituted alkyl, aryl, substituted aryl, heteroaryl, substituted heteroaryl, alkoxy, substituted alkoxy, alkenyl, substituted alkenyl, alkynyl, substituted alkynyl, and water solubilizing group (WSG), or
Y¹ and Y² together with the boron atom to which they are bound form a 5- or 6-membered ring (e.g., a 5- or 6-membered ring comprising a boron atom, carbon atoms and 0-3 heteroatoms selected from O, S and N), which ring may be unsubstituted or further substituted with a substituent independently selected from alkyl, substituted alkyl, alkoxy, substituted alkoxy, aryl, substituted aryl, heteroaryl, substituted heteroaryl, alkenyl, substituted alkenyl, alkynyl, substituted alkynyl, water solubilizing group (WSG), and -T¹-J¹; and
wherein at least one moiety selected from Y¹, Y² and R¹-R⁷ is a WSG, or at least one moiety selected from Y¹, Y² and R¹-R⁷ is substituted with a WSG, where the WSG is of the formula (Ia):
-(L¹)ₙ₁ - (X¹)ₙ₂ - ((L²)ₙ₃-Z¹)ₘ (Ia)
wherein:
n1, n2 and n3 are each independently 0 or 1;
L¹, if present, is a linker;
L² if present, is a linker;
X¹, if present, is a branching point;
Z¹ is a water soluble polymer comprising 6-24 monomeric units; and
m is an integer from 1 to 3.

2. The water-soluble dipyrromethene-based dye according to claim 1, having the formula (II): 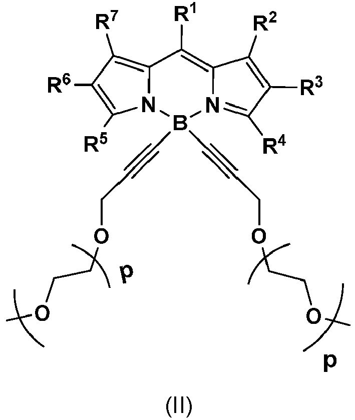 wherein p is an integer from 6-24.

3. The water-soluble dipyrromethene-based dye according to claim 2, having the formula (IIb): 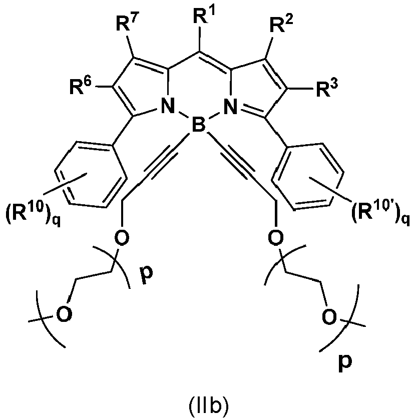 wherein:
each q is independently an integer from 0 to 5; and
R¹⁰ and R^{10'}, if present, are each independently selected from halogen, hydroxyl, cyano, carboxamide, substituted carboxamide amino, substituted amino, thiol, substituted thiol, alkyl, substituted alkyl, aryl, substituted aryl, heteroaryl, substituted heteroaryl, alkoxy, substituted alkoxy, alkenyl, substituted alkenyl, alkynyl, substituted alkynyl, and water soluble group (WSG), or
at least one R¹⁰ group is at an ortho position, and R¹⁰ and R⁶ together with the atoms to which they are bound form a 5- or 6-membered ring (e.g., a 5- or 6-membered ring comprising a boron atom, carbon atoms and 0-3 heteroatoms selected from O, S and N), which ring may be unsubstituted or further substituted with a substituent independently selected from alkyl, substituted alkyl, alkoxy, substituted alkoxy, aryl, substituted aryl, heteroaryl, substituted heteroaryl, alkenyl, substituted alkenyl, alkynyl, substituted alkynyl, and water solubilizing group (WSG); and
at least one R^{10'} is an ortho position, and R¹⁰ and R³ together with the atoms to which they are bound form a 5- or 6-membered ring (e.g., a 5- or 6-membered ring comprising a boron atom, carbon atoms and 0-3 heteroatoms selected from O, S and N), which ring may be unsubstituted or further substituted with a substituent independently selected from alkyl, substituted alkyl, alkoxy, substituted alkoxy, aryl, substituted aryl, heteroaryl, substituted heteroaryl, alkenyl, substituted alkenyl, alkynyl, substituted alkynyl, and water solubilizing group (WSG).

4. The water-soluble dipyrromethene-based dye according to claim 2, having the formula (IId): 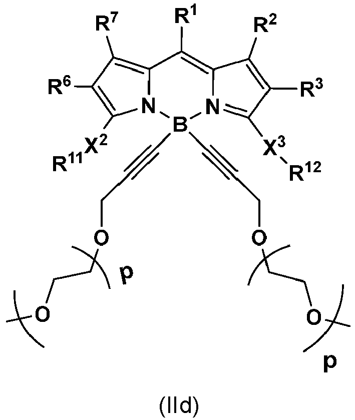 wherein:
X² and X³ are each independently selected from NR¹³, S and O;
R¹³ is selected from H, alkyl, and substituted alkyl;
R¹¹ and R¹² are each independently selected from aryl, substituted aryl, heteroaryl, substituted heteroaryl, and water soluble group (WSG), or
when X² is NR¹³, optionally R¹³ and R¹¹ together with the nitrogen atom to which they are attached, form a heterocyclic or spiro heterocyclic ring, which ring may be unsubstituted or further substituted with a substituent independently selected from alkyl, substituted alkyl, alkoxy, substituted alkoxy, aryl, substituted aryl, heteroaryl, substituted heteroaryl, alkenyl, substituted alkenyl, alkynyl, substituted alkynyl, and water solubilizing group (WSG), and
when X³ is NR¹³, optionally R¹³ and R¹² together with the nitrogen atom to which they are attached, form a heterocyclic or spiro heterocyclic ring, which ring may be unsubstituted or further substituted with a substituent independently selected from alkyl, substituted alkyl, alkoxy, substituted alkoxy, aryl, substituted aryl, heteroaryl, substituted heteroaryl, alkenyl, substituted alkenyl, alkynyl, substituted alkynyl, and water solubilizing group (WSG).

5. A yellow green dipyrromethene-based dye of formula (II): 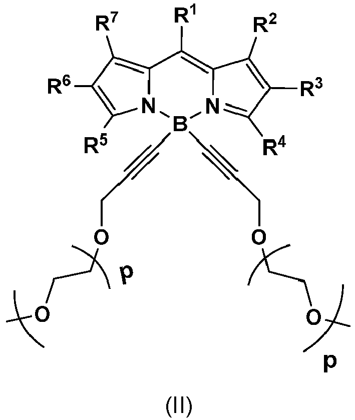 wherein:
R¹-R⁷ are each independently selected from H, alkyl, halogen, substituted alkyl, alkoxy, substituted alkoxy, amino, substituted amino, amido, substituted amido, thiol, substituted thiol, aryl, substituted aryl, heteroaryl, substituted heteroaryl, heterocycle, substituted heterocycle, alkenyl, substituted alkenyl, alkynyl, substituted alkynyl, water solubilizing group (WSG), and -T¹-Z¹, or
optionally any one or more pairs of substituents selected from R⁶ and R⁷, R² and R³, R⁵ and R⁶, R³ and R⁴, R² and R¹ and R⁷ and R¹, together form a divalent radical and are cyclically linked and together with the carbon atoms to which they are bound provide a 5- or 6-membered fused heterocycle, carbocycle, aryl or heteroaryl ring (e.g., a 5- or 6-membered ring comprising carbon atoms and 0-3 heteroatoms selected from O, S and N), which ring may be unsubstituted or further substituted with a substituent independently selected from alkyl, substituted alkyl, alkoxy, substituted alkoxy, aryl, substituted aryl, heteroaryl, substituted heteroaryl, alkenyl, substituted alkenyl, alkynyl, substituted alkynyl, water solubilizing group (WSG), and -T¹-Z¹;
T¹ is an optional linker;
Z¹ is a repeat unit of a compound of formula (I); and
p is an integer from 6-24.

6. The yellow green dipyrromethene-based dye according to claim 5, having the formula (IIb): 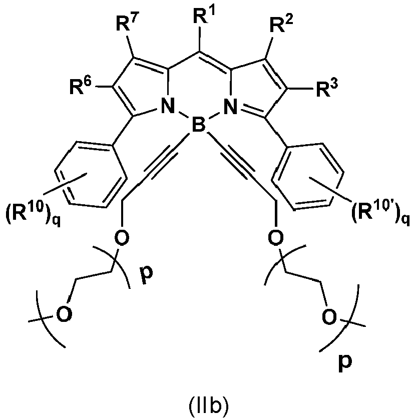 wherein:
each q is independently an integer from 0 to 5; and
R¹⁰ and R^{10'}, if present, are each independently selected from halogen, hydroxyl, cyano, amino, substituted amino, thiol, substituted thiol, alkyl, substituted alkyl, aryl, substituted aryl, heteroaryl, substituted heteroaryl, alkoxy, substituted alkoxy, alkenyl, substituted alkenyl, alkynyl, substituted alkynyl, and water soluble group (WSG), or
at least one R¹⁰ group is at an ortho position, and R¹⁰ and R⁶ together with the atoms to which they are bound form a 5- or 6-membered ring (e.g., a 5- or 6-membered ring comprising a boron atom, carbon atoms and 0-3 heteroatoms selected from O, S and N), which ring may be unsubstituted or further substituted with a substituent independently selected from alkyl, substituted alkyl, alkoxy, substituted alkoxy, aryl, substituted aryl, heteroaryl, substituted heteroaryl, alkenyl, substituted alkenyl, alkynyl, substituted alkynyl, and water solubilizing group (WSG); and
at least one R^{10'} is an ortho position, and R¹⁰ and R³ together with the atoms to which they are bound form a 5- or 6-membered ring (e.g., a 5- or 6-membered ring comprising a boron atom, carbon atoms and 0-3 heteroatoms selected from O, S and N), which ring may be unsubstituted or further substituted with a substituent independently selected from alkyl, substituted alkyl, alkoxy, substituted alkoxy, aryl, substituted aryl, heteroaryl, substituted heteroaryl, alkenyl, substituted alkenyl, alkynyl, substituted alkynyl, and water solubilizing group (WSG).

7. The yellow green dipyrromethene-based dye according to claim 5, having the formula (IId): 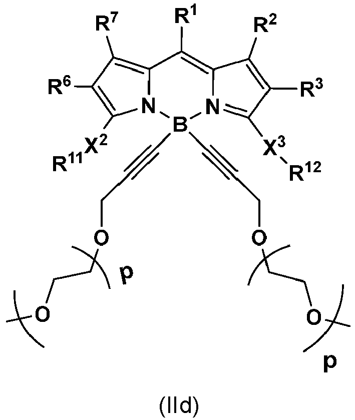 wherein:
X² and X³ are each independently selected from NR¹³, S and O;
R¹³ is selected from H, alkyl, and substituted alkyl;
R¹¹ and R¹² are each independently selected from aryl, substituted aryl, heteroaryl, substituted heteroaryl, and a water soluble group (WSG), or
when X² is NR¹³, optionally R¹³ and R¹¹ together with the nitrogen atom to which they are attached, form a heterocyclic or spiro heterocyclic ring, which ring may be unsubstituted or further substituted with a substituent independently selected from alkyl, substituted alkyl, alkoxy, substituted alkoxy, aryl, substituted aryl, heteroaryl, substituted heteroaryl, alkenyl, substituted alkenyl, alkynyl, substituted alkynyl, and a water solubilizing group (WSG); and
when X³ is NR¹³, optionally R¹³ and R¹² together with the nitrogen atom to which they are attached, form a heterocyclic or spiro heterocyclic ring, which ring may be unsubstituted or further substituted with a substituent independently selected from alkyl, substituted alkyl, alkoxy, substituted alkoxy, aryl, substituted aryl, heteroaryl, substituted heteroaryl, alkenyl, substituted alkenyl, alkynyl, substituted alkynyl, and a water solubilizing group (WSG).

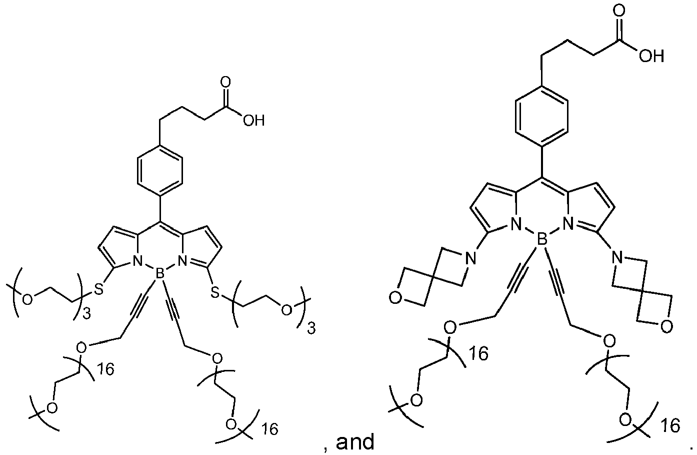

8. The water-soluble dye or yellow green dipyrromethene-based dye according to any one of the above claims, having a structure selected from:

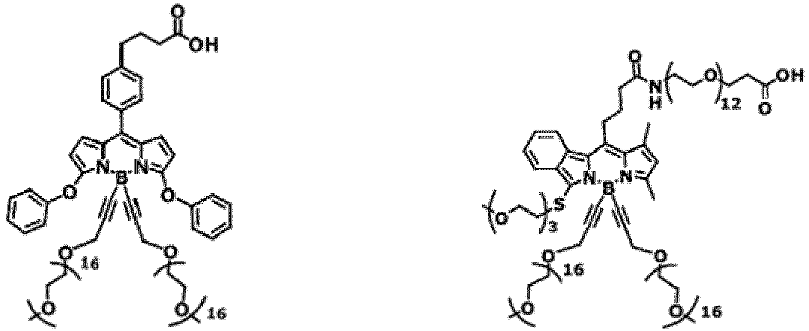

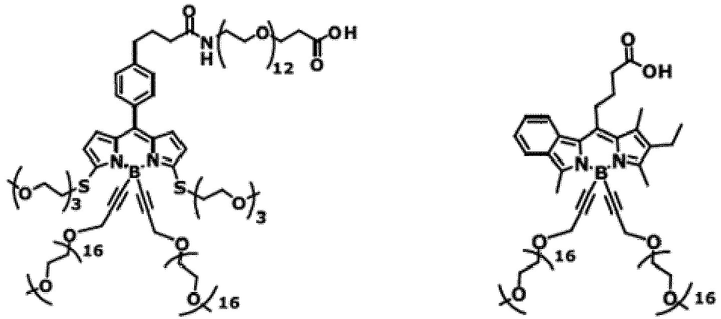

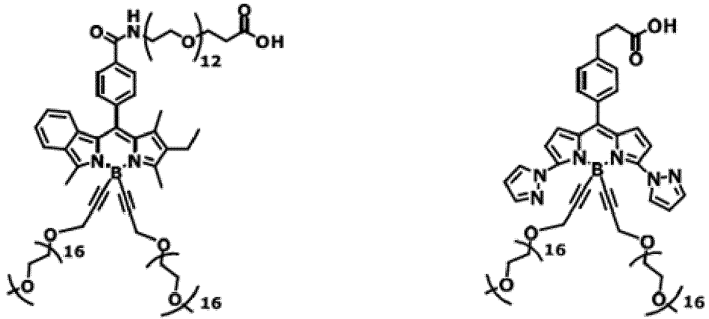

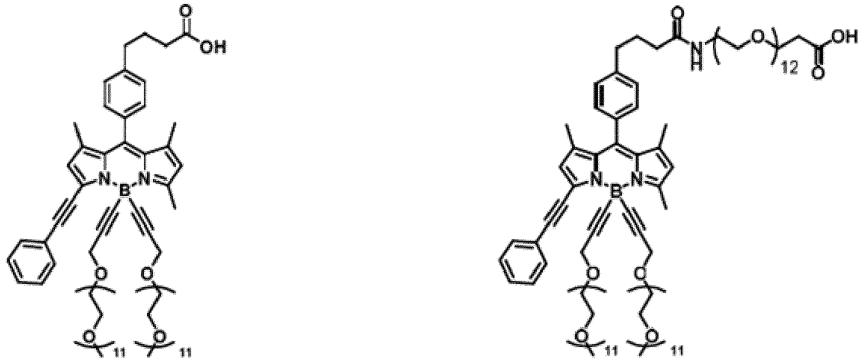

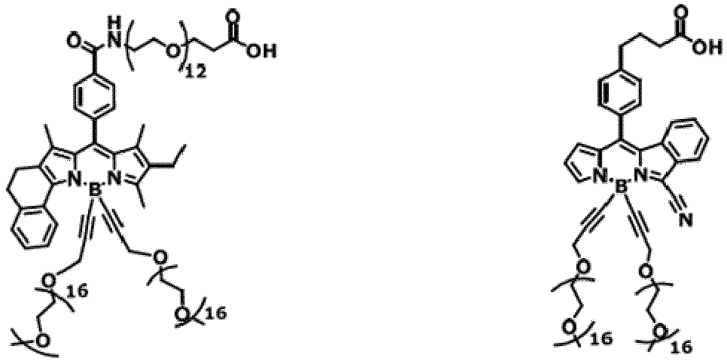

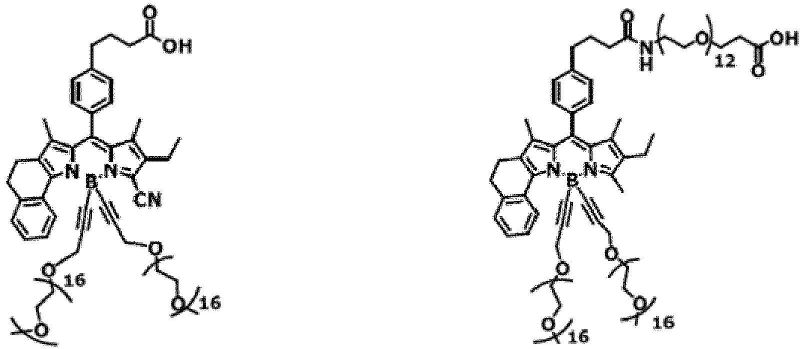

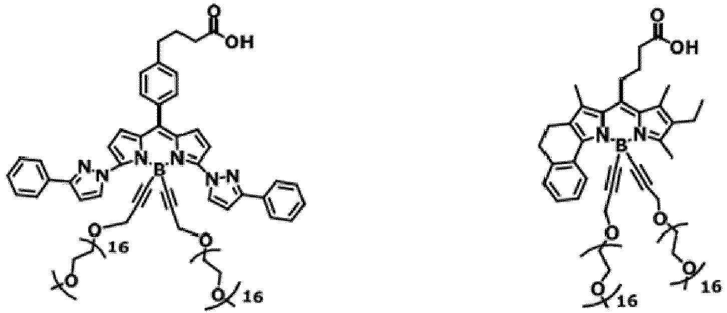

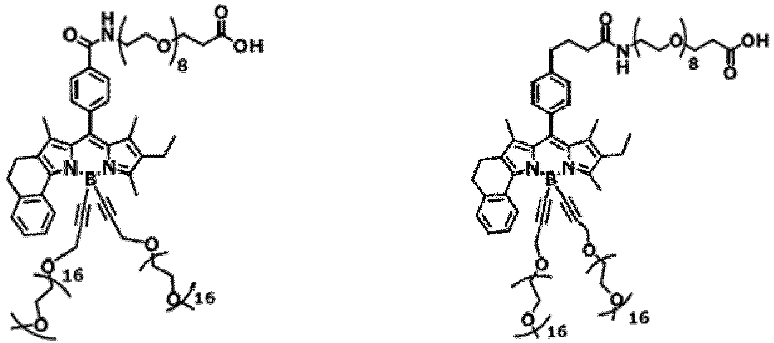

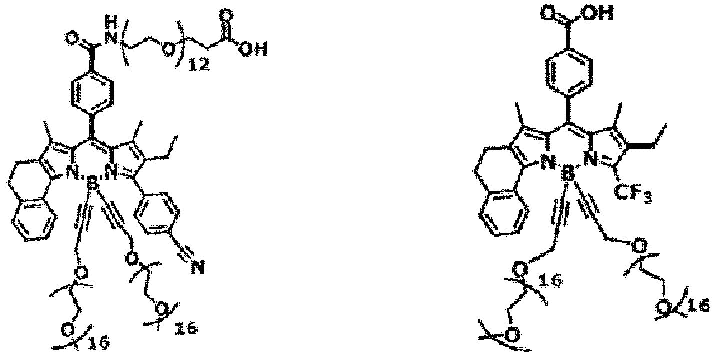

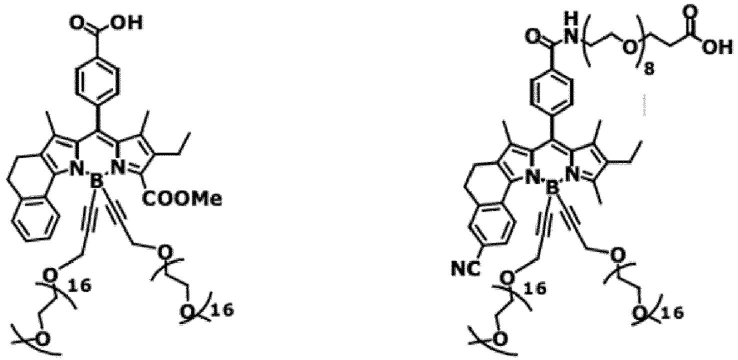

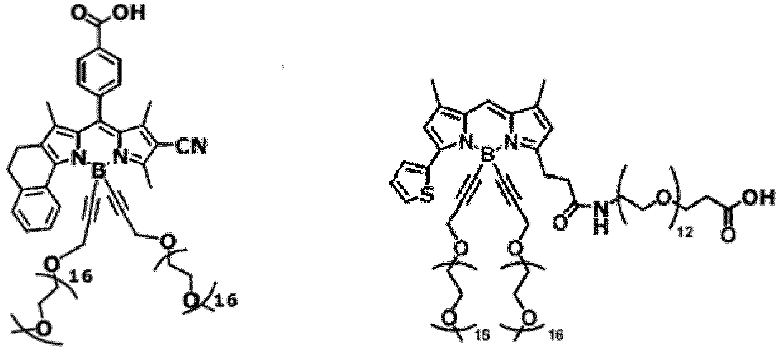

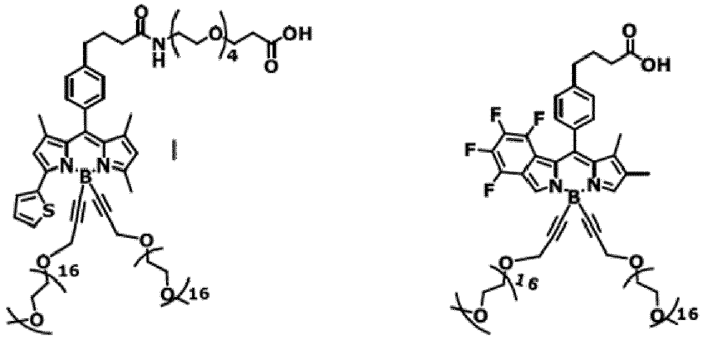

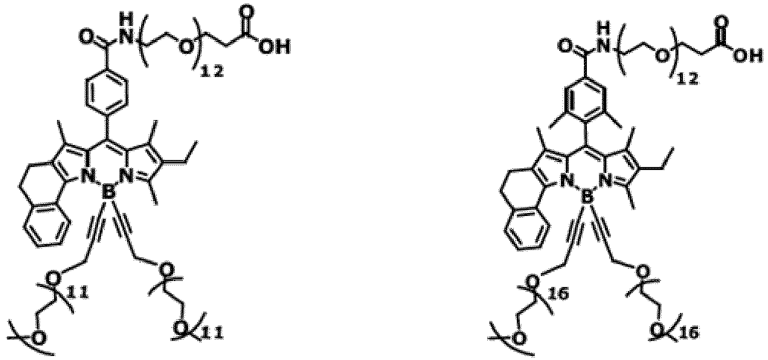

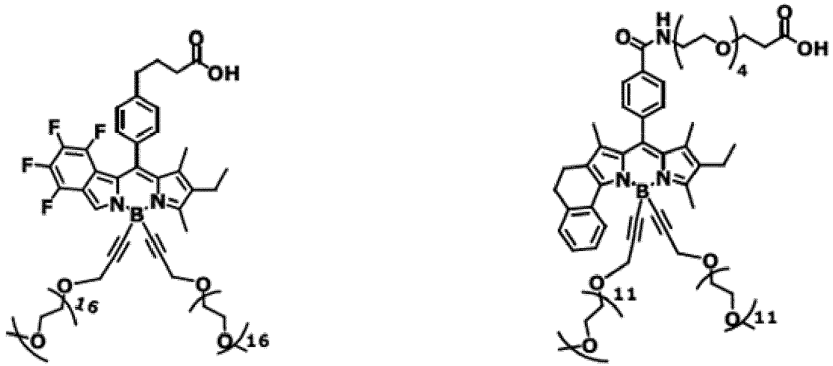

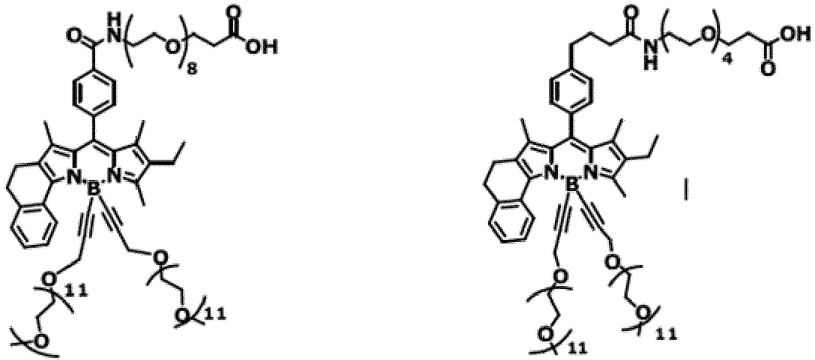

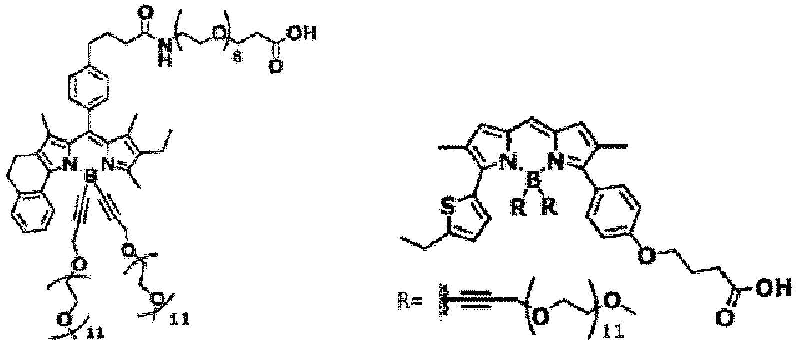

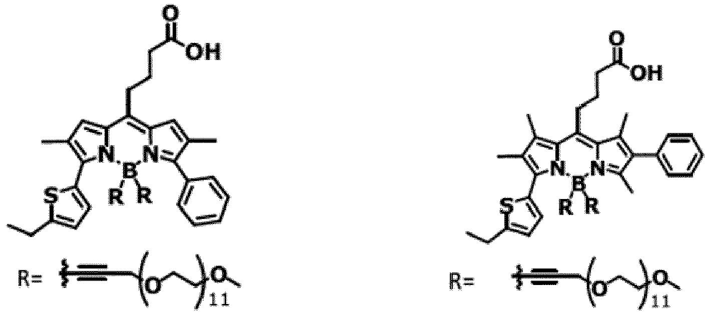

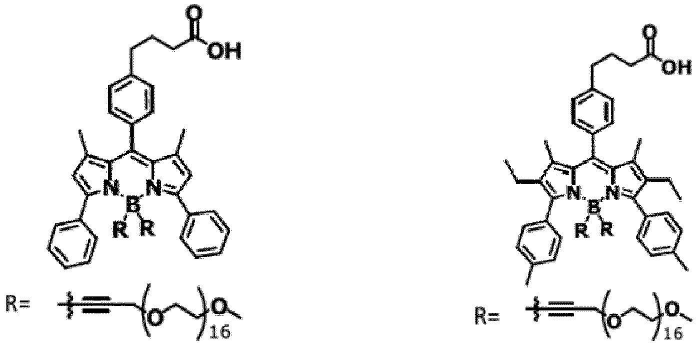

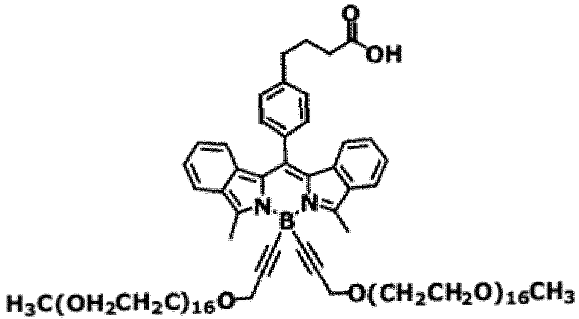

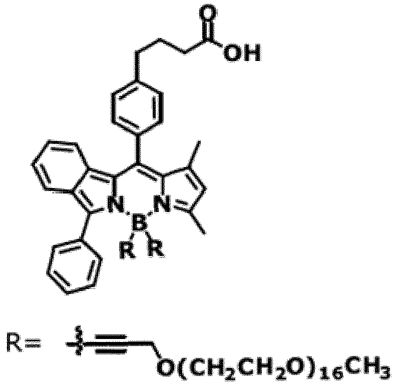

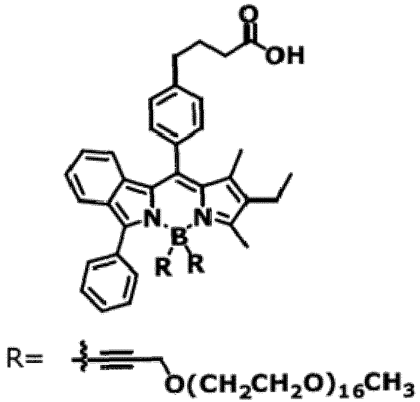

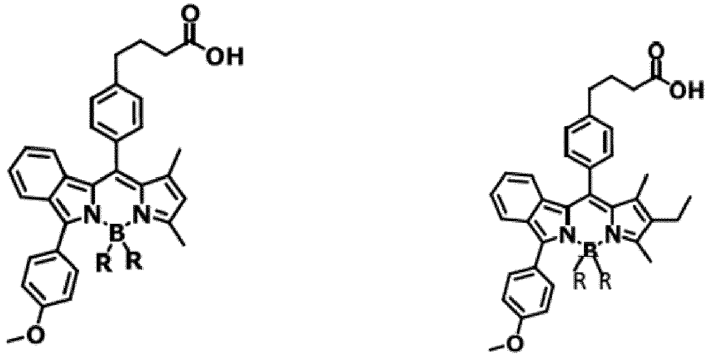

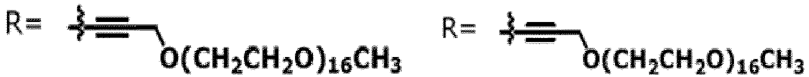

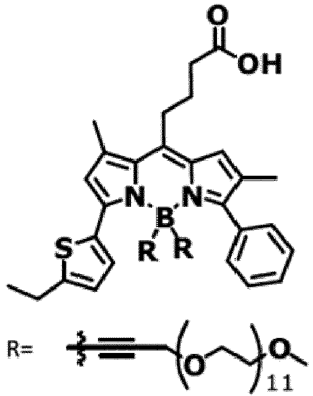

9. A tandem dye comprising:
a light harvesting chromophore comprising:
a backbone;
a donor chromophore linked to the backbone, wherein the donor chromophore is a water-soluble dipyrromethene-based dye according to any of claims 1 to 8.

10. The tandem dye according to claim 9, wherein the light harvesting chromophore is of formula (VA) or (VB): 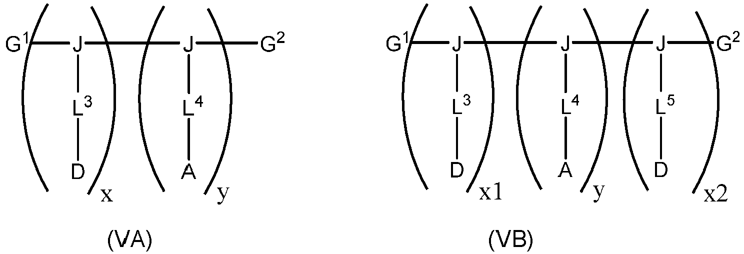 wherein:
each J are repeat units of the backbone;
L³-L⁵ are each independently a linker;
each D is a donor chromophore;
each A is an acceptor fluorophore;
G¹ and G² are each independently selected from the group consisting of terminal group, a polymer segment, a donor chromophore, an acceptor fluorophore, a linker, and a linked specific binding member;
x is 75 mol% or more;
x1 + X2 is 75% or more; and
y is 25 mol% or less.

11. A method of evaluating a sample for the presence of a target analyte, the method comprising:
(a) contacting the sample with an aggregation-resistant dye conjugate that specifically binds the target analyte to produce a labelling composition contacted sample, wherein the dye conjugate comprises:
(i) a tandem dye according to claim 9 or claim 10; and
(ii) a specific binding member linked to the tandem dye; and
(b) assaying the labelling composition contacted sample for the presence of a dye conjugate-target analyte binding complex to evaluate whether the target analyte is present in the sample.

12. A method of labelling a target molecule, the method comprising:
contacting the target molecule with a tandem dye to produce a labelled target molecule, wherein:
the tandem dye is a dye according to claim 9 or claim 10, and comprises a conjugation tag that covalently links to the target molecule.

13. The method according to claim 11 or claim 12, wherein the target molecule is an antibody.

14. A kit comprising:
a water-soluble dipyrromethene-based dye of any one of claims 1 to 8, or a tandem dye according to claim 9 or claim 10; and
a container.

15. The kit according to claim 14, wherein the tandem dye is covalently linked to an antibody.
